(19) Europäisches Patentamt
European Patent Office
Office européen des brevets

(11) **EP 4 545 559 A2**

(12) **EUROPEAN PATENT APPLICATION**
published in accordance with Art. 153(4) EPC

(43) Date of publication:
**30.04.2025 Bulletin 2025/18**

(21) Application number: **23768129.1**

(22) Date of filing: **26.06.2023**

(51) International Patent Classification (IPC):
**C07K 16/10** (2006.01)      **A61P 31/14** (2006.01)
**A61K 39/00** (2006.01)

(52) Cooperative Patent Classification (CPC):
**C07K 16/10; A61P 31/14;** C07K 2317/21;
C07K 2317/76; C07K 2317/92

(86) International application number:
**PCT/BR2023/050212**

(87) International publication number:
**WO 2023/245271 (28.12.2023 Gazette 2023/52)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **24.06.2022   BR 102022012744
15.07.2022   BR 102022014120**

(71) Applicant: **INSTITUTO BUTANTAN
05503900 São Paulo - SP (BR)**

(72) Inventors:
• **MORO, Ana Maria
01411-000 São Paulo - SP (BR)**
• **MANIERI, Tania Maria
Guarulhos - SP (BR)**

• **OLIVEIRA, Adriele Silva Alves De
06140-030 Osasco - SP (BR)**
• **CARVALHO, João Victor Batalha De
05540-120 São Paulo - SP (BR)**
• **CHUDZINSKI, Sonia Aparecida De Andrade
05363-000 São Paulo (BR)**
• **BOTOSSO, Viviane Fongaro
06036-050 Osasco - SP (BR)**

(74) Representative: **AWA Sweden AB
Matrosgatan 1
Box 5117
200 71 Malmö (SE)**

Remarks:
The complete document including Reference Table(s) and the Sequence Listing(s) can be downloaded from the EPO website

(54) **ANTI-SARS-COV-2 HUMAN MONOCLONAL ANTIBODY, PHARMACEUTICAL COMPOSITION, DIAGNOSTIC KIT AND USE THEREOF**

(57)     The present invention is part of the field of genetic engineering, more specifically, in the field of production of anti-sars-cov-2 human monoclonal antibodies. The neutralizing human antibodies of the present application can be used in the therapy of COVID19, avoiding hospitalization and aggravation of infected people with a predisposition to develop severe disease. Persons who are not infected but at risk (health and safety professionals, and others) may benefit from preventive treatment. The present invention also relates to a pharmaceutical composition comprising anti-SARS-COV-2 human monoclonal antibodies, as well as a diagnostic kit and use thereof.

Figure 1

EP 4 545 559 A2

**Description**

FIELD OF THE INVENTION:

The present invention is part of the field of genetic engineering, more specifically, in the field of production of anti-SARS-COV-2 human monoclonal antibodies. The present invention also relates to a pharmaceutical composition comprising anti-SARS-COV-2 human monoclonal antibodies, as well as the use thereof.

BACKGROUND OF THE INVENTION:

At the end of 2019, China reported the first case of COVID-19 (Coronavirus Disease 2019), a severe acute respiratory syndrome caused by SARS-CoV-2 (Severe Acute Respiratory Syndrome Coronavirus-2). The new virus spread rapidly around the world, reaching 167 million cases and 3.5 million deaths in May 2021 (JHUM, 2020). Although vaccines became available in less than a year, approaches to treating COVID-19 remain scarce. Many studies have sought different therapeutic interventions, ranging from the use of antiviral and antiparasitic drugs to immunotherapy with interleukins (for example, as interferon-alpha). NK cells or mesenchymal stem cells, as well as transfusion of plasma from a convalescent patient. Despite numerous studies, there is still no specific drug to combat COVID-19 and treatment remains mostly symptomatic.

SARS-CoV-2 belongs to a large family of viruses called the coronavirus. It is a positive single-stranded RNA (+ssRNA) virus. Coronaviruses have the ability to cause a variety of illnesses in humans, from the common cold to more serious illnesses such as Middle East respiratory syndrome (MERS). SARS-CoV-2 is the seventh coronavirus known to infect humans, the rest being 229E, NL63, OC43, HKU1, MERS-CoV and the original SARS-CoV.

Like the strain that caused the SARS outbreak in 2003, SARS-CoV-2 is a member of the Sarbecovirus subgenus (betacoronavirus lineage B). Its RNA sequence is approximately 30,000 nucleobases long. However, SARS-CoV-2 is the only one of the coronaviruses to incorporate a polybasic cleavage site, a feature known to increase the pathogenicity and reproductive rate of other viruses.

From a sufficient number of sequenced genomes, it is possible to reconstruct the phylogenetic tree of the mutation history of a family of viruses. On January 12, 2020, five SARS-CoV-2 genomes were isolated in Wuhan. As of January 30, 2020, 42 genomes were known. A phylogenetic analysis of these samples revealed that up to seven mutations are related to a common ancestor, which means that the first infection in humans occurred in November or December 2019. As of March 13, 2020, 410 genomes of SARS-CoV-2 were already sampled and publicly available.

On February 11, 2020, the International Committee on Taxonomy of Viruses announced that, in accordance with current rules that determine the hierarchical relationships between coronaviruses based on conserved nucleic acid sequences, the differences between SARS-CoV-2 and the SARS-CoV responsible for the SARS outbreak were insufficient to classify them as two different viral species. Thus, SARS-CoV-2 was classified as a strain of coronaviruses associated with acute respiratory syndrome (SARS-CoV).

Determining regions of complementarity

Complementarity-determining regions (CDRs) are part of the light and heavy variable chains (LC and HC) in immunoglobulins. The CDRs are where the antibody binds to a specific antigen. There are three CDRs (CDR1, CDR2, and CDR3) in each variable chain, arranged non-consecutively, in the amino acid sequence of an antibody variable domain. Since antibodies are typically composed of two variable domains (heavy and light chain), there are 6 CDRs. In view of the characteristics, functions and nature of antibodies, the recognition of an epitope is carried out by the LC-HC pair, that is, the 6 CDRs are the arrangement capable of recognizing specific regions of the antigens.

Strategically, more than the similarity of the amino acid sequence of the LC-HC pair of antibodies, the identification of an antibody is based on the assumption that the CDRs are the most variable regions of an antibody, therefore, they align in a very limited set, becoming unique according to the sequence and position of the amino acids of the 6 regions of the CDRs.

For prior analysis of the anti-Sars-Cov-2 monoclonal antibodies developed by the inventors of this application, the strategy used was based on the identification of documents that contained at least 2 equal CDRs (exact matches) and/or at least 2 CDRs with similarity greater than 70% (with mismatch). In this way, the identification of "exact matches" and "with mismatchs" occurred by the following groups:

(I)     HCDR1 + HCDR2 + HCRD3 + LCDR1 + LCDR2 + LCRD3;

(II)     HCDR1 + HCDR2 + HCDR3;

(III)         HCDR1 + HCDR2;

(IV)         HCDR1 + HCDR3;

(V)         HCDR2 + HCDR3;

(VI)         LCDR1 + LCDR2 + LCDR3;

(VII)         LCDR1 + LCDR2;

(VIII)         LCDR1 + LCDR3

(IX)         LCDR2 + LCDR3.

International patent application WO 2021/218947 filed on April 27, 2021 in the name of PEKING UNIVERSITY and entitled "ANTI-NOVEL CORONAVIRUS MONOCLONAL ANTIBODY AND APPLICATION THEREOF" describes an anti-novel coronavirus monoclonal antibody and a composition (for example, a diagnostic agent and a therapeutic agent) containing the same. Furthermore, the present invention also relates to the use of the antibody. The antibody of the present invention can be used to diagnose, prevent and/or treat novel coronavirus infections and/or diseases (e.g., novel coronavirus pneumonia) caused by the infections.

The international patent application WO 2021/228135 filed on May 12, 2021 in the name of PEKING UNIVERSITY and entitled "METHOD FOR PREPARING ANTIGEN-BINDING UNIT" describes a method for preparing an antibody, related to the field of immunology and the field of molecular virology, in particular, to the field of diagnosis, prevention and treatment of new coronaviruses. Specifically, a monoclonal antibody against the novel coronavirus and a composition (e.g., a diagnostic agent and a therapeutic agent) containing the antibody are provided. Antibody preparation, screening, and use are also provided.

The international patent application WO 2021/207152 filed on April 6, 2021 in the name of VANDERBILT UNIVERSITY and BOARD OF REGENTS, THE UNIVERSITY OF TEXAS SYSTEM and entitled "CROSS-REACTIVE CORONAVIRUS ANTIBODIES AND USES THEREOF" describes antibodies and their uses to treat, prevent and detect coronavirus infection.

The international patent application WO 2022/046888 filed on August 25, 2021 in the name of TARGET DISCOVERY MERGER SUB II, LLC and entitled "SARS-COV-2 ASSOCIATED ANTIBODY COMPOSITIONS AND METHODS OF USE" refers to new antibodies and antigen-binding fragments that are used in the treatment, prevention and diagnosis of COVID-19, the disease caused by SARS-CoV -2. The complete consensus polypeptide and nucleic acid sequences of the disclosed antibodies and antigen-binding fragments are reconstructed in silico.

International patent application WO 2021/242815 filed on May 26, 2021 in the name of REGENERON PHARMACEUTICALS, INC and entitled "ANTI-SARS-COV-2-SPIKE GLYCOPROTEIN ANTIBODIES AND ANTIGEN-BINDING FRAGMENTS" provides antibodies and antigen-binding fragments thereof that specifically bind to a coronavirus spike protein and methods of using such antibodies and fragments to treat or prevent viral infections (e.g., coronavirus infections).

The international patent application WO 2021/195326 filed on March 25, 2021 in the name of VANDERBILT UNIVERSITY and entitled "HUMAN MONOCLONAL ANTIBODIES TO SEVERE ACUTE RESPIRATORY SYNDROME CORONAVIRUS 2 (SARS-COV-2)" refers to antibodies that bind to and neutralize the coronavirus designated SARS-CoV-2 and methods for use of the same.

The international patent application WO 2021/244852 filed on May 19, 2021 in the name of CORAT THERAPEUTICS GMBH and entitled "THERAPEUTIC ANTIBODIES WITH NEUTRALIZING ACTIVITY AGAINST SARS-COV-2 GLYCOPROTEIN S" refers to antibodies and antigen-binding antibody fragments that specifically bind to SA glycoprotein S (spike) RS-CoV-2, as well as diagnostic and therapeutic methods of using these antibodies.

The international patent application WO 2021/226560 filed on May 7, 2021 in the name of VIR BIOTECHNOLOGY, INC. and HUMABS BIOMED SA and titled "ANTIBODIES AGAINST SARS-COV-2" refers to antibodies and antigen-binding fragments thereof that can bind to a SARS-CoV-2 antigen, and, in certain embodiments, are capable of potently neutralizing a SARS-CoV-2 infection. Also provided are polynucleotides encoding antibodies and antigen-binding fragments, vectors, host cells, and related compositions and uses, including for preventing, treating, and diagnosing an infection with SARS-CoV-2 or another coronavirus.

Chinese patent application CN 113402602 filed on September 17, 2021 in the name of HANGHAI XIANGYAO BIOTECHNOLOGY CO LTD and ZHEJIANG XIANGYAO BIOLOGICAL MEDICINE CO LTD. and titled "NEUTRALIZING ANTIBODY FOR NOVEL CORONAVIRUS SARS-COV-2 AND APPLICATION OF NEUTRALIZING ANTIBODY" refers to an antibody. The antibody can be linked to an RBD region of an S1 subunit of a Spike protein of a novel SARS-CoV-2 coronavirus, so that the binding activity between an ACE2 receptor on the surface of the host cell membrane and the Spike protein is blocked; the antibody is a wholly human-derived neutralizing antibody; and the antibody comprises a heavy chain VH and a light chain VL, and both the VH and the VL have variable regions CDR1, CDR2 and CDR3. The invention also relates to the related application of the antibody, in particular, the preparation of a complete IgG antibody and an affinity matured antibody and application of the corresponding antibody in the treatment and/or prevention of the SARS-CoV-2 novel coronavirus. The wholly human-derived single chain scFv antibody obtained by screening and the IgG antibody thereof can recognize and bind to the RBD region of Spike protein, can effectively block the binding activity between ACE2 and Spike protein, are not toxic to cells, and can remarkably inhibit a cytopathic effect caused by viral infection, and after the antibody affinity is matured, both the antibody affinity and the blocking effect on Spike protein and ACE2 are obviously increased.

Chinese patent application CN 112159469 filed on January 1, 2021 in the name of SHANGHAI PUBLIC HEALTH CLINICAL CT and entitled "ANTIBODIES OF CORONAVIRUS OR ANTIGEN BINDING FRAGMENTS OF ANTIBODIES" refers to antibodies of a coronavirus or antigen-binding fragments of antibodies, a nucleic acid molecule for encoding antibodies or their antigen-binding fragments antigen, a vector including the nucleic acid molecule, a host cell including the vector and applying the antibodies or antigen-binding fragments thereof to the aspect of preparing drugs for the treatment or prevention of diseases caused by the coronavirus and the aspect of detection products. The inventors use a monoclonal in vitro culture of B cells and high-throughput antibody screening technology to obtain a series of coronavirus antibodies and the antigen-binding fragments of the antibodies, and the antibodies and the antigen-binding fragments thereof have high binding and neutralizing capacity for the SARS-CoV-2 virus, can recognize and bind the S1 protein of the SARS-CoV-2 virus and a receptor binding domain (RBD) of the S1 protein, and have very strong affinity, and thus, one can speculate that antibodies and antigen-binding fragments possibly have the binding ability and neutralizing ability for other coronaviruses, and coronaviruses possibly appear in the future, and antibodies and antigen-binding fragments have good prospects for clinical application in the future.

The international patent application WO 2021/226405 filed on May 6, 2021 in the name of INTERNATIONAL AIDS VACCINE INITIATIVE INC. and THE SCRIPPS RESEARCH INSTITUTE and entitled "COVID-19 ANTIBODIES AND USES THEREOF" refers to recombinant monoclonal antibodies or antigen fragments thereof that bind to a Spike protein of SARS-CoV-2. Methods of using the antibodies to treat or prevent SARS-CoV-2 (COVID-19) are also disclosed.

International patent application WO 2021/183947 filed on March 12, 2021 in the name of UNIVERSITY OF PITTSBURGH - OF THE COMMONWEALTH SYSTEM OF HIGH EDUCATION and entitled "MOLECULES THAT BIND TO SARS-CoV-2" provides methods and materials involved in binding a ligand (e.g., an antibody, antigen-binding fragment and/or antibody domain) to a SARS-CoV-2 antigen. For example, ligands (e.g., antibodies, antigen-binding fragments, and antibody domains) that bind to a SARS-CoV-2 polypeptide, and methods and materials for using one or more of these binding molecules to treat a mammal (e.g., a human) with COVID-19 (or a viral infection caused by SARS-CoV-2).

No prior art document identified "exact matches" and/or "with mismatches" of the 6 CDRs (group I) present in the antibodies developed by the inventors of the present application.

As can be seen, no prior art document describes or suggests anti-sars-cov-2 human monoclonal antibodies, as disclosed by the present application.

## SUMMARY OF THE INVENTION

The present invention will provide significant advantages in relation to therapy with specific and neutralizing monoclonal antibodies, enabling an increase in its performance and presenting a more favorable cost/benefit ratio.

The pandemic caused by infection with the SARS-CoV-2 virus, which began more than 2 years ago, can cause severe and systemic conditions, leading to death. Infection control, especially severe cases, can be achieved by therapy with specific and neutralizing monoclonal antibodies, preferably human antibodies produced in vitro recombinantly, in unlimited quantities. Using a methodology described in the present application, the inventors of the present application isolated B lymphocytes from COVID19 convalescents, identified new sequences of human monoclonal antibodies, expressed antibodies that are shown to be neutralizing for the original SARS-CoV-2 virus (wild type) and variants.

The neutralizing human antibodies of this application can be used in the therapy of COVID19, avoiding hospitalization and aggravation of infected people with a predisposition to develop severe disease. Persons who are not infected but are at risk (health and safety professionals, and others) may benefit from preventive treatment.

In another embodiment, the present application refers to the use of generating cell lines expressing human monoclonal antibodies, derived from B cells of people infected and/or vaccinated with the SARS-CoV-2 virus

In a third embodiment, the present invention relates to the production of one or more antibodies that recognize the RBD region of SARS-CoV-2, including variants resulting from virus mutation.

In a fourth embodiment, the present invention relates to the treatment of COVID-19, neutralization of the SARS-CoV-2 virus, preventing systemic inflammation that can lead to death and other sequelae caused by COVID-19, viral inhibition, inhibition of inflammation caused by viral infection.

In a fifth embodiment, it refers to the prophylactic use of monoclonal antibodies recognizing the SARS-CoV-2 virus.

In a sixth embodiment, the present invention relates to the diagnostic use of such monoclonal antibodies.

In a seventh embodiment, the present invention refers to the use of anti-SARS-COV-2 human monoclonal antibodies for the production of a drug for the treatment of COVID-19.

BRIEF DESCRIPTION OF THE DRAWINGS

The structure and operation of the present invention, together with additional advantages thereof, may be better understood by referring to the attached drawings and the following descriptions:

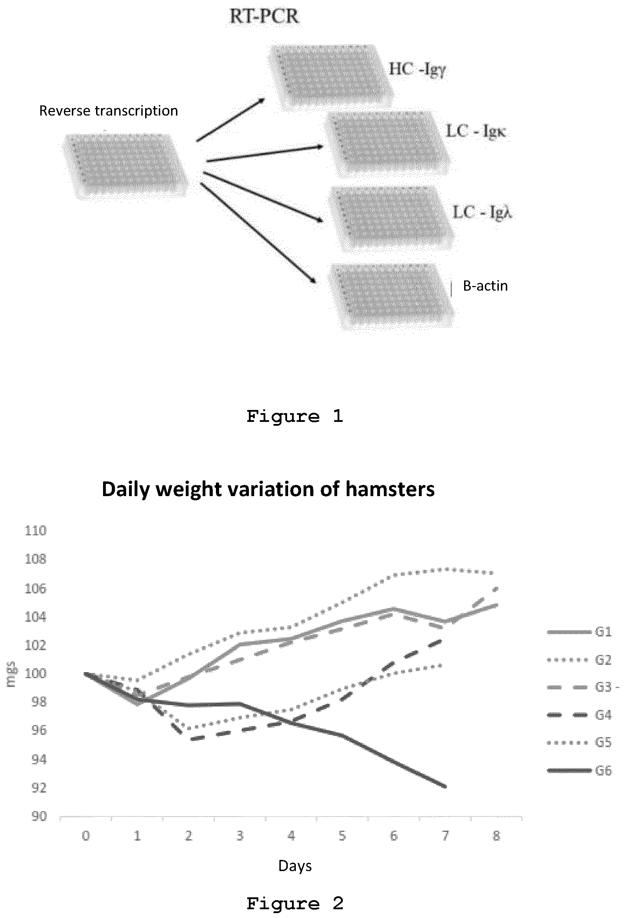

Figure 1 shows the original cDNA distribution scheme for the RT-PCR steps;

Figure 2 shows the average weight evolution of each animal, each day. The animals in the control group (G1 to G3) were euthanized for collection of blood, nasal turbinates, trachea, lung, liver and kidney on the 8th post-infection day. The animals in the infected groups (G4 to G6) were euthanized in subgroups of 6 animals each on days 3, 5 and 7 post infection for collection of the same samples;

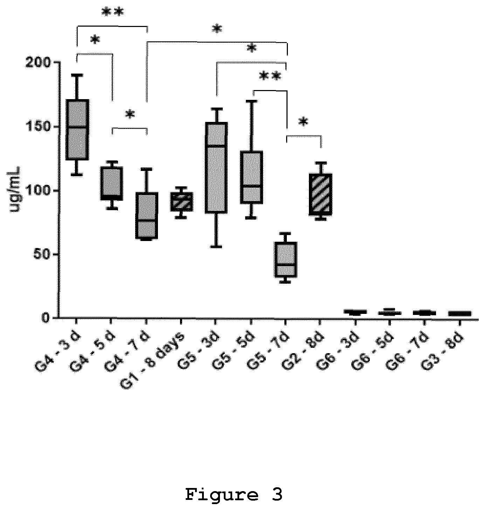

Figure 3 shows an ELISA graph evaluating the concentration (in $\mu$g/mL) of human antibody in the serum of each of the euthanized hamsters, according to the group to which they belonged. The columns in pink represent the mAb combination #1 (G4 at 3, 5 and 7 days post infection and G1 (uninfected) at 8 days. The columns in green represent the mAb combination #2 (G5 at 3, 5 and 7 days post infection and G2 (uninfected) at 8 days. The G6 and G3 groups were infected and did not receive antibodies. The results were evaluated by the Mann-Whitney test with statistical significance $p \leq 0.05$ (*) and $p \leq 0.01$ (**).

DETAILED DESCRIPTION OF THE INVENTION

While the present invention may be susceptible to different embodiments, preferred embodiments are shown in the following detailed description with the understanding that the present embodiments are to be considered exemplifying the principles of the invention and are not intended to limit the present invention to what has been illustrated and described herein.

People infected with SARS-CoV-2 or vaccinated volunteered to donate blood, in a project approved by Plataforma Brasil (CAAE 32707920.0.0000.5467). Blood collection was performed at the Butantan Institute Outpatient Clinic, by its professionals. On the day of donation, material was also collected for performing RT-PCR and confirming a negative result (confirmation of resolved disease). Donor blood was collected from July 2020 to July 2021, representing periods of predominance of variants of the original Wuhan SARS-Cov-2 virus (WT = wild type), for example, Beta, Gamma and Delta variants. Blood was also collected from people who were infected by SARS-CoV-2 and subsequently immunized with vaccines developed by different methods. The choice of convalescent donors followed a collection strategy over time, to represent infection with different variants of the virus. Another strategy consisted of the diversity of donors and courses of infection to represent a variety of immune responses.

The neutralizing human antibodies of this application can be used in the therapy of COVID19, avoiding hospitalization and aggravation of infected people with a predisposition to develop severe disease. Persons who are not infected but are at risk (health and safety professionals, and others) may benefit from preventive treatment.

In another embodiment, the present application refers to the use of generating cell lines expressing human monoclonal antibodies, derived from B cells of people infected and/or vaccinated with the SARS-CoV-2 virus

In a third embodiment, the present invention relates to the production of one or more antibodies that recognize the RBD region of SARS-CoV-2, including variants resulting from virus mutation.

In a fourth embodiment, the present invention relates to the treatment of COVID-19, neutralization of the SARS-CoV-2 virus, preventing systemic inflammation that can lead to death and other sequelae caused by COVID-19, viral inhibition, inhibition of inflammation caused by viral infection.

In a fifth embodiment, it refers to the prophylactic use of monoclonal antibodies recognizing the SARS-CoV-2 virus.

In a sixth embodiment, the present invention relates to the diagnostic use of such monoclonal antibodies.

In a seventh embodiment, the present invention refers to the use of anti-SARS-COV-2 human monoclonal antibodies for the production of a drug for the treatment of COVID-19.

PREFERRED EMBODIMENT

The collected blood was taken to the Biopharmaceuticals Laboratory, where it was immediately separated into mononuclear cells, which were frozen and stored in liquid nitrogen. A sample for serum preparation was collected from the same donor, processed, inactivated at 56°C, aliquoted and frozen on the same day at -20°C. until the VNT neutralization test was carried out in the NB3 laboratory at ICB/USP. Initially, the Wuhan strain neutralization index was established for each serum from the 41 donors.

Based on this titer and infection history, donor cells were thawed, enriched for B cells, and isolated by negative selection using the B cell isolation kit according to the Milenyi Biotec manufacturer's instructions (Robbiani et al, 2020). Then the mononuclear cells enriched in B cells were separated individually in a flow cytometer with a cell sorter (Aria III, Becton Dickinson). Twelve sortings were performed with different immunophenotyping strategies, using the RBD region or the Spike protein, from the original Wuhan strain (alpha) or variants. The strategy for choosing the bait (viral protein) for fishing for the B lymphocytes of interest had a great influence on the results achieved. The use of two proteins (RBD e/spike) or the same RBD protein labeled with two different fluorophores increased the efficiency of the sorting, or individualized separation of B cells. Throughout the sortings, the efficiency increased due to the change in strategy. We increased the efficiency of obtaining cDNA by changing the protocol that indicated the importance of ice at all stages, by using a freezing plate. When the plate was too cold, previously thawed samples were frozen for manipulation. Frozen samples cause lack of homogeneity leading to errors and even loss of material. Removing the plates 10 minutes before use greatly increased the efficiency of material recovery from each well.

The mRNA of each isolated cell was amplified by RT-PCR, from which the cDNA was generated, followed by other PCRs with specific oligos for amplification of genes related to the gamma region (heavy chain - HC - of IgG) and kappa or lambda genes (light chain - LC), in order to maintain the original nature of the antibody produced by the convalescent donor (Wardemann and Kofer, 2013).

The antibody molecule is formed by 2 identical HC and 2 identical LC, and the LC can be of the kappa or lambda type. The HC and LC form pairs (HC-LC), and the pairing is important for defining the specificity and affinity of the antibody. In the protocols described in the literature, each antibody has the HC-LC pair amplified for joint sequence analysis. In order to work with a large number of samples (4233), as shown in Table 1, the protocol was modified, in order to first amplify and sequence all the HC obtained from the randomly selected lymphocytes, resulting in a total of 2022 sequences. These sequences were analyzed for the gene family of the VDJ segment, following the IMGT numbering (Giudicelli et al, 2011) as well as the CDR3H sequence (Tiller et al, 2008).

These two analyzes allowed the grouping of clonally related sequences, due to the similarity of the CDR3H and belonging to the same family of the variable segment. The antibodies from these selected groups then underwent the second stage of amplification and sequencing of the respective LC, in a number of 313 kappa and 211 lambda, totaling 524.

Sequencing and analysis of the families of the VJ and CDR3L segments almost always confirmed the clonal groups initially identified for the HC. This strategy saved a lot of work, time, and reagent usage. It brought, on the other hand, a complication for the maintenance of the HC-LC pairing. According to the original protocol, the cDNA of each well of the 96-well plate obtained in the sorting is distributed in 4 plates, maintaining the same addressing, as shown in Figure 1. With the HC amplification in the first place, there is no reason to maintain the addressing for the LC RT-PCR, since the number of samples is much smaller.

First, different colors were standardized for the identification of HC, LC-K and LC-L sequences, facilitating the organization of plates in HC/LC pairs. To bring consistency and traceability, the samples are renamed according to the original position of the sorting board, the chain type (HC, K or L). To avoid errors in sample identification, we instituted an Excel spreadsheet that renames hundreds of samples by command line (CMD), in a few seconds and almost automatically.

The clonally related antibody sequences were subject to amplification of the HC and LC, using specific primers to recognize regions of the VDJ (HC) and VJ (kappa or lambda LC) families and addition of restriction sites for cloning in 3 vectors, one for the HC, one for the kappa LC and one for the lambda LC. The AbVec vectors (2.0-IGHG1, 1.1-IGKC and 1.1-IGLC2-XhoI) contain, respectively, gamma, kappa and lambda constant domain gene regions (Wardemann and Kofer, 2013). Thus, if the antibody contains LC kappa, the vectors used will be one for HC and another for LC kappa. The same occurs for a lambda LC, using the specific AbVec vector.

Table 1. Data on sortings, donors, B cell capture strategy by immunophenotyping with fluorophore-labeled antigens, number of plates, samples, and sequenced samples.

| Sorting | Donor | Antigen | Plates obtained in Sorting | PCR | | | | |
| | | | | | | Samples for sequencing | | |
| | | | | Plate | Number of samples | PCR Gamma | PCR Kappa | PCR Lambda |
|---|---|---|---|---|---|---|---|---|
| 1 | 1 | RBD-ALEXA | 1 | 1 | 37 | 11 | 11 | 0 |

(continued)

| Sorting | Donor | Antigen | Plates obtained in Sorting | PCR | | | | |
|---------|-------|---------|----------------------------|-------|-------------------|------------|------------|-------------|
| | | | | | | Samples for sequencing | | |
| | | | | Plate | Number of samples | PCR Gamma | PCR Kappa | PCR Lambda |
| 2 | 1 | RBD-ALEXA | 5 | 1 | 92 | 57 | 33 | 29 |
| | | | | 2 | 92 | 62 | 53 | 36 |
| | | | | 3 | 92 | 50 | 2 | 2 |
| | | | | 4 | 92 | 58 | 3 | 3 |
| | | | | 5 | 54 | 47 | 0 | 0 |
| 3 | 2 | RBD-ALEXA/ RBD-Biotin | 4 | 1 | 92 | 65 | 45 | 39 |
| | | | | 2 | 92 | 30 | 18 | 19 |
| | | | | 3 | 92 | 46 | 33 | 17 |
| | | | | 4 | 73 | 48 | 0 | 0 |
| 4 | 2 | Spike-ALEXA/ Spike-Biotin | 1 | 1 | 46 | 7 | 4 | 6 |
| 5 | 3 | Spike-ALEXA/ Spike-Biotin | 1 | 1 | 22 | 9 | 7 | 4 |
| 6 | 3 | Spike-ALEXA/ Spike-Biotin | 1 | 1 | 92 | 19 | 13 | 8 |
| 7 | 4 | Plasmablast | 15 | 1 | 92 | 29 | 2 | 0 |
| | | | | 2 | 92 | 18 | 0 | 0 |
| | | | | 3 | 92 | 17 | 0 | 0 |
| | | | | 4 | 92 | 19 | 0 | 0 |
| | | | | 5 | 92 | 21 | 0 | 0 |
| | | | | 6 | 92 | 24 | 0 | 0 |
| | | | | 7 | 92 | 15 | 0 | 0 |
| | | | | 8 | 92 | 17 | 1 | 1 |
| | | | | 9 | 92 | 14 | 0 | 0 |
| | | | | 10 | 92 | 19 | 0 | 1 |
| | | | | 11 | 92 | 22 | 1 | 0 |
| | | | | 12 | 92 | 21 | 4 | 0 |
| | | | | 13 | 92 | 18 | 0 | 0 |
| | | | | 14 | 92 | 11 | 0 | 0 |
| | | | | 15 | 92 | 19 | 0 | 0 |
| 8 | 5 | S1(D614G)-- ALEXA/ S1-Biotin | 1 and a half | 1 | 92 | 80 | 3 | 2 |
| | | | | 2 | 53 | 34 | 4 | 2 |

(continued)

| Sorting | Donor | Antigen | Plates obtained in Sorting | PCR | | | | |
|---|---|---|---|---|---|---|---|---|
| | | | | | | Samples for sequencing | | |
| | | | | Plate | Number of samples | PCR Gamma | PCR Kappa | PCR Lambda |
| 9 | 6 | S1(D614G)-- ALEXA/ S1- Biotin | 4 and a half | 1 | 92 | 78 | 12 | 6 |
| | | | | 2 | 92 | 71 | 7 | 3 |
| | | | | 3 | 92 | 86 | 11 | 3 |
| | | | | 4 | 92 | 82 | 9 | 5 |
| | | | | 5 | 38 | 36 | 5 | 1 |
| 10 | 7 | RBD- Alexa- Fluor 647 | 3 and a half | 1 | 92 | 25 | 2 | 3 |
| | | | | 2 | 92 | 17 | 1 | 0 |
| | | | | 3 | 92 | 23 | 0 | 0 |
| | | | | 4 | 35 | 11 | 1 | 0 |
| 11 | 8 | S1(D614G)-- ALEXA/ S1- Biotin | 8 plates | 1 | 92 | 74 | 12 | 8 |
| | | | | 2 | 92 | 73 | 6 | 4 |
| | | | | 3 | 92 | 60 | 12 | 3 |
| | | | | 4 | 92 | 71 | 13 | 6 |
| | | | | 5 | 92 | 69 | 6 | 2 |
| | | | | 6 | 92 | 60 | 8 | 4 |
| | | | | 7 | 92 | 75 | 10 | 10 |
| | | | | 8 | 92 | 69 | 13 | 4 |
| 12 | 9 | S1 + S2- Biotin / RBD- Biotin | 3 plates and 11 samples | 1 | 92 | 49 | 6 | 1 |
| | | | | 2 | 92 | 38 | 4 | 1 |
| | | | | 3 | 92 | 43 | 2 | 0 |
| | | | | 4 | 11 | 5 | 1 | 0 |
| | | | | 223 | 4233 | 2022 | 378 | 233 |

Another improvement consisted of the assignment of primers and verification of digestion sites through the creation of an Excel spreadsheet. In the HC-LC pair cloning step, a specific primer for the VDJ (HC) or VJ (LC) gene family to which each antibody belongs is used. An Excel spreadsheet allows the assignment of primers to be automated, although one or another sequence still needs manual analysis. Primer assignment time has been reduced from a few hours (or days) to a few minutes, eliminating human error. It is necessary to verify digestion sites by restriction enzymes in the sequences, using software where each sequence must be checked in search of the 2 possible restriction sites (for cloning in expression vectors). For 100 sequences there are 200 analyses. The Excel worksheet created does the checking automatically, with the return of information from the site that needs attention.

The cloned vectors with the variable regions were inserted into bacteria by heat shock transformation. These bacteria were inoculated onto agar plates for selection and colony growth. For each sample, 3 colonies were chosen, which underwent PCR and sequencing to confirm the expected nucleotide sequence. With this confirmation, pairs of vectors specific for the HC-LC combination were prepared and purified for transient co-transfection into ExpiCHO cells.

Transfection of cells allowed the expression of 65 mAbs, whose supernatants were tested by SPR (surface plasmon resonance) for binding to all or some of the following proteins immobilized on CM5 sensors: RBD WT, RBD E484K, RBD N501Y, RBD beta/gamma, RBD delta, Spike WT, Spike (HV69-70 deletion, Y144 deletion, N50 1Y, A570D, D614G, P681H, T716I, S982A, D1118H), NC WT, NC (D3L, R203K, G204R, S235F), gamma and delta. Analyzes were performed in BIAcore T200.

The 39 supernatants that showed binding to RBD or Spike were purified by protein A chromatography and retested by binding to sensors sensitized with the above recombinant proteins. Fourteen selected mAbs were tested for the neutralization of SARS-CoV-2 WT by the VNT100 test in VERO cells, with 14 having titers between 1/40 to 1/7680, being also tested for the neutralization of the P1 variant (gamma) by the same procedure in VERO cells. Two did not react and the rest titrated between 1/20 and > 1/2560 (last tested dilution).

The neutralization test was also performed with the delta variant of SARS-CoV2, with titers between 1/<20 and 1/2560 (last tested dilution). Only a few mAbs were tested for their ability to neutralize the omicron variant, with titer up to 1/80.

Among 37 mAbs expressed, purified and tested by binding to SARS-CoV-2 proteins, neutralization and/or kinetic affinity (appendix I), 3 mAbs were chosen with titles, respectively for WU, gamma and delta strains, 960/>2560/, 80/1280/320 and 1280/2560/1280. The gamma variant was chosen for the neutralizing title because it has so far been shown to cause the highest number of deaths.

Regarding the kinetic affinity, several mAbs (Appendix I) did not show dissociation with the target in the standard time used for the analysis (360 s or 6 min) and were evaluated up to the maximum time allowed by the equipment (36000 s or 10h), and some still did not dissociate. In this case the kinetic affinity is approximate. According to the law of mass action, when in equilibrium, there is association and dissociation between antigen and antibody, and long dissociation times are favorable to pharmacokinetics and therapeutic activity.

Production of anti-SARS-CoV mAbs:

The selected mAbs were obtained by transient transfection, requiring stable transfection for the development of permanent cell lines, with selection of clones by criteria of high productivity, stability, physical-chemical and biological analyzes and effector activity.

The main clone is selected through a matrix of all data, taking into account the desired characteristics for therapeutic antibodies (rebmab200, adabut).

The CHO cells are cultivated in a suspension bioreactor, by fed or continuous batch system, with perfusion of the culture medium. Chemically defined and animal-free culture media are used. The clarified supernatant is purified by 3 steps of chromatography (protein A capture, anionic and cationic ion exchange), which allow for approximate purity of 100%. Cell banks, MCB (master cell bank) and WCB (working cell bank) are created, from which ampoules stored in liquid nitrogen are removed for each production process.

Cell banks are certified by tests required by regulatory agencies (ANVISA, for example, or FDA, or WHO). The purified antibodies, after formulation, constitute the API (active pharmaceutical ingredient), evaluated by the quality criteria of microbiological monitoring and specific assays for purity, charge, isoelectric point, glycosylation profile, structure and potency, by standard methods for monoclonal antibodies. The API is ampoule in ampoules with the desired volume and submitted to microbiological and biological quality tests, constituting the final product. Stability tests are applied according to the recommendations of regulatory agencies.

Table 2 - CDR1, CDR2 and CDR3 regions of the heavy chain of the monoclonal antibody of the present invention

| SEQ ID NO | Name | Heavy chain aa sequence | CDR1-H | CDR2-H | CDR3-H |
|---|---|---|---|---|---|
| 1 | S.8.1.A4 | EVQLVESGGGLVQPGGSLRL SCAAS**GIIVSNNY**MSWVRQA PGKGLEWVSI**IYSGGST**FYA DSVKGRFTISRDNSKNTLYL QMNSLRVEDTAVYYC**ARDLG GLRLDY**WGQGTLVTVSS | **GIIVSN NY** | **IYSGGST** | **ARDLGGLRLDY** |

(continued)

| SEQ ID NO | Name | Heavy chain aa sequence | CDR1-H | CDR2-H | CDR3-H |
|---|---|---|---|---|---|
| 3 | S.9.1.A12 | EVQLVESGGGLVQPGGSLRL SCAVS**GFIVGRNY**MSWVRQA PGKGLEWVSV**IYPGGST**FYA DSVKGRFTISRDNSKSTLFL QMNSLRAEDTAVYYC**ARDRG EQFFDY**WGQGTLVTVSS | **GFIVGR NY** | **IYPGGST** | **ARDRGEQFFDY** |
| 5 | S.9.1.B11 | EVQLVETGGGXIQPGGSLRL SCAAS**GITVSRNY**MNWVRQA PGKGLEWVSL**IYSGGST**FYA DSVKGRFTISRDNSKNTLYL QMNSLRAEDTAVYYC**ARDLA DRGGMDV**WGQGTTVTVSS | **GITVSR NY** | **IYSGGST** | **ARDLADRGGMDV** |
| 7 | S.9.1.C10 | QVQLVQSGAEVKKPGASVKI SCKAS**GYTFIHYY**MHWVRQA PGQGLEWMGI**INPSGGGT**TY TQKFQGRVTMTRDTSTSTVY MELSSLRSEDTAVYYC**ARDL AGYTSEFDY**WGQGTLVTVSS | **GYTFIH YY** | **INPSGGG T** | **ARDLAGYTSEFD Y** |
| 9 | S.9.1.C9 | QVQLVESGGGVVQPGRSLGL SCAVS**GFTFSNYG**MDWVRQA PGKGLEWVAV**TSYDGSNK**YY ADSVKGRFTISRDNSKNTLY LQMNSLRTEDTAVYYC**AKHG GGPYCGGGTCYLAYFDY**WGQ GTLVTVSS | **GFTFSN YG** | **TSYDGSN K** | **AKHGGGPYCGGG TCYLAYFDY** |
| 11 | S.9.1.H5 | QVQLVESGGGVVQPGRSLRL SCAAS**GFTFSRYG**MHWVRQA PGKGLEWVAL**ISYDGTNK**YY ADSVKGRFTISRDNSKSTQY LQMNSLRAEDTAVYFC**AKES FGYASGSYYFDY**WGQGTLVT VSS | **GFTFSR YG** | **ISYDGTN K** | **AKESFGYASGSY YFDY** |

(continued)

| SEQ ID NO | Name | Heavy chain aa sequence | CDR1-H | CDR2-H | CDR3-H |
|---|---|---|---|---|---|
| 13 | S.9.2.B8 | EVQLVETGGGLIQPGGSLRLSCAAS**GLIVSSNY**MTWVRQAPGKGLEWVSV**LYSGGTT**YYADSVKGRVTISRDNSKNTLYLQVNSLTVADTAVYYC**ARDLADRGGMDV**WGQGTTVTVSS | **GLIVSSNY** | **LYSGGTT** | **ARDLADRGGMDV** |
| 15 | S.9.2.D3 | EVQLVESGGGLVQPGGSLRLSCAAS**EFIVSSNY**MSWVRQAPGKGLEWVSV**IYSGGST**FYADSVKGRFTISRDNSKNTLYLQMDALRAEDTAVYYC**ARDYGEFYFDY**WGQGTLVTVSS | **EFIVSSNY** | **IYSGGST** | **ARDYGEFYFDY** |
| 17 | S.9.2.D7 | EVQLLESGGGLIQTGGSLRLSCAAS**GFTFSSYA**MSWVRQAPGKGLEWVSV**ISDSGGST**YYADSVKGRLTISRDNSKNTLYVQMNSLRAEDTAVYYC**AKGKGGIAAAGPAFLFDY**WGQGTLVTVSS | **GFTFSSYA** | **ISDSGGST** | **AKGKGGIAAAGPAFLFDY** |
| 19 | S.9.2.E9 | QVQLVQSGAEVKKPGASVKVSCQAS**GYSFTYYY**MHWVRQAPGQGLEWMGI**INPSGGGT**SYAQKFQGRVTMTRDTSTSTVYMELSSLRSEDTAVYYC**ARDLAGVTSEFDY**WGQGTLVTVSS | **GYSFTYYY** | **INPSGGGT** | **ARDLAGVTSEFDY** |
| 21 | S.9.2.F4 | QVQLVESGGGVVQPGRSLRLSCAAS**GFTFSSYG**IHWVRQAPGKGLEWVAV**IANDGSNK**YYRDSVKGRFTISRDNSKNTLYLQMNSLRVEDTAVYYC**AKGLAGIAAAGTPFDY**WGQGTLVTVSS | **GFTFSSYG** | **IANDGSNK** | **AKGLAGIAAAGTPFDY** |

(continued)

| SEQ ID NO | Name | Heavy chain aa sequence | CDR1-H | CDR2-H | CDR3-H |
|---|---|---|---|---|---|
| 23 | S.9.3.B2 | EVQLVESGGGLVQPGGSLRLSCAAS**GLTVSSNY**MSWVRQAPGKGLEWVSI**IYSGGST**FYADSVKGRFTISRHNSKNTLYLQMNSLRAEDTAVYYC**ARDLVYYGMDV**WGQGTTVTVSS | **GLTVSSNY** | IYSGGST | ARDLVYYGMDV |
| 25 | S.9.3.G2 | EVQLVESGGGLVRPGGSLRLSCAAS**GIIVSRNY**MSWVRQAPGKGLEWVSV**IYSGGST**FYADSVKGRFTISRDNSKNTLYLQMNGLRAEDTAVYYC**ARDLSYYGMDV**WGQGTTVTVSS | **GIIVSRNY** | IYSGGST | ARDLSYYGMDV |
| 27 | S.9.4.C7 | QVQLVESGGGVVQPGRSLRLSCAAS**GFTFRNYA**MHWVRQAPGKGLEWVTV**ISYDGSDK**YYADSVKGRFTISRDNSKNTLYLQMNSLRAEDTAVYYC**AKDPVGGYCSGGRCYFHSHYFDY**WGQGTLVTVSS | **GFTFRNYA** | **ISYDGSDK** | **AKDPVGGYCSGGRCYFHSHYFDY** |
| 29 | S.9.4.F4 | QVQLVESGGGVVQPGRSLRLSCAAS**GFTFSHYG**MHWVRQAPGKGLEWVAVI**LYDGSNE**YYADSVKGRFTISRDNSKNTLYLQMNSLRAEDTAVYYC**AKGAGTYCSGGNCYVAYFDY**WGQGTLVTVSS | **GFTFSHYG** | **ILYDGSNE** | **AKGAGTYCSGGNCYVAYFDY** |
| 31 | S.9.4.H8 | QVQLVESGXGVVQPGRSLRLSCAAS**GFTFSTYG**MHWVRQAPGKGLEWVAV**ISYDGSNK**KFADSVKGRFTISRDTSKNTLYLQMNSLRADDTAVYYC**AKGAGTYCSAGNCYVAYFDY**WGQGTLVTVSS | **GFTFSTYG** | **ISYDGSNK** | **AKGAGTYCSAGNCYVAYFDY** |

(continued)

| SEQ ID NO | Name | Heavy chain aa sequence | CDR1-H | CDR2-H | CDR3-H |
|---|---|---|---|---|---|
| 33 | S.9.5.A9 | EVQLVESGGGLVQPGGSLRLSCAAS**GITVSSNY**MSWVRQAPGKGLEWVSV**IYSGGST**FYADSVKGRFTISRHNSKNTLYLQMNSLRPEDTAVYYC**ARDLGENAFDI**WGQGTMVTVSS | **GITVSSNY** | IYSGGST | ARDLGENAFDI |
| 35 | S.10.1.C6 | QLQLQESGPGLVKPSETLSLTCTVS**GGSISSSSYY**WGWIRQPPGKGLEWIGS**FYYSGGS**YYNPSLKSRVTISVDTSKNQFSLKLSSVTAADTAVYYC**ARDEHGDYAVGY**WGQGTLVTVSS | **GGSISSSSYY** | FYYSGGS | ARDEHGDYAVGY |
| 37 | S.10.1.D8 | EVQLVESGGGXVQPGGSLRLSCAAS**GLTVSSNY**MSWVRQAPGKGLEWVSL**IYSGGST**YYADSVKGRFTISRDNSKNTLYLQMNSLRAEDTAVYYC**ARDVLHRSGMDV**WGQGTTVTVSS | **GLTVSSNY** | IYSGGST | ARDVLHRSGMDV |
| 39 | S.10.2.D2 | EVQLVESGGGLVQPGXSLRLSCAAS**GVTVSSNY**MSWVRQAPGKGLEWVSV**IYSGGST**YYADSVKGRFTISRDNSKNTLHLQMNSLRAEDTAVYYC**ARDLGPRGAFDI**WGQGTMVTVSS | **GVTVSSNY** | **IYSGGST** | **ARDLGPRGAFDI** |
| 41 | S.11.2.A5 | QVQLVESGXGVVQPGRSLRLTCAAS**GFPFSTYV**MNWVRQAPGKGLEWVAS**ISNDGTRK**DYAGSVKGRFSISRDNFNSDLFLQMNTLKTEDTALYWCARTSGRGSRDRGRFDLWGQGTLVTVSS | **GFPFSTYV** | **ISNDGTRK** | **ARTSGRGSRDRGRFDL** |

(continued)

| SEQ ID NO | Name | Heavy chain aa sequence | CDR1-H | CDR2-H | CDR3-H |
|---|---|---|---|---|---|
| 43 | S.11.2.C5 | QVQLVQSGPEVKKPGSSVKVSCKGS**EGTFNVYA**ISWLRQAPGQGLEWMGG**IIPMHGKA**NLAQKFQGRVTIAADGNTNTGYMELSGLRSEDTAIYFC**ARDYRYCDSSTCYDFAFDI**WGQGTMVTVSS | **EGTFNVYA** | **IIPMHGKA** | **ARDYRYCDSSTCYDFAFDI** |
| 45 | S.11.3.A5 | QVQLVQSGAEVKKPGSSMKVSCKTS**GGTFNTYA**TNWVRQAPGQGLEWMGA**IIPIFGTT**NYAQKFQGRVTITADQSTTTSYMYLNSLTSEDTAVYYC**ARGTPVWGSSGPLGYNGMDV**WGQGTTVTVSS | **GGTFNTYA** | **IIPIFGTT** | **ARGTPVWGSSGPLGYNGMDV** |
| 47 | S.11.3.H5 | QVQLVQSGAEVRKPGASVEISCKAS**GYTFTNFY**IHWVRQAAGQGLEWMGL**INPSGAST**TYAPKFQGRITMTRDTSTPTVYMELSSLRSDDTAVYFC**ARDFSPSVHYCGGDCNSP**QYNWFDPWGQGTLVTVSS | **GYTFTNFY** | **INPSGAST** | **ARDFSPSVHYCGGDCNSPQYNWFDP** |
| 49 | S.11.4.F9 | EVQLVESGGGLIQPGGSLRLSCVVS**GLTVSSNY**MSWVRQAPGKGLEWVSV**IYSGGST**FYANSVKGRFTISRDNSKNTLYLQMNSLRAEDTAIYYC**TRDYGDFYMDV**WGKGTTVTVSS | **GLTVSSNY** | **IYSGGST** | **TRDYGDFYMDV** |
| 51 | S.1.1.D10 | EVQLLESGGGLVQPGGSLRLSCAVS**GFTFSSYA**MNWVRQAPGKGLEWVSA**ISGSGDST**YYADSVKGRFTISRDSSKNTLYLQMSSLRAGDTAVYYC**AKDTMGHSSLFDY**WGQGALVTVSS | **GFTFSSYA** | **ISGSGDST** | **AKDTMGHSSLFDY** |

(continued)

| SEQ ID NO | Name | Heavy chain aa sequence | CDR1-H | CDR2-H | CDR3-H |
|---|---|---|---|---|---|
| 53 | S.11.1.C7 | QVQLVQSGAEVKKPGASVRVSCKAS**GYIFSSYY**IHWVRQAPGQGLEWMGV**IYPSGGST**DYAQIFQGRVTMTRDTSTNTVYMELSGLRSEDTAVYYC**ARDGKDIVGATLPGRY**WGQGTLVTVSS | **GYIFSSYY** | **IYPSGGST** | **ARDGKDIVGATLPGRY** |
| 55 | S.11.1.C8 | QVQLVESGGGVVQPGRSLRLSCAAS**GFTFSTF**SMHWVRQAPGKGLEWVAV**ILYDGTYK**SYADSVKGRFTISRDNSGNTLFLQMNSLRAEDSAVYYC**ARDFGEMTTYF**DFWGQGTLVTVSS | **GFTFSTFS** | **ILYDGTYK** | **ARDFGEMTTYFDF** |
| 57 | S.11.1.D12 | EVQLLESGGGLVQPGGSLRLSCAAS**GFPFITYP**MSWVRQAPGKGLEWVSD**IGGIGGST**SYADSVKGRFTISRDNYQNTVYLQMHSLRAEDTAVYYC**AKWKSRVDSSYKGGGWFDS**WGPGTLVTVSS | **GFPFITYP** | **IGGIGGST** | **AKWKSRVDSSYKGGGWFDS** |
| 59 | S. 11. 1. F4 | QVQLVQSGAEVKKPGSSVRVSCKAS**AGTFSSYA**IRWVRQAPGQGLEWMG**TIIPIFGT**TNYAQKFQGRVSITADESTNTAYMELSSLRSEDTAVYYC**ARDLETSPLYGLDV**WGQGTTVTVSS | **AGTFSSYA** | **IIPIFGTT** | **ARDLETSPLYGLDV** |

(continued)

| SEQ ID NO | Name | Heavy chain aa sequence | CDR1-H | CDR2-H | CDR3-H |
|---|---|---|---|---|---|
| 61 | S.11.1.H1 | QVQLVESGGGVVQPGRSLTLSCSAS**GFPFSTYV**MHWVRQAPGKGLEWVAV**ISSDGSNE**NYADSVKGRFTVSRDNFGSTLFLQMSTLRPDDTAVYYC**ARTAGRGYADRGRLDT**WGQGTLVTVSS | **GFPFSTYV** | **ISSDGSNE** | **ARTAGRGYADRGRLDT** |
| 63 | S.11.1.H5 | EVQLVESAGGLVQPGGSLRLSCSAS**GFTFSSSP**MHWVRQAPGKGLQYVSA**VSSAGLT**TYYADSVKGRFTISRDNSKNTVFLQMSSLRADDTAVYYC**VKGWVGTFDP**WGQGTLVTVSS | **GFTFSSSP** | **VSSAGLTT** | **VKGWVGTFDP** |
| 65 | S.11.1.H8 | QVQLQESGPGQVKPSQTLSLSCTVS**GDSINSVGYY**WNWIRQHPGKGLEWIGN**IYFYGST**YYNPSLQSRVIISLDTSKNQFSLRLTSVTAADAAVYYC**ARSYTGYESDDALDI**WGQGTMVTVSS | **GDSINSVGYY** | **IYFYGST** | **ARSYTGYESDDALDI** |
| 67 | S.11.2.A3 | EVQLVESGGGLVKPGGSLSLSCAAS**GLTFSNVW**MNWVRQAPGKGLEWVGL**IKSATDGGTP**YYAAPVKGRFIIPRDDSKNTLYLQMNSLKTEDTAVYFC**TTEAPGYSYGYEDFDY**WGQGTLVTVSS | **GLTFSNVW** | **IKSATDGGTP** | **TTEAPGYSYGYEDFDY** |

(continued)

| SEQ ID NO | Name | Heavy chain aa sequence | CDR1-H | CDR2-H | CDR3-H |
|---|---|---|---|---|---|
| 69 | S.2.2.F11 | QVQLVQSGAEVKKPGSSVKV SCRAS**GGTFNNYA**ISWVRQA PGRGLEWMGG**IFPFRNTA**KY AQHFQGRVTITADESTDTAY MELSSLRSEDTAIYYC**ARGD TIFGVTMGYYAMD**VWGQGTT VTVSS | **GGTFNN YA** | **IFPFRNT A** | **ARGDTIFGVTMG YYAMDV** |
| 71 | S.2.2.G8 | QVQLVQSEAEVKKPGSSVKV SCRAS**GGTFNNYA**ISWVRQA PGQGLEWMGG**IFPFRNTA**KY AQHFQGRVTITADESTGTAY MELSSLRSEDTAIYYC**ARGD TIFGVTMGYYAMDV**WGQGTT VTVSS | **GGTFNN YA** | **IFPFRNT A** | **ARGDTIFGVTMG YYAMDV** |
| 73 | S.2.3.C4 | QVQLVRSGAEVKKPGASVKV SCKAS**GYSFTNYA**ISWVRQA PGQGLEWMGW**ISTYNGNP**KY AQKLQGRVTMTTDTSTSTAY MDLRSLRSDDTAVYYC**ARLP FYFYDSSGYDAFDI**WGQGTM VTVSS | **GYSFTN YA** | **ISTYNGN P** | **ARLPFYFYDSSG YDAFDI** |
| 75 | S.2.3.G7 | QVQLVESGGGLVKPGGSLRL SCAASG**FTFSDYY**MSWVRQA PGKGLEWVPY**ISSSGSSI**NY ADSVKGRFTISRDNAKNSLY LQMNNLRAEDTAVYYC**ARDP GPYYASGSYTTFYYRYGMDV** WGQGTTVTVSS | **GFTFSD YY** | **ISSSGSS I** | **ARDPGPYYASGS YTTFYYRYGMDV** |
| 77 | S.2.4.D2 | QVQLVESGGGVVQPGGSLRL SCAAS**GFTFSSYA**MHWVRQA PGKGLEWVAY**IQYDGTNK**FY ADSVKGRFTISRDNSKNTLY LQMNSLRVEDSAVFYC**AKSR** | **GFTFSS YA** | **IQYDGTN K** | **AKSRGSGSYSFF FHH** |

(continued)

| SEQ ID NO | Name | Heavy chain aa sequence | CDR1-H | CDR2-H | CDR3-H |
|---|---|---|---|---|---|
| | | **GSGSYSFFFH**HWGQGTLVTVSS | | | |
| 79 | S.2.4.E3 | EVQLLESGGDLVQPGGSLRLSCTAS**GFTFSSYA**MSWVRQAPGKGLEWVSR**ISGSGSGT**FYRDSVKGRFTVSRDNPNNTLFLQMNSLRAEDTAVYYC**AKDGPYYDILTGPGHPSYYYYGMDV**WGQGTTVTVSS | **GFTFSSYA** | **ISGSGSGT** | **AKDGPYYDILTGPGHPSYYYYGMDV** |
| 81 | S.2.4.E8 | EVQLVESGGVVVQPGGSLRVSCAAS**GFTFDDYA**MHWVRQAPGRGLEWVSV**ISWDASST**YYADSVKGRFTISRDNSKHSLFLQMNSLRPEDTAFYFC**AKDMTPYGSGSPDY**WGQGTLVTVSS | **GFTFDDYA** | **ISWDASST** | **AKDMTPYGSGSPDY** |
| 83 | S.3.1.B7 | QVQLVESGGGVVQPGGSLRLSCAAS**GFTFRNYA**LHWVRHTPGKGLEWVAV**IWYDENSK**YYAESVEGRFTVSRDNSKNTLFLQMNNLRAADAGVYYC**ARDPGSTSDSSGYYGIDF**WGQGTLVTVSS | **GFTFRNYA** | **IWYDENSK** | **ARDPGSTSDSSGYYGIDF** |
| 85 | S.3.1.E2 | QVQLQESGPGLVKPSETLSLTCTVS**GGSITSDH**WTWIRQPPGKGLEWIAY**MEHNGSP**TYNPSVRSPVAMSLDTSKNQLSLKVYSVTAADSAVYYC**ARAATSSAIDS**WGQGTLVTVSS | **GGSITSDH** | **MEHNGSP** | **ARAATSSAIDS** |

(continued)

| SEQ ID NO | Name | Heavy chain aa sequence | CDR1-H | CDR2-H | CDR3-H |
|---|---|---|---|---|---|
| 87 | S.3.1.G5 | RCTSSQLQQWGAGLLKPSET LSLTCSVY**GESFSGYY**WNWI RQPPGKRLEWIGE**INHSGSA** NYNPSLKSRVSILVDTSRHQ IFLRLSSVTAADTAVYYC**AR AYGSGSYTWGWAPFDY**WGQG SLVTVSS | **GESFSG YY** | **INHSGSA** | **ARAYGSGSYTWG WAPFDY** |
| 89 | S.3.2.C7 | QVQLVESGGALVKPGESLRL SCVGS**GFTFSDFY**MSWIRQA PGKGLEWVSA**ISSSGSNI**YH GDSLKGRFTISRDNAKNSMY LQMNSLRAEDTAVYYC**ARFL NRYYGSGSYGYYYHAVDV**WG QGTTVTVSS | **GFTFSD FY** | **ISSSGSN I** | **ARFLNRYYGSGS YGYYYHAVDV** |
| 91 | S.3.2.D9 | EVQLVESGGGLVQPGGSQRL SCVVS**GFTVSTFY**MSWVRQA PGKGLEWVSV**IFSGDST**YYA DSVKGRFTISRDDSKNILYL QMNSLRAEDTAVYFC**ARSLK GAYSTSWYFYYAMDV**WGQGT TVTVSS | **GFTVST FY** | **IFSGDST** | ARSLKGAYSTSW YFYYAMDV |
| 93 | S.8.1.C12 | QVQLVQSGAEVKKPGSSVKV SCKAS**GGTFSSYV**IGWVRQA PGQGLEWMGG**IIPIFGTT**NY AQKSQGRVTITADESSSTAY MELSGLRSEDTAVYYC**AGLS QWELMIR**WGQGTLVTVSS | **GGTFSS YV** | **IIPIFGT T** | **AGLSQWELMIR** |
| 95 | S.8.1.C2 | EVQLVESGGGLVQPGGSLRL SCVVS**GIIVSNNY**MSWVRQA PGKGLEWVAV**IYSGGST**FYA DSVKGRFTISRHNSKNTLYL QMNSLRTEDTALYYC**ARDLG NSEFDY**WGQGTLVTVSS | **GIIVSN NY** | **IYSGGST** | **ARDLGNSEFDY** |

(continued)

| SEQ ID NO | Name | Heavy chain aa sequence | CDR1-H | CDR2-H | CDR3-H |
|---|---|---|---|---|---|
| 97 | S. 9. 1. B7 | EVQLVESGGGLVQPGGSLRLSCAAS**GFTVSSNY**MSWVRQAPGKGLEWVSV**IYSGGST**FYADSVKGRFTISRDNSKNTLYLQMNSLRAEDTAVYYC**ARDLRGYNDY**WGQGTLVTVSS | **GFTVSSNY** | **IYSGGST** | **ARDLRGYNDY** |
| 99 | S.9.1.G7 | EVQLVETGGGLIQPGGSLRLSCAAS**GLTVSSNY**MSWVRQAPGKGLEWVSV**IYAGGST**FYADSVKGRFTISRDNSKNTLYLQMNGLRADDTAVYYC**ARDLVVYGMDV**WGQGTTVTVSS | **GLTVSSNY** | **IYAGGST** | **ARDLVVYGMDV** |
| 101 | S.9.3.A7 | QLQLQESGPGLVKPSETLSLTCTVS**GGSISSSSYY**WGWIRQPPGKGLEWIAS**LFYSGST**YYNPSLKSRVTISVDTSKNRFFLIPTSVTAADTAVYYC**ARDSSGSYSIDY**WGQGTLVTVSS | **GGSISSSSYY** | **LFYSGST** | **ARDSSGSYSIDY** |
| 103 | S.9.4.A9 | QVQLVQSGAEVKKPGASVKVSCKAS**GYTFISYG**ISWVRQAPGQELEWMGW**ISAYNGNT**NYAQKLQGRVTMTRDTSTTTAYMELRSLRSDDTAVYYC**ARVLGGLWFGEPKARHSDY**WGQGTLVTVSS | **GYTFISYG** | **ISAYNGNT** | **ARVLGGLWFGEPKARHSDY** |
| 105 | S.9.4.C6 | QVQLVQSGSELKKPGASVKVSCKAS**GYTFTNYA**MNWVRQAPGQGLEWMGW**IDTNAGNP**TYAQGFTGRFVFSLDTSVSTAYLQISSLKPEDTAVYFC**ARVQGVGVVTKSYYYYYAMDV**WGQGTTVTVSS | **GYTFTNYA** | **IDTNAGNP** | **ARVQGVGVVTKSYYYYYAMDV** |

(continued)

| SEQ ID NO | Name | Heavy chain aa sequence | CDR1-H | CDR2-H | CDR3-H |
|---|---|---|---|---|---|
| 107 | S.11.1.D4 | QVQLVESGGGVVQPGRSLRL SCVAS**GFSFSTYT**MNWVRQA PGKGLEWVTK**ISSDGTLK**DY ADSVKGRFTVSRDNYESTLY LEMNSLRTEDTAVYYC**ARTS GRGSEDRGRFDP**WGQGTLVT VSS | **GFSFST YT** | **ISSDGTL K** | **ARTSGRGSEDRG RFDP** |
| 109 | S.11.1.E10 | EVQLVESGGGLVQPGRSLRL SCEVS**GFTFEDYA**MHWVRQP PGKGLEWVSG**ISWNGDSI**GY AGAVRGRFTISRDNAKNSLS LQMNSLRVEDTALYYC**VKGR YVDWTNPLDV**WGQGTTVTVS S | **GFTFED YA** | **ISWNGDS I** | **VKGRYVDWTNPL DV** |
| 111 | S.11.1.F1 | QLQLQESGPGLVKPSETLSL TCTVSG**GSISSSSYY**WGWIR QTPGKGLEWIGS**IYYSGST**Y YRPSLKSRVTISVDTSKNQF SLKLSSVTAADTAVYYC**ARL ARFLEWQPIVSFDY**WGQGTL VTVSS | **GGSISS SSYY** | **IYYSGST** | **ARLARFLEWQPI VSFDY** |
| 113 | S.11.2.B6 | EVQLVESGGGLVQPGGSLRL SCAAS**EIIVSRNY**MNWVRQA PGKGLEWVSV**MYPGGST**FYA NSVKGRFTISRDNSQNTLFL QMNSLRAEDTAVYYC**ARDRG NDAFDV**WGQGTMVTVSS | **EIIVSR NY** | **MYPGGST** | **ARDRGNDAFDV** |
| 115 | S.11.2.E5 | EVQLVESGGGLIQPGGSLRL SCAAS**EIIVSSNY**MNWVRQA PGRGLEWVSV**IYSGGST**FYA DSVKGRFTISRDNSKNTLYL QMNSLRADDTAIYFC**ARDPY GGAFDV**WGQGILVTVSS | **EIIVSS NY** | **IYSGGST** | **ARDPYGGAFDV** |

(continued)

| SEQ ID NO | Name | Heavy chain aa sequence | CDR1-H | CDR2-H | CDR3-H |
|---|---|---|---|---|---|
| 117 | S.11.2.F5 | QVQLVESGGGVVQPGRSLRLSCAAS**GFTFSSYV**IHWVRQAPGKGLEWVAV**ISHDGSNK**YYADSVKGRFTISRDNSKNTLYLQMNSLRAEDTAVYYC**ARDRELGKSYGLDAYDI**WGQGTMVTVSS | **GFTFSSYV** | **ISHDGSNK** | **ARDRELGKSYGLDAYDI** |
| 119 | S.11.5.A2 | QVQLVQSGAEVKKPGSSVKVSCKAS**VGTFSSYA**ISWVRQAPGQGLEWMGR**IIPIFGTA**DYAQKFQGRVTITADESTNTAYMELSSLRSEDTAVYYC**ARDLETSPLYGMEV**WGQGTTVTVSS | **VGTFSSYA** | **IIPIFGTA** | **ARDLETSPLYGMEV** |
| 121 | S.11.5.C7 | QVQLQESGPGLVKPSQTLSLTCSVS**GDSISINAYY**WSWIRQHPGRGLEWIGY**MDYSGTT**SYSPSLKSRVSISVDTSKNQFSLKLSSVTVADTAVYYC**ARVGFSYGNPLDAFDV**WGQGTMVTVSS | **GDSISINAYY** | **MDYSGTT** | **ARVGFSYGNPLDAFDV** |
| 123 | S. 11. 6. B5 | EVQLVETGGGLIQPGGSLRLSCTAS**GLIVSSNY**MIWVRQAPGKGLEWVSV**IYPGGST**FYADSVKGRFTISRDNSKNTLYLQMNSLRAEDTAVYYC**ARDLVEFGMDV**WGQGTTVTVSS | **GLIVSSNY** | **IYPGGST** | **ARDLVEFGMDV** |
| 125 | S.11.6.G11 | EVQLVETGGGLIQPGGSLRLSCAAS**GVTVSSNY**MSWVRQAPGKGLEWVSV**MYSGGST**FYADSVKGRFTISRDNSKNTLYLQMNSLRAEDTAVYYC**ARDLVIWGMDV**WGQGTTVTVSS | **GVTVSSNY** | **MYSGGST** | **ARDLVIWGMDV** |

(continued)

| SEQ ID NO | Name | Heavy chain aa sequence | CDR1-H | CDR2-H | CDR3-H |
|---|---|---|---|---|---|
| 127 | S.11.7.A11 | QVQLVQSGTEVKKPGSSVKV SCMAS**GGSFSTSA**ISWVRQA PGQGLEWMGG**IIPITGTA**SY AQNFQGRVTITADESTSTAY MELSSLRSEDTAVYYC**ARGY RYCNSTRCYPWFDP**WGQGTL VTVSS | **GGSFST SA** | **IIPITGT A** | **ARGYRYCNSTRC YPWFDP** |
| 129 | S.11.7.A5 | EVQLVESGGGLVQPGRSLRL SCAAS**GFNFDDYA**MHWVRQG PGKGLEWVSG**ISYNSDSI**GY AASVKGRVTISRDNAKNSLY LQMNSLRAEDTALYYC**AKGR YFDWTHAMDV**WGQGTTVTVS S | **GFNFDD YA** | **ISYNSDS I** | **AKGRYFDWTHAM DV** |
| 131 | S.11.7.C8 | QVQLVQSGAEVKKPGSSVKV SCKAS**GGTFSTYA**FNWVRQA PGQGLEWMGG**IIPMFGTTHD ALGFQGT**NYAQRFQGRLTIT ADQSTNTAYMELSGLRSDDT AVYYC**ARGVPVWGSNGPLGY RAMDV**WGQGTTVTVSS | **GGTFST YA** | **IIPMFGT THDALGF QGT** | **ARGVPVWGSNGP LGYRAMDV** |
| 133 | S.11.7.F5 | QVQLVQSGAEVKKPGSSVKV SCKAS**GGTFSSHA**FSWVRQA PGQGLEWMGG**IIPIFGTS**HY AQKFQGRVTITADESTSTAY MELISLRSDDTAVYYC**ARYP YDILTGYLDY**WGQGTLVTVS S | **GGTFSS HA** | **IIPIFGT S** | **ARYPYDILTGYL DY** |

(continued)

| SEQ ID NO | Name | Heavy chain aa sequence | CDR1-H | CDR2-H | CDR3-H |
|---|---|---|---|---|---|
| 135 | S.11.7.G2 | QVQLVESGXGVVQPGRSLRL SCAAS**GFTFSNYA**IHWVRQA PAKGLEWVAV**ILSDGSGK**FY ADSVKGRFTISRDNSKNTLY LQMNSLRPDDTALYYC**ARDQ GELTTYFDY**WGQGTLVTVSS | **GFTFSN YA** | **ILSDGSG K** | **ARDQGELTTYFD Y** |
| 137 | S.11.7.G3 | EVQLVESGGGLFQPGGSLRL SCEVS**GFPFSNYW**MNWVRQA PGKGLVWISR**ISSDGSSA**TY ADSVRGRLIISRDNAKNTLY LQMNSLRADDTAVYYC**ARSE YLRGWFDP**WGQGTLVTVSS | **GFPFSN YW** | **ISSDGSS A** | **ARSEYLRGWFDP** |
| 139 | S.11.7.H6 | QVQLVQSGAEVKKPGASVKI SCEAS**GYSFTSYY**MHWVRQA PGQGLEWMGI**IYPSGGRT**DY AQKFQGRVTVTRDTSTSTVY LELTSLRSEDTAVYYC**ARDA KDIVGATLPGCH**WGQGTLVT VSS | **GYSFTS YY** | **IYPSGGR T** | **ARDAKDIVGATL PGCH** |
| 141 | S.11.3.D10 | EVQLVQSGAEVKKPGESLKI SCKAS**GYIFTNYW**IAWVRQM PGKGLEWMGI**IYPDDSDT**RY GPSFQGQVTISADKSISTAY LQLSSLKASDNAMYYC**ARHW RPTIAARPGYSYYMDV**WGKG TTVTVSS | **GYIFTN YW** | **IYPDDSD T** | **ARHWRPTIAARP GYSYYMDV** |
| 143 | S.11.3.G9 | QVQLVESGGGVVQPGRSLRL SCAAS**GFGLNTYV**MNWVRQS PGKGLQWVAG**ISSDGSKE**QY ADSVKGRFTISRDNYENALY LQMNSLRTEDTAVYYC**ARTS GRGFADRGRFDP**WGQGTLVT VSS | **GFGLNT YV** | **ISSDGSK E** | **ARTSGRGFADRG RFDP** |

(continued)

| SEQ ID NO | Name | Heavy chain aa sequence | CDR1-H | CDR2-H | CDR3-H |
|---|---|---|---|---|---|
| 145 | S.11.3.H8 | EVQLVETGGGLIQPGGSLRLSCAVS**ELIVSVNY**MSWVRQAPGKGLEWVSI**IYSGGSM**FYAGSVKGRFTISRDHSKNTLYLQMNSLRVDDTAVYYC**ARSYGDFYLDL**WGQGTLVTVSS | **ELIVSVNY** | **IYSGGSM** | **ARSYGDFYLDL** |
| 147 | S.11.4.A5 | QLQLQESGPGLLKPSETLSLTCSVS**GGSISSSSYY**WGWIRQAPGKGLEWIGN**IFYSGTA**YSSPSLQSRVTMSVDTSKNQFSLNVRSVTAADTAVYYC**ARLARFLEGQPTASFDY**WGPGALVTVSS | **GGSISSSSYY** | **IFYSGTA** | **ARLARFLEGQPTASFDY** |
| 149 | S.11.4.A6 | QVQLQESGPGLVKPSQTLSLTCSVS**GDSISINAYY**WNWIRQHPGKGLEWIGY**MDYSGST**SYSPSLKSRVSISVDTSKNQFSLKLSSVTVADTAVYYC**ARVGFSYGNPLDAFDV**WGQGTMVTVSS | **GDSISINAYY** | **MDYSGST** | **ARVGFSYGNPLDAFDV** |
| 151 | S.11.4.B5 | QVQLQESGPGLVKPSQTLSLTCSVS**GGSVTSGGYY**WSWTRQYPGRGLEWVGN**IYHSGST**NYHPSLRSRTTILVDTSKDQFSLKLSSVTAADTAVYYC**ARDPVAGRGQFDR**WGQGILVTVSS | **GGSVTSGGYY** | **IYHSGST** | **ARDPVAGRGQFDR** |
| 153 | S.11.4.C6 | EVQLVESGGGLIQPGGSLRLSCAAS**EFIVSKNY**MSWVRQAPGKGLEWVSV**IYPGGTT**YYADSVKGRFTISRDSSKNTLYLQMNSLKAEDTALYYC**ARDFGDFYFDY**WGQGTLVTVSS | **EFIVSKNY** | **IYPGGTT** | **ARDFGDFYFDY** |

(continued)

| SEQ ID NO | Name | Heavy chain aa sequence | CDR1-H | CDR2-H | CDR3-H |
|---|---|---|---|---|---|
| 155 | S.11.4.D7 | QVQLVESGGGVVQPGRSLRLSCGAS**GFPFITYT**MNWVRQAPGKGLEWVAL**ISSDGSNK**YYADFAKGRFTISRDNYDNTLYLQMNSLRSEDTAVYWC**ARTGGRGSEDRGRLDA**WGQGTLVTVSS | **GFPFITYT** | **ISSDGSNK** | **ARTGGRGSEDRGRLDA** |
| 157 | S.11.4.E7 | QVQLVQSGAEVKKPGASVTVSCEAS**GFPFTNYY**IHWVRQAPGQGLQWMGLI**NPSGGST**TYAQKFQGRLTMTRDTSAPIVYMELSSLTSEDTAVYYC**ARDSPPSVHHCGGDCLSPRYNWFDP**WGQGTLVTVSS | **GFPFTNYY** | **INPSGGST** | **ARDSPPSVHHCGGDCLSPRYNWFDP** |
| 159 | S. 11. 4.G9 | EVQLVETGGGLIQPGGSLRLSCAAS**GVTVSSNY**MTWVRQAPGKGLEWVAV**IYSGGST**FYADSVKGRFTISRDNSKNTLFLQMNSLRAEDTAVYYC**ARDLVYYGMDV**WGQGTTVTVSS | **GVTVSSNY** | **IYSGGST** | **ARDLVYYGMDV** |
| 161 | S.11.5.D12 | QVQLVESGGGVVQPGRSLTISCVAS**GFSFSTFS**MHWVRQTPGKGLEWVAV**ILYDGSYK**SYADSVKGRFTVSRDNSKNTLYLHMNSPKVEDSAVYYC**ARDFGEMTTHFDY**WGQGTLVTVSS | **GFSFSTFS** | ILYDGSYK | **ARDFGEMTTHFD Y** |
| 163 | S.11.6.B6 | QVQLVQSGPEVKKPGSSVKVSCKAS**GFTFSSSA**VQWVRQGRGHRLEWIGW**IVVGSGNT**NYVQKFQERVTISRDMSTNTAYMELSGLRSEDTAVYYC**AAPSCSNVLCFDGFDL**WGQGTMVTVSS | **GFTFSSSA** | **IVVGSGNT** | **AAPSCSNVLCFDGFDL** |

(continued)

| SEQ ID NO | Name | Heavy chain aa sequence | CDR1-H | CDR2-H | CDR3-H |
|---|---|---|---|---|---|
| 165 | S.11.7.B11 | EVQLVESGPGLVKPSETLSLTCTVS**GVSIRSYY**WSWIRQPPGKGLEWIGY**IFYSGST**NYNPSLKSRVTISVDTSKNQFSLKLSSVTAADTAVYYC**ASLGYGDKSWYFEL**WGRGTLVTVSS | **GVSIRSYY** | **IFYSGST** | **ASLGYGDKSWYFEL** |
| 167 | S.11.7.D12 | QVQLVESGGGMVQPGRSLRLSCAAS**GFPFITY**TMHWIRQAPGKGLEWVAG**VSSDGSDV**YSADSVKGRLTISRDNFVNTLHLQMNSLKTDDTAVYYC**ARTGGRGSQDRGRFDL**WGQGTLVTVSS | **GFPFITYT** | **VSSDGSDV** | **ARTGGRGSQDRGRFDL** |
| 169 | S.11.7.E6 | QVQLVQSGAEVKKPGSSVKVSCKAS**GGPFSIYS**ITWVRQAPGQGLEWMGG**IIPMRNTP**NYAQKFQGRVTITADASTTTGYMELSSLRSEDTAVYYC**ARDYRYCNDTTCYGFAFDI**WGQGTMVTVSS | **GGPFSIYS** | **IIPMRNTP** | **ARDYRYCNDTTCYGFAFDI** |
| 171 | S.11.8.D10 | QVQLVESGGGVVQPGRSLRLSCAAS**GFTFSTYV**MHWVRQAPGGGLEWVAV**ISSDGSNR**YNADSVKGRFTISRDNSKNTLYLQMNSLRTEDTAVYFC**ARDREVGKSYGLDGFDV**WGQGTMVTVXS | **GFTFSTYV** | **ISSDGSNR** | **ARDREVGKSYGLDGFDV** |

(continued)

| SEQ ID NO | Name | Heavy chain aa sequence | CDR1-H | CDR2-H | CDR3-H |
|---|---|---|---|---|---|
| 173 | S.11.8.E10 | QVQLVQSGPEVKKPGTSVKV SCKAS**GFTFSMSA**VQWVRQA RGQRLEWIGWI**VVGSGNT**EL AQKFQQRVIMTRDESTGTAY MELNSLTSEDTAAYYC**AAPS CTNVLCFDGFDL**WGQGTMVT VSS | **GFTFSM SA** | **IVVGSGN T** | **AAPSCTNVLCFD GFDL** |
| 175 | S.11.8.H11 | EVQLVETGGGLIQPGGSLRL SCTTS**GLIVSRNY**MHWVRQA PGKGLEWVAV**IYPGGTT**HYA DSVKGRFTISREHSNNTLSL QMKSLRAGDTAVYYC**ARDLD VLGGFDI**WGQGTMVTVSS | **GLIVSR NY** | **IYPGGTT** | **ARDLDVLGGFDI** |
| 177 | S.11.8.D6 | QVQLVESGGGVVQPGRSLRL SCAAS**GFTFSIYG**MHWVRQA PGKGLEWVAV**MSYDGSHV**YY GDSVKGRFTISRDNSKNTLY LQMNILRVEDTAVYYC**ARDY GDYAALDH**WGQGTLVTVSS | **GFTFSI YG** | **MSYDGSH V** | **ARDYGDYAALDH** |
| 179 | S.11.4.A8 | QVQLQESGPGLVKPSETLSL TCAVS**GYSISSGYY**WGWIRQ PPGKGLEWIGS**MHHSGNT**DY NPSLKSRATISIDTSKNQLS LKLSSVTAADTAVYYC**ARIM QGRIVVLISYIDF**WDQGSLV TVSS | **GYSISS GYY** | **MHHSGNT** | **ARIMQGRIVVLI SYIDF** |
| 181 | S.8.2.B2 | EVQLVESGGGWVQPGGSLRL SCSAS**GFSFSTYA**MNWVRQA PGKGLEYVSG**ISSNGDIT**YY ADSVKGRFTISRDNSKNTLY LQMSSLRTEDTAIYYC**VKDL GTVVTVDVFDI**WGQGTMVTV SS | **GFSFST YA** | **ISSNGDI T** | **VKDLGTVVTVDV FDI** |

(continued)

| SEQ ID NO | Name | Heavy chain aa sequence | CDR1-H | CDR2-H | CDR3-H |
|---|---|---|---|---|---|
| 183 | S.9.1.C11 | QVQLVQSGAEVKKPGASVRVSCKTS**GYTFTTYF**IHWVRQAPGRGLEWMG**IINPSGGST**SYALKFQGRVTMRDTSTSTVYMELSSLRSEDTAVYYC**AREEDIVVVPAASFDY**WGQGTLVTVSS | **GYTFTTYF** | **INPSGGS T** | **AREEDIVVVPAASFDY** |
| 185 | S.9.1.D10 | QVQLQESGPGLVKPSETLSLTCSVS**GGSINNYY**WSWIRQPPGKGLEWIGY**IYFSGNT**NYNPSLKSRVTISIDTSKNQFSLKLSSVTAADTAVYYC**AREGTGYCSGGTCYSAEEDAFDI**WGQGTMVTVSS | **GGSINNYY** | **IYFSGNT** | **AREGTGYCSGGTCYSAEEDAFDI** |
| 187 | S.9.2.B2 | QVQLQESGPGLVKPSQTLSLTCTVS**GGSISSGDYY**WSWIRQPPGKGLEWIGY**IYYSGST**YYNPSLRSRVSISVDTSKNQLSLKLRSVTAADTAVYYC**ARDHGGGYNWGPGFDS**WGQGTLVTVSS | **GGSISSGDYY** | **IYYSGST** | **ARDHGGGYNWGPGFDS** |
| 189 | S.9.3.H9 | EVQLVESGGGLVKPGGSLRLSCAAS**GFSFSSYS**MNWVRQTPGKGLEWVSS**ISSSSTSFI**YADSVMGRFTISRDNAKNSLYLQMNRPRAEDTAVYYC**ARDFTPSPRRYFDWLLPLGMDV**WGQGTTVTVSS | **GFSFSSYS** | **ISSSSTS FI** | **ARDFTPSPRRYFDWLLPLGMDV** |

(continued)

| SEQ ID NO | Name | Heavy chain aa sequence | CDR1-H | CDR2-H | CDR3-H |
|---|---|---|---|---|---|
| 191 | S.11.1.E2 | EVQLVESGGDLVQPGGSLRLSCAAS**GFSLSXHW**MSWVRQVPGKGLEWVAN**IKHDESET**YYVASVKGRFTISRDNAKNSLYLQMNSLTDEDSAIYYC**TREAKSGPGDY**WGRGTLVTVSS | **GFSLSXHW** | **IKHDESET** | **TREAKSGPGDY** |
| 193 | S.11.2.C6 | QVQLVESGGGVVQPGRSLRLSCAAS**GFGFNFYL**IHWVRQAPGKGLEWVAL**ISFDGTNE**YYADSVKGRFIISRDNSENTLYLQMNSLRTEDTAVYYC**ARTGGRGSADRGRFDL**WGQGTLVTVSS | **GFGFNFYL** | **ISFDGTNE** | **ARTGGRGSADRGRFDL** |
| 195 | S.11.2.F11 | EVQLLESGGGLVQPGGSQRLSCAAS**GFTFSSYP**MSWVRQAPGKGLEWVSF**ISGSGGTI**YYADSVKGRFTISRDNSKNTLYLQMNSLRAEDTAVYYC**AKDPSTAPIYYYFYMDV**WGKGTTVTVSS | **GFTFSSYP** | **ISGSGGTI** | **AKDPSTAPIYYYFYMDV** |
| 197 | S.11.3.B3 | QVQLVESGXGVVQPGGSRRLSCAAS**GFGLNTYV**LHWVRQPPGKGLEWVAL**ISSDGGNE**QFADSVKGRFTISRDNFDNKVFLQMNGLTTEDTAVYFC**ARTGGRGSADRGRFDL**WGHGALVTVSS | **GFGLNTYV** | **ISSDGGNE** | ARTGGRGSADRGRFDL |
| 199 | S.11.3.G8 | EVQLVESGGGLIQPGGSLRLSCAAS**EVIVSSNY**MSWVRQAPGKGLEWVSV**LFAGGST**FYAASVKGRFTISRDDVKNTLYLQMNSLSAEDTAVYYC**ARDRWEGAFDI**WGLGTMVTVSS | **EVIVSSNY** | **LFAGGST** | **ARDRWEGAFDI** |

(continued)

| SEQ ID NO | Name | Heavy chain aa sequence | CDR1-H | CDR2-H | CDR3-H |
|---|---|---|---|---|---|
| 201 | S.11.5.D7 | EVQLLESGGGLIQPGGSPRLSCAAS**GFTFSSSV**MTWVRQAPGKGLEWVSA**VSGTGGSI**YYADSVKGRFTISRDNSKETLYLQMNSLRAEDTAVYYC**AKEPPAARYFHHFYMDV**WGKGTTVTVSS | **GFTFSSSV** | **VSGTGGSI** | **AKEPPAARYFHHFYMDV** |
| 203 | S. 11. 6. F1 | QVQLQESGPGLVKASETLSLTCTVS**GVSIRSYY**WSWIRQPPGKGLEWIGY**IFYSGST**NYNPSLKSRVTMSVDTSKNQFSLKLSSVTAADTAVYYC**ASLGYGDASWYFDL**WGRGTLVTVSS | **GVSIRSYY** | **IFYSGST** | **ASLGYGDASWYFDL** |
| 205 | S.11.6.B11 | QVQLQESGPGLVTPSGTLSLTCVVS**GGSVSSTNW**WSWVRQPPGKGLEWIGEI**FQSGNT**HYNPSLKSRVSMSLDKSNNHFSLTLTSVTAADTAVYYC**ARGPLLGGGNYYLDV**WGKGTTVTVSS | **GGSVSSTNW** | **IFQSGNT** | **ARGPLLGGGNYYLDV** |
| 207 | S.11.7.B3 | QVQLVQSGAEVKKPGASVKVSCKAS**GYTFTNYH**LHWVRQAPGQGPEWMGI**FYPSGGST**GYARTFQGRVTMTRDTSTSTVYMELSSLRSDDTAVYYC**AREGGSGETYFDY**WGQGTLVTVSS | **GYTFTNYH** | **FYPSGGST** | **AREGGSGETYFDY** |
| 209 | S.11.7.E9 | EVQLLESGGGLVQPGGSLRLSCAAS**GFTFSSYV**VSWVRQAPGKGLEWVST**ISGIGIST**YYADSVRGRFTISRDKSKNTLYLEMNSLSAEDTAVYYC**AKDPSAAAHYYYYYMDV**WGKGTTVTVSS | **GFTFSSYV** | **ISGIGIST** | **AKDPSAAAHYYYYYMDV** |

(continued)

| SEQ ID NO | Name | Heavy chain aa sequence | CDR1-H | CDR2-H | CDR3-H |
|---|---|---|---|---|---|
| 211 | S.11.7.C11 | QVQLVESGGGVVQPGRSLRLSCAAS**GFIFSNYG**MHWVRQAPGKGLEWMAV**ILDDGSGK**FYADSVKGRFTISRDNSKNTLYLQLNSLREDDTAVYYC**ARDWGSLVTLFDY**WGQGTLVTVSS | **GFIFSNYG** | **ILDDGSGK** | ARDWGSLVTLFDY |
| 213 | S.11.8.F10 | EVQLVETGGGLIQPGGSLRLSCAAS**GVTVSRNY**MYWVRQAPGKGLEWVSV**IYSGGST**FYADSVKGRFTISRDNSNNTLYLQMNSLRAEDTAVYYC**ARDAVVYGMDV**WGQGTTVTVSS | **GVTVSRNY** | **IYSGGST** | **ARDAVVYGMDV** |
| 215 | S.12.1.A4 | QVQLVQSGTEVKKPGSSVKVSCKAS**GGTFSSSA**ISWVRQAPGQGLEWMGG**IITIFGTS**NIAQKFQGRVTITADAPSSTAYMGLSSLRSEDTAVYYC**ARSRGRYYSSSRVYHYYGMDV**WGQGTTVTVSS | **GGTFSSSA** | **IITIFGTS** | **ARSRGRYYSSSRVYHYYGMDV** |
| 217 | S.12.1.B7 | QVQLVQSGAEVKKPGSSVKVACRAS**GDTLSSYA**ISWVRQAPGQGLEWMGG**IIPMFGIA**DYAHKFQGRVTITADGSTSTAYMELSSLRSEDTAVYYC**ARTLAGQLGRVNYYYGMDV**WGLRGPRSPSPQ | **GDTLSSYA** | **IIPMFGIA** | **ARTLAGQLGRVNYYYGMDV** |
| 219 | S.12.1.C10 | EVQLLESGGDLIQPGGSLRLSCAAS**GFTFSTYA**MIWARQAPGKGLEWVSS**ITGSGDYT**YFADSVKGRFTMSRDNSKNTVYLQMNNLRVEDTALYFC**AKGGMTTAYY**WGQGTLVTVSS | **GFTFSTYA** | **ITGSGDYT** | **AKGGMTTAYY** |

(continued)

| SEQ ID NO | Name | Heavy chain aa sequence | CDR1-H | CDR2-H | CDR3-H |
|---|---|---|---|---|---|
| 221 | S.12.2.A3 | EVQLVESGGGLVEPGGSLRLSCAAS**GFTFTSYT**MNWVRQAPGKGLEWVSS**ISSSGRHI**HYGDSMKGRFHISRDNSKSLVFLEMNSLRAGDTAMYYC**ARGYDVLTGYYKIQKSRYYHMDV**WGQGTTVTVSS | **GFTFTSYT** | **ISSSGRHI** | **ARGYDVLTGYYKIQKSRYYHMDV** |
| 223 | S.12.2.C5 | QVQLVQSGAEVKKPGASVKVSCKAS**GYTFTNYY**IHWVRQAPGQGLEWMGM**INPAGGSI**AYAQKFQGRVTMTRDTSTSTVYMDLSSLTSEDTAVYYC**ARDDFVVPAAGPFDP**WGQGTLVTVSS | **GYTFTNYY** | **INPAGGSI** | **ARDDFVVPAAGPFDP** |
| 225 | S.12.2.G11 | EVQLVESGGGLVKPGGSLRLSCAAS**GFTFNNAW**MNWVRQAPGKGLEWVGR**IKSKTDGGTT**DYAAPVKGRFTISRDDSENTLFLQMNSLKTEDTAVYYC**TTDDSVLWFGELPYFDY**WGQGTLVTVSS | **GFTFNNAW** | **IKSKTDGGTT** | **TTDDSVLWFGELPYFDY** |
| 227 | S.12.3.H1 | EVQLVESGGGLVKPGGSLRLSCAVS**GFTFSNAW**MSWVRQAPGKGLEWVGR**VKSKSDGGTT**DYAAPVKGRFTISRDDSKNTLYLQMNSLKTEDTAVYYC**TTDDSVLWFGELSYFDY**WGQGTLVTVSS | **GFTFSNAW** | **VKSKSDGGTT** | **TTDDSVLWFGELSYFDY** |
| 229 | S.12.1.B12 | EVQLVESGGGLVQPGRSLRLSCAAS**GFTFDDYA**MHWVRQAPGKGLEWVSG**ISWNSGSI**GYADSVRGRFTISRDNAKNSLY | **GFTFDDYA** | **ISWNSGSI** | **AKDTHQLVEGDNWFDP** |

(continued)

| SEQ ID NO | Name | Heavy chain aa sequence | CDR1-H | CDR2-H | CDR3-H |
|---|---|---|---|---|---|
| | | LQMNSLRTEDTALYYC**AKDT HQLVEGDNWFD**PWGQGTLVT VSS | | | |
| 231 | S.12.4.A8 | EVQLVESGGGLVKPGGSLRL SCAAS**GFTFTNAW**MSWVRQP PGRGLEWVGR**IRSKSDGGTT** DYAAPVKGRFSISRDDSKNT LYLQMNSLKTEDTAVYYC**TT DDSVLWFGELPYFDY**WGQGA LVTVSS | **GFTFTN AW** | **IRSKSDG GTT** | **TTDDSVLWFGEL PYFDY** |
| 233 | S.11.1.F6 | QVQLQESGPGLVKPSETLSL TCSVS**GGSIINSAIW**WNWVR QTPGKGLEWIGE**VYHRGST**N YNPSLKSRVTLSLDKSKNQF SLKLTSLTAADTAVYYC**ASK LFDNYIRFES**WGQGTLVTVS S | **GGSIIN SAIW** | **VYHRGST** | **ASKLFDNYIRFE S** |
| 235 | S.11.3.E5 | QLQLQESGSGLVKPSQTLSL TCTVS**GGSIGSGGY**TWNWIR QAPGKGLESIGY**IYPGGSA**Y YNPSLKSRVTISVDGSKNQF SLKLSSVTAADTAVYYC**ARE YGIGRGYDDMPVYRRFDL**WG RGALVTVSS | **GGSIGS GGYT** | **IYPGGSA** | **AREYGIGRGYDD MPVYRRFDL** |
| 237 | S.12.2.G6 | EVQLVESGGGLVQPGRSLRL SCAAS**GFTFDDYA**MHWVRQA PGKGLEWVSG**ISWNSGSI**GY ADSVRGRFTISRDNAKNSLY LQMNSLRTEDTALYYC**AKDT HQLVEGDNWFD**PWGQGTLVT VSS | **GFTFDD YA** | **ISWNSGS I** | **AKDTHQLVEGDN WFDP** |

(continued)

| SEQ ID NO | Name | Heavy chain aa sequence | CDR1-H | CDR2-H | CDR3-H |
|---|---|---|---|---|---|
| 239 | S.11.4.E4 | QLQLQESGPGLVKPSETLSL TCTVS**GGSLYSGGHF**WGWFR QPPGKGLEWIGS**VFYSGIT**Y YNPSLEGRLTIFVDPAKNQF SLRLNSVTAADTAVYFC**ASL YDNSGLWQFDY**WGQGTLVTV SS | **GGSLYS GGHF** | **VFYSGIT** | **ASLYDNSGLWQF DY** |
| 241 | S.11.5.C2 | EVQLVESGGGLIQPGGSLRL SCEAS**GIIVSSNY**MTWVRQA PGKGLEWVSV**LYAGGST**FYT DSVKGRFTISRDNSXNXLYL QMNSLRAEDTAVYYC**ARDLA VFGVDI**WGQGTMVTVSS | **GIIVSS NY** | **LYAGGST** | **ARDLAVFGVDI** |
| 243 | S.11.8.G10 | QVQLQESGPGLVTPXGTLSL TCVXS**GGSVSSTN**WWSWVRQ PPGKGLEWIGE**IFQSGNT**HY NPSLKSRLSMSLDKSNNHFS LTLTSVTAADTAVYYC**ARGP LLGGGNYYLDV**WGKGTTVSV SS | **GGSVSS TNW** | **IFQSGNT** | **ARGPLLGGGNYY LDV** |
| 245 | S.12.1.A5 | QVQLQESGPRLVRPSQTLSL NCTVS**GDSLSRNIIY**WTWIR QRPGKGLEWIGY**IQHTGTT**F YSPSLKSRLIMSIDTSNNQF SLSLDSLTAADTAVYYC**VRG IYCGGGCYRGADH**WGPGIQV AVSS | **GDSLSR NIIY** | **IQHTGTT** | **VRGIYCGGGCYR GADH** |

(continued)

| SEQ ID NO | Name | Heavy chain aa sequence | CDR1-H | CDR2-H | CDR3-H |
|---|---|---|---|---|---|
| 247 | S.12.1.E10 | QVQLQESGPRLVRPSQTLSLNCTVS**GDSISRHIIY**WTWIRQRPGKGLEWIGY**IHHTGST**FYSPSLKSRLTMSIDTSNNQFSLSLGSLTAADTALYYC**ARGTYCGGACYRGTDH**WGPGIQVAVSS | **GDSISRHIIY** | **IHHTGST** | **ARGTYCGGACYRGTDH** |
| 249 | S.12.3.E12 | QVQLVESGGGLVKPGGSLRLSCAAS**GFTFSGHY**MAWIRQAPGKGLEWLSY**ISTSGSYT**EYVDSVKGRFTISRDNARNSLYLQMNSLRVDDTAIYYC**ARDHCGADCYSNWFDS**WGQGTLVTVSS | **GFTFSGHY** | **ISTSGSYT** | **ARDHCGADCYSNWFDS** |
| 251 | S.11.1.B8 | EVQLVESGGVLVQPGGSLRLSCAAS**GFSLSNHW**MSWVRQAPGKGLEWVAN**IKHDGSEK**YYVDSVKGRFTISRDNARNSLYLQMDSLRAEDTALYYC**AREAFSGPGDY**WGQGTLVTVSS | **GFSLSNHW** | **IKHDGSEK** | **AREAFSGPGDY** |
| 253 | S.11.1.E9 | EVQLLESGGGLVQPGGSLRLSCAAS**GFTFSDYV**MSWXRQAPGKGLEWVSA**TSGSGGSI**YYADSVKGRFTISRDNSKNTLYLQMNSLRAEDTAVYYC**AKDGPSGILTGPPPRVYFYYYMAV**WGIGTTVTVSS | **GFTFSDYV** | **TSGSGGSI** | **AKDGPSGILTGPPPRVYFYYYMAV** |
| 255 | S.11.2.E3 | EVQLLESGGGLVQPGGSLRLSCAASG**FPFITYA**MSWVRQAPGKGPEWVSG**ISSRGGKT**YYADSVKGRFTISRDNDGNILSLQMNSLRAEDTAVYYC**AKALLRSSGSYDGNYFYFYMDV**WGKGTTVTVSS | **GFPFITYA** | **ISSRGGKT** | **AKALLRSSGSYDGNYFYFYMDV** |

(continued)

| SEQ ID NO | Name | Heavy chain aa sequence | CDR1-H | CDR2-H | CDR3-H |
|---|---|---|---|---|---|
| 257 | S.11.2.H4 | EVQLVESGGGLVKPGGSLRLSCAAS**GFTLNTYG**MNWVRQAPGKGLEWVSS**ISGRSNFI**YYADSLRGRFTISRDNAKNSLYLQMNSLRAEDTAVYYC**ARDFPPETSRRFDWLLADGLDV**WGQGTTVTVSS | **GFTLNTYG** | **ISGRSNFI** | **ARDFPPETSRRFDWLLADGLDV** |
| 259 | S.11.3.A2 | QVQLVESGGGVVQPGRSLRLSCAAS**GFTFSNYG**MHWVRQAPGKGLEWVAV**MSYDGSHK**YYADSVKGRFTISRDNSKNTLYLQMNSLRGDDTAVYYC**ARDYGDYGAFDI**WGQGTMVTVSS | **GFTFSNYG** | **MSYDGSHK** | **ARDYGDYGAFDI** |
| 261 | S.11.3.E11 | EVQLVEPRWVLSQVQLQQSGPGLVKPSETLSLTCTVS**GGSISSYY**WSWIRQPPGKGLEWIGY**IYYTGST**NYNPSLKSRVTIAVDTSKNQFSLKLSSVTAADTAVYYC**ATYYYDSSGWDYGLDV**WGLGTTVTVSS | **GGSISSYY** | **IYYTGST** | **ATYYYDSSGWDYGLDV** |
| 263 | S.11.3.F2 | EVQLVESGGGLIQPGGSLRLSCGVS**GFTFSQYW**MNWVRQVPGKGLVWVAR**VSSDGSSA**TYADSVKGRFTISRDNAKNTLYLQMESLRVEETAVYYC**ARSAYLRGWFDP**WGQGTLVTVSS | **GFTFSQYW** | **VSSDGSS A** | **ARSAYLRGWFDP** |
| 265 | S.11.3.F9 | QVQLVESGGGVVQPGRSLRLSCAAS**GFPFLTYA**MHWVRQAPGKGLEWVAG**ISSDGDTK**SYADSVKGRSTISRDNYASTLYLQIDSLTTEDTAVYYC**ARTSGRGSNDRARFDP**WGQGTLVTVSS | **GFPFLTYA** | **ISSDGDT K** | **ARTSGRGSNDRARFDP** |

(continued)

| SEQ ID NO | Name | Heavy chain aa sequence | CDR1-H | CDR2-H | CDR3-H |
|---|---|---|---|---|---|
| 267 | S.11.4.D10 | QVQLQESGPGLVKPSETLSL TCTVS**GYSIRSGYY**WGWIRQ PPGKGLEWLGS**IHQSGNT**SY NPSLKRRVTMSVDTSKNQFS LKLSSVTAADTAVYYC**ARIM QGRIVVLITYMDY**WGQGTLV TVSS | **GYSIRS GYY** | **IHQSGNT** | **ARIMQGRIVVLI TYMDY** |
| 269 | S.11.4.F10 | EVQLVETGGGLIQPGGSRRL SCAAS**GITVSSNY**MNWVRQA PGKGLEWVAV**IYSGGST**FYA DSVKGRFTISRDNSKNTLYL QINSLRADDTAVYYC**ARGVW DDAFDI**WGQGTMVTVSSA | **GITVSS NY** | **IYSGGST** | **ARGVWDDAFDI** |
| 271 | S. 11. 4.G6 | QVQLVESGGGVVQPGRSLRL SCVAS**GFTFSNYG**MHWVRQA PGKGLEWVAV**ISYDGSMI**YF ADSVKGRSTIPRDNSKNTLY LQMNNLRPEDTAVYYC**ARDY GDYAALDF**WGQGTMVTVSS | **GFTFSN YG** | **ISYDGSM I** | **ARDYGDYAALDF** |
| 273 | S.11.6.H11 | QVQLQESGPGLVKPSETLSL TCTVS**GGSIISSAIW**WNWVR QPPGKGLEWIGE**VYHSGST**N YNPSLKSRVILSVDKSKNQF SLKLTSLTAADTAVYYC**ASK LYDYYIRYDS**WGQGTLVTVS S | **GGSIIS SAIW** | **VYHSGST** | **ASKLYDYYIRYD S** |
| 275 | S.11.7.A12 | QVQLQESGPGLVKPSETLSL ACAVS**GYSISSGYY**WAWLRQ PPGKGLEWIGS**IHQSGDT**SY SPSLKSRVAISIDKSKNQFS LKLSSVTAADTAVYYC**ARVM QGRIVVLIAYIDY**WGQGTLV TVSS | **GYSISS GYY** | **IHQSGDT** | **ARVMQGRIVVLI AYIDY** |

(continued)

| SEQ ID NO | Name | Heavy chain aa sequence | CDR1-H | CDR2-H | CDR3-H |
|---|---|---|---|---|---|
| 277 | S.11.7.A8 | EVQLVESGGGLIQPGGSLRL SCAVS**DLIVGDSY**MTWVRQA PGKGLEWVSV**IYRGGNV**YHA DSVKGRFTVTRDRATNTLFL HMNSLKAEDTAVYYC**ARRQS TSWSLDY**WGQGTLVTVSS | **DLIVGD SY** | **IYRGGNV** | **ARRQSTSWSLDY** |
| 279 | S.11.7.G6 | QVQLQESGPGLVKPSETLSL TCTVS**GGSVSGGTYY**WSWIR QHPGKGLEWIGNI**YFYGST**Y YNPSVGTRITLSLDTSKNHI YLRLTSVTAADTAVYYC**ARS YTGYQSDDALDI**WGQGTMVT VXS | **GGSVSG GTYY** | **IYFYGST** | **ARSYTGYQSDDA LDI** |
| 281 | S.11.8.B12 | QVQLVESGGGVVQPGRSLRL SCAAS**GFGITTYT**MNWVRQA PGMGLEWVAS**ISSDGSNT**YH ADSVKGRFTVSRDNYMNTLY LQLNSPRSEDTAVYYC**ARTS GRGSADRGRFDP**WGQGTLVT VSS | **GFGITT YT** | **ISSDGSN T** | **ARTSGRGSADRG RFDP** |
| 283 | S.11.8.B2 | QVQLVQSGAELKKPGASVEV SCKAS**GYTFTNSY**IHWVRQA PGQGLEWMGI**INPSGGST**SY AQKFQGRVTMTRDTSTPTVY MELSSLSSEDTAVYYC**ARDM GRNYDILTDYESFYYHAMDV** WGQGTTVTVSS | **GYTFTN SY** | **INPSGGS T** | **ARDMGRNYDILT DYESFYYHAMDV** |
| 285 | S.11.8.C3 | EVQLVETGGGLIQPGGSLRL SCAAS**GVTVSSNY**MSWVRQA PGKGLDWVSV**IYPGGTT**NYA DSVKGRFTISRDNSKNTLHL QMDSLRAEDTAVYYC**ARDFF EGAMDV**WGQGTTVTVSS | **GVTVSS NY** | **IYPGGTT** | **ARDFFEGAMDV** |

(continued)

| SEQ ID NO | Name | Heavy chain aa sequence | CDR1-H | CDR2-H | CDR3-H |
|---|---|---|---|---|---|
| 287 | S.11.8.D2 | QVQLQESGPGLVKPSETLSLTCAVS**GGAVSGSNWW**SWVRQPPGKGLEWIGE**VYHSGNT**NYNPSLKRRVVISVDRSKNQFSLHLTSLTAADTAVYFC**ARGPILGGGHYYLDV**WGSRTTVTVSS | **GGAVSGSNW** | **VYHSGNT** | **ARGPILGGGHYYLDV** |
| 289 | S.11.8.D9 | EVQLVQSAAEVRKPGESLKISCKVS**GYSFTNYW**IAWVRQMPGKGLEWMGI**IYPDDSDT**RYSPSFQGQVTISADKAVNTAYLQWSSLKASDTAMYYC**ARHWRPSMAARPGYQYYMDV**WGKGTTVTVSS | **GYSFTNYW** | **IYPDDSDT** | **ARHWRPSMAARPGYQYYMDV** |
| 291 | S.11.8.G11 | QVQLVQSGAEVKKPASSVKVSCKAS**GGTFNIYA**ITWVRQVPGQGLEWMGE**IIPISNSP**TYAQKFQGRILITADESSSTAFMELSSLSSEDTAVYCC**ARRGRFYYDSSGSYYTYFDY**WGQGTLVTVSS | **GGTFNIYA** | **IIPISNSP** | **ARRGRFYYDSSGSYYTYFDY** |
| 293 | S.11.8.G8 | EVQLLEAGGGLVQPGGSLTLSCEAS**GFPFITYA**MNWVRQAPGKGPEWISA**ISSGGDRT**FYADSVKGRFTVSRDNPGNILYLQMNSLSAEDTAVYYC**AKALLRGSGSFHGNYYYYYMDV**WGKGTTVTVSS | **GFPFITYA** | **ISSGGDRT** | **AKALLRGSGSFHGNYYYYYMDV** |

(continued)

| SEQ ID NO | Name | Heavy chain aa sequence | CDR1-H | CDR2-H | CDR3-H |
|---|---|---|---|---|---|
| 295 | S.3.1.A6 | QVQLVESGGGVVQPGRSLRLSCAAS**GFTFSSYG**MHWVRQAPGKGLEWVAV**IWYDGSNK**YYADSVKGRFTISRDNSKNTLYLQMNSLRAEDTAVYYC**AREGYSSSWYRTRFDY**WGQGTLVTVSS | **GFTFSSYG** | **IWYDGSNK** | **AREGYSSSWYRTRFDY** |
| 297 | S.3.2.F6 | EXGAEVKKPGESLKISCKGS**GYSFTSYW**IGWVRQMPGKGLEWMGI**IYPGDSDT**RYSPSFQGQVTISADKSISTAYLQWSSLKASDTAMYYC**ARLRGVIMPQYGMDV**WGXGTTVT | **GYSFTSYW** | **IYPGDSDT** | **ARLRGVIMPQYGMDV** |
| 299 | S.3.3.A4 | EVQLVETGGGVIQPGVSLRLSCAVS**GFNVGRTY**MSWVRQTPGRGLEWVAV**IYTDATT**YYPDSLRGRFSISRDNWKNTVSLQMSSLRGEDTAIYYC**AGGLKYPNTFDF**WGQGTLVTVSS | **GFNVGRTY** | **IYTDATT** | **AGGLKYPNTFDF** |
| 301 | S.3.3.E10 | EVQLVETGGGVIQPGVSPRLSCAAS**GFTVGSSY**MSWVRQAPGRGLEWVSV**IYKDGAP**YYADSVRGRFTISRDDSTNTVSLQMTRLRAEDTAMYFC**ARGLRLPNTFDQ**WGQGTLVTVSS | **GFTVGSSY** | **IYKDGAP** | **ARGLRLPNTFDQ** |
| 303 | S.3.3.G8 | QVQLQQWGAGLLKPSETLSLTCAVY**SESFRGYY**WSWIRQPPGKRLEWIGE**INHSGRT**NYNPSLKSRAAISVDTSRNQFSLILSSVTAADTAVYYC**ARGYGLGTYTWGWAPFDY**WGQGSLVTVSS | **SESFRGYY** | **INHSGRT** | **ARGYGLGTYTWGWAPFDY** |

(continued)

| SEQ ID NO | Name | Heavy chain aa sequence | CDR1-H | CDR2-H | CDR3-H |
|---|---|---|---|---|---|
| 305 | S.5.1.A5 | QVQLQQWGAGLLKPSETLSLSCAVY**GGSFSGYY**WSWIRQPPGKGLEWIGE**INHSGST**NYNPSLKSRVTISVDTSKNQFSLQLNSVSPEDTAVYYC**AMTTKNSEGYWYFDL**WGRGTLVTVSS | **GGSFSGYY** | **INHSGST** | **AMTTKNSEGYWYFDL** |
| 307 | S5.5.1.B8 | QVQLVESGGGVVQPGRSLRLSCVAS**GFTFSSYA**MHWVRQAPGKGLEWVAV**ISYDGGDR**YYTDSVKGRFTISRDNSKSTLYLQMNSLRAEDTAVYYC**AKDLSRGYDSDDY**WGQGTLVTVSS | **GFTFSSYA** | **ISYDGGDR** | **AKDLSRGYDSDDY** |
| 309 | S. 6. 1. F8 | EVQLVESGGGVVRPGGSLRLSCAAS**GFTFDDYG**MSWVRQAPGKGLEWVSG**INWNGGST**GYADSVKGRFTISRDNAKNSLYLQMNSLRAEDTALYYC**ARDPSIATTADYYYGMDV**WGQGTTVTVSS | **GFTFDDYG** | **INWNGGST** | **ARDPSIATTADYYYGMDV** |
| 311 | S.6.1.H8 | QVQLVESGGGVVQPGRSLRLSCAAS**GFTFKTYG**MHWVRQAPGKGLEWVAV**ISYDEKTT**YYADSVKGRFTISRDNSKNTLYLQIKNSRREDTAVYLC**AKGGSAAAVLDY**WGQGTLVTVSS | **GFTFKTYG** | **ISYDEKTT** | **AKGGSAAAVLDY** |
| 313 | S.7.11.B7 | QVQLVQSGAEVKKPGASVNVSCKAS**GYTFTSYG**ISWVRQAPGQGLEWMGW**ISAYNGNT**NYAQKIQGRVTMTTDTSTSTAYMELRSLRSDDTAVYYC**ARVSRSVWFGEARKSAMDV**WGQGTTVTVSS | **GYTFTSYG** | **ISAYNGNT** | **ARVSRSVWFGEARKSAMDV** |

(continued)

| SEQ ID NO | Name | Heavy chain aa sequence | CDR1-H | CDR2-H | CDR3-H |
|---|---|---|---|---|---|
| 315 | S.8.2.B6 | QVQLVQSGPEVRKPGASVKVSCKAS**GYIFSSYA**ISWVRQAPGQGLEWMGF**ISTYNGDA**KYAQKYQGRVTMTIDTSTSTAYMELRSLRSDDTAVYYC**ARDLTGEEYYYDSSDAFDI**WGQGTMVTVSS | **GYIFSSYA** | **ISTYNGDA** | **ARDLTGEEYYYDSSDAFDI** |
| 317 | S.8.2.E3 | EVQLLESGGGLVQPGGSLRLSCAGS**GFTFSSYA**MTWVRQAPGKGLEWVST**ISGGGGST**YYADSVKGRFTISRDNSKNTLYLQMNSLRAEDTALYYC**AKGERIIILVAVTFLDY**WGQGTLVTVSS | **GFTFSSYA** | **ISGGGGST** | **AKGERIIILVAVTFLDY** |
| 319 | S.8.2. F3 | QVQLQESGPGLVKPSQTLSLTCSVS**GGSITSGSY**FWSWIRQPAGKGLEWIGR**IYPTGST**NYNPSLKSRVSISVDTSKNQFSLRLSSVTAADTAVYYC**ARAPHRIYYYDRSGDYYDAFDI**WGQGSMVTVSS | **GGSITSGSYF** | **IYPTGST** | **ARAPHRIYYYDRSGDYYDAFDI** |
| 321 | S.9.1.B2 | QVQLVESGGGVVQPGRSLRLSCVAS**GFTFSRYG**MHWVRQAPGKGLEWVAV**ISYDGSNK**DYADSVKGRFTISRDISKNTLYLQMNSLRAEDTAVYYC**AKEVGYSDYDVGDY**WGQGTLVTVSS | **GFTFSRYG** | **ISYDGSNK** | **AKEVGYSDYDVGDY** |

(continued)

| SEQ ID NO | Name | Heavy chain aa sequence | CDR1-H | CDR2-H | CDR3-H |
|---|---|---|---|---|---|
| 323 | S.9.1.C7 | QVQLQESGPGLVKPSQTLSL TCTVS**GGSISSGSYY**WSWIR QPAGKGLEWIGR**IFPTGST**N YNPSLKSRVTISVDTSKNQF SLKLSSVTAADTAVYFC**ARE PFKMYYYDNSDYYYDAFDI**W GQGTMVTVSS | **GGSISS GSYY** | **IFPTGST** | **AREPFKMYYYDN SDYYYDAFDI** |
| 325 | S.9.1.D5 | EVQLVESGGGLVQPGGSLRL SCAGS**GFTVSSNY**MSWVRQA PGKGLEWVSV**IYSGGST**KYA DSVKGRFTISRDNSKNTLYL QMNSLRLEDTAVYYC**ARDLE GTD**WGQGTLVTVSS | **GFTVSS NY** | **IYSGGST** | **ARDLEGTD** |
| 327 | S. 9. 1. H9 | QLQLQESGPGLVKPSETLSL TCTVS**GDSISSSSYY**WGWIR QPPGKGLEWIGS**IFYSGST**Y YNPSLKSRVTISVDTSNNQF SLKLSSVTAADTAVYYC**ARL YDTYYYHHGMDV**WGQGTTVT VSS | **GDSISS SSYY** | **IFYSGST** | **ARLYDTYYYHHG MDV** |
| 329 | S.9.2.E3 | QVQLVQSGAEVKKPGASVKV SCKVS**GYTLTEVS**IHWVRQA PGKGLEWMGG**FDPEDAET**IY AQKFQGRVTMTEDTSTDTAY MELSSLRSDDTAVYYC**AAGV AVTGTPLAYSYYYGMDV**WGQ GTTVTVSS | **GYTLTE VS** | **FDPEDAE T** | **AAGVAVTGTPLA YSYYYGMDV** |

(continued)

| SEQ ID NO | Name | Heavy chain aa sequence | CDR1-H | CDR2-H | CDR3-H |
|---|---|---|---|---|---|
| 331 | S.9.2.F3 | EVQLLESGGXLVQPGGSLRLSCAAS**GFTFSNYV**MNWVRQAPGKGLEWVST**ISGSGSTT**YYADSVKGRFTISRDNSKNTLYLQMNSLRAEDTAVYYC**AKGERIIMIVVVTFFDY**WGQGTLVTVSS | **GFTFSNYV** | **ISGSGSTT** | **AKGERIIMIVVVTFFDY** |
| 333 | S.9.2.G1 | EVQLLESGGGLVQPGGSLRLSCAAS**GLTFSSYA**MSWVRQAPGKGLEWVSS**ISGGGGST**YYADSVKGRFTISGDNSKNTLFLQMNSLRAEDTAVYYC**AKNERIITMVVITLFDY**WGQGTLVTVSS | **GLTFSSYA** | **ISGGGGST** | **AKNERIITMVVITLFDY** |
| 335 | S.9.3.D5 | EVQLVESGGGLVKPGGSLRLSCAAS**GFTFSSYS**MNWVRQAPGKGLEWVSS**ISSSNSYI**YYADSVKGRFTISRDNAKNSLYLQMNSLRAEDTAVYYC**ARWKLPYYDSSGYYPEAFDI**WGQGTMVTVSS | **GFTFSSYS** | **ISSSNSYI** | **ARWKLPYYDSSGYYPEAFDI** |
| 337 | S.9.3.F7 | QVQLQESGPGLVKPSQTLSLTCTVS**GDSISSGDYY**WSWIRQPPGKGLEWIGY**IYYTGIT**YYNPSLKSRVTISVDTSKNQFSLKLSSVTAADTAMYYC**VRARVVPAAISDAQNYYYYYGMDV**WGQGTTVTVSS | **GDSISSGDYY** | **IYYTGIT** | **VRARVVPAAISDAQNYYYYYGMDV** |
| 339 | S.9.3.G1 | EVQLVESGGGLVKPGGSLRLSCAAS**GFAFSNAW**MNWVRQAPGKGLEWVGR**IKSETDGGTT**DYAAPVKGRFTISRDDSKNT | **GFAFSNAW** | **IKSETDGGTT** | **VTVSPGYNYGYEDFDH** |

(continued)

| SEQ ID NO | Name | Heavy chain aa sequence | CDR1-H | CDR2-H | CDR3-H |
|---|---|---|---|---|---|
| | | LYLQMNSLKTEDTAVYYC**VT VSPGYNYGYEDFDH**WGQGTL VTVSS | | | |
| 341 | 5.9.3.H10 | QVQLVQSGSELKKPGASVKV SCKAS**GYTFTTYA**MNWVRQA PGQGLEWMGW**INTNTGNP**MY AQGFRGRYVFSLDTSVSTAY LQISSLKAEDTAVYYC**ARDD VVSVVGTAHYYYYGMDV**WGQ GTTVTVSS | GYTFTT YA | INTNTGN P | ARDDVVSVVGTA HYYYYGMDV |
| 343 | S.9.4.B6 | QVQLQESGPGLVKPSGTLSL TCAVSG**ASISSGNW**WSWVRQ PPGKGLEWIGE**VYHSGNT**NY NPSLKSRVTMSVDKSKNQFS LKLSSVTAADTAVYYC**PRY CSSTSCPNWFDP**WGQGTLVT VSS | GASISS GNW | VYHSGNT | APRYCSSTSCPN WFDP |
| 345 | S. 9. 4.B8 | QVQLQESGPGLVKPSQTLSL TCTVSG**GSISSGSYY**WSWIR QPAGKGLELIGR**IYTSGGT**N YNPSLKSRVTISVDRSKNQF SLKLSSVTAADTAVYYC**ARY THRAYYYDSSSYYDAFDI**WG QGTMVTVSS | GGSISS GSYY | IYTSGGT | ARYTHRAYYYDS SSYYDAFDI |
| 347 | S.9.4.C4 | QVQLQESGPGLVKPSQTLSL TCTVS**GDSISSGDYY**WTWIR QPPGKGLEWIGY**IYYSGIT**Y YNPSLKSRVTISVDTSRNQF SLKLSSVTAADTAMYYC**ARI VVVPAAIAAERRDYYYYGM DV**WGQGTTVTVSS | GDSISS GDYY | IYYSGIT | ARIVVVPAAIAA ERRDYYYYYGMD V |

(continued)

| SEQ ID NO | Name | Heavy chain aa sequence | CDR1-H | CDR2-H | CDR3-H |
|---|---|---|---|---|---|
| 349 | S.9.4.E1 | QVQLVESGGGVVQPGRSLRLSCAAS**GFTFSSYA**MHWVRQAPGKGLEWVAL**ISYDGSNKYYA**DSVKGRFTISRDNSKNTLYLQMNSLRAEDTAVYYC**ARDSGSQLDY**WGQGTLVTVSS | GFTFSSYA | ISYDGSNK | ARDSGSQLDY |
| 351 | S.9.5.B2 | QVQLQESGPGLVKPSGTLSLTCTVSG**DSISSSNW**WSWVRQPPGKGLEWIGE**IYHSGNT**NYNPSLKSRVTISVDKAKNQFSLKLTSVTATDTAVYYC**ASRYCSSTACPNWFDP**WGQGTLVTVSS | GDSISSSNW | IYHSGNT | ASRYCSSTACPNWFDP |
| 353 | S.9.5.B8 | QVQLQESGPGLVKPSGTLSLTCAVS**GGSISSGDW**WSWVRQPPGKGLEWIGE**IYHSGNTN**YSPSLKSRVTISVDNSKNQFSLKLNSVTAADTAVYYC**ARRYCSSTTCPNWFDP**WGQGTLVTVSS | GGSISSGDW | IYHSGNT | ARRYCSSTTCPNWFDP |
| 355 | S.9.5.C6 | QVQLVQSGSELKKPGASVKVSCKAS**GYSFSSYA**MNWVRQAPGQGLEWMGW**INTNTGKP**TYAQDFTGRFVFSLDTSVNTAYLQISSLKAEDTAVYYC**ARVQGVGVVAESYYYYYAMDV**WGQGTTVTVSS | GYSFSSYA | INTNTGK P | ARVQGVGVVAESYYYYYAMDV |

(continued)

| SEQ ID NO | Name | Heavy chain aa sequence | CDR1-H | CDR2-H | CDR3-H |
|---|---|---|---|---|---|
| 357 | S.11.1.E5 | EVQLVESGGGLAQPGKSLRLSCTAS**GFTFDDY**AMHWVRQVPGKGLEWVSG**ISWNGDSI**GYGASVKGRFTISRDNAKNSLYLQMNSLRDEDTALYYC**AKGRYVDWTHAMDV**WGQGTTVTVSS | **GFTFDDY** | **ISWNGDS I** | **AKGRYVDWTHAMDV** |
| 359 | S. 11. 1. E5 | EVQLVESGGGLAQPGKSLRLSCTAS**GFTFDDY**AMHWVRQVPGKGLEWVSG**ISWNGDSI**GYGASVKGRFTISRDNAKNSLYLQMNSLRDEDTALYYC**AKGRYVDWTHAMDV**WGQGTTVTVSS | **GFTFDDY** | **ISWNGDSI** | **AKGRYVDWTHAMDV** |

Table 3 - CDR1, CDR2 and CDR3 regions of the light chain of the monoclonal antibody of the present invention

| SEQ ID NO | Nome | Sequência aa da cadeia leve | CDR1-L | CDR2-L | CDR3-L |
|---|---|---|---|---|---|
| 2 | S.8.1.A4_K, | DIQMTQSPSSLSASVGDRVTITCQAS**QDINIC**LNWYQQKPEKAPKLLIY**DAS**NLETGVPSRFSGSGSGTDFTFTISSLQPEDIATYYC**QQYDNLPQT**FGQGTKVEIK | **QDINIC** | **DAS** | **QQYDNLPQT** |
| 4 | S.9.1.A12_K | DIQMTQSPSSLSASVGDRVTITCRAS**QGISNY**LAWYQQKPGKAPKLLIY**AAS**TLQSGVPSRFSGSGSGTDFTLTISSLQPEDVATYYC**QKYNSAPPT**FGGGTKVEIK | **QGISNY** | **AAS** | **QKYNSAPPT** |

48

(continued)

| SEQ ID NO | Nome | Sequência aa da cadeia leve | CDR1-L | CDR2-L | CDR3-L |
|---|---|---|---|---|---|
| 6 | S.9.1.B11_L | SYELTQPPSVSVAPGKTARIT CGGN**NVGSQS**VHWYQQRSGQA PVLVVY**DDG**DRPSGIPGRFSG SNSGNTATLTISRVEAGDEAD YFC**QAWDSSRDHVV**FGGGTKL TVL | **NVGSQS** | **DDG** | **QAWDSSRDHVV** |
| 8 | S.9.1.C10_K | IQMTQSPSSLSASVGDRVTIT CRAS**QGIRND**LGWYQQKPGKA PKRLIY**AAS**SLQSGVPSRFSG SGSGTEFTLTISSLQPEDVAT YYC**LQHNSYPMCS**FGQGTKLE IK | **QGIRND** | **AAS** | **LQHNSYPMCS** |
| 10 | S.9.1.C9_K | DIQMTQSPSSLSASVRDRVTI TCQAS**QDISKF**LNWYQQKPGK APKLLIY**DAS**NLETGVPSRFS GSGSGTDFTFTISSLQPEDIA TYYC**QQYDNLPIT**FGQGTRLE IK | **QDISKF** | **DAS** | **QQYDNLPIT** |
| 12 | S.9.1.H5_K | DIQMTQSPSSLSASVGDRITI TCQAS**QDISNY**LNWYQQKPGK APKLMIY**DAS**NLETGVPSRFS GSGAGTDFTFTISSLQPEDFA RYYCQ**QYGNLPLT**FGGGTKVE IK | **QDISNY** | **DAS** | **QQYGNLPLT** |
| 14 | S.9.2.B8_L | SYELTQPPSVSVAPGQTARIT CGGN**NIGSQS**VHWYQQKPGQA PVLVVY**DDS**DRPSGIPERFSG SKSGNMATLTISRVEAGDEAD YYC**QVWDSSSDHVV**FGGGTKL TVL | **NIGSQS** | **DDS** | **QVWDSSSDHVV** |

(continued)

| SEQ ID NO | Nome | Sequência aa da cadeia leve | CDR1-L | CDR2-L | CDR3-L |
|---|---|---|---|---|---|
| 16 | S.9.2.D3_K | EIVLTQSPGTLSLSPGERATL SCRAS**QSVSNSY**LAWYQQKPG QAPRLLIY**GAS**SRATGIPDRF SGSGSGTDFTLTISRLEPEDF AVYYC**QQYDSSPRT**FGQGTKV EIK | **QSVSNS Y** | **GAS** | **QQYDSSPRT** |
| 18 | S.9.2.D7_K | DIQMTQSPSLLSASAGDRVTI SCRMS**QGISSY**LAWYQQKPGK APELLIS**SAS**TLQSGVPSRFS GSGSGTDFTLTISYLQSEDFA TYYC**QQYYSFPTT**FGGGTKVE IK | **QGISSY** | **SAS** | **QQYYSFPTT** |
| 20 | S.9.2.E9_K | IQMTQSPSSLSASVGDRVTIT CRAS**QDIRHD**LGWYQQKPGKA PKRLIY**AAS**SLHSGVPSRFSG SGSGTEFTLTISSLQPEDFAT YYC**LQHNSYPMCS**FGQGTKLE IK | **QDIRHD** | **AAS** | **LQHNSYPMCS** |
| 22 | S.9.2.F4_K | EIVLTQSPYLLSASTGDRVTI SCRMT**QGISSF**LAWYQQKPGK APELLIY**AAS**TLQSGVPSRFS GSGSGTDFTLTISSLQPEDFA TYYC**QQYYSFPPT**FGQGTKLE IK | **QGISSF** | **AAS** | **QQYYSFPPT** |
| 24 | S.9.3.B2_K | DIQLTQSPSFLSASVGDRVTI TCRAS**QGISNY**LAWYQQKPGK APKLLIY**AAS**TLQSGVPSRFS GSGSGTEFTLTISSLQPEDFA TYYC**QQLDSYPPLI**FGGGTKV EIK | **QGISNY** | **AAS** | **QQLDSYPPLI** |

(continued)

| SEQ ID NO | Nome | Sequência aa da cadeia leve | CDR1-L | CDR2-L | CDR3-L |
|---|---|---|---|---|---|
| 26 | S.9.3.G2_K | DIQLTQSPSFLSASVGDRVTITCRAS**QGISSY**LAWYQQKPGKAPKLLIY**AAS**TLQSGVPSRFSGSGSGTEFTLTISSLQPEDFATYYC**QQLNSYPCS**FGQGTKLEIK | **QGISSY** | **AAS** | **QQLNSYPCS** |
| 28 | S.9.4.C7_L | NFMLTQPHSVSESPGKTVTISCTRS**SGSIASYY**VQWYQQRPGSSPTTVIS**EDN**QRPSGVPDRFSGSIDSSSNSASLTISGLKTEDEADYYC**QSYDSNNHVI**FGGGTKLTVL | **SGSIASYY** | **EDN** | **QSYDSNNHVI** |
| 30 | S.9.4.F4_K | AIRMTQSPSSLSASVGDRVTITCQAS**QDISNY**LNWYQQKPGKAPKLLIY**DAS**NLETGVPSRFSGSGSGTDFTVTISSLQPEDFATYYC**QQYDNLPLT**FGGGTKVEIK | **QDISNY** | **DAS** | **QQYDNLPLT** |
| 32 | S.9.4.E3_K | DIQMTQSPSSXSAXVGXRVTITCQAS**QDINNY**LNWYQQKPGKAPKLLIY**DAS**NLETGVPSRFSGSGSGTDFTFTISSLKPEDIATYYC**QQYYSLPPVT**FGQGTRLEIK | **QDINNY** | **DAS** | **QQYYSLPPVT** |
| 34 | S.9.5.A9_K | DIQLTQSPSFLSASVGDRVTITCRAS**QGISSY**LAWYQQKPGKAPKLLIY**AAS**TLQSGVPSRFSGSGSGTEFTLTISSLQPEDFATYYC**QQLNSYPGFT**FGPGTKVDIK | **QGISSY** | **AAS** | **QQLNSYPGFT** |

(continued)

| SEQ ID NO | Nome | Sequência aa da cadeia leve | CDR1-L | CDR2-L | CDR3-L |
|---|---|---|---|---|---|
| 36 | S.10.1.C6_L | QSVLTQPPSVSGAPGQRVTIS CTGS**SSNIGAGYD**VHWYQQLP GTAPKLLIY**GNN**NRPSGVPDR FSGSKSGTSASLAITGLQAED EADYYC**QSYDSSLSALYV**FGT GTKVTVL | SSNIGA GYD | GNN | QSYDSSLSALY V |
| 38 | S.10.1.D8_K | DIQMTQSPSSXSASVGDRVTI TCQAS**QDISNY**LNWYQQKPGK APKLLIY**DAS**NLETGVPSRFS GSGSGTDFTFTISSLQPEDIA TYYC**QQYDNLPRT**FGQGTKVE IK | QDISNY | DAS | QQYDNLPRT |
| 40 | S.10.2.D2_K | EIVLTQSPGTXSXSPGERATL SCRAS**QSVSSTY**LAWYQQKPG QAPRLLIY**GAS**SRATGIP DRFSGSGSGTDFTLTISRLEP EDFAVYYC**QQYGSSPYS**FGQG TKLEIK | QSVSST Y | GAS | QQYGSSPYS |
| 42 | S.11.2.A5 K | DIVMTQSPLSLPVTPGEPASI SCRSS**QSLLDSNGYNY**LDWYL QKPGQSPQLLIY**LGS**NRASGV PDRFSGSGSGTDFTLKISRVE VEDVGVYYC**MQSLQGFT**FGPG TKVDIK | QSLLDS NGYNY | LGS | MQSLQGFT |
| 44 | S.11.2.C5_K | DIQMTQSPSSLSASVGDRVTM SCRAS**QNISTF**LNWYQQEPGK APKLLIY**AAS**SLQSGVPSRFS GSGSGTDFSLTIRSLQPEDFA TYYC**QQSYRSKS**FGQGTKLEI K | QNISTF | AAS | QQSYRSKS |

(continued)

| SEQ ID NO | Nome | Sequência aa da cadeia leve | CDR1-L | CDR2-L | CDR3-L |
|---|---|---|---|---|---|
| 46 | S.11.3.A5_K | DIQMTQSPSSLSASVGDRVTITCRAS**QSISTY**LNWYQQKPGKAPRLVIY**GAS**TLHSGVPSRFSGSGSGTAFTLTISNLQPDDFASYYC**QQSSSSLALT**FGGGTKVEIK | **QSISTY** | **GAS** | **QQSSSSLALT** |
| 48 | S.11.3.H5_K | EIVLTQSPATLSVSPGERVTLSCRAS**QIISRY**LAWYQQKPGQAPRLLIY**GAS**SRAAGVPVRFSGSGSGTDFTLTISSLQSEDFAVYFC**QQYNNWPLT**FGGGTKVEIK | **QIISRY** | **GAS** | **QQYNNWPLT** |
| 50 | S.11.4.F9_K | EIVLTQSPGTLSLSPGERATLSCRAS**QSVISSY**LAWYQQKPGQAPRLLIH**GAS**SRATGIPDRFSGSGSGTDFTLTIRRLEPEDFAVYYC**QHYGSSRT**FGQGTKVEIK | **QSVISSY** | **GAS** | **QHYGSSRT** |
| 52 | S.1.1.D10_K | EIVMTQSPVSLSVSPGERATLSCRAR**ESVSNN**VAWYQQKPGQAPRLLIY**GVS**TRATGIPARFSGSGSGTEFTLTISSLQSEDFAVYYC**QHYNNWPSWT**FGQGTKVEIK | **ESVSNN** | **GVS** | **QHYNNWPSWT** |
| 54 | S.11.1.C7_K | DIVMTQSPDSLAVSLGERATIYCKSS**QSVLNSSNKKNY**LAWYQQKPGQPPRLLIY**WAS**TRESGVPDRFSGSGSGTDFTLTITSLQAEDVAVYYC**QHYYSPPIT**FGQGTRLEIK | **QSVLNSSNKKNY** | **WAS** | **QHYYSPPIT** |

(continued)

| SEQ ID NO | Nome | Sequência aa da cadeia leve | CDR1-L | CDR2-L | CDR3-L |
|---|---|---|---|---|---|
| 56 | S.11.1.C8_K | DIQMTQPPSSLSASVGDRVTI TCRAS**QSIGNN**LNWYQQKPGK APKLLIY**VAS**SLQSGVPSRFS GSGSGTDFTLTISSLQPEDFA TYFC**QQSYSTPPWT**FGQGTKV EIK | **QSIGNN** | **VAS** | **QQSYSTPPWT** |
| 58 | S.11.1.D12_K | DIVMTQSPDSLAVSLGERATI HCKSS**QTVVYGSNNKNY**LAWY QQKPGQPPKLLIY**WAS**TRESG VPDRFSGSGSGTDFTLTISSL QAEDVAVYYC**QQYYGPPPWT**F GQGTKVEIK | **QTVVYG SNNKNY** | **WAS** | **QQYYGPPPWT** |
| 60 | S.11.1.F4_K | DIQMTQSPSSLSASVGDRVTI TCRAS**QTISRY**LNWYQQKPGK PPKLLIF**AAS**TLHSGVPSRFS GSGSGTEFTLTITSLHPDDFS TYYC**QQTYSSPHT**FGQGTKLE IK | **QTISRY** | **AAS** | **QQTYSSPHT** |
| 62 | 5.11.1.H1_K | DIVMTQSPLYLPVTPGEPASI SCRSS**QSLLHSNGYNY**LDWYL QKPGQSPQLLIY**LGS**NRASGV PDRFSGSGSGTDFTLKISRVE AEDVGVYYC**MQTLQTFT**FGPG TKVDIK | **QSLLHS NGYNY** | **LGS** | **MQTLQTFT** |
| 64 | S.11.1.H5_K | EIVLTQSPATLSVSPGERATL SCRAS**QSVSSN**LAWYQQKPGQ APRLLIY**GAS**TRATGIPARFS GSGSGTEFTLTISSLQSEDFA VYYC**QQYNNWPPYT**FGQGTKL EIK | **QSVSSN** | **GAS** | **QQYNNWPPYT** |
| 66 | S.11.1.H8_K | IQMTQSPSSLSASVGDRVTIT CRAS**QDIGSD**LGWFQQRPGKA | **QDIGSD** | **GAS** | **LQYNSYPLT** |

(continued)

| SEQ ID NO | Nome | Sequência aa da cadeia leve | CDR1-L | CDR2-L | CDR3-L |
|---|---|---|---|---|---|
| | | PKRLIY**GAS**SLQSGVPSRVSG SGSGTEFTLTISSLQPEDFAS YYC**LQYNSYPLT**FGGGTKVEI K | | | |
| 68 | S.11.2.A3_K | EIVLTQSPGTLSLSPGERATL SCRAS**QNIGMSY**VAWYQQKGG QAPRLLIY**GVS**SRATGIPDRF SGSGSGTDFTLTISRLEPKDS AVYYC**QQYGSSHRT**FGQGTKL EI | **QNIGMS Y** | **GVS** | **QQYGSSHRT** |
| 70 | S.2.2.F11_K | DIQMTQSPSSLSASVGDRVTI TCQAS**QDISNY**LNWYQQKPGK APKLLIY**DAS**NLETGVPSRFS GSGSGTDFTFTISSLQPEDIA TYYC**QQYDNLLT**FGGGTKVEI K | **QDISNY** | **DAS** | **QQYDNLLT** |
| 72 | S.2.2.G8_K | EIVLTQSPVSLSVSPGERATL SCRAR**ESVSNN**VAWYQQKPGQ APRLLIY**GVS**TRATGIPARFS GSGSGTEFTLTISSLQSEDFA VYYC**QHYNNWPSWT**FGQGTKV EIK | **ESVSNN** | **GVS** | **QHYNNWPSWT** |
| 74 | S.2.3.C4_K | IQMTQSPSAVSASVGDRVTIT CRAS**QGISDY**LVWFQQKPGKV PKRLIY**GAS**SLQSGVPSRFSG SGSGTEFTLTISSLQPEDFAT YYC**LQHKSYPYT**FGQGTKLEI K | **QGISDY** | **GAS** | **LQHKSYPYT** |
| 76 | S.2.3.G7_K | EIVLTQSPATLSLSPGERATL SCRAS**QSVSSY**LAWYQHKPGQ APRLLIY**DAS**NRATGIPARFS GSGSGTDFTLTISSLEPEDFA VYYC**QQRSNWPPLT**FGGGTKV EIK | **QSVSSY** | **DAS** | **QQRSNWPPLT** |

(continued)

| SEQ ID NO | Nome | Sequência aa da cadeia leve | CDR1-L | CDR2-L | CDR3-L |
|---|---|---|---|---|---|
| 78 | S.2.4.D2_K | DIQMTQSPFSLSASVGDRVTI TCRAS**PGISNY**LAWYQQKPGK VPKLLIY**AAS**TLQSGVPSRFS GSGSGTDFTLTITSLQPEDVA TYYC**QKYNNAPFT**FGPGTKVD IK | **PGISNY** | **AAS** | **QKYNNAPFT** |
| 80 | S.2.4.E3_K | IQMTQSPSSVSASVGDRVTIT CRAS**QHVSSS**LVWYQQKPGRA PRLLIF**AAS**KLQSGVSSRFSG SGSGTDFTLTISSLQPEDFAT YYC**QQANSFPYT**FGQGTKLEI K | **QHVSSS** | **AAS** | **QQANSFPYT** |
| 82 | S.2.4.E8_K | DIQMTQSPSSLSASVGDRVVI TCRAS**QSISGY**LNWYQQKPGK APKLLIH**GTS**SLQSGVPSRFS GSGAGTDFTLTISSLQPEDFA TYFC**QQSYSAPLWT**FGQGTKV EIK | **QSISGY** | **GTS** | **QQSYSAPLWT** |
| 84 | S.3.1.B7_K | DVVMTQSPLSLPVTLGQPASI SCRSS**QSLVYSDGNTY**LNWFH QRPGQSPRRLIY**RVS**KRDSGV PDRFSGSGSGNDFTLKISRVE AEDVGVYYC**MQGTHWPLT**FGG GTKVEIK | **QSLVYS DGNTY** | **RVS** | **MQGTHWPLT** |
| 86 | S.3.1.E2_K | DIQMTQSPXTXSASVGDRVTI TCRAS**QSISGG**LAWYQQKAGK APKLLIY**KAS**SLESGVPSRFS GSASGTEFTLTISSLQPDDFA TYYC**HQYNERWT**FGQGTKVEI K | **QSISGG** | **KAS** | **HQYNERWT** |
| 88 | S.3.1.G5_K | DIQLTQSPGTLSLSPGERATL SCRAS**QSISSTF**LAWYQQIPG QAPRLLIY**GAS**SRAIGIPDRF SGSGSGTDFTLTISRLEPQDF | **QSISST F** | **GAS** | **QQYGRSPHT** |

(continued)

| SEQ ID NO | Nome | Sequência aa da cadeia leve | CDR1-L | CDR2-L | CDR3-L |
|---|---|---|---|---|---|
| | | AVYYC**QQYGRSPHT**FGQGTKL EIK | | | |
| 90 | S.3.2.C7_L | QSALTQPASVSGSPGQSITIP CTGT**SSDVGGHNY**VSWYQHHP GKVPQLMIY**NVI**NRPSGVSNR FSGSKSGNTASLIISGLQAED EAEYYC**SSYIGTNNFGVYV**FG TGTKVTV | **SSDVGG HNY** | **NVI** | **SSYIGTNNFGV YV** |
| 92 | S.3.2.D9_K | DIVMTQSPATLSLPPGERATL SCRAS**RSISSN**VAWYQQKPGQ APRLLIY**DAS**NRATGIPARFS GSGSGADFTLTISSLEPEDFA FYYC**QQRSSLPPLT**FGGGTKL EIK | **RSISSN** | **DAS** | **QQRSSLPPLT** |
| 94 | S.8.1.C12_K | EIVLTQSPGTLSLSPGERATL SCRAS**QSISSSY**LAWYQQRPG QAPRLLIY**GAS**SRATGVPDRF SGSGSGTDFTLTISRLEPEDF AVYYC**QQYGLSPAWT**FGQGTK VEIK | **QSISSS Y** | **GAS** | **QQYGLSPAWT** |
| 96 | S.8.1.C2_K | DIQLTQSPSSLSASVGDRVTI TCRAS**QGISTY**LAWYQQKPGK APKLLIY**AAS**TLQSGVPSRFS GSGSGTDFTLTISSLQPEDFA TYYC**QQLNSYPSSYT**FGQGTK LEIK | **QGISTY** | **AAS** | **QQLNSYPSSYT** |
| 98 | S. 9.1.B7_K | IQMTQSPSSVPASVGDRVTIT CRAS**QGISSW**LAWYQQKPGKA PKLLIY**AA**SSLQSGVPSRFSG SGSGTDFTLTISSLQPEDFAT YYC**QQTNDFPPA**LGQGTKVEI KRT | **QGISSW** | **AAS** | **QQTNDFPPA** |
| 100 | S.9.1.G7_K | DIQLTQSPSFLSASVGDRVTI TCRASQ**GISSY**LAWYQQKPGK | **QGISSY** | **AAS** | **QHLNSYPA** |

(continued)

| SEQ ID NO | Nome | Sequência aa da cadeia leve | CDR1-L | CDR2-L | CDR3-L |
|-----------|------|----------------------------|--------|--------|--------|
| | | APKLLIY**AAS**TLQSGVPSRFS GSGSGTEFTLTISSLQPEDFA TYYC**QHLNSYPA**FGQGTKVEI K | | | |
| 102 | S.9.3.A7_L | QSVLTQPPSVSGAPGQRVTIS CSGS**SSNIGAGYD**VHWYQLLP GTAPKLLIY**GNS**NRPSGVPDR FSGSKSGTSASLAITGLQAED EADYYC**QSYDTSLSALYV**FGT GTKVTVL | **SSNIGA GYD** | **GNS** | **QSYDTSLSALY V** |
| 104 | S.9.4.A9_K | IQMTQSPSSLSASVGDRVTIT CRAS**QGINNY**LAWFQQKPGKA PKSLIY**AAS**SLHSGVPSRFSG SGSGTDFTLTISSLQPEDFAT YYC**QQYNTYPIT**FGQGTRLEI K | **QGINNY** | **AAS** | **QQYNTYPIT** |
| 106 | S.9.4.C6_L | SYELTQSPSVSVAPGKTARIT CGGN**DIGSKS**VHWYQQRPGQA PVLVVY**DDT**DRPSGIPERFSG SNSGNTATLTISRVEAGDEAD YYC**QVWDSSGV**FSGGTKLTVL | **DIGSKS** | **DDT** | **QVWDSSGV** |
| 108 | S.11.1.D4_K | DIVMTQSPLSLPVTPGESASI SCRSS**QSLLHSNGYNY**LDWYL QKPGQSPQLLIY**LGS**NRASGV PDRFSGSGSGRDFTLKISRVE AEDVGVYYC**MQSLQTFT**FGPG TKVDIK | **QSLLHS NGYNY** | **LGS** | **MQSLQTFT** |
| 110 | S.11.1.E10_L | SYELTQPPPVSVAPGKTARIT CGGD**NIGGKS**VHWYQQKPGLA PVMVVY**DDS**ARPSGIPERFSG SNSGNTATLTITRAEAGDEAD YYC**QVWDTGSDPLV**FGTGTKV TVL | **NIGGKS** | **DDS** | **QVWDTGSDPLV** |

(continued)

| SEQ ID NO | Nome | Sequência aa da cadeia leve | CDR1-L | CDR2-L | CDR3-L |
|---|---|---|---|---|---|
| 112 | S.11.1.F1_L | QSALTQPASVSGSPGQSIAIS CTGT**SSDVGSYNY**VSWYQQHP GKAPKLMIY**EVS**KRPSGVSNR FSGSKSGNTASLTISGLQAED EADYYC**CSYAGSSTVL**FGGGT KLTVL | SSDVGS YNY | EVS | CSYAGSSTVL |
| 114 | S.11.2.B6_K | DIQLTQSPSFLSASVGDRVTI TCRAS**QGISTY**LAWYQQKPGK APNLLIY**AAS**TLHSGVPSRFS GSGSGTEFTLTISSLQPEDFA TYYC**QQLNGYPGSN**FGQGTKV EIK | QGISTY | AAS | QQLNGYPGSN |
| 116 | S.11.2.E5_K | DIQMTQSPSTLSASVGDRVTI TCRAS**ESFSNW**LAWYQQKPGK APKLLIY**QAS**TLESGVPSRFS GSGSGTEFTLTISSLQPEDFA TYYC**QQYNSGYT**FGQGTKLEI K | ESFSNW | QAS | QQYNSGYT |
| 118 | S.11.2.F5 _K | DIQMTQSPSTLSASVGDRVTI TCRAS**QSISSW**LAWYQQKPGK APKLLIY**KAS**SLESGVPSRFS GSGSGTEFTLTISSLQPDDFA TYYC**QQSQT**FGQGTKVEIK | QSISSW | KAS | QQSQT |
| 120 | S.11.5.A2_K | DIQMTQSPSSLSASVGDRVTI TCRAS**QSISRF**LNWYQQKPGK APKLLIY**ATS**NLQTGVPSRFS GSGSGTDFTLTISSLQPEDFA TYFC**QQTYNTPHT**FGQGTKLE IK | QSISRF | ATS | QQTYNTPHT |
| 122 | S.11.5.C7_L | QSVLTQSPSVSGAPGQRVTIS CTG**SSSNIGAGYD**VHWYQQLP GTAPKIIIY**ANT**NRPSGVPDR FSGSKSGTSASLAITGLQAED | SSNIGA GYD | ANT | QSYDSSLSGYV V |

(continued)

| SEQ ID NO | Nome | Sequência aa da cadeia leve | CDR1-L | CDR2-L | CDR3-L |
|---|---|---|---|---|---|
| | | EADYYC**QSYDSSLSGYVV**FGG GTKLTVL | | | |
| 124 | S.11.6.B5_K | DIQLTQSPSFLSASVGDRVTI TCRAS**QGISNY**LAWYQQKPGK APNLLIY**GAF**ILQSGVPSRFS GSGSGTEFTLTISSLQPEDFA TYYC**QQLNSESRT**FGGGTKVE IK | **QGISNY** | **GAF** | **QQLNSESRT** |
| 126 | S.11.6.G11_K | DIQLTQSPSFLSASVGDRVTI TCRAS**QGISSY**LAWYQQKPGK APKLLIY**AAS**TLQSGVPTRFS GSGSGTEFTLTISSLQPEDFA TYYC**QLLNSYPPT**FGGGTKVE IK | **QGISSY** | **AAS** | **QLLNSYPPT** |
| 128 | S.11.7.A11_K | DIQMTQSPSSLSASVGDRVTI TCRAS**QSVSSY**LNWYQQKSGK APKLLIY**AAS**SLQSGVPSRFS GSGSGTDFTLTISSLQPEDFA TYFC**QQSYRSPLT**FGGGTKVE IK | **QSVSSY** | **AAS** | **QQSYRSPLT** |
| 130 | S.11.7.A5_L | SYELTQPPSVSVAPGKTARIT CGGN**NIGSKS**VNWYQQRPGQA PVLVVY**DDS**DRPSGIPERFSG SNSGNTATLTISRVEAGDEAD YYC**QVWDTGSDPLV**FGTGTKV TVL | **NIGSKS** | **DDS** | **QVWDTGSDPLV** |
| 132 | S.11.7.C8_K | DIQLTQSPSFLSASVGDRVTI TCRAS**QGISSY**LAWYQQKPGK APKLLIY**ASS**TLQSGVPSRFS GSESGTEFTLTISSLQPEDFA TYYC**QQLNSYPLT**FGGGTKVE IK | **QGISSY** | **ASS** | **QQLNSYPLT** |
| 134 | S.11.7.F5_L | SYELTQPPSVSVAPGKTARIT CGGA**NIGSKN**VHWYQQKPGQA | **NIGSKN** | **DDS** | **QVWDSGNFWV** |

(continued)

| SEQ ID NO | Nome | Sequência aa da cadeia leve | CDR1-L | CDR2-L | CDR3-L |
|---|---|---|---|---|---|
| | | PVLVVY**DDS**DRPSGIPERFSG SNSGNTATLTISRVEAGDEAD YYC**QVWDSGNFWV**FGGGTKLT VL | | | |
| 136 | S.11.7.G2_K | DIQMTQSPSSLSASVGDRVTI TCRAS**QSIGKY**LNWYQQKPGK APKLLIY**VAS**NLQSGVPSRFS GSGSGTDFTLTISSLQPEDFA TYYC**QQSYETPPWT**FGQGTKV EIK | **QSIGKY** | **VAS** | **QQSYETPPWT** |
| 138 | S.11.7.G3_L | NFMLTQPQSVSASPGKTVTIS CTGS**GGDVINNY**VQWYQQRPG SAPTTVIF**EDS**QRPSGVPDRF SGSIDSSSNSASLTISGLKTE DEADYYC**QSFDSRVHVV**FGGG TKLIVL | **GGDVIN NY** | **EDS** | **QSFDSRVHVV** |
| 140 | S.11.7.H6_K | DIVMTQSPDSLAVSLGERATI NCKSS**QSLLYISNNKNY**LAWY QQKPGQPPKLLIY**WAS**TRESG VPDRFSGSGSGTDFTLTISSL QAEDVAVYYC**QHYYSSPIT**FG QGTRLEIK | **QSLLYI SNNKNY** | **WAS** | **QHYYSSPIT** |
| 142 | S.11.3.D10_K | DIQMTQSPSSXSASVGDRVTI TCQAS**QDIDIY**LNWYQQTPGK PPKLLIF**DAS**NLETGVPSRFS GSGSGTDFSFTISRLQPEDTA TYYC**QQSDSLPLT**FGGGTKVE IK | **QDIDIY** | **DAS** | **QQSDSLPLT** |
| 144 | S.11.3.G9_K | DIVMTQSPLSLPVTPGEPASI SCRSS**QSLLDSNGKNY**LDWYL QKPGQSPQLLIY**LGS**NRASGV PDRFSGSGSGTDFTLKISRVE AEDVGVYYCM**EALQTFT**FGPG TKVDIK | **QSLLDS NGKNY** | **LGS** | **MEALQTFT** |

(continued)

| SEQ ID NO | Nome | Sequência aa da cadeia leve | CDR1-L | CDR2-L | CDR3-L |
|---|---|---|---|---|---|
| 146 | S.11.3.H8_K | EIVLTQSPATLSVSPGERATL SCRAS**QSVSSS**VAWYQQKPGQ APSLLIY**DVS**TRATETPARFS GSGSGTEFTLTISSLQSEDFA VYYC**QQYINWPGT**FGQGTKLE IK | **QSVSSS** | **DVS** | **QQYINWPGT** |
| 148 | S.11.4.A5_L | QSALTQPASVSGSPGQSITIS CTGT**SADVGSYNF**VSWYQQHP GKAPKLMIY**EVS**KRPSGVSYR FSGSKSGNTASLTISGLQADD EADY**YCYSYVGSTTV**LFGGGT KLTVL | **SADVGS YNF** | **EVS** | **YSYVGSTTVL** |
| 150 | S.11.4.A6_L | QSVLTQSPSVSGAPGQRVTIS CTGS**SSNIGAGYD**VHWYQQLP GTAPKIIIY**ANT**NRPSGVPDR FSGSKSGTSASLAITGLQAED EADYYC**QSYDSSLSGYVV**FGG GTKLTVL | **SSNIGA GYD** | **ANT** | **QSYDSSLSGYV V** |
| 152 | S.11.4.B5_K | DIQMTQSPSSLSASVGDRVTI TCQAS**QDISNY**LNWYQQKPGK APKLLIY**DAS**HLETGVPSRFS GSGSGTHFTFTISSLQPEDVA TYYC**QQCDNLPPPT**FGQGTKL EIK | **QDISNY** | **DAS** | **QQCDNLPPPT** |
| 154 | S.11.4.C6_K | EIVMTQSPATLSVAPGERATL SCRAS**QSISSN**LAWYQQKPGQ APRLLIY**DAS**TWATDIPARFS GSGSGTEFTLTISSLQSEDSA VYYCQ**QYYDRPRT**FGRGTKVE IK | **QSISSN** | **DAS** | **QQYYDRPRT** |
| 156 | S.11.4.D7_K | DIVMTQSPLSLPVTPGEPASI SCRSS**QSLLHTNGHNY**LDWYL QKPGQSPQLLIY**LGS**IRASGV PDRFSGSGSGTDFTLKISRVE | **QSLLHT NGHNY** | **LGS** | **MKTLQPFT** |

(continued)

| SEQ ID NO | Nome | Sequência aa da cadeia leve | CDR1-L | CDR2-L | CDR3-L |
|---|---|---|---|---|---|
| | | AEDVGVYYCM**KTLQPFT**FGPG TKVDIK | | | |
| 158 | S.11.4.E7_K | EIVLTQSLATLSVSPGERATL SCRAS**QSVSSN**LAWYQQKPGQ APRLLIY**GTS**IRATGIPARFS GSGSGTEFTLTISSLQSQDFV LYYC**QQYNNWPLT**FGGGTKVE IK | **QSVSSN** | **GTS** | **QQYNNWPLT** |
| 160 | S.11.4.G9_K | DIQLTQSPSFLSASVGDRVTI TCRAS**QGISNY**LAWYQQKPGK APKLLIYAASTLQSGVPSRFS GSGSGTEFTLTISSLQPEDCA TYYC**QQLNSHPPA**FGQGTKVE IK | **QGISNY** | **AAS** | **QQLNSHPPA** |
| 162 | S.11.5.D12_K | DIQMTQSPSSLSASVGDRVSI TCRAS**HSIGKS**LNWYQQIPGK APKLLIF**DTS**NLQSGVPSRFS GSGSGTDFTLTINSLQPEDFA TYYC**QQSYSTPPWT**FGQGTKV EIK | **HSIGKS** | **DTS** | **QQSYSTPPWT** |
| 164 | S.11.6.B6_K | EIVLTQSPGTLSLSPGERATL SCRAS**QSVRSGY**FGWYQQKPG QAPRLLIY**NAS**SRATGIPDRF SGSGSGTDFILTISGLGPEDF AVYYC**QQYDSSPWA**FGQGTKV EIK | **QSVRSG Y** | **NAS** | **QQYDSSPWA** |
| 166 | S.11.7.B11_L | SYELTQPPSVSVAPGMTARIT CGGS**NIETKS**VHWYQKKPDQA PVLVAY**DDS**DRPSGIPERFSG SNSGNTATLTISRVEAGDEAD YYC**QVWDSSSDHGV**FGGGTKL TVL | **NIETKS** | **DDS** | **QVWDSSSDHGV** |
| 168 | S.11.7.D12_K | DIVMTQSPLSLPVTPGEPASI SCRSS**QSLLHTNGHNY**LDWYL | **QSLLHT NGHNY** | **LGS** | **MQSLQSYT** |

(continued)

| SEQ ID NO | Nome | Sequência aa da cadeia leve | CDR1-L | CDR2-L | CDR3-L |
|---|---|---|---|---|---|
| | | QKPGQSPQLLIY**LGS**NRASGV PDRFSGSGSGTDFTLKISRVE AEDVGVYYC**MQSLQSYT**FGPG TKVDIK | | | |
| 170 | S.11.7.E6_K | DIQMTQSPSSLSASVGDRVTI TCRAS**QSINNF**LNWYQQKPGK APKLLIY**AAS**SLQSGVPSRFG GSGSGTDFTLTISSLQPDDFA TYYC**QQSYSSRT**FGQGTKLEI K | **QSINNF** | **AAS** | **QQSYSSRT** |
| 172 | S.11.8.D10_K | DIQMTQSPSTLSASVGDRVTI TCRAS**QNISPW**LAWYQQKPGR APKLLIY**KAS**TLESGVPSRFS GSGSGTEFTLTISSLQPDDFA TYYC**QQLQT**FGQGTKVEIK | **QNISPW** | **KAS** | **QQLQT** |
| 174 | S.11.8.E10_K | EIVLTQSPGTLSLSPGERATL SCRAS**QSVRSSY**LGWYQQKPG QAPRLLMY**NTS**SRATGIPDRF SGSGSGTAFTLTITRLEPEDI GVYHC**QQYDRSPWT**FGQGTKV EIK | **QSVRSS Y** | **NTS** | **QQYDRSPWT** |
| 176 | S.11.8.H11_K | AIRMTQSPSSFSASTGDRVTI SCRTS**QDINND**LAWYQQKPGK APNLLIY**GAS**TLQTGVPSRFS GSGSGTEFTLTITYVKSEDFA TYYC**QQYSNYPRT**FGQGTKVE IK | **QDINND** | **GAS** | **QQYSNYPRT** |
| 178 | S.11.8.D6_K | DIVMTQSPDSLAASLGERATI NCKSSQ**SVLYNSNNKNY**LAWY QQKPGQPPKLLIY**WAS**IRESG VPDRFSGSGSGTDFTLTISSL QAEDVAFYYC**QPYLPTPT**FGP GTKVDIK | **QSVLYN SNNKNY** | **WAS** | **QPYLPTPT** |

(continued)

| SEQ ID NO | Nome | Sequência aa da cadeia leve | CDR1-L | CDR2-L | CDR3-L |
|---|---|---|---|---|---|
| 180 | S.11.4.A8_L | QSVLTQPPSASGTPGQRVTIS CSGG**SSNIGRNT**VDWYQQLPG TAPKLLIY**SSN**QRPSGVPDRF SGSKSGTSASLAISGLQSGDE ADYYC**ASWDDTLNGQLV**FGGG TKLTVL | SSNIGR NT | SSN | ASWDDTLNGQL V |
| 182 | S.8.2.B2_K | DIQMTQSPSSLSASVGDRVTI TCRPS**QTINSY**LNWYQQKPGK APKLLIY**AAS**SLQSGVPSRFS GSGSGTDFTLTISSLQPEDFA TYYC**QQTYITPGT**FGQGTKVE IK | QTINSY | AAS | QQTYITPGT |
| 184 | S.9.1.C11_K | EIVLTQSPATLSLSPGERATL SCRAS**QSVSSY**FAWYQQKPGQ SPRLLIY**DAS**NRASGIPARFS GSGSGTDFTLTISSLEPEDFA VYYC**QQRSNWPPRIT**FGQGTR LEIK | QSVSSY | DAS | QQRSNWPPRIT |
| 186 | S.9.1.D10_K | DIQMTQSPSSLSASVGDRVTI TCRAS**QSISRY**LNWYQQKPGK APKLLIY**TAS**SLQSGVPSRFS GSGSGTDFTLTISSLQPEDFA TYYC**QQSHSTPWT**FGQGTKVE IK | QSISRY | TAS | QQSHSTPWT |
| 188 | S.9.2.B2_K | DIQMTQSPSSLSAYVGDRVTI TCRAS**QNISSY**LNWYQQKPGK APKFLIY**AAS**SLQSGVPSRFS GSGSGTNFTLTVSSLQPEDFA TYYC**QQSYSTLWT**FGQGTKVE IK | QNISSY | AAS | QQSYSTLWT |
| 190 | S.9.3.H9_L | QSALTQSRSVSGSPGESVAIS CTGT**SSDVGAYNY**VSWYQQHP GKAPKLMIY**DVN**KRPSGVPDR FSGSKSGNTASLTISGLQAED | SSDVGA YNY | DVN | CSYAGSYNWV |

(continued)

| SEQ ID NO | Nome | Sequência aa da cadeia leve | CDR1-L | CDR2-L | CDR3-L |
|---|---|---|---|---|---|
| | | EADYYC**CSYAGSYNWV**FGGGT KLTVL | | | |
| 192 | S.11.1.E2_L | QSVLTQPPSASGTPGQRVTIS CSGSNS**NIGSRF**VYWYQQLPG TAPTLLIY**RDD**QRPSGVPDRF SGSKSGTSASLAISGVRSEDE ADYYC**AVWDDNLTGWV**FGGGT KLTVL | **NSNIGS RF** | **RDD** | **AVWDDNLTGWV** |
| 194 | S.11.2.C6_K | IVMTQSPLSLSVTPGEPASIS CRSS**QSLLHSNGHTY**LDWYLQ KPGQSPQLLIY**LGS**NRASGVP DRFSGSGSGTDFTLKISRVEA EDVGIYYC**MQAIQSFT**FGPGT KVDIK | **QSLLHS NGHTY** | **LGS** | **MQAIQSFT** |
| 196 | S.11.2.F11_L | QSALTQPASVSGSPGQSITIS CTGT**SSNVGGYNL**VSWYQKHL GKAPKLMIF**DVN**KRPSGVSNR FSGSKSGNTASLTISGLQAED EADYY**CCSYAGRSTSYVV**FGG GTKLTVL | **SSNVGG YNL** | **DVN** | **CSYAGRSTSYV V** |
| 198 | S.11.3.B3_K | DIVMTQSPLFLPVTPGEPASI SCRSS**QSLLHSNGYNY**LDWYL QKPGQSPQLLIY**LGS**NRASGV PDRFSGSGSGTDFTLKISRVE AEDVGVYYC**MEALQAFT**FGPG TKVDIK | **QSLLHS NGYNY** | **LGS** | **MEALQAFT** |
| 200 | S.11.3.G8_K | DIQMTQSPSFLSASVGDRVTI TCRAS**QGISNY**LAWYQQKPGK APKLLIY**AAS**TLQSGVPSRFS GSGSGTEFTLTVSSLQPEDFA TYYC**QQLNSYPKIT**FGPGTKV DIK | **QGISNY** | **AAS** | **QQLNSYPKIT** |
| 202 | S.11.5.D7_L | QSALTQPASVSGSPGQSITIS CTGT**SSDVGSYDL**VSWYQQHP | **SSDVGS YDL** | **EVT** | **CSYAGRSTSYV V** |

(continued)

| SEQ ID NO | Nome | Sequência aa da cadeia leve | CDR1-L | CDR2-L | CDR3-L |
|---|---|---|---|---|---|
| | | GKAPELMIY**EVT**KRPSGVSDR FSGSKSGNTASLTISGLQAED EADYYC**CSYAGRSTSY**VVFGG GTKVTVV | | | |
| 204 | S.11.6.F1_L | SYELTQPPSVSVAPGKTARIT CGGN**NIGSKS**VHWYLQKPGQA PVLVVY**DDS**DRPSGIPERFSG SNSGNTATLTISRVEAGDEAD YYC**QVWDTSSDHGV**FGGGTKL TVL | **NIGSKS** | **DDS** | **QVWDTSSDHGV** |
| 206 | S.11.6.B11_L | QSALTQPASVSGSPGQSITIS CTGT**SSDVGSYNL**VSWYRQYP GKAPSLMIY**QVT**KRPSGVSDR FSGSKSGNTASLTIAGLQVED EGDYYC**CSYAGSDTQLL**FGGG TKLTVL | **SSDVGS YNL** | **QVT** | **CSYAGSDTQLL** |
| 208 | S.11.7.B3_L | SYELTQPPSVSVAPGKPARIT CGGS**YIGSKS**VHWYQQQPGQA PVLVVY**DDS**DRPSGIPERFSG SNSGNTATLTISRVEAGDEAD YYC**QVWDGSSDRQV**VFGGGTK LTVL | **YIGSKS** | **DDS** | QVWDGSSDRQV V |
| 210 | S.11.7.E9_L | QSALTQPASVSGSPGQSITIS CTGT**SSDVGNYNL**VSWYQQHP GKAPKLMIY**EVS**KRPSGVSNR FSGSKSGNTASLTISGLQAED EADYYC**CSYAGSSTSYVL**FGG GTKLTVL | **SSDVGN YNL** | **EVS** | CSYAGSSTSYV L |
| 212 | S.11.7.C11_K | DIQMTQSPSSLSASVGDRVTI TCRAS**QRIQTY**LNWYQQKPGK APKLLIH**SAS**SLQSGVPSRFS GSGSGTDFTLTISSLQPEDFG TYYC**QQSYETPPWT**FGQGTKV EIK | **QRIQTY** | **SAS** | **QQSYETPPWT** |

(continued)

| SEQ ID NO | Nome | Sequência aa da cadeia leve | CDR1-L | CDR2-L | CDR3-L |
|---|---|---|---|---|---|
| 214 | S.11.8.F10_K | DIQMTQSPSSLSASVGDRVTI TCRAS**QSISTY**LNWYQQKPGK APKLLIY**AAS**SLQSGVPSRFS GSGSGTDFTLTISSLQPEDFA TYYC**QQSYSTPPFT**FGPGTKV DIK | **QSISTY** | **AAS** | **QQSYSTPPFT** |
| 216 | S.12.1.A4_K | DIQMTQSPSSLSASVGDRVTI TCQAR**QDISNY**LNWYQQKPGK APKLLIY**DAS**NLETGVPSRFS GRGSGTHFTFTISSLQPEDIA TYYC**QQYDNLPLT**FGGGTKVE IK | **QDISNY** | **DAS** | **QQYDNLPLT** |
| 218 | S.12.1.B7_K | DIVMTQSPLSLPVTPGEPASI SCRSS**QSLLHSNGYNY**LDWYL QKPGQSPQLLIY**LGS**NRASGV PDRFSGSGSGTDFTLKISRVE AEDVGVYYC**MQALQTPP**TFGQ GTKVEIK | **QSLLHS NGYNY** | **LGS** | **MQALQTPPT** |
| 220 | S.12.1.C10_K | DIVMTQSPDSLAVSLGERATI NCKSS**QTILHNSHNRNC**LAWF QQKPGQPPKLLIY**WAS**TRESG VPDRFSGSGSGTDFTLTINNL QAEDVAVYYC**QQYYTNPRWT**F GQGTKVEIK | **QTILHN SHNRNC** | **WAS** | **QQYYTNPRWT** |
| 222 | S.12.2.A3_L | QSVLTQPPSASGTPGQRVTVS CSGS**SSNIGSNT**VNWYQQIPG TAPKLLMY**GNN**QGASGVPDRF SGAKSGTSASLAISGLQSEDE ADYYC**EAWDDSLNGRV**FGTGT KVTVL | **SSNIGS NT** | **GNN** | **EAWDDSLNGRV** |
| 224 | S.12.2.C5_K | DIQMTQSPSTLSASVGDRVTI TCRAS**QSISSW**LAWYQQKPGK APKLLIY**KAS**SLQSGVPSRFS GSGSGTEFTLTISSLQPDDFA | **QSISSW** | **KAS** | **QHYNGYSKT** |

(continued)

| SEQ ID NO | Nome | Sequência aa da cadeia leve | CDR1-L | CDR2-L | CDR3-L |
|---|---|---|---|---|---|
| | | TYYC**QHYNGYSKT**FGQGTKVE IK | | | |
| 226 | S.12.2.G11_K | EIVLTQSPGTLSLSPGERATL SCRAS**QSVSSSF**LVWYQQKPG QAPRLLMY**GAS**TRATGIPDRF SGSGSGTDFTLTISRLEPEDF AVYYC**QHYGNSPPRT**FGQGTK VEIK | **QSVSSS F** | **GAS** | **QHYGNSPPRT** |
| 228 | S.12.3.H1_K | EIVLTQSPGTLSLPPGERATL SCRAS**QSVSSSF**LAWYQQKPG QAPRLIMY**GAS**TRATGIPDRF SGSGSGTDFTLTISRLEPEDF AVYYC**RHYGNSPPRT**FGQGTK VEIK | **QSVSSS F** | **GAS** | **RHYGNSPPRT** |
| 230 | S.12.1.B12_K | IQMTQSPSSVSASVGDRVTIT CRAS**QDISSW**LAWYQQKPGRA PKLLIY**AAS**SLQSGVPSRFSG SGSGTDFTLTISSLQPEDFAT YYC**QQANNFPLT**FGGGTKVEI K | **QDISSW** | **AAS** | **QQANNFPLT** |
| 232 | S.12.4.A8_K | EIVLTQSPGTLSLSPGERATL SCRAS**QSISASY**LAWYQQKPG QAPRLLMY**GAS**SRATGIPDRF SGSGSGTDFTLTISRLEPEDF AVYYC**QQYGSSPPRT**FGQGTK VEIK | **QSISAS Y** | **GAS** | **QQYGSSPPRT** |
| 234 | S.11.1.F6_K | EIVLTQSPGTLALSPGESGTL SCRAN**ESLNSNY**VAWLQQKPG QAPRLLIY**ATS**TRATGIPVRF SGSGSGTDFTLTVSSLEPEDF AVYYC**QQYDRPPVT**FGAGTKV EIK | **ESLNSN Y** | **ATS** | **QQYDRPPVT** |
| 236 | S.11.3.E5_K | DIQMTQSPSSLSASVGDRVSI TCRAS**QNISSY**LNWYQQKPGK | **QNISSY** | **GAF** | **QQTYGTPRT** |

# EP 4 545 559 A2

(continued)

| SEQ ID NO | Nome | Sequência aa da cadeia leve | CDR1-L | CDR2-L | CDR3-L |
|---|---|---|---|---|---|
|  |  | APELLIY**GAF**SLESGVPSRFS GSGSGTDFTLTITSLQPEDFA TYSC**QQTYGTPRT**FGQGTKVE IK |  |  |  |
| 238 | S.12.2.G6_K | IQMTQSPSSVSASVGDRVTIT CRAS**QDISSW**LAWYQQKPGRA PKLLIY**AAS**SLQSGVPSRFSG SGSGTDFTLTISSLQPEDFAT YYC**QQANNFPLT**FGGGTKVEI K | **QDISSW** | **AAS** | **QQANNFPLT** |
| 240 | S.11.4.E4_K | IQMTQSPSSLSASVGDRVTIS CRAS**QGVGDD**LAWYQQKPGRA PKRLIY**SAS**SLQSGVPSRFSG SGYGTEFTLTITSLQPEDFAT YYC**LQYNSYPLT**FGGGTKVEI K | **QGVGDD** | **SAS** | **LQYNSYPLT** |
| 242 | S.11.5.C2_K | DIQLTQSPSFLSAFVGDRVTI TCRAS**QGISSD**LAWYQQQPGK APKLLIY**AAS**TLQSGVPSRFS GSGSGTEFTLTISSLQPEDFA TYYC**QHLNSNPGIFT**FGPGTK VDIK | **QGISSD** | **AAS** | **QHLNSNPGIFT** |
| 244 | S.11.8.G10_L | QSALTQPASVSGSPGQSITIS CTGT**SSDIGSYNL**VSWYRQYP GEAPSLVIY**QVS**KRPSGVSDR FSGSKSGNTASLTIAGLQAED EADYYC**CSYAGSDTQLF**FGGG TRLTVL | **SSDIGS YNL** | **QVS** | **CSYAGSDTQLF** |
| 246 | S.12.1.A5_K | DIVMTQSPLSLPVTPGEPASI SCRSS**QSLLHSNGYTY**LDWYL QKPGQSPQLLIY**LGS**YRASGV PDRFSGSGSGTDFTLKIGRVE AEDVGIYYC**MQGLQTPY**SFGQ GTKLEIK | **QSLLHS NGYTY** | **LGS** | **MQGLQTPYS** |

70

(continued)

| SEQ ID NO | Nome | Sequência aa da cadeia leve | CDR1-L | CDR2-L | CDR3-L |
|---|---|---|---|---|---|
| 248 | S.12.1.E10_K | DIVMTQSPLSLPVTPGEPASI SCRSS**QSLLHNNGYTY**LDWYL QKPGQSPQLLIY**LGS**YRASGV PDRFSGSGSGTDFTLKIGRVE AEDVGIYYC**MQGLQTPY**SFGQ GTKLEIK | **QSLLHN NGYTY** | **LGS** | **MQGLQTPYS** |
| 250 | S.12.3.E12_K | DIVMTQSPDSLAVSLGERAAI TCKSS**QSVLYSSSNRNY**LAWY QQKPGQSPKLLIY**WAS**TRASG VPDRFSGSGSETDFTLTISSL QTEDVAVYYC**QQYYNTPLT**FG GGTKVEIK | **QSVLYS SSNRNY** | **WAS** | **QQYYNTPLT** |
| 252 | S.11.1.B8_L | QSVLTQPPSASGTPGQRVTVS CSGR**GSNIGNNF**VYWYQQLPG TAPKLLIY**RND**QRPSGAPDRF SGSKSGTSASLAISGLRSEDE ADYYC**AAWDDSLRGW**VFGGGT KLTVL | **GSNIGN NF** | **RND** | **AAWDDSLRGWV** |
| 254 | S.11.1.E9_L | QSALTQPASVSGSPGQSITIS CTGS**SSDVGSYNL**VSWYQQHP GKAPKLMIY**EVS**KRPSGVSNR FSGSKSGNTASLTISGLQAED EADYYC**CSYAGSNTYVA**FGGG TRLTVLGQP | **SSDVGS YNL** | **EVS** | **CSYAGSNTYVA** |
| 256 | S.11.2.E3_L | QSALTQPASVSGSPGQSLTIS CTGT**SSDIGAYNY**VSWYQQYP GKAPKLIIY**DAD**KRPSGASDR FSGSKSGNTASLTISGLQAED EADYYC**NSYTTANTGV**FGGGT KLTVL | **SSDIGA YNY** | **DAD** | **NSYTTANTGV** |
| 258 | S.11.2.H4_L | QSALTQPPSASGSPGQSVTIS CTGT**SSDIGAYNY**VSWYQQHP GKAPKFLIY**GVT**QRPSGVPDR FSGSKSGNTASLTVSGLQAED | **SSDIGA YNY** | **GVT** | **SSYAGNKVI** |

71

(continued)

| SEQ ID NO | Nome | Sequência aa da cadeia leve | CDR1-L | CDR2-L | CDR3-L |
|---|---|---|---|---|---|
| | | EADYYC**SSYAGNKVI**FGGGTK LTVL | | | |
| 260 | S.11.3.A2_K | DIVMTQSPDSLAVSLGERATI NCKSS**QSVLYSSNNKNY**LAWY QQKPGQPPKLLIY**WAS**TRESG VPDRFSGSGSGTDFTLTISSL QAEDVAVYY**CHQYHTPPLT**FA GGTKVEIK | QSVLYS SNNKNY | WAS | HQYHTPPLT |
| 262 | S.11.3.E11_K | DIQMTQSPSSLSASVGDRVTI TCRTS**QRISSF**LNWYQQRPGK APKLLIY**GAS**TLQSGVPSRFS GSGSATDFTLTISSLQPEDFA TYYC**QRSYGTPPALT**FGGGTK VEIK | QRISSF | GAS | QRSYGTPPALT |
| 264 | S.11.3.F2_L | NFMLTQPHSVSESPGKTVTIT CTGS**SGSVANNY**VQWYQQRPG SAPTLVIF**EDS**HRPSGVPDRF SASVDISSNSASLTISGLKTE DEADYYC**QSLDSSVHVV**FGGG TRLTVL | SGSVAN NY | EDS | QSLDSSVHVV |
| 266 | 5.11.3.F9_K | DIVMTQSPVSLPVTPGEPATI SCRSS**QSLLDSNGHNY**LDWYL QKPGQSPQLLIY**LGS**TRASGV PDRFSGSGSGTDFTLKISRVE AEDVGVYYC**MQALQTFT**FGPG TKVDIK | QSLLDS NGHNY | LGS | MQALQTFT |
| 268 | S.11.4.D10_L | QSVLTQPPSASGTPGQRVTIX CSGS**SSNIGRNA**VDWYQQVPG TAPRLLIY**SNY**QRPSGVPARF SGSKSGTSASLAISGLXSEDE ADYYC**AAWDDSLDGQVV**FGGG TKLTVL | SSNIGR NA | SNY | AAWDDSLDGQV V = |
| 270 | S.11.4.F10_K | DIQLTQSPSFLSASVGDRVTI TCRAS**QGISHY**LAWYQQKPGK | QGISHY | SAS | QRLNSSPSIFT |

(continued)

| SEQ ID NO | Nome | Sequência aa da cadeia leve | CDR1-L | CDR2-L | CDR3-L |
|---|---|---|---|---|---|
| | | APKLLIY**SAS**TLQSGVPSRFS GSGSGTEFTLTISSLQPEDFA TYYC**QRLNSSPSIFT**FGPGTK VDIK | | | |
| 272 | S.11.4.G6_K | DIVMTQSPDSLAVSLGERATI NCKSS**QSVLFSSTNKNN**LAWY QQKPGQPPTLLIY**WAS**ARQSG VPDRFSGSGSGTDFTLTITGL QAEDVAVYYC**QQYHSPPLT**FG GGTKVEIK | **QSVLFS STNKNN** | **WAS** | **QQYHSPPLT** |
| 274 | S.11.6.H11_K | EIVLTQSPGTLSLSPGETGTL SCRAS**QSLSSNY**VAWLQQKPG QAPRLLIY**ATS**TRATGIPVRF SGSGSGTDFTLTVSRLEPEDF AVYYC**QQYASSPVT**FGAGTKV EIK | **QSLSSN Y** | **ATS** | **QQYASSPVT** |
| 276 | S.11.7.A12_L | QSVLTQPPSASGTPGQRVTIS CSGS**SSNIGPNA**VDWYQQLPG TAPKLLIY**SNN**QRPSGVPARF SGSKSGTSASLAISGLQSEDE ADYYC**AAWDDSLGGQVV**FGGG TKLTVL | **SSNIGP NA** | **SNN** | **AAWDDSLGGQV V** |
| 278 | 11.7.A8_L | QSALTQPPSASGSPGQSVTIS CTGT**SSDVGGYKY**VSWYQQRP GRAPKLIIS**DVT**ERPSGVPDR FSGSKSGNTASLTVSGLQAED EADYYC**SSYAGSNNLV**FGGGT KLTVLGQPKAAPSVTL | **SSDVGG YKY** | **DVT** | **SSYAGSNNLV** |
| 280 | S.11.7.G6_K | IQMTQSPSSLSASEGDRVTIT CRAS**QDIGSD**LGWYRQKPGKA PKRLIY**AAS**SLQSGVPSRFSG SGSGTEFTLTISSLQPEDFAT YYC**LQHNSYPLT**FGGGTKVEI K | **QDIGSD** | **AAS** | **LQHNSYPLT** |

# EP 4 545 559 A2

(continued)

| SEQ ID NO | Nome | Sequência aa da cadeia leve | CDR1-L | CDR2-L | CDR3-L |
|---|---|---|---|---|---|
| 282 | S.11.8.B12_K | DIVMTQSPLSLPVIPGEPASI SCRSS**QSLLGSNGHSY**LDWYL QKPGQSPQLLIY**LGS**NRASGV PDRFSGSGSGTDFTLKISRVE AEDVGVYYC**MQALESFT**FGPG TKVDIK | **QSLLGS NGHSY** | **LGS** | **MQALESFT** |
| 284 | S.11.8.B2_L | SYELTQPPSVSVAPGKTASIT CGGN**NIGSKS**VHWYQRKPGQA PVLVVY**DDS**DRPSGIPERFSG SKSGNTATLTISRVDAGDEAD YYC**QVWDSSSDPVV**FGGGTKL TVLGQPKAAPSVTL | **NIGSKS** | **DDS** | **QVWDSSSDPVV** |
| 286 | S.11.8.C3_K | DIQLTQSPSFLSASVGDRVTI TCRAS**QGISSY**LAWYQQKPGR APKLLIY**AAS**TLQSGVPSRFS GSGSGTEFTLTISSLQPEDFA TYYC**QQLNSYPRGT**FGGGTKV EIK | **QGISSY** | **AAS** | **QQLNSYPRGT** |
| 288 | S.11.8.D2_L | QSALTQPASVSGSPGQSITIS CTGT**SSDVGSYNL**VSWYQQHP GKAPKLMIF**QVA**KRPSGVSQR FSGSKSGNTASLTISGLQAED EADYYCCSYA**GSDTYLL**FGGG TKLTVL | **SSDVGS YNL** | **QVA** | **CSYAGSDTYLL** |
| 290 | 11.8.D9_K | DIQMTQSPSSLSASVGDRVTI TCQASQ**DIDIY**LNWYQQKPRK APKLLIF**DAS**NLETGVPSRFS GSGSGTHFTFTISSLQPEDIA TYYC**QQYDNLPLT**FGGGTK* | **QDIDIY** | **DAS** | **QQYDNLPLT** |
| 292 | S.11.8.G11_K | DIQMTQSPSSLSASVGDRVTI TCRAS**QSINNY**LNWYQHKPGK APKLLIY**AAS**SLHSGVPSRFS GSGSGTDFTLTISSLQPEDFA | **QSINNY** | **AAS** | **QQSYSTPA** |

74

(continued)

| SEQ ID NO | Nome | Sequência aa da cadeia leve | CDR1-L | CDR2-L | CDR3-L |
|---|---|---|---|---|---|
| | | TYYC**QQSYSTPA**FGQGTKVEIK | | | |
| 294 | S.11.8.G8_L | QSALTQPASVSGSPGQSITISCTGT**STDVGGYDY**VSWYQQHPGKAPKLIIY**DVT**KRPSGVSDRFSGSKSGNTASLTISGLQADDEAHFYC**TSYTATTVAL**FGGGTKLTVL | **STDVGGYDY** | **DVT** | **TSYTATTVAL** |
| 296 | S.3.1. A6_K | DIQMTQSPSSLSASVGDRVTITCRAS**QSISSY**LNWYQQKPGKAPKLLIY**AAS**SLQSGVPSRFSGSGSGTDFTLTISSLQPEDFATYYC**QQSYSYT**FGQGTKLEIK | **QSISSY** | **AAS** | **QQSYSYT** |
| 298 | S.3.2.F6_K | LSASVGDRVTITCRAS**QSISSW**LAWYQQKPGKAPKLLIY**KAS**SLESGVPSRFSGSGSGTEFTLTISSLQPDDFATYYC**QQYNSXSRT**FGXGTXLEIX | **QSISSW** | **KAS** | **QQYNSXSRT** |
| 300 | S.3.3.A4_K | DIVMTQSPLSLPVTPGEPASISCRSS**QSLLNNDGNIY**LDWYLQKPGQSPQLLIY**LAS**IRASGVPDRFSGSGSGTDFTLKISRVEAEDVGLYYC**MQGQYAPT**FGQGTRLEIK | **QSLLNNDGNIY** | **LAS** | **MQGQYAPT** |
| 302 | S.3.3.A4_K | DIVMTQSPLSLPVTPGEPASISCRSS**QSLLNNDGNIY**LDWYLQKPGQSPQLLIY**LAS**IRASGVPDRFSGSGSGTDFTLKISRVEAEDVGLYYC**MQGQYAPT**FGQGTRLEIK | **QSLLNNDGNIY** | **LAS** | **MQGQYAPT** |
| 304 | S.3.3.G8_K | EIVLTQSPGTLSLSPGERATLSCTAS**QSVSSS**YLAWYQQKPGQAPRLLTY**GAS**SRATAIPHRFSGGGSGTDFTLTISRLEPEDF | **QSVSSSY** | **GAS** | **QQYGSAPHT** |

(continued)

| SEQ ID NO | Nome | Sequência aa da cadeia leve | CDR1-L | CDR2-L | CDR3-L |
|---|---|---|---|---|---|
|  |  | AVYYC**QQYGSAPHT**FGQGTKV EIK |  |  |  |
| 306 | S.5.1.A5_K | EIVLTQSPATLSLSPGERATL SCRAS**QSVSSY**LAWYQQKPGQ APRLLIY**DAS**NRATGIPTRFS GSGSGTDFTLTISNLEPGDFA VYYC**QQRSDWPPYT**FGQGTKL EIK | **QSVSSY** | **DAS** | **QQRSDWPPYT** |
| 308 | S.5.1.B8_L | QSVLTQPPSASGTPGQRVTIS CSGS**SSNIGSNY**VYWYQQLPG TAPQLLIY**TNN**QRPSGIPDRF SGSKSGTSASLAISGLRSEDE ADYYC**ATWDDSLSGW**VFGGGT KLTVL | **SSNIGS NY** | **TNN** | **ATWDDSLSGWV** |
| 310 | S.5.1.A11_K | MTQSPASLSASVGDRVTITCR AS**QGIRND**LGWYQQKPGKAPK RLIYA**ASS**LQSGVPSRFSGSG SGTEFTLTISSLQPEDFATYY C**LQHNSYPST**WTFGQGTKVEI K | **QGIRND** | **ASS** | **LQHNSYPSTWT** |
| 312 | S.6.1.H8_K | DIVMTQSPDSLTVPLGERATI SCKSS**QSLFYRSNSKNY**LAWY QQKAGQPPTPLIY**WAS**TRESG VPDRFSGSGSGTDFTLTISSL QAEDVAVYYC**QQYYRTPLT**FG GGTKVEIK | **QSLFYR SNSKNY** | **WAS** | **QQYYRTPLT** |
| 314 | S.7.11.B7_K | DIQMTQSPSTLSASVGDRVTI TCRAS**QSISSW**LAWYQQKPGK APKLLIY**KAS**NLQDGVPSRFS GSGSGTEFTLTISSLQPDDFA TYYC**QQCNTYPWT**FGQGTKVE IK | **QSISSW** | **KAS** | **QQCNTYPWT** |
| 316 | S. 8.2. B6 K | DIQMTQSPSTLSASEGDRVTI TCRAS**QSISSW**LAWYQQKPGK | **QSISSW** | **KAS** | **QQYHSYSLT** |

(continued)

| SEQ ID NO | Nome | Sequência aa da cadeia leve | CDR1-L | CDR2-L | CDR3-L |
|---|---|---|---|---|---|
| | | APKLLIY**KAS**SLESGVPSRFS GSGSGTEFTLTISSLQPDDFA TYYC**QQYHSYSLT**FGGGTKVE IK | | | |
| 318 | S.8.2.E3_L | QSVLXQPPSVSAAPGQKVTIS CSGG**SSNIGN**NYVSWFQHLPG TAPKLLIY**DSN**KRPSGIPDRF SGSKSGTSATLGITGLQTGDE ADYYC**GTWDSSLSAYV**FGTGT KVT | SSNIGN NY | DSN | GTWDSSLSAYV |
| 320 | S.8.2.F3_L | QSVLTQPPSASGTPGQRVTIS CSGS**SSNIGS**NTVNWYQQLPG TAPKLLIY**SNN**QRPSGVPDRF SGSTSGTSASLAISGLQSEDE ADYYC**ATWDDSLNGYVV**FGGG TKLTV | SSNIGS NT | SNN | ATWDDSLNGYV V |
| 322 | S.9.1.B2_K | DIQMTQSPSSLSTSVGDRVTI TCQAS**QDIRNY**LNWYQQKPGK APKLLIY**DAS**NLEIGVPSRFS GSGSGTDFTFTISSLQPEDIA TYYC**QQYANLPLT**FGGGTKVE IK | QDIRNY | DAS | QQYANLPLT |
| 324 | S.9.1.C7_L | QSALTQPRSVSGSPGQSVTIS CTGT**SSDVGGYNY**VSWYQHHP GKAPKLMIY**DVS**KRPSGVPDR FSGSKSGNTASLTISGLQAED EADYYC**CSYAGRYTYV**FGTGT KVTVL | SSDVGG YNY | DVS | CSYAGRYTYV |
| 326 | S.9.1.D5_K | EIVMTQSPATLSVSPGERATL SCRAS**QSVSTN**LAWYQQKPGQ APRLLIY**GAS**TRATSIPARFS GSGSGTEFTLTISSLQSGDFA VYYC**QQYNNWPRT**FGQGTKVE IK | QSVSTN | GAS | QQYNNWPRT |

(continued)

| SEQ ID NO | Nome | Sequência aa da cadeia leve | CDR1-L | CDR2-L | CDR3-L |
|---|---|---|---|---|---|
| 328 | S.9.1.H9_K | IQMTQSPSSLSASVGDRVTIT CRAS**QDIRND**LGWYQQKPGKA PKRLIY**AAS**SLQSGVPSRFSG SGSGTEFTLTITSLQPEDFAT YSC**LQHNSYPLT**FGGGTKVEI K | **QDIRND** | **ASS** | **LQHNSYPLT** |
| 330 | S.9.2.E3_K | MTQSPATLSVSPEQRATLSCR AS**QSVSSN**LAWYQQKPGRAPR LLIY**GTS**TRATGIPARFSGSG SGTEFTLTISSLQSEDFAVYY C**QQYNNWPLT**FGGGTKVEIK | **QSVSSN** | **GTS** | **QQYNNWPLT** |
| 332 | S.9.2.F3_L | QSVLTQPPSVSAAPGQKVTIS CSGS**SSNIGNNY**VSWFQQLPG TAPKLLIY**DNN**KRPSGIPDRF SGSKSGTSATLGITGLQTGDE ADYYC**GTWDSSLSAVV**FGGGT KLTV | **SSNIGN NY** | **DNN** | **GTWDSSLSAVV** |
| 334 | S.9.2.G1_L | QSVLTQPPSVSAAPGQKVTIS CSGS**SSNIGKNY**VSWYQRLPG TAPKLLIY**DNN**KRPSGIPDRF SGSKSGTSATLGITGLQPGDE ADYYC**GTWDRSLSAWV**FGGGT KLTV | **SSNIGK NY** | **DNN** | **GTWDRSLSAWV** |
| 336 | S.9.3.D5_K | EIVMTQSPATLSVSPGERATL SCRAS**QSVSNN**LAWYQQKPGQ APRLLIY**GAS**TRATGFPARFS GSGSGTEFTLTISSLQSEDFA VYYC**QQYYNWPPWT**FGQGTKV EIK | **QSVSNN** | **GAS** | **QQYYNWPPWT** |
| 338 | S.9.3.F7_K | DIVMTQSPLSLPVTPGEPASI SCRSS**QSLLHSNGYNY**LDWYL QKPGQSPQLLIY**LGS**NRASGV PDRFSGSGSGTDFTLEISRVE | **QSLLHS NGYNY** | **LGS** | **MQALQTPFT** |

(continued)

| SEQ ID NO | Nome | Sequência aa da cadeia leve | CDR1-L | CDR2-L | CDR3-L |
|---|---|---|---|---|---|
| | | AEDVGVYYC**MQALQTPFT**FGQ GTRLEIK | | | |
| 340 | S.9.3.G1_K | EIVLTQSPGTLSLSPGERATL SCRAS**QSFSRSY**LAWYHQKPG QAPRLLIY**GAS**SRATGIPDRF SGSGSGTDFTLTISRLEPEDF AVYYC**QHYGSSPPIT**FGQGTR LEIK | **QSFSRS Y** | **GAS** | **QHYGSSPPIT** |
| 342 | S.9.3.H10_L | QSVLTQPPSASATPGQXVTIS CSGS**SSNIGSNF**VYWYQQLPG TAPKLLIY**SND**QRPSGVPDRF SGSKSGTSASLAISGLRSEDE ADYYC**AAWDDSLRGLWV**FGGG TKLTVL | **SSNIGS NF** | **SND** | **AAWDDSLRGLW V** |
| 344 | S.9.4.B6_L | QSALTQPASVSGSPGQSITIS CTGT**SSDVGSYNV**VSWYQQHP GKAPKVMIY**EAS**KRPSGVSNR FSGSKSGNTASLTISGLQAED EADYYC**CSYAGSSTWV**FGGGT KLTVL | **SSDVGS YNV** | **EAS** | **CSYAGSSTWV** |
| 346 | S.9.4.B8_L | QXALTQPPSASGSRGQSVTIS CTGT**SSDVGGYNY**VSWYQQHP GKAPKLMIY**EVS**KRPSGVPDR FFGSKSGNTASLTVSGLQAED EADYYC**SSYGGSNNYV**FGTGT KVTVL | **SSDVGG YNY** | **EVS** | **SSYGGSNNYV** |
| 348 | S.9.4.C4_K | DXVMTQSPLSPPVTPGEPASI SXRSS**QSLLHSXGYNY**LDWYL QKPGQSPQLLIY**LGS**NRASGV PDRFSGSGSGTDFTLKISRVE AEDVGVYYC**MQALQTPFT**FGP GTKVDIK | **QSLLHS XGYNY** | **LGS** | **MQALQTPFT** |
| 350 | S.9.4.E1_L | SYELTQAPSVSVSPGQTASIT CSGD**KXGDKY**ACWYQQKPGQS | **KXGDKY** | **QDS** | **QAWDSRTVV** |

(continued)

| SEQ ID NO | Nome | Sequência aa da cadeia leve | CDR1-L | CDR2-L | CDR3-L |
|---|---|---|---|---|---|
| | | PVLVIY**QDS**KRPSGIPERFSG SNSGNTATLTISGTQAMDEAD YYC**QAWDSRTVV**FGGGTKLTV L | | | |
| 352 | S.9.5.B2_L | QSALTQPASVSGSPGQSITIS CTGT**SSDVGSYNL**VSWYQQHP DKAPKLMIY**EVN**KRPSGVSNR FSGSKSGNTASLTISGLRAED EADYYC**CSYAGSSTFVV**LGGG TKLTVL | **SSDVGS YNL** | **EVN** | **CSYAGSSTFVV** |
| 354 | S.9.5.B8_K | DIQMTQSPSSLSASVGDRVTI TCRAS**QVISNS**LAWYQQKPGK APKFLIY**AAS**TLESGVPSRFS GSGSGTDFTLTISSLQPEDFA TYYC**QQYYDAPRT**FGQGTKVE IK | **QVISNS** | **AAS** | **QQYYDAPRT** |
| 356 | S.9.5.C6_K | EIVLTQSPATLSLSPGERATL SCRAS**QNISSF**LGWYQQKPGQ APRLLIY**DAS**NRATGIPARFS GSGSGTDFTLTISSLETEDFA IYYC**QQRSKWPPEGT**FGQGTK LEIK | **QNISSF** | **DAS** | **QQRSKWPPEGT** |
| 358 | S.11.1.E10_L | SYELTQPPPVSVAPGKTARIT CGGD**NIGGKS**VHWYQQKPGLA PVMVVY**DDS**ARPSGIPERFSG SNSGNTATLTITRAEAGDEAD YYC**QVWDTGSDPLV**FGTGTKV TVL | **NIGGKS** | **DDS** | **QVWDTGSDPLV** |
| 360 | S.11.7.A5_L | SYELTQPPSVSVAPGKTARIT CGGN**NIGSKS**VNWYQQRPGQA PVLVVY**DDS**DRPSGIPERFSG SNSGNTATLTISRVEAGDEAD YYC**QVWDTGSDPLV**FGTGTKV TVL | **NIGSKS** | **DDS** | **QVWDTGSDPLV** |

Pre-clinical study:

Among the antiSARS-CoV-2 mAbs, 3 were selected for in vivo preclinical testing. The 3 mAbs were combined into 2 groups of 2 mAbs each, with 1 mAb being common to both compositions (mAbs #1 and mAbs #2). mAbs composition #3 was not used in the preclinical trial.

    mAbs #1 (S.9.2.B8 + S.9.4.C7)
    mAbs #2 (5.9.2.D7 + S.9.4.C7)
    mAbs #3 (s.9.2.B8 + S.9.2.D7)

The two compositions mAbs #1 and mAbs #2 were submitted to proof of concept in assay of golden hamsters infected with the virus SARS-CoV-2 WU (B.1.1.28), according to the standard protocol. Figure 2 shows the weight of the animals over the 7 days for the different groups analyzed, detailed below. The animals were transferred from the vivarium at the Butantan Institute to the NB3 laboratory at the ICB/USP, where they were acclimatized for 1 week. The 7th day was considered day 0 (zero) when the animals were weighed and randomized into 100mgs each.

The animals in the infected groups were inoculated with virus intranasally on day zero. On the following day, animals from groups G4 and G5 were injected intraperitoneally with mAbs #1 and #2, respectively. Groups G1 and G2 were also injected intraperitoneally with mAbs #1 and #2, respectively, for experiment control and adverse effect verification (scheme below):

```
                         ┌ G1 (n = 6) - received mAbs #1
Not infected (control)  ┤  G2 (n = 6) - received mAbs #2
                         └ G3 (n = 6) - received only PBS (buffered saline)


                         ┌ G4 (n = 18) - received mAbs #1
       Infected (test)  ┤  G5 (n = 18) - received mAbs #2
                         └ G6 (n = 18) - received only PBS
```

A very important piece of data in the experiment is the weight variation of the animals, which lose weight due to SARS-CoV-2 infection. Figure 2 shows the average weight evolution of each animal, each day. The animals in the control group (G1 to G3) were euthanized for collection of blood, nasal turbinates, trachea, lung, liver and kidney on the 8th post-infection day. The animals in the infected groups (G4 to G6) were euthanized in subgroups of 6 animals each on days 3, 5 and 7 post infection for collection of the same samples.

In Figure 2, each animal was weighed daily until the day of euthanasia with the removal of blood and organ samples.

By weight, it is possible to conclude that the mAbs did not show toxicity (groups G1-G2 compared to the control group G3) and the groups that received treatment with the mAbs were able to recover the weight loss caused by the SARS-CoV-2 infection (groups G4-G5 compared to the untreated group G6). Pathophysiological analyzes are ongoing, as well as viral load analyzes in the collected organs.

The serum derived from the blood of each animal, collected on days 3, 5 and 7 (infected animals, G4 to G6) and day 8 (control animals < G1 to G3) was tested by ELISA to determine the concentration of circulating human antibody in the animal. Figure 3 presents the results obtained in the ELISA test, showing favorable pharmacokinetics for therapeutic antibodies (antibodies present in the blood circulation throughout the treatment period) .

Figure 3 shows an ELISA graph evaluating the concentration (in $\mu$g/mL) of human antibody in the serum of each of the euthanized hamsters, according to the group to which they belonged. The columns in pink represent the mAb combination #1 (G4 at 3, 5 and 7 days post infection and G1 (uninfected) at 8 days. The columns in green represent the mAb combination #2 (G5 at 3, 5 and 7 days post infection and G2 (uninfected) at 8 days. The G6 and G3 groups were infected and did not receive antibodies. The results were evaluated by the Mann-Whitney test with statistical significance $p \leq 0.05$ (*) and $p \leq 0.01$ (**).

Sequences of pairs of anti-SARS-CoV-2 monoclonal antibodies, with identification of the CDRs, binding (in RU) to recombinant SARS-CoV-2 proteins, title of the neutralization of the live virus in VERO cells (VNT), affinity kinetics (KD)

**Proteins used for binding and affinity kinetics assays**

RBD WT (original Wuhan)
RBD E484K single mutation
RBD N501Y single mutation
RBD Beta K417N/E484K/N501Y
RBD Gamma (K417T/E484K/N501Y)
RBD Delta (L452R/T478K)
RBD Delta plus (K417N/L452R/ T478K)
RBD Omicron (G339D, S371L, S373P, S375F, K417N, N440K, G446S, S477N, T478K, E484A, Q493R, G496S, Q498R, N501Y, Y505H) (Arg319-Phe541)
Spike S1+S2 Alpha (HV69-70 deletion, Y144 deletion, N501Y, A570D, D614G,P681H, T716I, S982A, D1118H)
Spike S1+S2 Gamma (L18F, T20N, P26S, D138Y, R190S, K417T, E484K, N501Y, D614G, H655Y, T1027I, V1176F)
Spike S1+S2 Delta (T19R, E156G, 157-158 deletion, L452R, T478K, D614G, F817P, A892P, A899P, A942P, D950N, K986P, V987P)
Spike S1+S2 trimer Protein Omicron (A67V, Δ69-70, T95I, G142D/Δ143-145, Δ211/L212I, ins214EPE, G339D, S371L, S373P, S375F, K417N, N440K, G446S, S477N, T478K, E484A, Q493R, G496S, Q498R, N501Y, Y505H, T547K, D614G, H655Y, N679K, P681H, N764K, D796Y, N856K, Q954H, N969K, L981F)
N WT protein (original Wuhan)
Mutated N protein (D3L/R203K/G204R/S235F)

Monoclonal antibody S.8.1.A4

Heavy chain amino acid sequence - SEQ ID NO: 1 (with underlined CDRs):

EVQLVESGGGLVQPGGSLRLSCAAS<u>GIIVSNNYMS</u>WVRQAPGKGLEWVS<u>IIYSGGSTFY</u>
ADSVKGRFTISRDNSKNTLYLQMNSLRVEDTAVYYC<u>ARDLGGLRLDY</u>WGQGTLVTVSS

K light chain amino acid sequence - SEQ ID NO: 2 (with underlined CDRs):

DIQMTQSPSSLSASVGDRVTITCQAS<u>QDINIC</u>LNWYQQKPEKAPKLLIY<u>DASN</u>LETGVP
SRFSGSGSGTDFTFTISSLQPEDIATYYC<u>QQYDNLPQT</u>FGQGTKVEIK

Table 4 - Binding of mAb S.8.1.A4 (expressed in CHO cells and purified) to the RBD, Spike and N proteins of the SARS-CoV-2 virus by Surface Plasm Resonance (SPR), with units in RU (resonance units), neutralization of SARS-CoV-2 by VNT100 (title of mAb S.8.1.A4 dilution):

| S.8.1.A4 | | | RU | VNT100 |
|---|---|---|---|---|
| RBD | | WT | 204 | 7680 |
| | | E484K | 118 | |
| | | N501Y | 162 | |
| | | BETA | 52 | |
| | | GAMMA | 30 | 20 |
| | | DELTA | 278 | 2560 |
| | | OMICRON | 11 | 10 |
| SPIKE | | WT | 11 | |
| | | MUTATED | 6 | |
| | | GAMMA | 0 | |
| | | DELTA | 86 | |
| | | OMICRON | 5 | |

(continued)

| S.8.1.A4 | | RU | VNT100 |
|---|---|---|---|
| | WT | 0 | |
| | MUTATED | 2 | |

Monoclonal antibody S.9.1.A12

Heavy chain amino acid sequence - (with underlined CDRs): SEQ ID NO: 3

EVQLVESGGGLVQPGGSLRLSCAVSGFIVGRNYMSWVRQAPGKGLEWVSVIYPGGSTFY
ADSVKGRFTISRDNSKSTLFLQMNSLRAEDTAVYYCARDRGEQFFDYWGQGTLVTVSS

K light chain amino acid sequence (with underlined CDRs): SEQ ID NO: 4

DIQMTQSPSSLSASVGDRVTITCRASQGISNYLAWYQQKPGKAPKLLIYAASTLQSGVP
SRFSGSGSGTDFTLTISSLQPEDVATYYCQKYNSAPPTFGGGTKVEIK

Table 5 - Binding of mAb S.9.1.A12 (expressed in CHO cells and purified) to the RBD, Spike and N proteins of the SARS-CoV-2 virus by Surface Plasm Resonance (SPR), with units in RU (resonance units), neutralization of SARS-CoV-2 by VNT100 (title of the dilution of mAb S.9.1.A12):

| S.9.1.A12 | | RU | VNT100 |
|---|---|---|---|
| RBD | WT | 268 | 1280 |
| | E484K | 156 | |
| | N501Y | 165 | |
| | BETA | 82 | |
| | GAMMA | 183 | 20 |
| | DELTA | 916 | 2560 |
| | OMICRON | 6 | |
| SPIKE | WT | 13 | |
| | MUTATED | 6 | |
| N | WT | 1 | |
| | MUTATED | 4 | |

Monoclonal antibody S.9.1.B11

Heavy chain amino acid sequence (with underlined CDRs): SEQ ID NO: 5

EVQLVETGGGXIQPGGSLRLSCAASGITVSRNYMNWVRQAPGKGLEWVSLIYSGGSTFY
ADSVKGRFTISRDNSKNTLYLQMNSLRAEDTAVYYCARDLADRGGMDVWGQGTTVTVSS

L light chain amino acid sequence (with underlined CDRs): SEQ ID NO: 6

SYELTQPPSVSVAPGKTARITCGGN<u>NVGSQS</u>VHWYQQRSGQAPVLVVY<u>DDG</u>DRPSGIPG

RFSGSNSGNTATLTISRVEAGDEADYFC<u>QAWDSSRDHVV</u>FGGGTKLTVL

Table 6 - Binding of mAb S.9.1.B11 (expressed in CHO cells and purified) to the RBD, Spike and N proteins of the SARS-CoV-2 virus by Surface Plasmon Resonance (SPR), with units in RU (resonance units), neutralization of SARS-CoV-2 by VNT100 (title of the dilution of mAb S.9.1.B11) and affinity kinetics (KD):

| S.9.1.B11 | | RU | VNT100 | KD (M) |
|---|---|---|---|---|
| RBD | WT | | 200 | |
| | GAMMA | 132 | 480 | 1,07E-09*** |
| | DELTA | 334 | 800 | 1,64E-12*** |
| | OMICRON | 51 | 80 | 6,80E-08*** |
| SPIKE | GAMMA | 7 | | |
| | DELTA | 60 | | |
| | OMICRON | 13 | | |
| * dissociation in 360sec<br>** dissociation in 36000sec<br>*** did not dissociate until 36000sec | | | | |

Monoclonal antibody S.9.1.C10

Heavy chain amino acid sequence (with underlined CDRs): SEQ ID NO: 7

QVQLVQSGAEVKKPGASVKISCKAS<u>GYTFIHYYMH</u>WVRQAPGQGLEWMGI<u>INPSGGGTT</u>

YTQKFQGRVTMTRDTSTSTVYMELSSLRSEDTAVYYC<u>ARDLAGYTSEFDY</u>WGQGTLVTV

SS

K light chain amino acid sequence (with underlined CDRs): SEQ ID NO: 8

IQMTQSPSSLSASVGDRVTITCRAS<u>QGIRND</u>LGWYQQKPGKAPKRLIY<u>AAS</u>SLQSGVPS

RFSGSGSGTEFTLTISSLQPEDVATYYC<u>LQHNSYPMCS</u>FGQGTKLEIK

Binding of mAb S.9.1.C10 (expressed in CHO cells and purified) to the RBD, Spike and N proteins of the SARS-CoV-2 virus by Surface Plasm Resonance (SPR), with units in RU (resonance units), neutralization of SARS-CoV-2 by VNT100 (title of the dilution of mAb S.9.1.C10):

| S.9.1.C10 | | RU | VNT100 |
|---|---|---|---|
| RBD | WT | 397 | 640 |
| | E484K | 198 | |
| | N501Y | 270 | |
| | BETA | 133 | |
| | GAMMA | 86 | <20 |
| | DELTA | 480 | 320 |
| | OMICRON | 1 | |

(continued)

| S.9.1.C10 | | RU | VNT100 |
|---|---|---|---|
| SPIKE | WT | 19 | |
| | MUTATED | 9 | |
| | GAMMA | 0 | |
| | DELTA | 98 | |
| | OMICRON | 6 | |
| N | WT | 1 | |
| | MUTATED | 3 | |

Monoclonal antibody S.9.1.C9

Heavy chain amino acid sequence (with underlined CDRs): SEQ ID NO: 9

QVQLVESGGGVVQPGRSLGLSCAVS<u>GFTFSNYGMD</u>WVRQAPGKGLEWVAV<u>TSYDGSNKY</u>
YADSVKGRFTISRDNSKNTLYLQMNSLRTEDTAVYYC<u>AKHGGGPYCGGGTCYLAYFDY</u>W
GQGTLVTVSS

K light chain amino acid sequence (with underlined CDRs): SEQ ID NO: 10

DIQMTQSPSSLSASVRDRVTITCQAS<u>QDISKF</u>LNWYQQKPGKAPKLLIY<u>DAS</u>NLETGVP
SRFSGSGSGTDFTFTISSLQPEDIATYYC<u>QQYDNLPIT</u>FGQGTRLEIK

Table 8 - Binding of mAb S.9.1.C9 (expressed in CHO cells and purified) to the RBD, Spike and N proteins of the SARS-CoV-2 virus by Surface Plasmonic Resonance (SPR), with units in RU (resonance units), neutralization of SARS-CoV-2 by VNT100 (title of the dilution of mAb S.9.1.C9) and affinity kinetics (KD):

| S.9.1.C9 | | RU | VNT100 | KD (M) |
|---|---|---|---|---|
| RBD | WT | 474 | 40 | 2, 14E-10* |
| | E484K | 392 | | |
| | N501Y | 349 | | |
| | BETA | 458 | | |
| | GAMMA | 294 | 20 | 3,62E-10*** |
| | DELTA | 636 | <20 | 4, 55E-10*** |
| | OMICRON | 137 | <20 | 2, 41E-10* |
| SPIKE | WT | 22 | | |
| | MUTATED | 9 | | |
| | GAMMA | 18 | | |
| | DELTA | 118 | | |
| | OMICRON | 44 | | |

(continued)

| S.9.1.C9 | | RU | VNT100 | KD (M) |
|---|---|---|---|---|
| N | WT | 0 | | |
| | MUTATED | 1 | | |

* dissociation in 360sec
** dissociation in 36000sec
*** did not dissociate until 36000sec

Monoclonal antibody S.9.1.H5

Heavy chain amino acid sequence (with underlined CDRs): SEQ ID NO: 11

QVQLVESGGGVVQPGRSLRLSCAASGFTFSRYGMHWVRQAPGKGLEWVALISYDGTNKY
YADSVKGRFTISRDNSKSTQYLQMNSLRAEDTAVYFCAKESFGYASGSYYFDYWGQGTL
VTVSS

K light chain amino acid sequence (with underlined CDRs): SEQ ID NO: 12

DIQMTQSPSSLSASVGDRITITCQASQDISNYLNWYQQKPGKAPKLMIYDASNLETGVP
SRFSGSGAGTDFTFTISSLQPEDFARYYCQQYGNLPLTFGGGTKVEIK

Table 9 - Binding of mAb S.9.1.H5 (expressed in CHO cells and purified) to the RBD, Spike and N proteins of the SARS-CoV-2 virus by Surface Plasm Resonance (SPR), with units in RU (resonance units), neutralization of SARS-CoV-2 by VNT100 (title of the dilution of mAb S.9.1.H5) and affinity kinetics (KD) :

| S.9.1.H5 | | RU | VNT100 | KD |
|---|---|---|---|---|
| RBD | WT | 623 | 40 | |
| | E484K | 526 | | |
| | N501Y | 469 | | |
| | BETA | 582 | | |
| | GAMMA | 376 | 40 | 1, 11E-09* |
| | DELTA | 942 | 320 | 1,97E-10*** |
| | OMICRON | 175 | <20 | 1, 83E-09* |
| SPIKE | WT | 31 | | |
| | MUTATED | 14 | | |
| | GAMMA | 28 | | |
| | DELTA | 153 | | |
| | OMICRON | 58 | | |
| N | WT | 0 | | |
| | MUTATED | 0, 5 | | |

* dissociation in 360sec
** dissociation in 36000sec
*** did not dissociate until 36000sec

Monoclonal antibody S.9.2.B8

Heavy chain amino acid sequence (with underlined CDRs): SEQ ID NO: 13

EVQLVETGGGLIQPGGSLRLSCAAS<u>GLIVSSNYMT</u>WVRQAPGKGLEWVS<u>VLYSG
GTT</u>YYADSVKGRVTISRDNSKNTLYLQVNSLTVADTAVYYC<u>ARDLADRGGMDV</u>WGQGTT
VTVSS

L light chain amino acid sequence (with underlined CDRs): SEQ ID NO: 14

SYELTQPPSVSVAPGQTARITCGGNN<u>IGSQS</u>VHWYQQKPGQAPVLVVY<u>DDS</u>DRP
SGIPERFSGSKSGNMATLTISRVEAGDEADYYC<u>QVWDSSSDHVV</u>FGGGTKLTVL

Table 10 - Binding of mAb S.9.2.B8 (expressed in CHO cells and purified) to the RBD, Spike and N proteins of the SARS-CoV-2 virus by Surface Plasmon Resonance (SPR), with units in RU (resonance units), neutralization of SARS-CoV-2 by VNT100 (title of the dilution of mAb S.9.2.B8) and affinity kinetics (KD):

| S.9.2.B8 | | RU | VNT100 | KD |
|---|---|---|---|---|
| RBD | WT | 170 | 1280 | 1,99E-09* |
| | E484K | 84 | | |
| | N501Y | 120 | | |
| | BETA | 171 | | |
| | GAMMA | 74 | 2560 | 1,17E-09*** |
| | DELTA | 163 | 1280 | 8,36E-13*** |
| | OMICRON | 22 | 20 | 1,77E-09*** |
| SPIKE | WT | 15 | | |
| | MUTATED | 10 | | |
| | GAMMA | 3 | | |
| | DELTA | 53 | | |
| | OMICRON | 7 | | |
| N | WT | 0 | | |
| | MUTATED | 6 | | |
| *dissociation in 360sec<br>**dissociation in 36000sec<br>***did not dissociate until 36000sec | | | | |

Monoclonal antibody S.9.2.D3

Heavy chain amino acid sequence (with underlined CDRs): SEQ ID NO: 15

EVQLVESGGGLVQPGGSLRLSCAAS<u>EFIVSSNY</u>MSWVRQAPGKGLEWVS<u>VIYSG</u>
<u>GST</u>FYADSVKGRFTISRDNSKNTLYLQMDALRAEDTAVYYC<u>ARDYGEFYFDY</u>WGQGTLV
TVSS

K light chain amino acid sequence (with underlined CDRs): SEQ ID NO: 16

EIVLTQSPGTLSLSPGERATLSCRAS<u>QSVSNSY</u>LAWYQQKPGQAPRLLIY<u>GASS</u>
<u>RAT</u>GIPDRFSGSGSGTDFTLTISRLEPEDFAVYYC<u>QQYDSSPRT</u>FGQGTKVEIK

Tabela 11 - Ligação do mAb S.9.2.D3 (expresso em célula CHO e purificado) às proteínas RBD, Spike e N do virus SARS-CoV-2 por Ressonância Plasmônica de Superfície (SPR), com unidades em RU (unidades de ressonância), neutralização do SARS-CoV-2 por VNT100 (titulo da diluição do mAb S.9.2.D3):

| S.9.2.D3 | | RU | VNT100 |
|---|---|---|---|
| RBD | WT | 201 | 3840 |
| | E484K | 108 | |
| | N501Y | 112 | |
| | BETA | 92 | |
| | GAMMA | 28 | 20 |
| | DELTA | 234 | 2560 |
| | OMICRON | 6 | <20 |
| SPIKE | WT | 11 | |
| | MUTATED | 5 | |
| | GAMMA | 0 | |
| | DELTA | 68 | |
| | OMICRON | 3 | |
| N | WT | 8 | |
| | MUTATED | 18 | |

<u>Monoclonal antibody S.9.2.D7</u>

Heavy chain amino acid sequence (with underlined CDRs): SEQ ID NO: 17

EVQLLESGGGLIQTGGSLRLSCAAS<u>GFTFSSYA</u>MSWVRQAPGKGLEWVS<u>VISDS</u>
<u>GGS</u>TYYADSVKGRLTISRDNSKNTLYVQMNSLRAEDTAVYYC<u>AKGKGGIAAAGPAFLFD</u>
<u>Y</u>WGQGTLVTVSS

K light chain amino acid sequence (with underlined CDRs): SEQ ID NO: 18

DIQMTQSPSLLSASAGDRVTISCRMS<u>QGISSY</u>LAWYQQKPGKAPELLISS<u>AST</u>L
QSGVPSRFSGSGSGTDFTLTISYLQSEDFATYYC<u>QQYYSFPTT</u>FGGGTKVEIK

Tabela 12 - Ligação do mAb S.9.2.D7 (expresso em célula CHO e purificado) às proteínas RBD, Spike e N do virus SARS-CoV-2 por Ressonância Plasmônica de Superfície (SPR), com unidades em RU (unidades de ressonância), neutralização do SARS-CoV-2 por VNT100 (titulo da diluição do mAb S.9.2.D7) e cinética de afinidade (KD):

| S.9.2.D7 | | RU | VNT100 | KD |
|---|---|---|---|---|
| RBD | WT | 60 | 960 | 4,85E-09** |
| | E484K | 42 | | |
| | N501Y | 44 | | |
| | BETA | 40 | | |
| | GAMMA | 50 | >2560 | 2,37E-13*** |
| | DELTA | 0 | | 1, 84E-12** |
| | OMICRON | 0 | <20 | 6,29E-12*** |
| SPIKE | WT | 6 | | |
| | MUTATED | 3 | | |
| | GAMMA | 1 | | |
| | DELTA | 0 | | |
| | OMICRON | 0 | | |
| N | WT | 1 | | |
| | MUTATED | 4 | | |
| * dissociation in 360sec<br>** dissociation in 36000sec<br>*** did not dissociate until 36000sec | | | | |

Monoclonal antibody S.9.2.E9

Heavy chain amino acid sequence (with underlined CDRs): SEQ ID NO: 19

QVQLVQSGAEVKKPGASVKVSCQASGYSFTYYYMHWVRQAPGQGLEWMGIINPSGGGTS YAQKFQGRVTMTRDTSTSTVYMELSSLRSEDTAVYYCARDLAGVTSEFDYWGQGTLVTV SS

K light chain amino acid sequence (with underlined CDRs): SEQ ID NO: 20

IQMTQSPSSLSASVGDRVTITCRASQDIRHDLGWYQQKPGKAPKRLIYAASSLHSGVPS RFSGSGSGTEFTLTISSLQPEDFATYYCLQHNSYPMCSFGQGTKLEIK

Binding of mAb S.9.2.E9 (expressed in CHO cells and purified) to the RBD, Spike and N proteins of the SARS-CoV-2 virus by Surface Plasm Resonance (SPR), with units in RU (resonance units), neutralization of SARS-CoV-2 by VNT100 (title of mAb S.9.2.E9 dilution):

| S.9.2.E9 | | RU | VNT100 |
|---|---|---|---|
| RBD | WT | 581 | 2560 |
| | E484K | 330 | |
| | N501Y | 427 | |
| | BETA | 412 | |
| | GAMMA | 154 | 320 |
| | DELTA | 544 | 1280 |
| | OMICRON | 0 | <20 |
| SPIKE | WT | 32 | |
| | MUTATED | 12 | |
| | GAMMA | 4 | |
| | DELTA | 125 | |
| | OMICRON | 0 | |
| N | WT | 0 | |
| | MUTATED | 0 | |

Monoclonal antibody S.9.2.F4

Heavy chain amino acid sequence (with underlined CDRs): SEQ ID NO: 21

QVQLVESGGGVVQPGRSLRLSCAAS<u>GFTFSSYGIH</u>WVRQAPGKGLEWVAV<u>IANDGSNKY</u>
YRDSVKGRFTISRDNSKNTLYLQMNSLRVEDTAVYYC<u>AKGLAGIAAAGTPFDY</u>WGQGTL
VTVSS

K light chain amino acid sequence (with underlined CDRs): SEQ ID NO: 22

EIVLTQSPYLLSASTGDRVTISCRMT<u>QGISSF</u>LAWYQQKPGKAPELLIY<u>AAS</u>TLQSGVP
SRFSGSGSGTDFTLTISSLQPEDFATYYC<u>QQYYSFPPT</u>FGQGTKLEIK

Table 14 - Binding of mAb S.9.2.F4 (expressed in CHO cells and purified) to the RBD, Spike and N proteins of the SARS-CoV-2 virus by Surface Plasm Resonance (SPR), with units in RU (resonance units), neutralization of SARS-CoV-2 by VNT100 (title of mAb S.9.2.F4 dilution):

| S.9.2.F4 | | RU | VNT100 |
|---|---|---|---|
| RBD | WT | 43 | 60 |
| | E484K | 36 | |
| | N501Y | 37 | |
| | BETA | 38 | |
| | GAMMA | 195 | 160 |
| | DELTA | 15 | |
| | OMICRON | 1 | <20 |

(continued)

| S.9.2.F4 | | | RU | VNT100 |
|---|---|---|---|---|
| SPIKE | WT | | 5 | |
| | MUTATED | | 3 | |
| | GAMMA | | 12 | |
| | DELTA | | 4 | |
| | OMICRON | | 6 | |
| N | WT | | 1 | |
| | MUTATED | | 3 | |

Monoclonal antibody S.9.3.B2

Heavy chain amino acid sequence (with underlined CDRs): SEQ ID NO: 23

EVQLVESGGGLVQPGGSLRLSCAASGLTVSSNYMSWVRQAPGKGLEWVSIIYSGGSTFY
ADSVKGRFTISRHNSKNTLYLQMNSLRAEDTAVYYCARDLVYYGMDVWGQGTTVTVSS

K light chain amino acid sequence (with underlined CDRs): SEQ ID NO: 24

DIQLTQSPSFLSASVGDRVTITCRASQGISNYLAWYQQKPGKAPKLLIYAASTLQSGVP
SRFSGSGSGTEFTLTISSLQPEDFATYYCQQLDSYPPLIFGGGTKVEIK

Table 15 - Binding of mAb S.9.3.B2 (expressed in CHO cells and purified) to the RBD, Spike and N proteins of the SARS-CoV-2 virus by Surface Plasm Resonance (SPR), with units in RU (resonance units), neutralization of SARS-CoV-2 by VNT100 (title of mAb S.9.3.B2 dilution):

| S.9.3.B2 | | | RU | VNT100 |
|---|---|---|---|---|
| RBD | WT | | 427 | 640 |
| | E484K | | 247 | |
| | N501Y | | 287 | |
| | BETA | | 372 | |
| | GAMMA | | 267 | 2560 |
| | DELTA | | 777 | 2560 |
| | OMICRON | | 47 | <20 |
| SPIKE | WT | | 21 | |
| | MUTATED | | 7 | |
| | GAMMA | | 15 | |
| | DELTA | | 125 | |
| | OMICRON | | 11 | |
| N | WT | | 0 | |
| | MUTATED | | 0 | |

Monoclonal antibody S.9.3.G2

Heavy chain amino acid sequence (with underlined CDRs): SEQ ID NO: 25

EVQLVESGGGLVRPGGSLRLSCAASGIIVSRNYMSWVRQAPGKGLEWVSVIYSGGSTFY
ADSVKGRFTISRDNSKNTLYLQMNGLRAEDTAVYYCARDLSYYGMDVWGQGTTVTVSS

K light chain amino acid sequence (with underlined CDRs): SEQ ID NO: 26

DIQLTQSPSFLSASVGDRVTITCRASQGISSYLAWYQQKPGKAPKLLIYAASTLQSGVP
SRFSGSGSGTEFTLTISSLQPEDFATYYCQQLNSYPCSFGQGTKLEIK

Table 16 - Binding of mAb S.9.3.G2 (expressed in CHO cells and purified) to RBD, Spike and N proteins of the SARS-CoV-2 virus by Surface Plasm Resonance (SPR), with units in RU (resonance units), neutralization of SARS-CoV-2 by VNT100 (title of mAb S.9.3.G2 dilution):

| S.9.3.G2 | | RU | VNT100 |
|---|---|---|---|
| RBD | WT | 381 | 1280 |
| | E484K | 201 | |
| | N501Y | 256 | |
| | BETA | 305 | |
| | GAMMA | 253 | 80 |
| | DELTA | 710 | 2560 |
| | OMICRON | 10 | <20 |
| SPIKE | WT | 18 | |
| | MUTATED | 5 | |
| | GAMMA | 11 | |
| | DELTA | 110 | |
| | OMICRON | 3 | |
| N | WT | 0 | |
| | MUTATED | 0 | |

Monoclonal antibody S.9.4.C7

Heavy chain amino acid sequence (with underlined CDRs): SEQ ID NO: 27

QVQLVESGGGVVQPGRSLRLSCAASGFTFRNYAMHWVRQAPGKGLEWVTVISYD
GSDKYYADSVKGRFTISRDNSKNTLYLQMNSLRAEDTAVYYCAKDPVGGYCSGGRCYFH
SHYFDYWGQGTLVTVSS

L light chain amino acid sequence (with underlined CDRs): SEQ ID NO: 28

NFMLTQPHSVSESPGKTVTISCTRSS<u>GSIASYY</u>VQWYQQRPGSSPTTVIS<u>EDNQ</u>

RPSGVPDRFSGSIDSSSNSASLTISGLKTEDEADYYCQ<u>SYDSNNHVI</u>FGGGTKLTVL

Table 17 - Binding of mAb S.9.4.C7 (expressed in CHO cells and purified) to the RBD, Spike and N proteins of the SARS-CoV-2 virus by Surface Plasmon Resonance (SPR), with units in RU (resonance units), neutralization of SARS-CoV-2 by VNT100 (title of the dilution of mAb S.9.4.C7) and affinity kinetics (KD):

| S.9.4.C7 | | | RU | VNT100 | KD |
|---|---|---|---|---|---|
| RBD | WT | | 539 | 80 | 4, 07E-11* |
| | E484K | | 529 | | |
| | N501Y | | 388 | | |
| | BETA | | 615 | | |
| | GAMMA | | 481 | 1280 | 2,05E-10*** |
| | DELTA | | 1153 | 320 | 1,40E-10*** |
| | OMICRON | | 160 | <20 | 1, 31E-09* |
| SPIKE | WT | | 27 | | |
| | MUTATED | | 13 | | |
| | GAMMA | | 30 | | |
| | DELTA | | 162 | | |
| | OMICRON | | 44 | | |
| N | WT | | 0 | | |
| | MUTATED | | 6 | | |
| *dissociation in 360sec<br>**dissociation in 36000sec<br>***did not dissociate until 36000sec | | | | | |

Monoclonal antibody S.9.4.F4

Heavy chain amino acid sequence (with underlined CDRs): SEQ ID NO: 29

QVQLVESGGGVVQPGRSLRLSCAAS<u>GFTFSHYG</u>MHWVRQAPGKGLEWVAV<u>ILYDGSNEY</u>YADSVKGRFTISRDNSKNTLYLQMNSLRAEDTAVYYC<u>AKGAGTYCSGGNCYVAYFDY</u>WGQGTLVTVSS

K light chain amino acid sequence (with underlined CDRs): SEQ ID NO: 30

AIRMTQSPSSLSASVGDRVTITCQAS<u>QDISNYL</u>NWYQQKPGKAPKLLIY<u>DAS</u>NLETGVPSRFSGSGSGTDFTVTISSLQPEDFATYYC<u>QQYDNLPLT</u>FGGGTKVEIK

Table 18 - Binding of mAb S.9.4.F4 (expressed in CHO cells and purified) to RBD, Spike and N proteins of the SARS-CoV-2 virus by Surface Plasm Resonance (SPR), with units in RU (resonance units), neutralization of SARS-CoV-2 by VNT100 (title of mAb S.9.4.F4 dilution) and affinity kinetics (KD):

| S.9.4.F4 | | | RU | VNT100 | KD |
|---|---|---|---|---|---|
| RBD | WT | | 413 | 40 | 3,36E-11*** |
| | E484K | | 363 | | |
| | N501Y | | 309 | | |
| | BETA | | 402 | | |
| | GAMMA | | 160 | 80 | 1,87E-11*** |
| | DELTA | | 373 | <20 | 1,22E-12*** |
| | OMICRON | | 74 | <20 | 2, 31E-10** |
| SPIKE | WT | | 19 | | |
| | MUTATED | | 9 | | |
| | GAMMA | | 11 | | |
| | DELTA | | 89 | | |
| | OMICRON | | 29 | | |
| N | WT | | 1 | | |
| | MUTATED | | 3 | | |
| *dissociation in 360sec<br>**dissociation in 36000sec<br>***did not dissociate until 36000sec | | | | | |

Monoclonal antibody S.9.4.H8

Heavy chain amino acid sequence (with underlined CDRs): SEQ ID NO: 31

QVQLVESGXGVVQPGRSLRLSCAAS<u>GFTFSTYGMH</u>WVRQAPGKGLEWVAV<u>ISYDGSNKK</u>FADSVKGRFTISRDTSKNTLYLQMNSLRADDTAVYYC<u>AKGAGTYCSAGNCYVAYFDY</u>WGQGTLVTVSS

K light chain amino acid sequence (with underlined CDRs): SEQ ID NO: 32

DIQMTQSPSSXSAXVGXRVTITCQAS<u>QDINNYLN</u>WYQQKPGKAPKLLIY<u>DASN</u>LETGVPSRFSGSGSGTDFTFTISSLKPEDIATYYC<u>QQYYSLPPVT</u>FGQGTRLEIK

Table 19 - Binding of mAb S.9.4.H8 (expressed in CHO cells and purified) to the RBD, Spike and N proteins of the SARS-CoV-2 virus by Surface Plasm Resonance (SPR), with units in RU (resonance units):

| S.9.4.H8 | | RU |
|---|---|---|
| RBD | WT | |
| | GAMMA | 211 |
| | DELTA | 591 |
| | OMICRON | 88 |

(continued)

| S.9.4.H8 | | RU |
|---|---|---|
| SPIKE | GAMMA | 15 |
| | DELTA | 107 |
| | OMICRON | 36 |

Monoclonal antibody S.9.5.A9

Heavy chain amino acid sequence (with underlined CDRs): SEQ ID NO: 33

EVQLVESGGGLVQPGGSLRLSCAAS<u>GITVSSNYM</u>SWVRQAPGKGLEWVSV<u>IYSG</u>
<u>GST</u>FYADSVKGRFTISRHNSKNTLYLQMNSLRPEDTAVYYC<u>ARDLGENAFDI</u>WGQGTMV
TVSS

K light chain amino acid sequence (with underlined CDRs): SEQ ID NO: 34

DIQLTQSPSFLSASVGDRVTITCRAS<u>QGISSYLA</u>WYQQKPGKAPKLLIY<u>AAS</u>TL
QSGVPSRFSGSGSGTEFTLTISSLQPEDFATYYC<u>QQLNSYPGFT</u>FGPGTKVDIK

Table 20 - Binding of mAb S.9.5.A9 (expressed in CHO cells and purified) to the RBD, Spike and N proteins of the SARS-CoV-2 virus by Surface Plasm Resonance (SPR), with units in RU (resonance units), neutralization of SARS-CoV-2 by VNT100 (title of mAb S.9.5.A9 dilution):

| s.9.5.A9 | | RU | VNT100 |
|---|---|---|---|
| RBD | WT | 320 | 1280 |
| | E484K | 185 | |
| | N501Y | 111 | |
| | BETA | 19 | |
| | GAMMA | 12 | 0 |
| | DELTA | 390 | 1280 |
| | OMICRON | 0 | <20 |
| SPIKE | WT | 15 | |
| | MUTATED | 1 | |
| | GAMMA | 0 | |
| | DELTA | 87 | |
| | OMICRON | 0 | |
| N | WT | 0 | |
| | MUTATED | 0 | |

Monoclonal antibody S.10.1.C6

Heavy chain amino acid sequence (with underlined CDRs): SEQ ID NO: 35

QLQLQESGPGLVKPSETLSLTCTVSGGSISSSSYYWGWIRQPPGKGLEWIGSFYYSGGS
YYNPSLKSRVTISVDTSKNQFSLKLSSVTAADTAVYYCARDEHGDYAVGYWGQGTLVTV
SS

L light chain amino acid sequence (with underlined CDRs): SEQ ID NO: 36

QSVLTQPPSVSGAPGQRVTISCTGSSSNIGAGYDVHWYQQLPGTAPKLLIYGNNNRPSG
VPDRFSGSKSGTSASLAITGLQAEDEADYYCQSYDSSLSALYVFGTGTKVTVL

Table 21 - Binding of the S.10.1.C6 mAb (expressed in CHO cells and purified) to the RBD, Spike and N proteins of the SARS-CoV-2 virus by Surface Plasm Resonance (SPR), with units in RU (resonance units), neutralization of SARS-CoV-2 by VNT100 (title of the S.10.1.C6 mAb dilution) and affinity kinetics (KD):

| S.10.1.C6 | | RU | VNT100 | KD |
|---|---|---|---|---|
| RBD | WU | | 20 | |
| | GAMMA | 134 | <20 | 2,35E-10*** |
| | DELTA | 343 | 20 | 8,12E-12*** |
| | OMICRON | 66 | <20 | 8,04E-10*** |
| SPIKE | GAMMA | 9 | | |
| | DELTA | 91 | | |
| | OMICRON | 29 | | |
| *dissociation in 360sec <br> **dissociation in 36000sec <br> ***did not dissociate until 36000sec | | | | |

Monoclonal antibody S.10.1.D8

Heavy chain amino acid sequence (with underlined CDRs): SEQ ID NO: 37

EVQLVESGGGXVQPGGSLRLSCAASGLTVSSNYMSWVRQAPGKGLEWVSLIYSGGSTYY
ADSVKGRFTISRDNSKNTLYLQMNSLRAEDTAVYYCARDVLHRSGMDVWGQGTTVTVSS

K light chain amino acid sequence (with underlined CDRs): SEQ ID NO: 38

DIQMTQSPSSXSASVGDRVTITCQASQDISNYLNWYQQKPGKAPKLLIYDASNLETGVP
SRFSGSGSGTDFTFTISSLQPEDIATYYCQQYDNLPRTFGQGTKVEIK

Table 22 - Binding of mAb S.10.1.D8 (expressed in CHO cells and purified) to the RBD, Spike and N proteins of the SARS-CoV-2 virus by Surface Plasm Resonance (SPR), with units in RU (resonance units), neutralization of SARS-CoV-2 by VNT100 (title of mAb S.10.1.D8 dilution):

| S.10.1.D8 | | RU | VNT100 |
|---|---|---|---|
| RBD | WT | | 640 |
| | GAMMA | 100 | 60 |
| | DELTA | 270 | 1200 |
| | OMICRON | 4 | <20 |
| SPIKE | GAMMA | 4 | |
| | DELTA | 48 | |
| | OMICRON | 2 | |

Monoclonal antibody S.10.2.D2

Heavy chain amino acid sequence (with underlined CDRs): SEQ ID NO: 39

EVQLVESGGGLVQPGXSLRLSCAASGVTVSSNYMSWVRQAPGKGLEWVSVIYSGGSTYY
ADSVKGRFTISRDNSKNTLHLQMNSLRAEDTAVYYCARDLGPRGAFDIWGQGTMVTVSS

K light chain amino acid sequence (with underlined CDRs): SEQ ID NO: 40

EIVLTQSPGTXSXSPGERATLSCRASQSVSSTYLAWYQQKPGQAPRLLIYGASSRATGI
PDRFSGSGSGTDFTLTISRLEPEDFAVYYCQQYGSSPYSFGQGTKLEIK

Table 23 - Binding of mAb S.10.2.D2 (expressed in CHO cells and purified) to RBD, Spike and N proteins of the SARS-CoV-2 virus by Surface Plasm Resonance (SPR), with units in RU (resonance units), neutralization of SARS-CoV-2 by VNT100 (title of mAb S.10.2.D2 dilution):

| S.10.2.D2 | | RU | VNT100 |
|---|---|---|---|
| RBD | WT | | 100 |
| | GAMMA | 89 | <20 |
| | DELTA | 207 | 200 |
| | OMICRON | 0 | <20 |
| SPIKE | GAMMA | 2 | |
| | DELTA | 28 | |
| | OMICRON | 1 | |

Monoclonal antibody S.11.2.A5

Heavy chain amino acid sequence (with underlined CDRs): SEQ ID NO: 41

QVQLVESGXGVVQPGRSLRLTCAASGFPFSTYVMNWVRQAPGKGLEWVASISNDGTRKD

YAGSVKGRFSISRDNFNSDLFLQMNTLKTEDTALYWCARTSGRGSRDRGRFDLWGQGTL

VTVSS

K light chain amino acid sequence (with underlined CDRs): SEQ ID NO: 42

DIVMTQSPLSLPVTPGEPASISCRSSQSLLDSNGYNYLDWYLQKPGQSPQLLIYLGSNR

ASGVPDRFSGSGSGTDFTLKISRVEVEDVGVYYCMQSLQGFTFGPGTKVDIK

Table 24 - Test of mAb S.11.2.A5 (expressed in CHO cell and purified) by neutralization of SARS-CoV-2 by VNT100 (title of mAb S.11.2.A5 dilution):

| S.11.2.A5 | | VNT100 |
|---|---|---|
| RBD | WT | >2560 |
| | GAMMA | <20 |
| | DELTA | 320 |
| | OMICRON | 10 |

Monoclonal antibody S.11.2.C5

Heavy chain amino acid sequence (with underlined CDRs): SEQ ID NO: 43

QVQLVQSGPEVKKPGSSVKVSCKGSEGTFNVYAISWLRQAPGQGLEWMGGIIPMHGKAN

LAQKFQGRVTIAADGNTNTGYMELSGLRSEDTAIYFCARDYRYCDSSTCYDFAFDIWGQ

GTMVTVSS

K light chain amino acid sequence (with underlined CDRs): SEQ ID NO: 44

DIQMTQSPSSLSASVGDRVTMSCRASQNISTFLNWYQQEPGKAPKLLIYAASSLQSGVP

SRFSGSGSGTDFSLTIRSLQPEDFATYYCQQSYRSKSFGQGTKLEIK

Table 25 - Test of mAb S.11.2.C5 (expressed in CHO cell and purified) by neutralization of SARS-CoV-2 by VNT100 (title of mAb S.11.2.C5 dilution):

| S.11.2.C5 | | VNT100 |
|---|---|---|
| RBD | WT | >2560 |
| | GAMMA | 1920 |
| | DELTA | >2560 |
| | OMICRON | <20 |

Monoclonal antibody S.11.3.A5

Heavy chain amino acid sequence SEQ ID NO: 45 (with underlined CDRs):

QVQLVQSGAEVKKPGSSMKVSCKTS<u>GGTFNTYATN</u>WVRQAPGQGLEWMGA<u>IIPIFGTTN</u>YAQKFQGRVTITADQSTTTSYMYLNSLTSEDTAVYYC<u>ARGTPVWGSSGPLGYNGMDV</u>WGQGTTVTVSS

K light chain amino acid sequence SEQ ID NO: 46 (with underlined CDRs):

DIQMTQSPSSLSASVGDRVTITCRAS<u>QSISTYLN</u>WYQQKPGKAPRLVIY<u>GASTLHS</u>GVPSRFSGSGSGTAFTLTISNLQPDDFASYYC<u>QQSSSSLALT</u>FGGGTKVEIK

Monoclonal antibody S.11.3.H5

Heavy chain amino acid sequence (with underlined CDRs): SEQ ID NO: 47

QVQLVQSGAEVRKPGASVEISCKAS<u>GYTFTNFY</u>IHWVRQAAGQGLEWMGL<u>INPS</u><u>GAST</u>TYAPKFQGRITMTRDTSTPTVYMELSSLRSDDTAVYFC<u>ARDFSPSVHYCGGDCNSPQYNWFDP</u>WGQGTLVTVSS

K light chain amino acid sequence (with underlined CDRs): SEQ ID NO: 48

EIVLTQSPATLSVSPGERVTLSCRAS<u>QIISRYLA</u>WYQQKPGQAPRLLIY<u>GASSR</u>AAGVPVRFSGSGSGTDFTLTISSLQSEDFAVYFC<u>QQYNNWPLT</u>FGGGTKVEIK

Table 26 - Test of mAb S.11.3.H5 (expressed in CHO cell and purified) by neutralization of SARS-CoV-2 by VNT100 (title of mAb S.11.3.H5 dilution):

| S.11.3.H5 | | VNT100 |
|---|---|---|
| RBD | WT | 40 |
| | GAMMA | 20 |
| | DELTA | 20 |
| | OMICRON | <20 |

Monoclonal antibody S.11.4.F9

Heavy chain amino acid sequence (with underlined CDRs): SEQ ID NO: 49

EVQLVESGGGLIQPGGSLRLSCVVS<u>GLTVSSNYMS</u>WVRQAPGKGLEWVSV<u>IYSGGST</u>FYANSVKGRFTISRDNSKNTLYLQMNSLRAEDTAIYYC<u>TRDYGDFYMDV</u>WGKGTTVTVSS

K light chain amino acid sequence (with underlined CDRs): SEQ ID NO: 50

EIVLTQSPGTLSLSPGERATLSCRAS<u>QSVISSY</u>LAWYQQKPGQAPRLLIH<u>GASS</u>
RATGIPDRFSGSGSGTDFTLTIRRLEPEDFAVYYC<u>QHYGSSRT</u>FGQGTKVEIK

Table 27 - Test of mAb S.11.4.F9 (expressed in CHO cell and purified) by neutralization of SARS-CoV-2 by VNT100 (title of mAb S.11.4.F9 dilution):

| S.11.4.F9 | | VNT100 |
|---|---|---|
| RBD | WT | 960 |
| | GAMMA | 200 |
| | DELTA | 1920 |
| | OMICRON | <20 |

Monoclonal antibody S.1.1.D10

Heavy chain amino acid sequence SEQ ID NO: 51 (with underlined CDRs):

EVQLLESGGGLVQPGGSLRLSCAVS<u>GFTFSSYAMN</u>WVRQAPGKGLEWVS<u>AISGSGDSTY</u>
YADSVKGRFTISRDSSKNTLYLQMSSLRAGDTAVYYC<u>AKDTMGHSSLFDY</u>WGQGALVTV
SS

K light chain amino acid sequence SEQ ID NO: 52 (with underlined CDRs):

EIVMTQSPVSLSVSPGERATLSCRAR<u>ESVSNNVA</u>WYQQKPGQAPRLLIY<u>GVS</u>TRATGIP
ARFSGSGSGTEFTLTISSLQSEDFAVYYC<u>QHYNNWPSWT</u>FGQGTKVEIK

Monoclonal antibody S.11.1.C7

Heavy chain amino acid sequence (with underlined CDRs): SEQ ID NO: 53

QVQLVQSGAEVKKPGASVRVSCKAS<u>GYIFSSYY</u>IHWVRQAPGQGLEWMGV<u>IYPS</u>
<u>GGST</u>DYAQIFQGRVTMTRDTSTNTVYMELSGLRSEDTAVYYC<u>ARDGKDIVGATLPGRYW</u>
GQGTLVTVSS

K light chain amino acid sequence (with underlined CDRs): SEQ ID NO: 54

DIVMTQSPDSLAVSLGERATIYCKSS<u>QSVLNSSNKKNY</u>LAWYQQKPGQPPRLLI
Y<u>WAS</u>TRESGVPDRFSGSGSGTDFTLTITSLQAEDVAVYYC<u>QHYYSPPIT</u>FGQGTRLEIK

Table 28 - Binding of mAb S.11.1.C7 (expressed in CHO cell and purified) to the RBD, Spike and N proteins of the SARS-CoV-2 virus by Surface Plasm Resonance (SPR), with units in RU (resonance units):

| S.11.1.C7 | | RU |
|---|---|---|
| RBD | WT | 641 |
| | E484K | 336 |
| | N501Y | 422 |
| | BETA | 514 |
| | GAMMA | 253 |
| | DELTA | 867 |
| | OMICRON | 33 |
| SPIKE | WT | 29 |
| | MUTATED | 11 |
| | GAMMA | 18 |
| | DELTA | 124 |
| | OMICRON | 2 |
| N | WT | 0 |
| | MUTATED | 0 |

Monoclonal antibody S.11.1.C8

Heavy chain amino acid sequence (with underlined CDRs): SEQ ID NO: 55

QVQLVESGGGVVQPGRSLRLSCAAS<u>GFTFSTFS</u>MHWVRQAPGKGLEWVAV<u>ILYDGTYKS</u>YADSVKGRFTISRDNSGNTLFLQMNSLRAEDSAVYYC<u>ARDFGEMTTYFDF</u>WGQGTLVTVSS

K light chain amino acid sequence (with underlined CDRs): SEQ ID NO: 56

DIQMTQPPSSLSASVGDRVTITCRAS<u>QSIGNN</u>LNWYQQKPGKAPKLLIY<u>VAS</u>SLQSGVPSRFSGSGSGTDFTLTISSLQPEDFATYFC<u>QQSYSTPPWT</u>FGQGTKVEIK

Table 29 - Binding of mAb S.11.1.C8 (expressed in CHO cells and purified) to the RBD, Spike and N proteins of the SARS-CoV-2 virus by Surface Plasm Resonance (SPR), with units in RU (resonance units) and affinity kinetics (KD):

| S.11.1.C8 | | RU | KD (M) |
|---|---|---|---|
| RBD | WT | 479 | |
| | E484K | 477 | |
| | N501Y | 335 | |
| | BETA | 571 | |
| | GAMMA | 360 | 1.03E-10*** |
| | DELTA | 799 | 1.31E-11*** |
| | OMICRON | 166 | 6.15E-10* |

(continued)

| S.11.1.C8 | | RU | KD (M) |
|---|---|---|---|
| SPIKE | WT | 26 | |
| | MUTATED | 11 | |
| | GAMMA | 26 | |
| | DELTA | 120 | |
| | OMICRON | 55 | |
| N | WT | 0 | |
| | MUTATED | 0 | |
| *dissociation in 360sec<br>**dissociation in 36000sec<br>***did not dissociate until 36000sec | | | |

Monoclonal antibody S.11.1.D12

Heavy chain amino acid sequence (with underlined CDRs): SEQ ID NO: 57

    EVQLLESGGGLVQPGGSLRLSCAASGFPFITYPMSWVRQAPGKGLEWVSDIGGIGGSTS
    YADSVKGRFTISRDNYQNTVYLQMHSLRAEDTAVYYCAKWKSRVDSSYKGGGWFDSWGP
    GTLVTVSS

K light chain amino acid sequence(with underlined CDRs): SEQ ID NO: 58

    DIVMTQSPDSLAVSLGERATIHCKSSQTVVYGSNNKNYLAWYQQKPGQPPKLLIYWAST
    RESGVPDRFSGSGSGTDFTLTISSLQAEDVAVYYCQQYYGPPPWTFGQGTKVEIK

Table 30 - Binding of mAb S.11.1.D12 (expressed in CHO cells and purified) to the RBD, Spike and N proteins of the SARS-CoV-2 virus by Surface Plasm Resonance (SPR), with units in RU (resonance units) and affinity kinetics (KD):

| S.11.1.D12 | | RU | KD (M) |
|---|---|---|---|
| RBD | WT | 623 | |
| | E484K | 111 | |
| | N501Y | 421 | |
| | BETA | 111 | |
| | GAMMA | 44 | 1, 10E-08*** |
| | DELTA | 771 | 6, 36E-10* |
| | OMICRON | 145 | 3,73E-09* |
| SPIKE | WT | 31 | |
| | MUTATED | 13 | |
| | GAMMA | 2 | |
| | DELTA | 94 | |
| | OMICRON | 35 | |

(continued)

| S.11.1.D12 | | RU | KD (M) |
|---|---|---|---|
| N | WT | 0 | |
| | MUTATED | 0 | |

| *dissociation in 360sec<br>**dissociation in 36000sec<br>***did not dissociate until 36000sec |
|---|

Monoclonal antibody S.11.1.F4

Heavy chain amino acid sequence (with underlined CDRs): SEQ ID NO: 59

QVQLVQSGAEVKKPGSSVRVSCKAS<u>AGTFSSYA</u>IRWVRQAPGQGLEWMGT<u>IIPI</u>
<u>FGTT</u>NYAQKFQGRVSITADESTNTAYMELSSLRSEDTAVYYC<u>ARDLETSPLYGLDV</u>WGQ
GTTVTVSS

K light chain amino acid sequence (with underlined CDRs): SEQ ID NO: 60

DIQMTQSPSSLSASVGDRVTITCRAS<u>QTISRYL</u>NWYQQKPGKPPKLLIF<u>AAST</u>L
HSGVPSRFSGSGSGTEFTLTITSLHPDDFSTYYC<u>QQTYSSPHT</u>FGQGTKLEIK

Table 31 - Binding of the S.11.1.F4 mAb (expressed in CHO cells and purified) to the RBD, Spike and N proteins of the SARS-CoV-2 virus by Surface Plasm Resonance (SPR), with units in RU (resonance units) and affinity kinetics (KD):

| S.11.1.F4 | | RU | KD (M) |
|---|---|---|---|
| RBD | WT | 192 | |
| | E484K | 400 | |
| | N501Y | 130 | |
| | BETA | 401 | |
| | GAMMA | 218 | 1, 21E-09* |
| | DELTA | 743 | 1, 89E-09* |
| | OMICRON | 153 | 5, 84E-10* |
| SPIKE | WT | 11 | |
| | MUTATED | 4 | |
| | GAMMA | 17 | |
| | DELTA | 75 | |
| | OMICRON | 50 | |
| N | WT | 0 | |
| | MUTATED | 0 | |

| *dissociation in 360sec<br>**dissociation in 36000sec<br>***did not dissociate until 36000sec |
|---|

103

Monoclonal antibody S.11.1.H1

Heavy chain amino acid sequence (with underlined CDRs): SEQ ID NO: 61

QVQLVESGGGVVQPGRSLTLSCSAS<u>GFPFSTYVMH</u>WVRQAPGKGLEWVAV<u>ISSDGSNEN
YADSVKG</u>RFTVSRDNFGSTLFLQMSTLRPDDTAVYYC<u>ARTAGRGYADRGRLDT</u>WGQGTL
VTVSS

K light chain amino acid sequence (with underlined CDRs): SEQ ID NO: 62

DIVMTQSPLYLPVTPGEPASISCRSS<u>QSLLHSNGYNYLD</u>WYLQKPGQSPQLLIY<u>LGSNR
A</u>SGVPDRFSGSGSGTDFTLKISRVEAEDVGVYYC<u>MQTLQTFT</u>FGPGTKVDIK

Table 32 - Binding of mAb S.11.1.H1 (expressed in CHO cells and purified) to the RBD, Spike and N proteins of the SARS-CoV-2 virus by Surface Plasm Resonance (SPR), with units in RU (resonance units) and affinity kinetics (KD):

| S.11.1.H1 | | RU | KD (M) |
|---|---|---|---|
| RBD | WT | 202 | |
| | E484K | 18 | |
| | N501Y | 133 | |
| | BETA | 9 | |
| | GAMMA | 4 | 1,37E-08*** |
| | DELTA | 913 | 5, 48E-10* |
| | OMICRON | 55 | 9, 76E-09* |
| SPIKE | WT | 13 | |
| | MUTATED | 6 | |
| | GAMMA | 0 | |
| | DELTA | 91 | |
| | OMICRON | 8 | |
| N | WT | 0 | |
| | MUTATED | 0 | |
| *dissociation in 360sec<br>**dissociation in 36000sec<br>***did not dissociate until 36000sec | | | |

Monoclonal antibody S.11.1.H5

Heavy chain amino acid sequence SEQ ID NO: 63 (with underlined CDRs):

EVQLVESAGGLVQPGGSLRLSCSAS<u>GFTFSSSPMH</u>WVRQAPGKGLQYVSA<u>VSSAGLTTY
YADSVKG</u>RFTISRDNSKNTVFLQMSSLRADDTAVYYC<u>VKGWVGTFDP</u>WGQGTLVTVSS

K light chain amino acid sequence SEQ ID NO: 64 (with underlined CDRs):

EIVLTQSPATLSVSPGERATLSCRAS<u>QSVSSNLA</u>WYQQKPGQAPRLLIY<u>GAS</u>TRATGIP
ARFSGSGSGTEFTLTISSLQSEDFAVYYC<u>QQYNNWPPYT</u>FGQGTKLEIK

Monoclonal antibody S.11.1.H8

Heavy chain amino acid sequence SEQ ID NO: 65 (with underlined CDRs):

QVQLQESGPGQVKPSQTLSLSCTVS<u>GDSINSVGYYW</u>NWIRQHPGKGLEWIG<u>NIYFYGST</u>
YYNPSLQSRVIISLDTSKNQFSLRLTSVTAADAAVYYC<u>ARSYTGYESDDALDI</u>WGQGTM
VTVSS

K light chain amino acid sequence SEQ ID NO: 66 (with underlined CDRs):

IQMTQSPSSLSASVGDRVTITCRAS<u>QDIGSDLG</u>WFQQRPGKAPKRLIY<u>GAS</u>SLQSGVPS
RVSGSGSGTEFTLTISSLQPEDFASYYC<u>LQYNSYPLT</u>FGGGTKVEIK

Monoclonal antibody S.11.2.A3

Heavy chain amino acid sequence SEQ ID NO: 67 (with underlined CDRs):

EVQLVESGGGLVKPGGSLSLSCAAS<u>GLTFSNVWM</u>NWVRQAPGKGLEWVG<u>LIKSATDGGT</u>
<u>P</u>YYAAPVKGRFIIPRDDSKNTLYLQMNSLKTEDTAVYFC<u>TTEAPGYSYGYEDFDY</u>WGQG
TLVTVSS

K light chain amino acid sequence SEQ ID NO: 68 (with underlined CDRs):

EIVLTQSPGTLSLSPGERATLSCRAS<u>QNIGMSYVA</u>WYQQKGGQAPRLLIY<u>GVS</u>SRATGI
PDRFSGSGSGTDFTLTISRLEPKDSAVYYC<u>QQYGSSHRT</u>FGQGTKLEI

Monoclonal antibody S.2.2.F11

Heavy chain amino acid sequence SEQ ID NO: 69 (with underlined CDRs):

QVQLVQSGAEVKKPGSSVKVSCRAS<u>GGTFNNYAI</u>SWVRQAPGRGLEWMGG<u>IFPFRNTAK</u>
YAQHFQGRVTITADESTDTAYMELSSLRSEDTAIYYC<u>ARGDTIFGVTMGYYAMDV</u>WGQG
TTVTVSS

K light chain amino acid sequence SEQ ID NO: 70 (with underlined CDRs):

DIQMTQSPSSLSASVGDRVTITCQAS<u>QDISNYL</u>NWYQQKPGKAPKLLIY<u>DAS</u>NLETGVP
SRFSGSGSGTDFTFTISSLQPEDIATYYC<u>QQYDNLLT</u>FGGGTKVEIK

Monoclonal antibody S.2.2.G8

Heavy chain amino acid sequence SEQ ID NO: 71 (with underlined CDRs):

QVQLVQSEAEVKKPGSSVKVSCRAS<u>GGTFNNYA</u>ISWVRQAPGQGLEWMGG<u>IFPFRNTAK</u>
YAQHFQGRVTITADESTGTAYMELSSLRSEDTAIYYC<u>ARGDTIFGVTMGYYAMDV</u>WGQG
TTVTVSS

K light chain amino acid sequence SEQ ID NO: 72 (with underlined CDRs):

EIVLTQSPVSLSVSPGERATLSCRAR<u>ESVSNNVA</u>WYQQKPGQAPRLLIY<u>GVS</u>RATGIP
ARFSGSGSGTEFTLTISSLQSEDFAVYYC<u>QHYNNWPSWT</u>FGQGTKVEIK

Table 33 - Binding of mAb S.2.2.G8 (expressed in CHO cell and purified) to RBD, Spike and N proteins of the SARS-CoV-2 virus by Surface Plasm Resonance (SPR), with units in RU (resonance units):

| S.2.2.G8 | | RU |
|---|---|---|
| RBD | WT | 30 |
| | E484K | 24 |
| | N501Y | 23 |
| | BETA | 56 |
| SPIKE | WT | 35 |
| | MUTATED | 24 |
| N | WT | 26 |
| | MUTATED | 106 |

Monoclonal antibody S.2.3.C4

Heavy chain amino acid sequence SEQ ID NO: 73 (with underlined CDRs):

QVQLVRSGAEVKKPGASVKVSCKAS<u>GYSFTNYA</u>ISWVRQAPGQGLEWMGW<u>ISTYNGNPK</u>
YAQKLQGRVTMTTDTSTSTAYMDLRSLRSDDTAVYYC<u>ARLPFYFYDSSGYDAFDI</u>WGQG
TMVTVSS

K light chain amino acid sequence SEQ ID NO: 74 (with underlined CDRs):

IQMTQSPSAVSASVGDRVTITCRAS<u>QGISDYL</u>VWFQQKPGKVPKRLIY<u>GAS</u>SLQSGVPS
RFSGSGSGTEFTLTISSLQPEDFATYYC<u>LQHKSYPYT</u>FGQGTKLEIK

Monoclonal antibody S.2.3.G7

Heavy chain amino acid sequence SEQ ID NO: 75 (with underlined CDRs):

QVQLVESGGGLVKPGGSLRLSCAASGFTFSDYYMSWVRQAPGKGLEWVPYISSSGSSIN
YADSVKGRFTISRDNAKNSLYLQMNNLRAEDTAVYYCARDPGPYYASGSYTTFYYRYGM
DVWGQGTTVTVSS

K light chain amino acid sequence SEQ ID NO: 76 (with underlined CDRs):

EIVLTQSPATLSLSPGERATLSCRASQSVSSYLAWYQHKPGQAPRLLIYDASNRATGIP
ARFSGSGSGTDFTLTISSLEPEDFAVYYCQQRSNWPPLTFGGGTKVEIK

Monoclonal antibody S.2.4.D2

Heavy chain amino acid sequence SEQ ID NO: 77 (with underlined CDRs):

QVQLVESGGGVVQPGGSLRLSCAASGFTFSSYAMHWVRQAPGKGLEWVAYIQYDGTNKF
YADSVKGRFTISRDNSKNTLYLQMNSLRVEDSAVFYCAKSRGSGSYSFFFHHWGQGTLV
TVSS

K light chain amino acid sequence SEQ ID NO: 78 (with underlined CDRs):

DIQMTQSPFSLSASVGDRVTITCRASPGISNYLAWYQQKPGKVPKLLIYAASTLQSGVP
SRFSGSGSGTDFTLTITSLQPEDVATYYCQKYNNAPFTFGPGTKVDIK

Monoclonal antibody S.2.4.E3

Heavy chain amino acid sequence SEQ ID NO: 79 (with underlined CDRs):

EVQLLESGGDLVQPGGSLRLSCTASGFTFSSYAMSWVRQAPGKGLEWVSRISGSGSGTF
YRDSVKGRFTVSRDNPNNTLFLQMNSLRAEDTAVYYCAKDGPYYDILTGPGHPSYYYG
MDVWGQGTTVTVSS

K light chain amino acid sequence SEQ ID NO: 80 (with underlined CDRs):

IQMTQSPSSVSASVGDRVTITCRASQHVSSSLVWYQQKPGRAPRLLIFAASKLQSGVSS
RFSGSGSGTDFTLTISSLQPEDFATYYCQQANSFPYTFGQGTKLEIK

Monoclonal antibody S.2.4.E8

Heavy chain amino acid sequence SEQ ID NO: 81 (with underlined CDRs):

EVQLVESGGVVVQPGGSLRVSCAAS<u>GFTFDDYAMH</u>WVRQAPGRGLEWVS<u>VISWDASSTY</u>
<u>YADSVKG</u>RFTISRDNSKHSLFLQMNSLRPEDTAFYFC<u>AKDMTPYGSGSPDY</u>WGQGTLVT
VSS

K light chain amino acid sequence SEQ ID NO: 82 (with underlined CDRs):

DIQMTQSPSSLSASVGDRVVITCRAS<u>QSISGYLN</u>WYQQKPGKAPKLLIH<u>GTSSLQS</u>GVP
SRFSGSGAGTDFTLTISSLQPEDFATYFC<u>QQSYSAPLWT</u>FGQGTKVEIK

<u>Monoclonal antibody S.3.1.B7</u>

Heavy chain amino acid sequence SEQ ID NO: 83 (with underlined CDRs):

QVQLVESGGGVVQPGGSLRLSCAAS<u>GFTFRNYALH</u>WVRHTPGKGLEWVA<u>VIWYDENSKY</u>
<u>YAESVEG</u>RFTVSRDNSKNTLFLQMNNLRAADAGVYYC<u>ARDPGSTSDSSGYYGIDF</u>WGQG
TLVTVSS

K light chain amino acid sequence SEQ ID NO: 84 (with underlined CDRs):

DVVMTQSPLSLPVTLGQPASISCRSS<u>QSLVYSDGNTYLN</u>WFHQRPGQSPRRLIY<u>RVSKR</u>
<u>DS</u>GVPDRFSGSGSGNDFTLKISRVEAEDVGVYYC<u>MQGTHWPLT</u>FGGGTKVEIK

<u>Monoclonal antibody S.3.1.E2</u>

Heavy chain amino acid sequence SEQ ID NO: 85 (with underlined CDRs):

QVQLQESGPGLVKPSETLSLTCTVS<u>GGSITSDHWT</u>WIRQPPGKGLEWIAY<u>MEHNGSPTY</u>
<u>NPSVRS</u>PVAMSLDTSKNQLSLKVYSVTAADSAVYYC<u>ARAATSSAIDS</u>WGQGTLVTVSS

K light chain amino acid sequence SEQ ID NO: 86 (with underlined CDRs):

DIQMTQSPXTXSASVGDRVTITCRAS<u>QSISGGLA</u>WYQQKAGKAPKLLIY<u>KAS</u>SLESGVP
SRFSGSASGTEFTLTISSLQPDDFATYYC<u>HQYNERWT</u>FGQGTKVEIK

<u>Monoclonal antibody S.3.1.G5</u>

Heavy chain amino acid sequence SEQ ID NO: 87 (with underlined CDRs):

RCTSSQLQQWGAGLLKPSETLSLTCSVY<u>GESFSGYY</u>WNWIRQPPGKRLEWIGE<u>INHSGS</u>

<u>A</u>NYNPSLKSRVSILVDTSRHQIFLRLSSVTAADTAVYYC<u>ARAYGSGSYTWGWAPFDYW</u>G

QGSLVTVSS

K light chain amino acid sequence SEQ ID NO: 88 (with underlined CDRs):

DIQLTQSPGTLSLSPGERATLSCRAS<u>QSISSTFLA</u>WYQQIPGQAPRLLIY<u>GAS</u>SRAIGI

PDRFSGSGSGTDFTLTISRLEPQDFAVYYC<u>QQYGRSPHT</u>FGQGTKLEIK

<u>Monoclonal antibody S.3.2.C7</u>

Heavy chain amino acid sequence SEQ ID NO: 89 (with underlined CDRs):

QVQLVESGGALVKPGESLRLSCVGS<u>GFTFSDFYMS</u>WIRQAPGKGLEWVS<u>AISSSGSNIY</u>

HGDSLKGRFTISRDNAKNSMYLQMNSLRAEDTAVYYC<u>ARFLNRYYGSGSYGYYYHAVDV</u>

<u>W</u>GQGTTVTVSS

L light chain amino acid sequence SEQ ID NO: 90 (with underlined CDRs):

TGSWAQSALTQPASVSGSPGQSITIPCTGT<u>SSDVGGHNYVS</u>WYQHHPGKVPQLMIY<u>NVI</u>

NRPSGVSNRFSGSKSGNTASLIISGLQAEDEAEYYC<u>SSYIGTNNFGVYV</u>FGTGTKVTV

<u>Monoclonal antibody S.3.2.D9</u>

Heavy chain amino acid sequence SEQ ID NO: 91 (with underlined CDRs):

EVQLVESGGGLVQPGGSQRLSCVVS<u>GFTVSTFYMS</u>WVRQAPGKGLEWVS<u>VIFSGDSTYY</u>

ADSVKGRFTISRDDSKNILYLQMNSLRAEDTAVYFC<u>ARSLKGAYSTSWYFYYAMDV</u>WGQ

GTTVTVSS

K light chain amino acid sequence SEQ ID NO: 92 (with underlined CDRs):

DIVMTQSPATLSLPPGERATLSCRAS<u>RSISSNVA</u>WYQQKPGQAPRLLIY<u>DAS</u>NRATGIP

ARFSGSGSGADFTLTISSLEPEDFAFYYC<u>QQRSSLPPLT</u>FGGGTKLEIK

<u>Monoclonal antibody S.8.1.C12</u>

Heavy chain amino acid sequence SEQ ID NO: 93 (with underlined CDRs):

QVQLVQSGAEVKKPGSSVKVSCKAS<u>GGTFSSYV</u>IGWVRQAPGQGLEWMGG<u>IIPIFGTTN</u>
YAQKSQGRVTITADESSSTAYMELSGLRSEDTAVYYC<u>AGLSQWELMIR</u>WGQGTLVTVSS

K light chain amino acid sequence SEQ ID NO: 94 (with underlined CDRs):

EIVLTQSPGTLSLSPGERATLSCRAS<u>QSISSSY</u>LAWYQQRPGQAPRLLIY<u>GAS</u>SRATGV
PDRFSGSGSGTDFTLTISRLEPEDFAVYYC<u>QQYGLSPAWT</u>FGQGTKVEIK

Table 34 - Binding of mAb S.8.1.C12 (expressed in CHO cells and purified) to RBD, Spike and N proteins of the SARS-CoV-2 virus by Surface Plasm Resonance (SPR), with units in RU (resonance units):

| S.8.1.C12 | | RU |
|---|---|---|
| RBD | WT | 36 |
| | E484K | 18 |
| | N501Y | 14 |
| | BETA | 31 |
| SPIKE | WT | 6 |
| | MUTATED | 2 |
| N | WT | 0 |
| | MUTATED | 1 |

Monoclonal antibody S.8.1.C2

Heavy chain amino acid sequence SEQ ID NO: 95 (with underlined CDRs):

EVQLVESGGGLVQPGGSLRLSCVVS<u>GIIVSNNYM</u>SWVRQAPGKGLEWVAV<u>IYSGGST</u>FY
ADSVKGRFTISRHNSKNTLYLQMNSLRTEDTALYYC<u>ARDLGNSEFDY</u>WGQGTLVTVSS

K light chain amino acid sequence SEQ ID NO: 96 (with underlined CDRs):

DIQLTQSPSSLSASVGDRVTITCRAS<u>QGISTY</u>LAWYQQKPGKAPKLLIY<u>AAS</u>TLQSGVP
SRFSGSGSGTDFTLTISSLQPEDFATYYC<u>QQLNSYPSSYT</u>FGQGTKLEIK

Table 35 - Binding of mAb S.8.1.C2 (expressed in CHO cells and purified) to the RBD, Spike and N proteins of the SARS-CoV-2 virus by Surface Plasm Resonance (SPR), with units in RU (resonance units):

| S.8.1.C2 | | RU |
|---|---|---|
| RBD | WT | 247 |
| | E484K | 135 |
| | N501Y | 110 |
| | BETA | 0 |
| SPIKE | WT | 10 |
| | MUTATED | 1 |

(continued)

| S.8.1.C2 | | RU |
|---|---|---|
| N | WT | 0 |
| | MUTATED | 1 |

Monoclonal antibody S.9.1.B7

Heavy chain amino acid sequence SEQ ID NO: 97 (with underlined CDRs):

        EVQLVESGGGLVQPGGSLRLSCAASGFTVSSNYMSWVRQAPGKGLEWVSVIYSG
    GSTFYADSVKGRFTISRDNSKNTLYLQMNSLRAEDTAVYYCARDLRGYNDYWGQGTLVT
    VSS

K light chain amino acid sequence SEQ ID NO: 98 (with underlined CDRs):

        IQMTQSPSSVPASVGDRVTITCRASQGISSWLAWYQQKPGKAPKLLIYAASSLQ
    SGVPSRFSGSGSGTDFTLTISSLQPEDFATYYCQQTNDFPPALGQGTKVEIKRT

Table 36 - Binding of mAb S.9.1.B7 Binding of mAb S.2.2.G8 (expressed in CHO cell and purified) (expressed in CHO cell and purified) to the RBD, Spike and N proteins of the SARS-CoV-2 virus by Surface Plasmonic Resonance (SPR), with units in RU (resonance units):

| S.8.1.B7 | | RU |
|---|---|---|
| RBD | WT | 187 |
| | E484K | 70 |
| | N501Y | 114 |
| | BETA | 3 |
| | DELTA | 70 |
| SPIKE | WT | 7 |
| | MUTATED | 2 |
| N | WT | 0 |
| | MUTATED | 0 |

Monoclonal antibody S.9.1.G7

Heavy chain amino acid sequence SEQ ID NO: 99 (with underlined CDRs):

    EVQLVETGGGLIQPGGSLRLSCAASGLTVSSNYMSWVRQAPGKGLEWVSVIYAGGSTFY
    ADSVKGRFTISRDNSKNTLYLQMNGLRADDTAVYYCARDLVVYGMDVWGQGTTVTVSS

K light chain amino acid sequence SEQ ID NO: 100(with underlined CDRs):

    DIQLTQSPSFLSASVGDRVTITCRASQGISSYLAWYQQKPGKAPKLLIYAASTLQSGVP

SRFSGSGSGTEFTLTISSLQPEDFATYYC<u>QHLNSYPA</u>FGQGTKVEIK

Table 37 - Binding of mAb S.9.1.G7 (expressed in CHO cells and purified) to the RBD, Spike and N proteins of the SARS-CoV-2 virus by Surface Plasm Resonance (SPR), with units in RU (resonance units):

| S.9.1.G7 | | RU |
|---|---|---|
| RBD | WT | 210 |
| | E484K | 99 |
| | N501Y | 118 |
| | BETA | 235 |
| | GAMMA | 116 |
| | DELTA | 332 |
| | OMICRON | 25 |
| SPIKE | WT | 11 |
| | MUTATED | 4 |
| | GAMMA | 11 |
| | DELTA | 58 |
| | OMICRON | 6 |
| N | WT | 3 |
| | MUTATED | 3 |

<u>Monoclonal antibody S.9.3.A7</u>

Heavy chain amino acid sequence SEQ ID NO: 101 (with underlined CDRs):

QLQLQESGPGLVKPSETLSLTCTVS<u>GGSISSSSYY</u>WGWIRQPPGKGLEWIAS<u>LFYSGST</u> YYNPSLKSRVTISVDTSKNRFFLIPTSVTAADTAVYYC<u>ARDSSGSYSIDY</u>WGQGTLVTV SS

L light chain amino acid sequence SEQ ID NO: 102 (with underlined CDRs):

QSVLTQPPSVSGAPGQRVTISCSGS<u>SSNIGAGYD</u>VHWYQLLPGTAPKLLIY<u>GNS</u>NRPSG VPDRFSGSKSGTSASLAITGLQAEDEADYYC<u>QSYDTSLSALYV</u>FGTGTKVTVL

Table 38 - Binding of mAb S.9.3.A7 (expressed in CHO cells and purified) to the RBD, Spike and N proteins of the SARS-CoV-2 virus by Surface Plasm Resonance (SPR), with units in RU (resonance units):

| S.9.3.A7 | | RU |
|---|---|---|
| RBD | WT | 16 |
| | E484K | 15 |
| | N501Y | 10 |
| | BETA | 8 |

(continued)

| S.9.3.A7 | | RU |
|---|---|---|
| SPIKE | WT | 20 |
| | MUTATED | 17 |
| N | WT | 4 |
| | MUTATED | 7 |

Monoclonal antibody S.9.4.A9

Heavy chain amino acid sequence SEQ ID NO: 103 (with underlined CDRs):

QVQLVQSGAEVKKPGASVKVSCKASGYTFISYGISWVRQAPGQELEWMGWISAYNGNTN
YAQKLQGRVTMTRDTSTTTAYMELRSLRSDDTAVYYCARVLGGLWFGEPKARHSDYWGQ
GTLVTVSS

K light chain amino acid sequence SEQ ID NO: 104 (with underlined CDRs):

IQMTQSPSSLSASVGDRVTITCRASQGINNYLAWFQQKPGKAPKSLIYAASSLHSGVPS
RFSGSGSGTDFTLTISSLQPEDFATYYCQQYNTYPITFGQGTRLEIK

Table 39 - Binding of mAb S.9.4.A9 (expressed in CHO cells and purified) to the RBD, Spike and N proteins of the SARS-CoV-2 virus by Surface Plasmon Resonance (SPR), with units in RU (resonance units), neutralization of SARS-CoV-2 by VNT100 (title of the purified S.9.4.A9 mAb dilution) and affinity kinetics (KD):

| S.9.3.A9 | | RU | KD (M) |
|---|---|---|---|
| RBD | WT | 16 | |
| | E484K | 4 | |
| | N501Y | 4 | |
| | BETA | 2 | |
| | GAMMA | 3 | 2, 54E-10** |
| | DELTA | 4 | 3, 16E-11*** |
| | OMICRON | 1 | 7, 96E08*** |
| SPIKE | WT | 23 | |
| | MUTATED | 20 | |
| | GAMMA | 18 | |
| | DELTA | 101 | |
| | OMICRON | 41 | |
| N | WT | 0 | |
| | MUTATED | 0 | |
| *dissociation in 360sec<br>**dissociation in 36000sec<br>***did not dissociate until 36000sec | | | |

Monoclonal antibody S.9.4.C6

Heavy chain amino acid sequence SEQ ID NO: 105 (with underlined CDRs):

QVQLVQSGSELKKPGASVKVSCKAS<u>GYTFTNYAMN</u>WVRQAPGQGLEWMG<u>WIDTNAGNPT
YAQGFTG</u>RFVFSLDTSVSTAYLQISSLKPEDTAVYFC<u>ARVQGVGVVTKSYYYYYAMDV</u>W
GQGTTVTVSS

L light chain amino acid sequence SEQ ID NO: 106 (with underlined CDRs):

SYELTQSPSVSVAPGKTARITCGGN<u>DIGSKS</u>VHWYQQRPGQAPVLVVY<u>DDT</u>DRPSGIPE
RFSGSNSGNTATLTISRVEAGDEADYYC<u>QVWDSSGV</u>FSGGTKLTVL

Table 40 - Binding of mAb S.9.4.C6 (expressed in CHO cells and purified) to the RBD, Spike and N proteins of the SARS-CoV-2 virus by Surface Plasm Resonance (SPR), with units in RU (resonance units):

| S.9.4.C6 | | RU |
|---|---|---|
| RBD | WT | 98 |
| | E484K | 60 |
| | N501Y | 57 |
| | BETA | 58 |
| | GAMMA | 125 |
| | DELTA | 375 |
| | OMICRON | 45 |
| SPIKE | WT | 10 |
| | MUTATED | 8 |
| | GAMMA | 11 |
| | DELTA | 97 |
| | OMICRON | 22 |
| N | WT | 0 |
| | MUTATED | 6 |

Monoclonal antibody S.11.1.D4

Heavy chain amino acid sequence SEQ ID NO: 107 (with underlined CDRs):

QVQLVESGGGVVQPGRSLRLSCVAS<u>GFSFSTYTMN</u>WVRQAPGKGLEWVT<u>KISSDGTLKD
YADSVKG</u>RFTVSRDNYESTLYLEMNSLRTEDTAVYYC<u>ARTSGRGSEDRGRFDP</u>WGQGTL
VTVSS

K light chain amino acid sequence SEQ ID NO: 108 (with underlined CDRs):

DIVMTQSPLSLPVTPGESASISCRSS<u>QSLLHSNGYNYL</u>DWYLQKPGQSPQLLIY<u>LGSNR</u>
ASGVPDRFSGSGSGRDFTLKISRVEAEDVGVYYC<u>MQSLQTFT</u>FGPGTKVDIK

Monoclonal antibody S.11.1.E10

Heavy chain amino acid sequence SEQ ID NO: 109 (with underlined CDRs):

EVQLVESGGGLVQPGRSLRLSCEVS<u>GFTFEDYAMH</u>WVRQPPGKGLEWVSG<u>ISWNGDSIG</u>
<u>YAGA</u>VRGRFTISRDNAKNSLSLQMNSLRVEDTALYYC<u>VKGRYVDWTNPLDV</u>WGQGTTVT
VSS

L light chain amino acid sequence SEQ ID NO: 110 (with underlined CDRs):

SYELTQPPPVSVAPGKTARITCGGD<u>NIGGKS</u>VHWYQQKPGLAPVMVVY<u>DDS</u>ARPSGIPE
RFSGSNSGNTATLTITRAEAGDEADYYC<u>QVWDTGSDPL</u>VFGTGTKVTVL

Monoclonal antibody S.11.1.F1

Heavy chain amino acid sequence SEQ ID NO: 111 (with underlined CDRs):

QLQLQESGPGLVKPSETLSLTCTVS<u>GGSISSSSYYW</u>GWIRQTPGKGLEWIGS<u>IYYSGST</u>
<u>YYR</u>PSLKSRVTISVDTSKNQFSLKLSSVTAADTAVYYC<u>ARLARFLEWQPIVSFDY</u>WGQG
TLVTVSS

L light chain amino acid sequence SEQ ID NO: 112 (with underlined CDRs):

QSALTQPASVSGSPGQSIAISCTGT<u>SSDVGSYNYVS</u>WYQQHPGKAPKLMIY<u>EVS</u>KRPSG
VSNRFSGSKSGNTASLTISGLQAEDEADYYC<u>CSYAGSSTVL</u>FGGGTKLTVL

Monoclonal antibody S.11.2.B6

Heavy chain amino acid sequence SEQ ID NO: 113 (with underlined CDRs):

EVQLVESGGGLVQPGGSLRLSCAAS<u>EIIVSRNYMN</u>WVRQAPGKGLEWVSV<u>MYPGGSTFY</u>
<u>A</u>NSVKGRFTISRDNSQNTLFLQMNSLRAEDTAVYYC<u>ARDRGNDAFDV</u>WGQGTMVTVSS

K light chain amino acid sequence SEQ ID NO: 114 (with underlined CDRs):

DIQLTQSPSFLSASVGDRVTITCRAS<u>QGISTYLA</u>WYQQKPGKAPNLLIY<u>AAS</u>TLHSGVP
SRFSGSGSGTEFTLTISSLQPEDFATYYC<u>QQLNGYPGSNF</u>GQGTKVEIK

Monoclonal antibody S.11.2.E5

Heavy chain amino acid sequence SEQ ID NO: 115 (with underlined CDRs):

EVQLVESGGGLIQPGGSLRLSCAAS<u>EIIVSSNYMN</u>WVRQAPGRGLEWVS<u>VIYSGGSTFY</u>
ADSVKGRFTISRDNSKNTLYLQMNSLRADDTAIYFC<u>ARDPYGGAFDV</u>WGQGILVTVSS

K light chain amino acid sequence SEQ ID NO: 116 (with underlined CDRs):

DIQMTQSPSTLSASVGDRVTITCRAS<u>ESFSNWLA</u>WYQQKPGKAPKLLIY<u>QAS</u>TLESGVP

SRFSGSGSGTEFTLTISSLQPEDFATYYC<u>QQYNSGYT</u>FGQGTKLEIK

Monoclonal antibody S.11.2.F5

Heavy chain amino acid sequence SEQ ID NO: 117 (with underlined CDRs):

QVQLVESGGGVVQPGRSLRLSCAAS<u>GFTFSSYVIH</u>WVRQAPGKGLEWVAV<u>ISHDGSNKY</u>
YADSVKGRFTISRDNSKNTLYLQMNSLRAEDTAVYYC<u>ARDRELGKSYGLDAYDI</u>WGQGT
MVTVSS

K light chain amino acid sequence SEQ ID NO: 118 (with underlined CDRs):

DIQMTQSPSTLSASVGDRVTITCRAS<u>QSISSWLA</u>WYQQKPGKAPKLLIY<u>KAS</u>SLESGVP
SRFSGSGSGTEFTLTISSLQPDDFATYYC<u>QQSQT</u>FGQGTKVEIK

Monoclonal antibody S.11.5.A2

Heavy chain amino acid sequence SEQ ID NO: 119 (with underlined CDRs):

QVQLVQSGAEVKKPGSSVKVSCKAS<u>VGTFSSYAIS</u>WVRQAPGQGLEWMGR<u>IIPIFGTAD</u>
YAQKFQGRVTITADESTNTAYMELSSLRSEDTAVYYC<u>ARDLETSPLYGMEV</u>WGQGTTVT
VSS

K light chain amino acid sequence SEQ ID NO: 120 (with underlined CDRs):

DIQMTQSPSSLSASVGDRVTITCRAS<u>QSISRFLN</u>WYQQKPGKAPKLLIY<u>ATS</u>NLQTGVP
SRFSGSGSGTDFTLTISSLQPEDFATYFC<u>QQTYNTPHT</u>FGQGTKLEIK

Monoclonal antibody S.11.5.C7

Heavy chain amino acids sequence [89] SEQ ID NO: 121 (with underlined CDRs):

QVQLQESGPGLVKPSQTLSLTCSVSGDSISINAYYWSWIRQHPGRGLEWIGYMDYSGTT
SYSPSLKSRVSISVDTSKNQFSLKLSSVTVADTAVYYCARVGFSYGNPLDAFDVWGQGT
MVTVSS

L light chain amino acid sequence SEQ ID NO: 122 (with underlined CDRs):

QSVLTQSPSVSGAPGQRVTISCTGSSSNIGAGYDVHWYQQLPGTAPKIIIYANTNRPSG
VPDRFSGSKSGTSASLAITGLQAEDEADYYCQSYDSSLSGYVVFGGGTKLTVL

Monoclonal antibody S.11.6.B5

Heavy chain amino acid sequence SEQ ID NO: 123 (with underlined CDRs):

EVQLVETGGGLIQPGGSLRLSCTASGLIVSSNYMIWVRQAPGKGLEWVSVIYPGGSTFY
ADSVKGRFTISRDNSKNTLYLQMNSLRAEDTAVYYCARDLVEFGMDVWGQGTTVTVSS

K light chain amino acid sequence SEQ ID NO: 124 (with underlined CDRs):

DIQLTQSPSFLSASVGDRVTITCRASQGISNYLAWYQQKPGKAPNLLIYGAFILQSGVP
SRFSGSGSGTEFTLTISSLQPEDFATYYCQQLNSESRTFGGGTKVEIK

Monoclonal antibody S.11.6.G11

Heavy chain amino acid sequence SEQ ID NO: 125 (with underlined CDRs):

EVQLVETGGGLIQPGGSLRLSCAASGVTVSSNYMSWVRQAPGKGLEWVSVMYSGGSTFY
ADSVKGRFTISRDNSKNTLYLQMNSLRAEDTAVYYCARDLVIWGMDVWGQGTTVTVSS

K light chain amino acid sequence SEQ ID NO: 126 (with underlined CDRs):

DIQLTQSPSFLSASVGDRVTITCRASQGISSYLAWYQQKPGKAPKLLIYAASTLQSGVP
TRFSGSGSGTEFTLTISSLQPEDFATYYCQLLNSYPPTFGGGTKVEIK

Monoclonal antibody S.11.7.A11

Heavy chain amino acid sequence SEQ ID NO: 127 (with underlined CDRs):

QVQLVQSGTEVKKPGSSVKVSCMASGGSFSTSAISWVRQAPGQGLEWMGGIIPITGTAS
YAQNFQGRVTITADESTSTAYMELSSLRSEDTAVYYCARGYRYCNSTRCYPWFDPWGQG
TLVTVSS

K light chain amino acid sequence SEQ ID NO: 128 (with underlined CDRs):

DIQMTQSPSSLSASVGDRVTITCRAS<u>QSVSSY</u>LNWYQQKSGKAPKLLIY<u>AAS</u>SLQSGVP
SRFSGSGSGTDFTLTISSLQPEDFATYFC<u>QQSYRSPLT</u>FGGGTKVEIK

Monoclonal antibody S.11.7.A5

Heavy chain amino acid sequence SEQ ID NO: 129 (with underlined CDRs):

EVQLVESGGGLVQPGRSLRLSCAAS<u>GFNFDDYAMH</u>WVRQGPGKGLEWVSG<u>ISYNSDSIG</u>
YAASVKGRVTISRDNAKNSLYLQMNSLRAEDTALYYC<u>AKGRYFDWTHAMDV</u>WGQGTTVT
VSS

L light chain amino acid sequence SEQ ID NO: 130 (with underlined CDRs):

SYELTQPPSVSVAPGKTARITCGGN<u>NIGSKS</u>VNWYQQRPGQAPVLVVY<u>DDS</u>DRPSGIPE
RFSGSNSGNTATLTISRVEAGDEADYYC<u>QVWDTGSDPLV</u>FGTGTKVTVL

Monoclonal antibody S.11.7.C8

Heavy chain amino acid sequence SEQ ID NO: 131 (with underlined CDRs):

QVQLVQSGAEVKKPGSSVKVSCKAS<u>GGTFSTYAFN</u>WVRQAPGQGLEWMGG<u>IIPMFGTTH</u>
<u>DALGFQG</u>TNYAQRFQGRLTITADQSTNTAYMELSGLRSDDTAVYYC<u>ARGVPVWGSNGPL</u>
<u>GYRAMDV</u>WGQGTTVTVSS

K light chain amino acid sequence SEQ ID NO: 132 (with underlined CDRs):

DIQLTQSPSFLSASVGDRVTITCRAS<u>QGISSY</u>LAWYQQKPGKAPKLLIY<u>ASS</u>TLQSGVP
SRFSGSESGTEFTLTISSLQPEDFATYYC<u>QQLNSYPLT</u>FGGGTKVEIK

Monoclonal antibody S.11.7.F5

Heavy chain amino acid sequence SEQ ID NO: 133 (with underlined CDRs):

QVQLVQSGAEVKKPGSSVKVSCKAS<u>GGTFSSHAFS</u>WVRQAPGQGLEWMGG<u>IIPIFGTSH</u>
YAQKFQGRVTITADESTSTAYMELISLRSDDTAVYYC<u>ARYPYDILTGYLDY</u>WGQGTLVT
VSS

L light chain amino acid sequence SEQ ID NO: 134 (with underlined CDRs):

SYELTQPPSVSVAPGKTARITCGGA<u>NIGSKN</u>VHWYQQKPGQAPVLVVY<u>DDS</u>DRPSGIPE
RFSGSNSGNTATLTISRVEAGDEADYYC<u>QVWDSGNFWV</u>FGGGTKLTVL

Monoclonal antibody S.11.7.G2

Heavy chain amino acid sequence SEQ ID NO: 135 (with underlined CDRs):

QVQLVESGXGVVQPGRSLRLSCAASGFTFSNYAIHWVRQAPAKGLEWVAVILSDGSGKF

YADSVKGRFTISRDNSKNTLYLQMNSLRPDDTALYYCARDQGELTTYFDYWGQGTLVTV

SS

K light chain amino acid sequence SEQ ID NO: 136 (with underlined CDRs):

DIQMTQSPSSLSASVGDRVTITCRASQSIGKYLNWYQQKPGKAPKLLIYVASNLQSGVP

SRFSGSGSGTDFTLTISSLQPEDFATYYCQQSYETPPWTFGQGTKVEIK

Monoclonal antibody S.11.7.G3

Heavy chain amino acid sequence SEQ ID NO: 137 (with underlined CDRs):

EVQLVESGGGLFQPGGSLRLSCEVSGFPFSNYWMNWVRQAPGKGLVWISRISSD

GSSATYADSVRGRLIISRDNAKNTLYLQMNSLRADDTAVYYCARSEYLRGWFDPWGQGT

LVTVSS

L light chain amino acid sequence SEQ ID NO: 138 (with underlined CDRs):

NFMLTQPQSVSASPGKTVTISCTGSGGDVINNYVQWYQQRPGSAPTTVIFEDSQRPSGV

PDRFSGSIDSSSNSASLTISGLKTEDEADYYCQSFDSRVHVVFGGGTKLIVL

Monoclonal antibody S.11.7.H6

Heavy chain amino acid sequence SEQ ID NO: 139 (with underlined CDRs):

QVQLVQSGAEVKKPGASVKISCEASGYSFTSYYMHWVRQAPGQGLEWMGIIYPSGGRTD

YAQKFQGRVTVTRDTSTSTVYLELTSLRSEDTAVYYCARDAKDIVGATLPGCHWGQGTL

VTVSS

K light chain amino acid sequence SEQ ID NO: 140 (with underlined CDRs):

DIVMTQSPDSLAVSLGERATINCKSSQSLLYISNNKNYLAWYQQKPGQPPKLLIYWAST

RESGVPDRFSGSGSGTDFTLTISSLQAEDVAVYYCQHYYSSPITFGQGTRLEIK

Monoclonal antibody S.11.3.D10

Heavy chain amino acid sequence SEQ ID NO: 141 (with underlined CDRs):

EVQLVQSGAEVKKPGESLKISCKAS<u>GYIFTNYWIA</u>WVRQMPGKGLEWMGI<u>IYPDDSDTR
YGPSFQG</u>QVTISADKSISTAYLQLSSLKASDNAMYYC<u>ARHWRPTIAARPGYSYYMDV</u>WG

KGTTVTVSS

K light chain amino acid sequence SEQ ID NO: 142 (with underlined CDRs):

DIQMTQSPSSXSASVGDRVTITCQAS<u>QDIDIY</u>LNWYQQTPGKPPKLLIF<u>DASN</u>LETGVP
SRFSGSGSGTDFSFTISRLQPEDTATYYC<u>QQSDSLPLT</u>FGGGTKVEIK

Monoclonal antibody S.11.3.G9

Heavy chain amino acid sequence SEQ ID NO: 143 (with underlined CDRs):

QVQLVESGGGVVQPGRSLRLSCAAS<u>GFGLNTYVMN</u>WVRQSPGKGLQWVAG<u>ISSDGSKEQ
YADSVKG</u>RFTISRDNYENALYLQMNSLRTEDTAVYYC<u>ARTSGRGFADRGRFDP</u>WGQGTL
VTVSS

K light chain amino acid sequence SEQ ID NO: 144 (with underlined CDRs):

DIVMTQSPLSLPVTPGEPASISCRSS<u>QSLLDSNGKNYLD</u>WYLQKPGQSPQLLIY<u>LGSNR
AS</u>GVPDRFSGSGSGTDFTLKISRVEAEDVGVYYC<u>MEALQTFT</u>FGPGTKVDIK

Monoclonal antibody S.11.3.H8

Heavy chain amino acid sequence SEQ ID NO: 145 (with underlined CDRs):

EVQLVETGGGLIQPGGSLRLSCAVS<u>ELIVSVNYMS</u>WVRQAPGKGLEWVSI<u>IYSGGSMFY
</u>AGSVKGRFTISRDHSKNTLYLQMNSLRVDDTAVYYC<u>ARSYGDFYLDL</u>WGQGTLVTVSS

K light chain amino acid sequence SEQ ID NO: 146 (with underlined CDRs):

EIVLTQSPATLSVSPGERATLSCRAS<u>QSVSSSVA</u>WYQQKPGQAPSLLIY<u>DVS</u>RATETP
ARFSGSGSGTEFTLTISSLQSEDFAVYYC<u>QQYINWPGT</u>FGQGTKLEIK

Monoclonal antibody S.11.4.A5

Heavy chain amino acid sequence SEQ ID NO: 147 (with underlined CDRs):

QLQLQESGPGLLKPSETLSLTCSVSG<u>GSISSSSYY</u>WGWIRQAPGKGLEWIG<u>NIFYSGTA</u>YSSPSLQSRVTMSVDTSKNQFSLNVRSVTAADTAVYYC<u>ARLARFLEGQPTASFDY</u>WGPGALVTVSS

L light chain amino acid sequence SEQ ID NO: 148 (with underlined CDRs):

QSALTQPASVSGSPGQSITISCTGT<u>SADVGSYNF</u>VSWYQQHPGKAPKLMIY<u>EVS</u>KRPSGVSYRFSGSKSGNTASLTISGLQADDEADYYC<u>YSYVGSTTVL</u>FGGGTKLTVL

Monoclonal antibody S.11.4.A6

Heavy chain amino acid sequence SEQ ID NO: 149 (with underlined CDRs):

QVQLQESGPGLVKPSQTLSLTCSVSG<u>DSISINAYY</u>WNWIRQHPGKGLEWIG<u>YMDYSGST</u>SYSPSLKSRVSISVDTSKNQFSLKLSSVTVADTAVYYC<u>ARVGFSYGNPLDAFDV</u>WGQGTMVTVSS

L light chain amino acid sequence SEQ ID NO: 150 (with underlined CDRs):

QSVLTQSPSVSGAPGQRVTISCTGS<u>SSNIGAGYD</u>VHWYQQLPGTAPKIIIY<u>ANT</u>NRPSGVPDRFSGSKSGTSASLAITGLQAEDEADYYC<u>QSYDSSLSGYVV</u>FGGGTKLTVL

Monoclonal antibody S.11.4.B5

Heavy chain amino acid sequence SEQ ID NO: 151 (with underlined CDRs):

QVQLQESGPGLVKPSQTLSLTCSVSG<u>GSVTSGGYY</u>WSWTRQYPGRGLEWVG<u>NIYHSGST</u>NYHPSLRSRTTILVDTSKDQFSLKLSSVTAADTAVYYC<u>ARDPVAGRGQFDR</u>WGQGILVTVSS

K light chain amino acid sequence SEQ ID NO: 152 (with underlined CDRs):

DIQMTQSPSSLSASVGDRVTITCQAS<u>QDISNYLN</u>WYQQKPGKAPKLLIY<u>DAS</u>HLETGVPSRFSGSGSGTHFTFTISSLQPEDVATYYC<u>QQCDNLPPPT</u>FGQGTKLEIK

Monoclonal antibody S.11.4.C6

Heavy chain amino acid sequence SEQ ID NO: 153 (with underlined CDRs):

EVQLVESGGGLIQPGGSLRLSCAAS<u>EFIVSKNYMS</u>WVRQAPGKGLEWVS<u>VIYPGGTTYY</u>ADSVKGRFTISRDSSKNTLYLQMNSLKAEDTALYYC<u>ARDFGDFYFDY</u>WGQGTLVTVSS

K light chain amino acid sequence SEQ ID NO: 154 (with underlined CDRs):

EIVMTQSPATLSVAPGERATLSCRAS<u>QSISSNLA</u>WYQQKPGQAPRLLIY<u>DAS</u>TWATDIP

ARFSGSGSGTEFTLTISSLQSEDSAVYYC<u>QQYYDRPRT</u>FGRGTKVEIK

Monoclonal antibody S.11.4.D7

Heavy chain amino acid sequence SEQ ID NO: 155 (with underlined CDRs):

QVQLVESGGGVVQPGRSLRLSCGAS<u>GFPFITYTMN</u>WVRQAPGKGLEWVAL<u>ISSDGSNKY</u>

YADFAKGRFTISRDNYDNTLYLQMNSLRSEDTAVYWC<u>ARTGGRGSEDRGRLDA</u>WGQGTL

VTVSS

K light chain amino acid sequence SEQ ID NO: 156 (with underlined CDRs):

DIVMTQSPLSLPVTPGEPASISCRSS<u>QSLLHTNGHNYLD</u>WYLQKPGQSPQLLIY<u>LGSIR</u>

ASGVPDRFSGSGSGTDFTLKISRVEAEDVGVYYC<u>MKTLQPFT</u>FGPGTKVDIK

Monoclonal antibody S.11.4.E7

Heavy chain amino acid sequence SEQ ID NO: 157 (with underlined CDRs):

QVQLVQSGAEVKKPGASVTVSCEAS<u>GFPFTNYYIH</u>WVRQAPGQGLQWMGL<u>INPSGGSTT</u>

YAQKFQGRLTMTRDTSAPIVYMELSSLTSEDTAVYYC<u>ARDSPPSVHHCGGDCLSPRYNW</u>

<u>FDPW</u>GQGTLVTVSS

K light chain amino acid sequence SEQ ID NO: 158 (with underlined CDRs):

EIVLTQSLATLSVSPGERATLSCRAS<u>QSVSSNLA</u>WYQQKPGQAPRLLIY<u>GTS</u>IRATGIP

ARFSGSGSGTEFTLTISSLQSDFVLYYC<u>QQYNNWPLT</u>FGGGTKVEIK

Monoclonal antibody S.11.4.G9

Heavy chain amino acid sequence SEQ ID NO: 159 (with underlined CDRs):

EVQLVETGGGLIQPGGSLRLSCAAS<u>GVTVSSNYMT</u>WVRQAPGKGLEWVAV<u>IYSGGSTFY</u>

ADSVKGRFTISRDNSKNTLFLQMNSLRAEDTAVYYC<u>ARDLVYYGMDV</u>WGQGTTVTVSS

K light chain amino acid sequence SEQ ID NO: 160 (with underlined CDRs):

DIQLTQSPSFLSASVGDRVTITCRAS<u>QGISNY</u>LAWYQQKPGKAPKLLIY<u>AAS</u>TLQSGVP
SRFSGSGSGTEFTLTISSLQPEDCATYYC<u>QQLNSHPPA</u>FGQGTKVEIK

<u>Monoclonal antibody S.11.5.D12</u>

Heavy chain amino acid sequence SEQ ID NO: 161 (with underlined CDRs):

QVQLVESGGGVVQPGRSLTISCVAS<u>GFSFSTFSMH</u>WVRQTPGKGLEWVAV<u>ILYDGSYKS</u>
YADSVKGRFTVSRDNSKNTLYLHMNSPKVEDSAVYYC<u>ARDFGEMTTHFDY</u>WGQGTLVTV
SS

Light chain amino acids sequence [130] SEQ ID NO: 162 K (with underlined CDRs):

DIQMTQSPSSLSASVGDRVSITCRAS<u>HSIGKS</u>LNWYQQIPGKAPKLLIF<u>DTSNLQSGVP
SRFSGSGSGTDFTLTINSLQPEDFATYYC<u>QQSYSTPPWT</u>FGQGTKVEIK

Monoclonal antibody S.11.6.B6

Heavy chain amino acid sequence SEQ ID NO: 163 (with underlined CDRs):

QVQLVQSGPEVKKPGSSVKVSCKAS<u>GFTFSSSAVQ</u>WVRQGRGHRLEWIG<u>WIVVGSGNTN</u>
YVQKFQERVTISRDMSTNTAYMELSGLRSEDTAVYYC<u>AAPSCSNVLCFDGFDL</u>WGQGTM
VTVSS

K light chain amino acid sequence SEQ ID NO: 164 (with underlined CDRs):

EIVLTQSPGTLSLSPGERATLSCRAS<u>QSVRSGYF</u>GWYQQKPGQAPRLLIY<u>NASSRATGI
PDRFSGSGSGTDFILTISGLGPEDFAVYYC<u>QQYDSSPWA</u>FGQGTKVEIK

Monoclonal antibody S.11.7.B11

Heavy chain amino acid sequence SEQ ID NO: 165 (with underlined CDRs):

EVQLVESGPGLVKPSETLSLTCTVS<u>GVSIRSYYWS</u>WIRQPPGKGLEWIG<u>YIFYSGSTNY</u>
NPSLKSRVTISVDTSKNQFSLKLSSVTAADTAVYYC<u>ASLGYGDKSWYFEL</u>WGRGTLVTV
SS

L light chain amino acid sequence SEQ ID NO: 166 (with underlined CDRs):

SYELTQPPSVSVAPGMTARITCGGS<u>NIETKS</u>VHWYQKKPDQAPVLVAY<u>DDS</u>DRPSGIPE
RFSGSNSGNTATLTISRVEAGDEADYYC<u>QVWDSSSDHGV</u>FGGGTKLTVL

Monoclonal antibody S.11.7.D12

Heavy chain amino acid sequence SEQ ID NO: 167 (with underlined CDRs):

QVQLVESGGGMVQPGRSLRLSCAAS<u>GFPFITYTMH</u>WIRQAPGKGLEWVAG<u>VSSDGSDVY</u>
SADSVKGRLTISRDNFVNTLHLQMNSLKTDDTAVYYC<u>ARTGGRGSQDRGRFDL</u>WGQGTL
VTVSS

K light chain amino acid sequence SEQ ID NO: 168 (with underlined CDRs):

DIVMTQSPLSLPVTPGEPASISCRSS<u>QSLLHTNGHNYLD</u>WYLQKPGQSPQLLIY<u>LGSNR</u>
ASGVPDRFSGSGSGTDFTLKISRVEAEDVGVYYC<u>MQSLQSYT</u>FGPGTKVDIK

Monoclonal antibody S.11.7.E6

Heavy chain amino acid sequence SEQ ID NO: 169 (with underlined CDRs):

QVQLVQSGAEVKKPGSSVKVSCKAS<u>GGPFSIYS</u>ITWVRQAPGQGLEWMGG<u>IIPMRNTPN</u>
YAQKFQGRVTITADASTTTGYMELSSLRSEDTAVYYC<u>ARDYRYCNDTTCYGFAFDI</u>WGQ
GTMVTVSS

K light chain amino acid sequence SEQ ID NO: 170 (with underlined CDRs):

DIQMTQSPSSLSASVGDRVTITCRAS<u>QSINNFLN</u>WYQQKPGKAPKLLIY<u>AASSLQS</u>GVP
SRFGGSGSGTDFTLTISSLQPDDFATYYC<u>QQSYSSRT</u>FGQGTKLEIK

Monoclonal antibody S.11.8.D10

Heavy chain amino acid sequence SEQ ID NO: 171 (with underlined CDRs):

QVQLVESGGGVVQPGRSLRLSCAAS<u>GFTFSTYVMH</u>WVRQAPGGGLEWVAV<u>ISSDGSNRY</u>
NADSVKGRFTISRDNSKNTLYLQMNSLRTEDTAVYFC<u>ARDREVGKSYGLDGFDV</u>WGQGT
MVTVXS

K light chain amino acid sequence SEQ ID NO: 172 (with underlined CDRs):

DIQMTQSPSTLSASVGDRVTITCRAS<u>QNISPWLA</u>WYQQKPGRAPKLLIY<u>KAST</u>LESGVP
SRFSGSGSGTEFTLTISSLQPDDFATYYC<u>QQLQT</u>FGQGTKVEIK

Monoclonal antibody S.11.8.E10

Heavy chain amino acid sequence SEQ ID NO: 173 (with underlined CDRs):

QVQLVQSGPEVKKPGTSVKVSCKAS<u>GFTFSMSAVQ</u>WVRQARGQRLEWIG<u>WIVVGSGNTE</u>
LAQKFQQRVIMTRDESTGTAYMELNSLTSEDTAAYYC<u>AAPSCTNVLCFDGFDL</u>WGQGTM
VTVSS

K light chain amino acid sequence SEQ ID NO: 174 (with underlined CDRs):

EIVLTQSPGTLSLSPGERATLSCRAS<u>QSVRSSYLG</u>WYQQKPGQAPRLLMY<u>NTSSRAT</u>GI
PDRFSGSGSGTAFTLTITRLEPEDIGVYHC<u>QQYDRSPWT</u>FGQGTKVEIK

Monoclonal antibody S.11.8.H11

Heavy chain amino acid sequence SEQ ID NO: 175 (with underlined CDRs):

EVQLVETGGGLIQPGGSLRLSCTTS<u>GLIVSRNYMH</u>WVRQAPGKGLEWVA<u>VIYPGGTTHY</u>
ADSVKGRFTISREHSNNTLSLQMKSLRAGDTAVYYC<u>ARDLDVLGGFDIW</u>GQGTMVTVSS

K light chain amino acid sequence SEQ ID NO: 176 (with underlined CDRs):

AIRMTQSPSSFSASTGDRVTISCRTS<u>QDINNDLA</u>WYQQKPGKAPNLLIY<u>GAST</u>LQTGVP
SRFSGSGSGTEFTLTITYVKSEDFATYYC<u>QQYSNYPRT</u>FGQGTKVEIK

Monoclonal antibody S.11.8.D6

Heavy chain amino acid sequence SEQ ID NO: 177 (with underlined CDRs):

QVQLVESGGGVVQPGRSLRLSCAAS<u>GFTFSIYGMH</u>WVRQAPGKGLEWVA<u>VMSYDGSHVY</u>
YGDSVKGRFTISRDNSKNTLYLQMNILRVEDTAVYYC<u>ARDYGDYAALDH</u>WGQGTLVTVS
S

K light chain amino acid sequence SEQ ID NO: 178 (with underlined CDRs):

DIVMTQSPDSLAASLGERATINCKSS<u>QSVLYNSNNKNYLA</u>WYQQKPGQPPKLLIY<u>WASI</u>
RESGVPDRFSGSGSGTDFTLTISSLQAEDVAFYYC<u>QPYLPTPT</u>FGPGTKVDIK

Monoclonal antibody S.11.4.A8

Heavy chain amino acid sequence SEQ ID NO: 179 (with underlined CDRs):

QVQLQESGPGLVKPSETLSLTCAVS<u>GYSISSGYYW</u>GWIRQPPGKGLEWIG<u>SMHHSGNTD</u>
YNPSLKSRATISIDTSKNQLSLKLSSVTAADTAVYYC<u>ARIMQGRIVVLISYIDFW</u>DQGS

```
                                      LVTVSS
```

L light chain amino acid sequence SEQ ID NO: 180 (with underlined CDRs):

```
QSVLTQPPSASGTPGQRVTISCSGGSSNIGRNTVDWYQQLPGTAPKLLIYSSNQRPSGV
PDRFSGSKSGTSASLAISGLQSGDEADYYCASWDDTLNGQLVFGGGTKLTVL
```

Monoclonal antibody S.8.2.B2

Heavy chain amino acid sequence SEQ ID NO: 181 (with underlined CDRs):

```
EVQLVESGGGWVQPGGSLRLSCSASGFSFSTYAMNWVRQAPGKGLEYVSGISSNGDITY
YADSVKGRFTISRDNSKNTLYLQMSSLRTEDTAIYYCVKDLGTVVTVDVFDIWGQGTMV
TVSS
```

K light chain amino acid sequence SEQ ID NO: 182 (with underlined CDRs):

```
DIQMTQSPSSLSASVGDRVTITCRPSQTINSYLNWYQQKPGKAPKLLIYAASSLQSGVP
SRFSGSGSGTDFTLTISSLQPEDFATYYCQQTYITPGTFGQGTKVEIK
```

Monoclonal antibody S.9.1.C11

Heavy chain amino acid sequence SEQ ID NO: 183 (with underlined CDRs):

```
QVQLVQSGAEVKKPGASVRVSCKTSGYTFTTYFIHWVRQAPGRGLEWMGIINPSGGSTS
YALKFQGRVTMTRDTSTSTVYMELSSLRSEDTAVYYCAREEDIVVVPAASFDYWGQGTL
VTVSS
```

K light chain amino acid sequence SEQ ID NO: 184 (with underlined CDRs):

```
EIVLTQSPATLSLSPGERATLSCRASQSVSSYFAWYQQKPGQSPRLLIYDASNRASGIP
ARFSGSGSGTDFTLTISSLEPEDFAVYYCQQRSNWPPRITFGQGTRLEIK
```

Monoclonal antibody S.9.1.D10

Heavy chain amino acid sequence SEQ ID NO: 185 (with underlined CDRs):

```
QVQLQESGPGLVKPSETLSLTCSVSGGSINNYYWSWIRQPPGKGLEWIGYIYFSGNTNY
NPSLKSRVTISIDTSKNQFSLKLSSVTAADTAVYYCAREGTGYCSGGTCYSAEEDAFDI
WGQGTMVTVSS
```

K light chain amino acid sequence SEQ ID NO: 186 (with underlined CDRs):

DIQMTQSPSSLSASVGDRVTITCRASQSISRYLNWYQQKPGKAPKLLIYTASSLQSGVP
SRFSGSGSGTDFTLTISSLQPEDFATYYCQQSHSTPWTFGQGTKVEIK

Monoclonal antibody S.9.2.B2

Heavy chain amino acid sequence SEQ ID NO: 187 (with underlined CDRs):

QVQLQESGPGLVKPSQTLSLTCTVSGGSISSGDYYWSWIRQPPGKGLEWIGYIYYSGST
YYNPSLRSRVSISVDTSKNQLSLKLRSVTAADTAVYYCARDHGGGYNWGPGFDSWGQGT
LVTVSS

K light chain amino acid sequence SEQ ID NO: 188 (with underlined CDRs):

DIQMTQSPSSLSAYVGDRVTITCRASQNISSYLNWYQQKPGKAPKFLIYAASSLQSGVP
SRFSGSGSGTNFTLTVSSLQPEDFATYYCQQSYSTLWTFGQGTKVEIK

Monoclonal antibody S.9.3.H9

Heavy chain amino acid sequence SEQ ID NO: 189 (with underlined CDRs):

EVQLVESGGGLVKPGGSLRLSCAASGFSFSSYSMNWVRQTPGKGLEWVSSISSSSTSFI
YYADSVMGRFTISRDNAKNSLYLQMNRPRAEDTAVYYCARDFTPSPRRYFDWLLPLGMD
VWGQGTTVTVSS

L light chain amino acid sequence SEQ ID NO: 190 (with underlined CDRs):

QSALTQSRSVSGSPGESVAISCTGTSSDVGAYNYVSWYQQHPGKAPKLMIYDVNKRPSG
VPDRFSGSKSGNTASLTISGLQAEDEADYYCCSYAGSYNWVFGGGTKLTVL

Monoclonal antibody S.11.1.E2

Heavy chain amino acid sequence SEQ ID NO: 191 (with underlined CDRs):

EVQLVESGGDLVQPGGSLRLSCAASGFSLSXHWMSWVRQVPGKGLEWVANIKHDESETY
YVASVKGRFTISRDNAKNSLYLQMNSLTDEDSAIYYCTREAKSGPGDYWGRGTLVTVSS

L light chain amino acid sequence SEQ ID NO: 192 (with underlined CDRs):

QSVLTQPPSASGTPGQRVTISCSGSNSNIGSRFVYWYQQLPGTAPTLLIYRDDQRPSGV
PDRFSGSKSGTSASLAISGVRSEDEADYYCAVWDDNLTGWVFGGGTKLTVL

Monoclonal antibody S.11.2.C6

Heavy chain amino acid sequence SEQ ID NO: 193 (with underlined CDRs):

QVQLVESGGGVVQPGRSLRLSCAASGFGFNFYLIHWVRQAPGKGLEWVALISFDGTNEY
YADSVKGRFIISRDNSENTLYLQMNSLRTEDTAVYYCARTGGRGSADRGRFDLWGQGTL
VTVSS

K light chain amino acid sequence SEQ ID NO: 194 (with underlined CDRs):

IVMTQSPLSLSVTPGEPASISCRSSQSLLHSNGHTYLDWYLQKPGQSPQLLIYLGSNRA
SGVPDRFSGSGSGTDFTLKISRVEAEDVGIYYCMQAIQSFTFGPGTKVDIK

Monoclonal antibody S.11.2.F11

Heavy chain amino acid sequence SEQ ID NO: 195 (with underlined CDRs):

EVQLLESGGGLVQPGGSQRLSCAASGFTFSSYPMSWVRQAPGKGLEWVSFISGSGGTIY
YADSVKGRFTISRDNSKNTLYLQMNSLRAEDTAVYYCAKDPSTAPIYYYFYMDVWGKGT
TVTVSS

L light chain amino acid sequence SEQ ID NO: 196 (with underlined CDRs):

QSALTQPASVSGSPGQSITISCTGTSSNVGGYNLVSWYQKHLGKAPKLMIFDVNKRPSG
VSNRFSGSKSGNTASLTISGLQAEDEADYYCCSYAGRSTSYVVFGGGTKLTVL

Monoclonal antibody S.11.3.B3

Heavy chain amino acid sequence SEQ ID NO: 197 (with underlined CDRs):

QVQLVESGXGVVQPGGSRRLSCAASGFGLNTYVLHWVRQPPGKGLEWVALISSDGGNEQ
FADSVKGRFTISRDNFDNKVFLQMNGLTTEDTAVYFCARTGGRGSADRGRFDLWGHGAL
VTVSS

K light chain amino acid sequence SEQ ID NO: 198 (with underlined CDRs):

DIVMTQSPLFLPVTPGEPASISCRSSQSLLHSNGYNYLDWYLQKPGQSPQLLIYLGSNR
ASGVPDRFSGSGSGTDFTLKISRVEAEDVGVYYCMEALQAFTFGPGTKVDIK

Monoclonal antibody S.11.3.G8

Heavy chain amino acid sequence SEQ ID NO: 199 (with underlined CDRs):

EVQLVESGGGLIQPGGSLRLSCAAS<u>EVIVSSNYMS</u>WVRQAPGKGLEWVS<u>VLFAGGSTFY</u>AASVKGRFTISRDDVKNTLYLQMNSLSAEDTAVYYC<u>ARDRWEGAFDI</u>WGLGTMVTVSS

K light chain amino acid sequence SEQ ID NO: 200 (with underlined CDRs):

DIQMTQSPSFLSASVGDRVTITCRAS<u>QGISNYLA</u>WYQQKPGKAPKLLIY<u>AAS</u>TLQSGVPSRFSGSGSGTEFTLTVSSLQPEDFATYYC<u>QQLNSYPKIT</u>FGPGTKVDIK

Monoclonal antibody S.11.5.D7

Heavy chain amino acid sequence SEQ ID NO: 201 (with underlined CDRs):

EVQLLESGGGLIQPGGSPRLSCAAS<u>GFTFSSSVMT</u>WVRQAPGKGLEWVS<u>AVSGTGGSIY</u>YADSVKGRFTISRDNSKETLYLQMNSLRAEDTAVYYC<u>AKEPPAARYFHHFYMDV</u>WGKGTTVTVSS

L light chain amino acid sequence SEQ ID NO: 202 (with underlined CDRs):

QSALTQPASVSGSPGQSITISCTGT<u>SSDVGSYDLVS</u>WYQQHPGKAPELMIY<u>EVT</u>KRPSGVSDRFSGSKSGNTASLTISGLQAEDEADYYC<u>CSYAGRSTSYVV</u>FGGGTKVTVV

Monoclonal antibody S.11.6.F1

Heavy chain amino acid sequence SEQ ID NO: 203 (with underlined CDRs):

QVQLQESGPGLVKASETLSLTCTVS<u>GVSIRSYYWS</u>WIRQPPGKGLEWIG<u>YIFYSGSTNY</u>NPSLKSRVTMSVDTSKNQFSLKLSSVTAADTAVYYC<u>ASLGYGDASWYFDL</u>WGRGTLVTVSS

L light chain amino acid sequence SEQ ID NO: 204 (with underlined CDRs):

SYELTQPPSVSVAPGKTARITCGGN<u>NIGSKSV</u>HWYLQKPGQAPVLVVY<u>DDS</u>DRPSGIPERFSGSNSGNTATLTISRVEAGDEADYYC<u>QVWDTSSDHGV</u>FGGGTKLTVL

<u>Monoclonal antibody S.11.6.B11</u>

Heavy chain amino acid sequence SEQ ID NO: 205 (with underlined CDRs):

QVQLQESGPGLVTPSGTLSLTCVVS<u>GGSVSSTNW</u>WSWVRQPPGKGLEWIG<u>EIFQSGNTH</u>YNPSLKSRVSMSLDKSNNHFSLTLTSVTAADTAVYYC<u>ARGPLLGGGNYYLDV</u>WGKGTTVTVSS

L light chain amino acid sequence SEQ ID NO: 206 (with underlined CDRs):

QSALTQPASVSGSPGQSITISCTGT<u>SSDVGSYNLV</u>SWYRQYPGKAPSLMIY<u>QVT</u>KRPSG
VSDRFSGSKSGNTASLTIAGLQVEDEGDYYC<u>CSYAGSDTQLL</u>FGGGTKLTVL

<u>Monoclonal antibody S.11.7.B3</u>

Heavy chain amino acid sequence SEQ ID NO: 207 (with underlined CDRs):

QVQLVQSGAEVKKPGASVKVSCKAS<u>GYTFTNYHLH</u>WVRQAPGQGPEWMGI<u>FYPSGGSTG</u>
YARTFQGRVTMTRDTSTSTVYMELSSLRSDDTAVYYC<u>AREGGSGETYFDY</u>WGQGTLVTV
SS

L light chain amino acid sequence SEQ ID NO: 208 (with underlined CDRs):

SYELTQPPSVSVAPGKPARITCGGS<u>YIGSKS</u>VHWYQQQPGQAPVLVVY<u>DDS</u>DRPSGIPE
RFSGSNSGNTATLTISRVEAGDEADYYC<u>QVWDGSSDRQVV</u>FGGGTKLTVL

<u>Monoclonal antibody S.11.7.E9</u>

Heavy chain amino acid sequence SEQ ID NO: 209 (with underlined CDRs):

EVQLLESGGGLVQPGGSLRLSCAAS<u>GFTFSSYVVS</u>WVRQAPGKGLEWVST<u>ISGIGISTY</u>
YADSVRGRFTISRDKSKNTLYLEMNSLSAEDTAVYYC<u>AKDPSAAAHYYYYYMDV</u>WGKGT
TVTVSS

L light chain amino acid sequence SEQ ID NO: 210 (with underlined CDRs):

QSALTQPASVSGSPGQSITISCTGT<u>SSDVGNYNLV</u>SWYQQHPGKAPKLMIY<u>EVS</u>KRPSG
VSNRFSGSKSGNTASLTISGLQAEDEADYYC<u>CSYAGSSTSYVL</u>FGGGTKLTVL

<u>Monoclonal antibody S.11.7.C11</u>

Heavy chain amino acid sequence SEQ ID NO: 211 (with underlined CDRs):

QVQLVESGGGVVQPGRSLRLSCAAS<u>GFIFSNYGMH</u>WVRQAPGKGLEWMAV<u>ILDDGSGKF</u>
YADSVKGRFTISRDNSKNTLYLQLNSLREDDTAVYYC<u>ARDWGSLVTLFDY</u>WGQGTLVTV
SS

K light chain amino acid sequence SEQ ID NO: 212(with underlined CDRs):

DIQMTQSPSSLSASVGDRVTITCRAS<u>QRIQTYLN</u>WYQQKPGKAPKLLIH<u>SAS</u>SLQSGVP

SRFSGSGSGTDFTLTISSLQPEDFGTYYC<u>QQSYETPPWT</u>FGQGTKVEIK

Monoclonal antibody S.11.8.F10

Heavy chain amino acid sequence SEQ ID NO: 213 (with underlined CDRs):

EVQLVETGGGLIQPGGSLRLSCAAS<u>GVTVSRNYMY</u>WVRQAPGKGLEWVS<u>VIYSGGSTFY</u>

ADSVKGRFTISRDNSNNTLYLQMNSLRAEDTAVYYC<u>ARDAVVYGMDV</u>WGQGTTVTVSS

K light chain amino acid sequence SEQ ID NO: 214 (with underlined CDRs):

DIQMTQSPSSLSASVGDRVTITCRAS<u>QSISTYLN</u>WYQQKPGKAPKLLIY<u>AAS</u>SLQSGVP

SRFSGSGSGTDFTLTISSLQPEDFATYYC<u>QQSYSTPPFT</u>FGPGTKVDIK

Monoclonal antibody S.12.1.A4

Heavy chain amino acid sequence SEQ ID NO: 215 (with underlined CDRs):

QVQLVQSGTEVKKPGSSVKVSCKAS<u>GGTFSSSAIS</u>WVRQAPGQGLEWMGG<u>IITIFGTSN</u>

<u>IA</u>QKFQGRVTITADAPSSTAYMGLSSLRSEDTAVYYC<u>ARSRGRYYSSSRVYHYYGMDV</u>W

GQGTTVTVSS

K light chain amino acid sequence SEQ ID NO: 216 (with underlined CDRs):

DIQMTQSPSSLSASVGDRVTITCQAR<u>QDISNYLN</u>WYQQKPGKAPKLLIY<u>DAS</u>NLETGVP

SRFSGRGSGTHFTFTISSLQPEDIATYYC<u>QQYDNLPLT</u>FGGGTKVEIK

Monoclonal antibody S.12.1.B7

Heavy chain amino acid sequence SEQ ID NO: 217 (with underlined CDRs):

QVQLVQSGAEVKKPGSSVKVACRAS<u>GDTLSSYAIS</u>WVRQAPGQGLEWMGG<u>IIPMFGIAD</u>

YAHKFQGRVTITADGSTSTAYMELSSLRSEDTAVYYC<u>ARTLAGQLGRVNYYYGMDV</u>WGL

RGPRSPSPQ

K light chain amino acid sequence SEQ ID NO: 218 (with underlined CDRs):

DIVMTQSPLSLPVTPGEPASISCRSS<u>QSLLHSNGYNYLD</u>WYLQKPGQSPQLLIY<u>LGSNR</u>

ASGVPDRFSGSGSGTDFTLKISRVEAEDVGVYYC<u>MQALQTPPT</u>FGQGTKVEIK

Monoclonal antibody S.12.1.C10

Heavy chain amino acid sequence SEQ ID NO: 219 (with underlined CDRs):

EVQLLESGGDLIQPGGSLRLSCAASGFTFSTYAMIWARQAPGKGLEWVSSITGSGDYTY
FADSVKGRFTMSRDNSKNTVYLQMNNLRVEDTALYFCAKGGMTTAYYWGQGTLVTVSS

K light chain amino acid sequence SEQ ID NO: 220 (with underlined CDRs):

DIVMTQSPDSLAVSLGERATINCKSSQTILHNSHNRNCLAWFQQKPGQPPKLLIYWAST
RESGVPDRFSGSGSGTDFTLTINNLQAEDVAVYYCQQYYTNPRWTFGQGTKVEIK

Monoclonal antibody S.12.2.A3

Heavy chain amino acid sequence SEQ ID NO: 221 (with underlined CDRs):

EVQLVESGGGLVEPGGSLRLSCAASGFTFTSYTMNWVRQAPGKGLEWVSSISSSGRHIH
YGDSMKGRFHISRDNSKSLVFLEMNSLRAGDTAMYYCARGYDVLTGYYKIQKSRYYHMD
VWGQGTTVTVSS

L light chain amino acid sequence SEQ ID NO: 222 (with underlined CDRs):

QSVLTQPPSASGTPGQRVTVSCSGSSSNIGSNTVNWYQQIPGTAPKLLMYGNNQGASGV
PDRFSGAKSGTSASLAISGLQSEDEADYYCEAWDDSLNGRVFGTGTKVTVL

Monoclonal antibody S.12.2.C5

Heavy chain amino acid sequence SEQ ID NO: 223 (with underlined CDRs):

QVQLVQSGAEVKKPGASVKVSCKASGYTFTNYYIHWVRQAPGQGLEWMGMINPAGGSIA
YAQKFQGRVTMTRDTSTSTVYMDLSSLTSEDTAVYYCARDDFVVPAAGPFDPWGQGTLV
TVSS

K light chain amino acid sequence SEQ ID NO: 224 (with underlined CDRs):

DIQMTQSPSTLSASVGDRVTITCRASQSISSWLAWYQQKPGKAPKLLIYKASSLQSGVP
SRFSGSGSGTEFTLTISSLQPDDFATYYCQHYNGYSKTFGQGTKVEIK

Monoclonal antibody S.12.2.G11

Heavy chain amino acid sequence SEQ ID NO: 225 (with underlined CDRs):

EVQLVESGGGLVKPGGSLRLSCAASGFTFNNAWMNWVRQAPGKGLEWVGRIKSKTDGGT
TDYAAPVKGRFTISRDDSENTLFLQMNSLKTEDTAVYYCTTDDSVLWFGELPYFDYWGQ
GTLVTVSS

K light chain amino acid sequence SEQ ID NO: 226 (with underlined CDRs):

EIVLTQSPGTLSLSPGERATLSCRASQSVSSSFLVWYQQKPGQAPRLLMYGASTRATGI
PDRFSGSGSGTDFTLTISRLEPEDFAVYYCQHYGNSPPRTFGQGTKVEIK

Monoclonal antibody S.12.3.H1

Heavy chain amino acid sequence SEQ ID NO: 227 (with underlined CDRs):

EVQLVESGGGLVKPGGSLRLSCAVSGFTFSNAWMSWVRQAPGKGLEWVGRVKSKSDGGT
TDYAAPVKGRFTISRDDSKNTLYLQMNSLKTEDTAVYYCTTDDSVLWFGELSYFDYWGQ
GTLVTVSS

K light chain amino acid sequence SEQ ID NO: 228 (with underlined CDRs):

EIVLTQSPGTLSLPPGERATLSCRASQSVSSSFLAWYQQKPGQAPRLIMYGASTRATGI
PDRFSGSGSGTDFTLTISRLEPEDFAVYYCRHYGNSPPRTFGQGTKVEIK

Monoclonal antibody S.12.1.B12

Heavy chain amino acid sequence SEQ ID NO: 229 (with underlined CDRs):

EVQLVESGGGLVQPGRSLRLSCAASGFTFDDYAMHWVRQAPGKGLEWVSGISWNSGSIG
YADSVRGRFTISRDNAKNSLYLQMNSLRTEDTALYYCAKDTHQLVEGDNWFDPWGQGTL
VTVSS

K light chain amino acid sequence SEQ ID NO: 230 (with underlined CDRs):

IQMTQSPSSVSASVGDRVTITCRASQDISSWLAWYQQKPGRAPKLLIYAASSLQSGVPS
RFSGSGSGTDFTLTISSLQPEDFATYYCQQANNFPLTFGGGTKVEIK

Monoclonal antibody S.12.4.A8

Heavy chain amino acid sequence SEQ ID NO: 231 (with underlined CDRs):

EVQLVESGGGLVKPGGSLRLSCAAS<u>GFTFTNAWM</u>SWVRQPPGRGLEWVGR<u>IRSKSDGGT</u><u>T</u>DYAAPVKGRFSISRDDSKNTLYLQMNSLKTEDTAVYYC<u>TTDDSVLWFGELPYFDY</u>WGQGALVTVSS

K light chain amino acid sequence SEQ ID NO: 232 (with underlined CDRs):

EIVLTQSPGTLSLSPGERATLSCRAS<u>QSISASYLA</u>WYQQKPGQAPRLLMY<u>GAS</u>SRATGIPDRFSGSGSGTDFTLTISRLEPEDFAVYYC<u>QQYGSSPPRT</u>FGQGTKVEIK

Monoclonal antibody S.11.1.F6

Heavy chain amino acid sequence SEQ ID NO: 233 (with underlined CDRs):

QVQLQESGPGLVKPSETLSLTCSVS<u>GGSIINSAIW</u>WNWVRQTPGKGLEWIG<u>EVYHRGST</u>NYNPSLKSRVTLSLDKSKNQFSLKLTSLTAADTAVYYC<u>ASKLFDNYIRFES</u>WGQGTLVTVSS

K light chain amino acid sequence SEQ ID NO: 234 (with underlined CDRs):

EIVLTQSPGTLALSPGESGTLSCRAN<u>ESLNSNYVA</u>WLQQKPGQAPRLLIY<u>ATS</u>RATGIPVRFSGSGSGTDFTLTVSSLEPEDFAVYYC<u>QQYDRPPVT</u>FGAGTKVEIK

Monoclonal antibody S.11.3.E5

Heavy chain amino acid sequence SEQ ID NO: 235 (with underlined CDRs):

QLQLQESGSGLVKPSQTLSLTCTVS<u>GGSIGSGGYT</u>WNWIRQAPGKGLESIG<u>YIYPGGSA</u>YYNPSLKSRVTISVDGSKNQFSLKLSSVTAADTAVYYC<u>AREYGIGRGYDDMPVYRRFDL</u>WGRGALVTVSS

K light chain amino acid sequence SEQ ID NO: 236 (with underlined CDRs):

DIQMTQSPSSLSASVGDRVSITCRAS<u>QNISSYLN</u>WYQQKPGKAPELLIY<u>GAF</u>SLESGVPSRFSGSGSGTDFTLTITSLQPEDFATYSC<u>QQTYGTPRT</u>FGQGTKVEIK

Monoclonal antibody S.12.2.G6

Heavy chain amino acid sequence SEQ ID NO: 237 (with underlined CDRs):

EVQLVESGGGLVQPGRSLRLSCAASGFTFDDYAMHWVRQAPGKGLEWVSGISWNSGSIG
YADSVRGRFTISRDNAKNSLYLQMNSLRTEDTALYYCAKDTHQLVEGDNWFDPWGQGTL
VTVSS

K light chain amino acid sequence SEQ ID NO: 238 (with underlined CDRs):

IQMTQSPSSVSASVGDRVTITCRASQDISSWLAWYQQKPGRAPKLLIYAASSLQSGVPS
RFSGSGSGTDFTLTISSLQPEDFATYYCQQANNFPLTFGGGTKVEIK

Monoclonal antibody S.11.4.E4

Heavy chain amino acid sequence SEQ ID NO: 239 (with underlined CDRs):

QLQLQESGPGLVKPSETLSLTCTVSGGSLYSGGHFWGWFRQPPGKGLEWIGSVFYSGIT
YYNPSLEGRLTIFVDPAKNQFSLRLNSVTAADTAVYFCASLYDNSGLWQFDYWGQGTLV
TVSS

K light chain amino acid sequence SEQ ID NO: 240 (with underlined CDRs):

IQMTQSPSSLSASVGDRVTISCRASQGVGDDLAWYQQKPGRAPKRLIYSASSLQSGVPS
RFSGSGYGTEFTLTITSLQPEDFATYYCLQYNSYPLTFGGGTKVEIK

Monoclonal antibody S.11.5.C2

Heavy chain amino acid sequence SEQ ID NO: 240 (with underlined CDRs):

EVQLVESGGGLIQPGGSLRLSCEASGIIVSSNYMTWVRQAPGKGLEWVSVLYAGGSTFY
TDSVKGRFTISRDNSXNXLYLQMNSLRAEDTAVYYCARDLAVFGVDIWGQGTMVTVSS

K light chain amino acid sequence SEQ ID NO: 242 (with underlined CDRs):

DIQLTQSPSFLSAFVGDRVTITCRASQGISSDLAWYQQQPGKAPKLLIYAASTLQSGVP
SRFSGSGSGTEFTLTISSLQPEDFATYYCQHLNSNPGIFTFGPGTKVDIK

Monoclonal antibody S.11.8.G10

Heavy chain amino acid sequence SEQ ID NO: 243 (with underlined CDRs):

QVQLQESGPGLVTPXGTLSLTCVXSGGSVSSTNWWSWVRQPPGKGLEWIGEIFQSGNTH
YNPSLKSRLSMSLDKSNNHFSLTLTSVTAADTAVYYCARGPLLGGGNYYLDVWGKGTTV
SVSS

L light chain amino acid sequence SEQ ID NO: 244 (with underlined CDRs):

QSALTQPASVSGSPGQSITISCTGTSSDIGSYNLVSWYRQYPGEAPSLVIYQVSKRPSG
VSDRFSGSKSGNTASLTIAGLQAEDEADYYCCSYAGSDTQLFFGGGTRLTVL

Monoclonal antibody S.12.1.A5

Heavy chain amino acid sequence SEQ ID NO: 245 (with underlined CDRs):

QVQLQESGPRLVRPSQTLSLNCTVSGDSLSRNIIYWTWIRQRPGKGLEWIGYIQHTGTT
FYSPSLKSRLIMSIDTSNNQFSLSLDSLTAADTAVYYCVRGIYCGGGCYRGADHWGPGI
QVAVSS

K light chain amino acid sequence SEQ ID NO: 246 (with underlined CDRs):

DIVMTQSPLSLPVTPGEPASISCRSSQSLLHSNGYTYLDWYLQKPGQSPQLLIYLGSYR
ASGVPDRFSGSGSGTDFTLKIGRVEAEDVGIYYCMQGLQTPYSFGQGTKLEIK

Monoclonal antibody S.12.1.E10

Heavy chain amino acid sequence SEQ ID NO: 247 (with underlined CDRs):

QVQLQESGPRLVRPSQTLSLNCTVSGDSISRHIIYWTWIRQRPGKGLEWIGYIHHTGST
FYSPSLKSRLTMSIDTSNNQFSLSLGSLTAADTALYYCARGTYCGGACYRGTDHWGPGI
QVAVSS

K light chain amino acid sequence SEQ ID NO: 248 (with underlined CDRs):

DIVMTQSPLSLPVTPGEPASISCRSSQSLLHNNGYTYLDWYLQKPGQSPQLLIYLGSYR
ASGVPDRFSGSGSGTDFTLKIGRVEAEDVGIYYCMQGLQTPYSFGQGTKLEIK

Monoclonal antibody S.12.3.E12

Heavy chain amino acid sequence SEQ ID NO: 249 (with underlined CDRs):

QVQLVESGGGLVKPGGSLRLSCAASGFTFSGHYMAWIRQAPGKGLEWLSYISTSGSYTE
YVDSVKGRFTISRDNARNSLYLQMNSLRVDDTAIYYCARDHCGADCYSNWFDSWGQGTL
VTVSS

K light chain amino acid sequence SEQ ID NO: 250 (with underlined CDRs):

DIVMTQSPDSLAVSLGERAAITCKSSQSVLYSSSNRNYLAWYQQKPGQSPKLLIYWAST
RASGVPDRFSGSGSETDFTLTISSLQTEDVAVYYCQQYYNTPLTFGGGTKVEIK

In summary, the invention refers to the following aspects, as defined in the following numbered items:

1. Anti-SARS-COV-2 human monoclonal antibody, wherein the antibody comprises an antibody heavy chain and an antibody light chain, said chains comprising complementarity determining regions (CDRs) selected from (i) - (clxxv):

(i) heavy chain CDR1, CDR2 and CDR3 regions having the amino acid sequences of positions 26-33, positions 51-57 and positions 96-106 of SEQ ID NO: 1, respectively; and light chain CDR1, CDR2 and CDR3 regions with the amino acid sequences of positions 27-32, positions 50-52 and positions 89-97 of SEQ ID NO: 2, respectively;

(ii) heavy chain CDR1, CDR2 and CDR3 regions having the amino acid sequences from positions 26-33, positions 50-57 and positions 96-106 of SEQ ID NO: 3, respectively; and light chain CDR1, CDR2 and CDR3 regions with the amino acid sequences of positions 27-32, positions 50-52 and positions 89-97 of SEQ ID NO: 4, respectively;

(iii) heavy chain CDR1, CDR2 and CDR3 regions having the amino acid sequences from positions 26-33, positions 51-57 and positions 96-107 of SEQ ID NO: 5, respectively; and light chain CDR1, CDR2 and CDR3 regions having the amino acid sequences of positions 26-31, positions 49-51 and positions 88-98 of SEQ ID NO: 6, respectively;

(iv) heavy chain CDR1, CDR2 and CDR3 regions with the amino acid sequences of positions 26-33, positions 51-58 and positions 97-109 of SEQ ID NO: 7, respectively; and light chain CDR1, CDR2 and CDR3 regions having the amino acid sequences of positions 26-31, positions 49-51 and positions 88-97 of SEQ ID NO: 8, respectively;

(v) heavy chain CDR1, CDR2 and CDR3 regions with the amino acid sequences of positions 26-33, positions 51-58 and positions 97-117 of SEQ ID NO: 9, respectively; and light chain CDR1, CDR2 and CDR3 regions having the amino acid sequences of positions 27-32, positions 50-52 and positions 89-97 of SEQ ID NO: 10, respectively;

(vi) heavy chain CDR1, CDR2 and CDR3 regions with the amino acid sequences of positions 26-33, positions 51-58 and positions 97-112 of SEQ ID NO: 11, respectively; and light chain CDR1, CDR2 and CDR3 regions with the amino acid sequences of positions 27-32, positions 50-52 and positions 89-97 of SEQ ID NO: 12, respectively;

(vii) heavy chain CDR1, CDR2 and CDR3 regions with the amino acid sequences of positions 26-33, positions 51-57 and positions 96-107 of SEQ ID NO: 13, respectively; and light chain CDR1, CDR2 and CDR3 regions having the amino acid sequences of positions 26-31, positions 49-51 and positions 88-98 of SEQ ID NO: 14, respectively;

(viii) heavy chain CDR1, CDR2 and CDR3 regions with the amino acid sequences of positions 26-33, positions 51-57 and positions 106-116 of SEQ ID NO: 15, respectively; and light chain CDR1, CDR2 and CDR3 regions having the amino acid sequences of positions 27-33, positions 51-53 and positions 90-98 of SEQ ID NO: 16, respectively;

(ix) heavy chain CDR1, CDR2 and CDR3 regions with the amino acid sequences of positions 26-33, positions 51-58 and positions 97-114 of SEQ ID NO: 17, respectively; and light chain CDR1, CDR2 and CDR3 regions having the amino acid sequences of positions 27-32, positions 50-52 and positions 89-97 of SEQ ID NO: 18, respectively;

(x) heavy chain CDR1, CDR2 and CDR3 regions with the amino acid sequences of positions 26-33, positions 51-58 and positions 97-109 of SEQ ID NO: 19, respectively; and light chain CDR1, CDR2 and CDR3 regions with the amino acid sequences of positions 26-31, positions 49-51 and positions 88-97 of SEQ ID NO: 20, respectively;

(xi) heavy chain CDR1, CDR2 and CDR3 regions with the amino acid sequences of positions 26-33, positions 51-58 and positions 97-112 of SEQ ID NO: 21, respectively; and light chain CDR1, CDR2 and CDR3 regions with the amino acid sequences of positions 27-32, positions 50-52 and positions 89-97 of SEQ ID NO: 22, respectively,

(xii) heavy chain CDR1, CDR2 and CDR3 regions with the amino acid sequences of positions 26-33, positions 51-57 and positions 96-106 of SEQ ID NO: 23, respectively; and light chain CDR1, CDR2 and CDR3 regions with the amino acid sequences of positions 27-32, positions 50-52 and positions 89-98 of SEQ ID NO: 24, respectively,

(xiii) heavy chain CDR1, CDR2 and CDR3 regions with the amino acid sequences of positions 26-33, positions 51-57 and positions 96-106 of SEQ ID NO: 25, respectively; and light chain CDR1, CDR2 and CDR3 regions with the amino acid sequences of positions 27-32, positions 50-52 and positions 89-97 of SEQ ID NO: 26, respectively,

(xiv) heavy chain CDR1, CDR2 and CDR3 regions with the amino acid sequences of positions 26-33, positions 51-58 and positions 97-119 of SEQ ID NO: 27, respectively; and light chain CDR1, CDR2 and CDR3 regions with the amino acid sequences of positions 26-33, positions 51-53 and positions 92-101 of SEQ ID NO: 28, respectively;

(xv) heavy chain CDR1, CDR2 and CDR3 regions with the amino acid sequences of positions 26-33, positions 51-58 and positions 97-116 of SEQ ID NO: 29, respectively; and light chain CDR1, CDR2 and CDR3 regions having the amino acid sequences of positions 27-32, positions 50-52 and positions 89-97 of SEQ ID NO: 30, respectively;

(xvi) heavy chain CDR1, CDR2 and CDR3 regions with the amino acid sequences of positions 26-33, positions 51-58 and positions 97-116 of SEQ ID NO: 31, respectively; and light chain CDR1, CDR2 and CDR3 regions with the amino acid sequences of positions 27-32, positions 50-52 and positions 89-98 of SEQ ID NO: 32, respectively;

(xvii) heavy chain CDR1, CDR2 and CDR3 regions with the amino acid sequences of positions 26-33, positions 51-57 and positions 96-106 of SEQ ID NO: 33, respectively; and light chain CDR1, CDR2 and CDR3 regions having the amino acid sequences of positions 27-32, positions 50-52 and positions 89-98 of SEQ ID NO: 34, respectively;

(xviii) heavy chain CDR1, CDR2 and CDR3 regions with the amino acid sequences of positions 26-35, positions 53-59 and positions 98-109 of SEQ ID NO: 35, respectively; and light chain CDR1, CDR2 and CDR3 regions with the amino acid sequences of positions 25-34, positions 52-54 and positions 91-102 of SEQ ID NO: 36, respectively;

(xix) heavy chain CDR1, CDR2 and CDR3 regions with the amino acid sequences of positions 26-37, positions 51-57 and positions 96-107 of SEQ ID NO: 37, respectively; and light chain CDR1, CDR2 and CDR3 regions having the amino acid sequences of positions 27-32, positions 50-52 and positions 89-97 of SEQ ID NO: 38, respectively;

(xx) heavy chain CDR1, CDR2 and CDR3 regions with the amino acid sequences of positions 26-33, positions 51-57 and positions 96-107 of SEQ ID NO: 39, respectively; and light chain CDR1, CDR2 and CDR3 regions with the amino acid sequences of positions 27-33, positions 51-53 and positions 90-98 of SEQ ID NO: 40, respectively;

(xxi) heavy chain CDR1, CDR2 and CDR3 regions having the amino acid sequences of positions 26-33, positions 51-58 and positions 97-112 of SEQ ID NO: 41, respectively; and light chain CDR1, CDR2 and CDR3 regions having the amino acid sequences of positions 27-37, positions 55-57 and positions 94-101 of SEQ ID NO: 42, respectively;

(xxii) heavy chain CDR1, CDR2 and CDR3 regions with the amino acid sequences of positions 26-33, positions 51-58 and positions 97-115 of SEQ ID NO: 43, respectively; and light chain CDR1, CDR2 and CDR3 regions having the amino acid sequences of positions 27-32, positions 50-52 and positions 89-96 of SEQ ID NO: 44, respectively;

(xxiii) heavy chain CDR1, CDR2 and CDR3 regions with the amino acid sequences of positions 26-33, positions 51-58 and positions 97-116 of SEQ ID NO: 45, respectively; and light chain CDR1, CDR2 and CDR3 regions having the amino acid sequences of positions 27-32, positions 50-52 and positions 89-98 of SEQ ID NO: 46, respectively;

(xxiv) heavy chain CDR1, CDR2 and CDR3 regions with the amino acid sequences of positions 26-33, positions 51-58 and positions 97-121 of SEQ ID NO: 47, respectively; and light chain CDR1, CDR2 and CDR3 regions having the amino acid sequences of positions 27-32, positions 50-52 and positions 89-97 of SEQ ID NO: 48, respectively;

(xxv) heavy chain CDR1, CDR2 and CDR3 regions with the amino acid sequences of positions 26-33, positions 51-57 and positions 96-106 of SEQ ID NO: 49, respectively; and light chain CDR1, CDR2 and CDR3 regions having the amino acid sequences of positions 27-33, positions 51-53 and positions 90-97 of SEQ ID NO: 50, respectively;

(xxvi) heavy chain CDR1, CDR2 and CDR3 regions with the amino acid sequences of positions 26-33, positions 51-58 and positions 97-109 of SEQ ID NO: 51, respectively; and light chain CDR1, CDR2 and CDR3 regions having the amino acid sequences of positions 27-32, positions 50-52 and positions 89-98 of SEQ ID NO: 52, respectively;

(xxvii) heavy chain CDR1, CDR2 and CDR3 regions with the amino acid sequences of positions 26-33, positions 51-58 and positions 97-112 of SEQ ID NO: 53, respectively; and light chain CDR1, CDR2 and CDR3 regions having the amino acid sequences of positions 27-38, positions 56-58 and positions 95-103 of SEQ ID NO: 54, respectively;

(xxviii) heavy chain CDR1, CDR2 and CDR3 regions with the amino acid sequences of positions 26-33, positions 51-58 and positions 97-108 of SEQ ID NO: 55, respectively; and light chain CDR1, CDR2 and CDR3 regions with the amino acid sequences of positions 27-32, positions 50-52 and positions 89-98 of SEQ ID NO: 56, respectively

(xxix) heavy chain CDR1, CDR2 and CDR3 regions with the amino acid sequences of positions 26-33, positions 51-58 and positions 97-115 of SEQ ID NO: 57, respectively; and light chain CDR1, CDR2 and CDR3 regions having the amino acid sequences of positions 27-38, positions 56-58 and positions 95-104 of SEQ ID NO: 58, respectively;

(xxx) heavy chain CDR1, CDR2 and CDR3 regions with the amino acid sequences of positions 26-33, positions 51-58 and positions 97-110 of SEQ ID NO: 59, respectively; and light chain CDR1, CDR2 and CDR3 regions with the amino acid sequences of positions 27-31, positions 50-52 and positions 89-97 of SEQ ID NO: 60, respectively;

(xxxi) heavy chain CDR1, CDR2 and CDR3 regions with the amino acid sequences of positions 26-33, positions 51-58 and positions 97-112 of SEQ ID NO: 61, respectively; and light chain CDR1, CDR2 and CDR3 regions with the amino acid sequences of positions 27-37, positions 55-57 and positions 94-101 of SEQ ID NO: 62, respectively;

(xxxii) heavy chain CDR1, CDR2 and CDR3 regions with the amino acid sequences of positions 26-33, positions 51-58 and positions 97-106 of SEQ ID NO: 63, respectively; and light chain CDR1, CDR2 and CDR3 regions with the amino acid sequences of positions 27-32, positions 50-52 and positions 89-98 of SEQ ID NO: 64, respectively;

(xxxiii) heavy chain CDR1, CDR2 and CDR3 regions with the amino acid sequences of positions 26-35, positions 53-59 and positions 98-112 of SEQ ID NO: 65, respectively; and light chain CDR1, CDR2 and CDR3 regions having the amino acid sequences of positions 26-31, positions 49-51 and positions 88-96 of SEQ ID NO: 66, respectively;

(xxxiv) heavy chain CDR1, CDR2 and CDR3 regions with the amino acid sequences of positions 26-33, positions 51-60 and positions 99-114 of SEQ ID NO: 67, respectively; and light chain CDR1, CDR2 and CDR3 regions with the amino acid sequences of positions 27-33, positions 51-53 and positions 90-98 of SEQ ID NO: 68, respectively;

(xxxv) heavy chain CDR1, CDR2 and CDR3 regions with the amino acid sequences of positions 26-33, positions 51-58 and positions 97-113 of SEQ ID NO: 69, respectively; and light chain CDR1, CDR2 and CDR3 regions having the amino acid sequences of positions 27-32, positions 50-52 and positions 89-96 of SEQ ID NO: 70, respectively;

(xxxvi) heavy chain CDR1, CDR2 and CDR3 regions with the amino acid sequences of positions 26-33, positions 51-58 and positions 97-115 of SEQ ID NO: 71, respectively; and light chain CDR1, CDR2 and CDR3 regions having the amino acid sequences of positions 27-32, positions 50-52 and positions 89-98 of SEQ ID NO: 72, respectively;

(xxxvii) heavy chain CDR1, CDR2 and CDR3 regions with the amino acid sequences of positions 26-33, positions 51-58 and positions 97-114 of SEQ ID NO: 73, respectively; and light chain CDR1, CDR2 and CDR3 regions having the amino acid sequences of positions 26-31, positions 49-51 and positions 88-96 of SEQ ID NO: 74, respectively;

(xxxviii) heavy chain CDR1, CDR2 and CDR3 regions with the amino acid sequences of positions 26-33, positions 51-58 and positions 97-120 of SEQ ID NO: 75, respectively; and light chain CDR1, CDR2 and CDR3 regions having the amino acid sequences of positions 27-32, positions 50-52 and positions 89-98 of SEQ ID NO: 76, respectively;

(xxxxi) heavy chain CDR1, CDR2 and CDR3 regions with the amino acid sequences of positions 26-33, positions 51-58 and positions 97-111 of SEQ ID NO: 77, respectively; and light chain CDR1, CDR2 and CDR3 regions having the amino acid sequences of positions 27-32, positions 50-52 and positions 89-97 of SEQ ID NO: 78, respectively;

(xl) heavy chain CDR1, CDR2 and CDR3 regions with the amino acid sequences of positions 26-33, positions 51-58 and positions 97-121 of SEQ ID NO: 79, respectively; and light chain CDR1, CDR2 and CDR3 regions with the amino acid sequences of positions 26-31, positions 49-51 and positions 88-96 of SEQ ID NO: 80, respectively;

(xli) heavy chain CDR1, CDR2 and CDR3 regions having the amino acid sequences of positions 26-33, positions 51-58 and positions 97-110 of SEQ ID NO: 81, respectively; and light chain CDR1, CDR2 and CDR3 regions having the amino acid sequences of positions 27-32, positions 50-52 and positions 89-98 of SEQ ID NO: 82, respectively;

(xlii) heavy chain CDR1, CDR2 and CDR3 regions with the amino acid sequences of positions 26-33, positions 51-58 and positions 97-114 of SEQ ID NO: 83, respectively; and light chain CDR1, CDR2 and CDR3 regions having the amino acid sequences of positions 27-37, positions 55-57 and positions 94-102 of SEQ ID NO: 84, respectively;

(xliii) heavy chain CDR1, CDR2 and CDR3 regions with the amino acid sequences of positions 27-33, positions 51-57 and positions 96-106 of SEQ ID NO: 85, respectively; and light chain CDR1, CDR2 and CDR3 regions having the amino acid sequences of positions 27-32, positions 50-52 and positions 89-96 of SEQ ID NO: 86, respectively;

(xliv) heavy chain CDR1, CDR2 and CDR3 regions with the amino acid sequences of positions 29-36, positions 54-60 and positions 99-116 of SEQ ID NO: 87, respectively; and light chain CDR1, CDR2 and CDR3 regions having the amino acid sequences of positions 27-33, positions 51-53 and positions 91-98 of SEQ ID NO: 88, respectively;

(xlv) heavy chain CDR1, CDR2 and CDR3 regions with the amino acid sequences of positions 26-33, positions 51-58 and positions 97-118 of SEQ ID NO: 89, respectively; and light chain CDR1, CDR2 and CDR3 regions having the amino acid sequences of positions 31-39, positions 57-59 and positions 99-108 of SEQ ID NO: 90, respectively; or

(xlvi) heavy chain CDR1, CDR2 and CDR3 regions with the amino acid sequences of positions 26-33, positions 51-57 and positions 96-115 of SEQ ID NO: 91, respectively; and light chain CDR1, CDR2 and CDR3 regions with the amino acid sequences of positions 27-32, positions 50-52 and positions 89-98 of SEQ ID NO: 92, respectively.

(xlvii) heavy chain CDR1, CDR2 and CDR3 regions with the amino acid sequences of positions 26-33, positions 51-58 and positions 97-107 of SEQ ID NO: 93, respectively; and light chain CDR1, CDR2 and CDR3 regions having the amino acid sequences of positions 27-32, positions 51-53 and positions 90-99 of SEQ ID NO: 94, respectively;

(xlxi) heavy chain CDR1, CDR2 and CDR3 regions with the amino acid sequences of positions 26-33, positions 51-57 and positions 96-106 of SEQ ID NO: 95, respectively; and light chain CDR1, CDR2 and CDR3 regions having the amino acid sequences of positions 27-32, positions 50-52 and positions 89-99 of SEQ ID NO: 96, respectively;

(l) heavy chain CDR1, CDR2 and CDR3 regions with the amino acid sequences of positions 26-33, positions 51-57 and positions 96-105 of SEQ ID NO: 97, respectively; and light chain CDR1, CDR2 and CDR3 regions with the amino acid sequences of positions 26-31, positions 49-51 and positions 88-96 of SEQ ID NO: 98, respectively;

(li) heavy chain CDR1, CDR2 and CDR3 regions having the amino acid sequences of positions 26-33, positions 51-57 and positions 96-106 of SEQ ID NO: 99, respectively; and light chain CDR1, CDR2 and CDR3 regions having the amino acid sequences of positions 27-32, positions 50-52 and positions 89-96 of SEQ ID NO: 100, respectively;

(lii) heavy chain CDR1, CDR2 and CDR3 regions with the amino acid sequences of positions 26-35, positions 53-59 and positions 98-109 of SEQ ID NO: 101, respectively; and light chain CDR1, CDR2 and CDR3 regions having the amino acid sequences of positions 26-34, positions 52-524 and positions 91-102 of SEQ ID NO: 102, respectively;

(liii) heavy chain CDR1, CDR2 and CDR3 regions with the amino acid sequences of positions 26-33, positions 51-58 and positions 97-115 of SEQ ID NO: 103, respectively; and light chain CDR1, CDR2 and CDR3 regions having the amino acid sequences of positions 26-31, positions 49-51 and positions 88-96 of SEQ ID NO: 104, respectively;

(liii) heavy chain CDR1, CDR2 and CDR3 regions with the amino acid sequences of positions 26-33, positions 51-58 and positions 97-117 of SEQ ID NO: 105, respectively; and light chain CDR1, CDR2 and CDR3 regions having the amino acid sequences of positions 26-31, positions 49-51 and positions 88-95 of SEQ ID NO: 106, respectively;

(liv) heavy chain CDR1, CDR2 and CDR3 regions with the amino acid sequences of positions 26-33, positions 51-58 and positions 97-112 of SEQ ID NO: 107, respectively; and light chain CDR1, CDR2 and CDR3 regions having the amino acid sequences of positions 27-37, positions 55-57 and positions 89-101 of SEQ ID NO: 108, respectively;

(lv) heavy chain CDR1, CDR2 and CDR3 regions with the amino acid sequences of positions 26-33, positions 51-58 and positions 97-110 of SEQ ID NO: 107, respectively; and light chain CDR1, CDR2 and CDR3 regions having the amino acid sequences of positions 26-31, positions 49-51 and positions 88-98 of SEQ ID NO: 110, respectively;

(lvi) heavy chain CDR1, CDR2 and CDR3 regions with the amino acid sequences of positions 26-35, positions 53-59 and positions 98-115 of SEQ ID NO: 109, respectively; and light chain CDR1, CDR2 and CDR3 regions with the amino acid sequences of positions 26-34, positions 52-54 and positions 91-100 of SEQ ID NO: 112, respectively;

(lvii) heavy chain CDR1, CDR2 and CDR3 regions with the amino acid sequences of positions 26-33, positions 51-57 and positions 96-106 of SEQ ID NO: 113, respectively; and light chain CDR1, CDR2 and CDR3 regions with the amino acid sequences of positions 27-32, positions 50-52 and positions 89-98 of SEQ ID NO: 114, respectively;

(lviii) heavy chain CDR1, CDR2 and CDR3 regions with the amino acid sequences of positions 26-33, positions 51-57 and positions 96-106 of SEQ ID NO: 115, respectively; and light chain CDR1, CDR2 and CDR3 regions having the amino acid sequences of positions 27-32, positions 50-52 and positions 89-96 of SEQ ID NO: 116, respectively;

(lvix) heavy chain CDR1, CDR2 and CDR3 regions with the amino acid sequences of positions 26-33, positions 51-58 and positions 97-113 of SEQ ID NO: 117, respectively; and light chain CDR1, CDR2 and CDR3 regions having the amino acid sequences of positions 27-32, positions 50-52 and positions 89-93 of SEQ ID NO: 118, respectively;

(lx) heavy chain CDR1, CDR2 and CDR3 regions with the amino acid sequences of positions 26-33, positions 51-58 and positions 97-110 of SEQ ID NO: 119, respectively; and light chain CDR1, CDR2 and CDR3 regions with the amino acid sequences of positions 27-32, positions 50-52 and positions 89-97 of SEQ ID NO: 120, respectively;

(lxi) heavy chain CDR1, CDR2 and CDR3 regions having the amino acid sequences of positions 26-35, positions 53-59 and positions 98-113 of SEQ ID NO: 121, respectively; and light chain CDR1, CDR2 and CDR3 regions having the amino acid sequences of positions 26-34, positions 52-54 and positions 91-102 of SEQ ID NO: 122, respectively;

(lxii) heavy chain CDR1, CDR2 and CDR3 regions with the amino acid sequences of positions 26-33, positions 51-57 and positions 96-106 of SEQ ID NO: 123, respectively; and light chain CDR1, CDR2 and CDR3 regions with the amino acid sequences of positions 27-32, positions 50-52 and positions 89-97 of SEQ ID NO: 124, respectively;

(lxiii) heavy chain CDR1, CDR2 and CDR3 regions with the amino acid sequences of positions 26-33, positions 51-57 and positions 96-106 of SEQ ID NO: 125, respectively; and light chain CDR1, CDR2 and CDR3 regions with the amino acid sequences of positions 27-32, positions 50-52 and positions 89-97 of SEQ ID NO: 126, respectively;

(lxiv) heavy chain CDR1, CDR2 and CDR3 regions with the amino acid sequences of positions 26-33, positions 51-58 and positions 97-114 of SEQ ID NO: 127, respectively; and light chain CDR1, CDR2 and CDR3 regions having the amino acid sequences of positions 27-32, positions 50-52 and positions 89-97 of SEQ ID NO: 128, respectively;

(lxv) heavy chain CDR1, CDR2 and CDR3 regions with the amino acid sequences of positions 26-33, positions 51-58 and positions 97-110 of SEQ ID NO: 129, respectively; and light chain CDR1, CDR2 and CDR3 regions having the amino acid sequences of positions 26-31, positions 49-51 and positions 88-98 of SEQ ID NO: 130, respectively;

(lxvi) heavy chain CDR1, CDR2 and CDR3 regions with the amino acid sequences of positions 26-33, positions 51-67 and positions 106-125 of SEQ ID NO: 131, respectively; and light chain CDR1, CDR2 and CDR3 regions having the amino acid sequences of positions 27-32, positions 50-52 and positions 89-97 of SEQ ID NO: 132, respectively;

(lxvii) heavy chain CDR1, CDR2 and CDR3 regions with the amino acid sequences of positions 26-33, positions 51-58 and positions 97-110 of SEQ ID NO: 133, respectively; and light chain CDR1, CDR2 and CDR3 regions having the amino acid sequences of positions 26-31, positions 49-51 and positions 88-97 of SEQ ID NO: 134, respectively;

(lxviii) heavy chain CDR1, CDR2 and CDR3 regions with the amino acid sequences of positions 26-33, positions 51-58 and positions 97-109 of SEQ ID NO: 135, respectively; and light chain CDR1, CDR2 and CDR3 regions having the amino acid sequences of positions 27-32, positions 50-52 and positions 89-98 of SEQ ID NO: 136, respectively;

(lxix) heavy chain CDR1, CDR2 and CDR3 regions with the amino acid sequences of positions 26-33, positions 51-58 and positions 97-108 of SEQ ID NO: 137, respectively; and light chain CDR1, CDR2 and CDR3 regions having the amino acid sequences of positions 26-33, positions 51-53 and positions 92-101 of SEQ ID NO: 138, respectively;

(lxx) heavy chain CDR1, CDR2 and CDR3 regions with the amino acid sequences of positions 26-33, positions 51-58 and positions 97-112 of SEQ ID NO: 139, respectively; and light chain CDR1, CDR2 and CDR3 regions with the amino acid sequences of positions 27-38, positions 56-58 and positions 95-103 of SEQ ID NO: 140, respectively;

(lxxi) heavy chain CDR1, CDR2 and CDR3 regions with the amino acid sequences of positions 26-33, positions 51-58 and positions 97-115 of SEQ ID NO: 141, respectively; and light chain CDR1, CDR2 and CDR3 regions with the amino acid sequences of positions 27-32, positions 50-52 and positions 89-97 of SEQ ID NO: 142, respectively;

(lxxii) heavy chain CDR1, CDR2 and CDR3 regions with the amino acid sequences of positions 26-33, positions 51-58 and positions 98-112 of SEQ ID NO: 143, respectively; and light chain CDR1, CDR2 and CDR3 regions with the amino acid sequences of positions 27-37, positions 55-57 and positions 94-101 of SEQ ID NO: 144, respectively;

(lxxiii) heavy chain CDR1, CDR2 and CDR3 regions with the amino acid sequences of positions 26-33, positions 51-57 and positions 99-106 of SEQ ID NO: 145, respectively; and light chain CDR1, CDR2 and CDR3 regions with the amino acid sequences of positions 27-32, positions 50-52 and positions 99-108 of SEQ ID NO: 146, respectively;

(lxxiv) heavy chain CDR1, CDR2 and CDR3 regions with the amino acid sequences of positions 26-35, positions 53-59 and positions 98-114 of SEQ ID NO: 147, respectively; and light chain CDR1, CDR2 and CDR3 regions having the amino acid sequences of positions 27-34, positions 52-54 and positions 91-100 of SEQ ID NO: 148, respectively;

(lxxv) heavy chain CDR1, CDR2 and CDR3 regions with the amino acid sequences of positions 26-35, positions 53-59 and positions 98-112 of SEQ ID NO: 149, respectively; and light chain CDR1, CDR2 and CDR3 regions having the amino acid sequences of positions 26-34, positions 52-54 and positions 91-102 of SEQ ID NO: 150, respectively;

(lxxvi) heavy chain CDR1, CDR2 and CDR3 regions with the amino acid sequences of positions 26-35, positions 53-59 and positions 98-110 of SEQ ID NO: 151, respectively; and light chain CDR1, CDR2 and CDR3 regions having the amino acid sequences of positions 27-32, positions 50-52 and positions 89-98 of SEQ ID NO: 152, respectively;

(lxxvii) heavy chain CDR1, CDR2 and CDR3 regions with the amino acid sequences of positions 26-33, positions 52-57 and positions 96-106 of SEQ ID NO: 153, respectively; and light chain CDR1, CDR2 and CDR3 regions having the amino acid sequences of positions 27-32, positions 50-52 and positions 89-97 of SEQ ID NO: 154, respectively;

(lxxviii) heavy chain CDR1, CDR2 and CDR3 regions with the amino acid sequences of positions 26-33, positions 51-58 and positions 97-112 of SEQ ID NO: 155, respectively; and light chain CDR1, CDR2 and CDR3 regions with the amino acid sequences of positions 27-37, positions 55-57 and positions 94-101 of SEQ ID NO: 156, respectively;

(lxxix) heavy chain CDR1, CDR2 and CDR3 regions with the amino acid sequences of positions 26-33, positions 51-58 and positions 97-121 of SEQ ID NO: 157, respectively; and light chain CDR1, CDR2 and CDR3 regions having the amino acid sequences of positions 27-32, positions 50-52 and positions 89-97 of SEQ ID NO: 158, respectively;

(lxxx) heavy chain CDR1, CDR2 and CDR3 regions with the amino acid sequences of positions 26-33, positions 51-57 and positions 96-106 of SEQ ID NO: 159, respectively; and light chain CDR1, CDR2 and CDR3 regions with the amino acid sequences of positions 27-32, positions 50-52 and positions 89-97 of SEQ ID NO: 160, respectively;

(lxxxi) heavy chain CDR1, CDR2 and CDR3 regions having the amino acid sequences of positions 26-33, positions 51-59 and positions 97-109 of SEQ ID NO: 161, respectively; and light chain CDR1, CDR2 and CDR3 regions with the amino acid sequences of positions 27-32, positions 50-52 and positions 89-98 of SEQ ID NO: 162, respectively;

(lxxxii) heavy chain CDR1, CDR2 and CDR3 regions with the amino acid sequences of positions 26-33, positions 51-58 and positions 97-112 of SEQ ID NO: 163, respectively; and light chain CDR1, CDR2 and CDR3 regions with the amino acid sequences of positions 27-33, positions 51-53 and positions 90-98 of SEQ ID NO: 164, respectively;

(lxxxiii) heavy chain CDR1, CDR2 and CDR3 regions with the amino acid sequences of positions 26-33, positions 51-57 and positions 96-109 of SEQ ID NO: 165, respectively; and light chain CDR1, CDR2 and CDR3 regions having the amino acid sequences of positions 26-31, positions 49-51 and positions 88-98 of SEQ ID NO: 166, respectively;

(lxxxiv) heavy chain CDR1, CDR2 and CDR3 regions with the amino acid sequences of positions 26-33, positions 51-58 and positions 97-112 of SEQ ID NO: 167, respectively; and light chain CDR1, CDR2 and CDR3 regions with the amino acid sequences of positions 27-32, positions 55-57 and positions 94-101 of SEQ ID NO: 168, respectively;

(lxxxv) heavy chain CDR1, CDR2 and CDR3 regions with the amino acid sequences of positions 26-33, positions 51-58 and positions 97-115 of SEQ ID NO: 169, respectively; and light chain CDR1, CDR2 and CDR3 regions having the amino acid sequences of positions 27-32, positions 50-52 and positions 89-96 of SEQ ID NO: 170, respectively;

(lxxxvi) heavy chain CDR1, CDR2 and CDR3 regions with the amino acid sequences of positions 26-33, positions 51-57 and positions 97-113 of SEQ ID NO: 171, respectively; and light chain CDR1, CDR2 and CDR3 regions with the amino acid sequences of positions 27-32, positions 50-52 and positions 89-93 of SEQ ID NO: 172, respectively;

(lxxxvii) heavy chain CDR1, CDR2 and CDR3 regions with the amino acid sequences of positions 26-33, positions 51-57 and positions 96-107 of SEQ ID NO: 173, respectively; and light chain CDR1, CDR2 and CDR3 regions with the amino acid sequences of positions 27-33, positions 51-53 and positions 90-98 of SEQ ID NO: 174, respectively;

(lxxxviii) heavy chain CDR1, CDR2 and CDR3 regions with the amino acid sequences of positions 26-33, positions 51-58 and positions 97-115 of SEQ ID NO: 175, respectively; and light chain CDR1, CDR2 and CDR3 regions having the amino acid sequences of positions 27-32, positions 50-52 and positions 89-96 of SEQ ID NO: 176, respectively;

(lxxxix) heavy chain CDR1, CDR2 and CDR3 regions with the amino acid sequences of positions 26-33, positions 51-58 and positions 97-108 of SEQ ID NO: 177, respectively; and light chain CDR1, CDR2 and CDR3 regions having the amino acid sequences of positions 27-38, positions 56-58 and positions 95-102 of SEQ ID NO: 178, respectively;

(xc) heavy chain CDR1, CDR2 and CDR3 regions with the amino acid sequences from positions 26-34, positions 52-58 and positions 97-113 of SEQ ID NO: 179, respectively; and light chain CDR1, CDR2 and CDR3 regions with the amino acid sequences of positions 26-33, positions 51-53 and positions 90-101 of SEQ ID NO: 180, respectively;

(xci) heavy chain CDR1, CDR2 and CDR3 regions having the amino acid sequences of positions 26-33, positions 51-58 and positions 97-111 of SEQ ID NO: 181, respectively; and light chain CDR1, CDR2 and CDR3 regions having the amino acid sequences of positions 27-32, positions 50-52 and positions 89-97 of SEQ ID NO: 182, respectively;

(xcii) heavy chain CDR1, CDR2 and CDR3 regions with the amino acid sequences of positions 26-33, positions 51-58 and positions 97-112 of SEQ ID NO: 183, respectively; and light chain CDR1, CDR2 and CDR3 regions with the amino acid sequences of positions 27-32, positions 50-52 and positions 89-99 of SEQ ID NO: 184, respectively;

(xciii) heavy chain CDR1, CDR2 and CDR3 regions with the amino acid sequences of positions 26-33, positions 51-57 and positions 96-117 of SEQ ID NO: 185, respectively; and light chain CDR1, CDR2 and CDR3 regions with the amino acid sequences of positions 27-32, positions 50-52 and positions 89-97 of SEQ ID NO: 186, respectively;

(xciv) heavy chain CDR1, CDR2 and CDR3 regions with the amino acid sequences from positions 26-35, positions 53-59 and positions 98-112 of SEQ ID NO: 187, respectively; and light chain CDR1, CDR2 and CDR3 regions with the amino acid sequences of positions 27-32, positions 50-52 and positions 89-97 of SEQ ID NO: 188, respectively;

(xcv) heavy chain CDR1, CDR2 and CDR3 regions with the amino acid sequences of positions 26-33, positions 51-57 and positions 98-119 of SEQ ID NO: 189, respectively; and light chain CDR1, CDR2 and CDR3 regions having the amino acid sequences of positions 26-34, positions 52-54 and positions 91-100 of SEQ ID NO: 190, respectively;

(xcvi) heavy chain CDR1, CDR2 and CDR3 regions with the amino acid sequences of positions 26-33, positions 51-58 and positions 97-107 of SEQ ID NO: 191, respectively; and light chain CDR1, CDR2 and CDR3 regions with the amino acid sequences of positions 26-33, positions 51-53 and positions 90-100 of SEQ ID NO: 192, respectively;

(xcvii) heavy chain CDR1, CDR2 and CDR3 regions with the amino acid sequences of positions 26-33, positions 51-58 and positions 97-112 of SEQ ID NO: 193, respectively; and light chain CDR1, CDR2 and CDR3 regions with the amino acid sequences of positions 26-36, positions 54-56 and positions 93-100 of SEQ ID NO: 194, respectively;

(xcviii) heavy chain CDR1, CDR2 and CDR3 regions with the amino acid sequences of positions 26-33, positions 51-58 and positions 97-113 of SEQ ID NO: 195, respectively; and light chain CDR1, CDR2 and CDR3 regions with the amino acid sequences of positions 26-34, positions 52-54 and positions 91-102 of SEQ ID NO: 196, respectively;

(xcix) heavy chain CDR1, CDR2 and CDR3 regions with the amino acid sequences of positions 26-33, positions 51-58 and positions 97-112 of SEQ ID NO: 197, respectively; and light chain CDR1, CDR2 and CDR3 regions with the amino acid sequences of positions 27-37, positions 55-57 and positions 94-101 of SEQ ID NO: 198, respectively;

(c) heavy chain CDR1, CDR2 and CDR3 regions with the amino acid sequences of positions 26-33, positions 51-57 and positions 96-106 of SEQ ID NO: 199, respectively; and light chain CDR1, CDR2 and CDR3 regions with the amino acid sequences of positions 27-32, positions 50-52 and positions 89-98 of SEQ ID NO: 200, respectively;

(ci) heavy chain CDR1, CDR2 and CDR3 regions with the amino acid sequences of positions 26-33, positions 51-58 and positions 97-113 of SEQ ID NO: 201, respectively; and light chain CDR1, CDR2 and CDR3 regions having the amino acid sequences of positions 26-33, positions 52-54 and positions 91-102 of SEQ ID NO: 202, respectively;

(cii) heavy chain CDR1, CDR2 and CDR3 regions with the amino acid sequences of positions 26-33, positions 51-57 and positions 96-109 of SEQ ID NO: 203, respectively; and light chain CDR1, CDR2 and CDR3 regions having the amino acid sequences of positions 26-31, positions 49-51 and positions 88-98 of SEQ ID NO: 204, respectively;

(ciii) heavy chain CDR1, CDR2 and CDR3 regions with the amino acid sequences of positions 26-34, positions 52-58 and positions 97-111 of SEQ ID NO: 205, respectively; and light chain CDR1, CDR2 and CDR3 regions having the amino acid sequences of positions 26-34, positions 52-54 and positions 91-101 of SEQ ID NO: 206, respectively;

(civ) heavy chain CDR1, CDR2 and CDR3 regions with the amino acid sequences of positions 26-33, positions 51-58 and positions 97-109 of SEQ ID NO: 207, respectively; and light chain CDR1, CDR2 and CDR3 regions having the amino acid sequences of positions 26-31, positions 49-51 and positions 88-99 of SEQ ID NO: 208, respectively;

(cv) heavy chain CDR1, CDR2 and CDR3 regions with the amino acid sequences of positions 26-33, positions 51-58 and positions 97-113 of SEQ ID NO: 209, respectively; and light chain CDR1, CDR2 and CDR3 regions having the amino acid sequences of positions 26-32, positions 52-54 and positions 91-102 of SEQ ID NO: 210, respectively;

(cvi) heavy chain CDR1, CDR2 and CDR3 regions with the amino acid sequences of positions 26-33, positions 51-58 and positions 97-109 of SEQ ID NO: 211, respectively; and light chain CDR1, CDR2 and CDR3 regions having the amino acid sequences of positions 27-32, positions 50-52 and positions 91-112 of SEQ ID NO: 212, respectively;

(cvii) heavy chain CDR1, CDR2 and CDR3 regions with the amino acid sequences of positions 26-33, positions 51-57 and positions 96-106 of SEQ ID NO: 213, respectively; and light chain CDR1, CDR2 and CDR3 regions with the amino acid sequences of positions 27-32, positions 50-52 and positions 89-98 of SEQ ID NO: 214, respectively;

(cviii) heavy chain CDR1, CDR2 and CDR3 regions with the amino acid sequences of positions 26-33, positions 51-58 and positions 97-117 of SEQ ID NO: 215, respectively; and light chain CDR1, CDR2 and CDR3 regions with the amino acid sequences of positions 27-32, positions 50-52 and positions 89-97 of SEQ ID NO: 216, respectively;

(cix) heavy chain CDR1, CDR2 and CDR3 regions with the amino acid sequences of positions 26-33, positions 51-58 and positions 97-115 of SEQ ID NO: 217, respectively; and light chain CDR1, CDR2 and CDR3 regions with the amino acid sequences of positions 27-36, positions 55-57 and positions 94-102 of SEQ ID NO: 218, respectively;

(cx) heavy chain CDR1, CDR2 and CDR3 regions with the amino acid sequences of positions 26-33, positions 51-58 and positions 97-106 of SEQ ID NO: 219, respectively; and light chain CDR1, CDR2 and CDR3 regions with the amino acid sequences of positions 27-38, positions 56-58 and positions 95-104 of SEQ ID NO: 220, respectively;

(cxi) heavy chain CDR1, CDR2 and CDR3 regions having the amino acid sequences of positions 26-33, positions 51-58 and positions 97-119 of SEQ ID NO: 221, respectively; and light chain CDR1, CDR2 and CDR3 regions having the amino acid sequences of positions 25-33, positions 51-53 and positions 90-100 of SEQ ID NO: 222, respectively;

(cxii) heavy chain CDR1, CDR2 and CDR3 regions with the amino acid sequences of positions 26-33, positions 51-58 and positions 97-111 of SEQ ID NO: 223, respectively; and light chain CDR1, CDR2 and CDR3 regions with the amino acid sequences of positions 27-32, positions 50-52 and positions 89-97 of SEQ ID NO: 224, respectively;

(cxiii) heavy chain CDR1, CDR2 and CDR3 regions with the amino acid sequences of positions 26-33, positions 51-60 and positions 99-115 of SEQ ID NO: 225, respectively; and light chain CDR1, CDR2 and CDR3 regions with the amino acid sequences of positions 27-33, positions 51-53 and positions 90-99 of SEQ ID NO: 226, respectively;

(cxiv) heavy chain CDR1, CDR2 and CDR3 regions with the amino acid sequences of positions 26-33, positions 51-60 and positions 99-115 of SEQ ID NO: 227, respectively; and light chain CDR1, CDR2 and CDR3 regions with the amino acid sequences of positions 27-33, positions 51-53 and positions 90-99 of SEQ ID NO: 228, respectively;

(cxv) heavy chain CDR1, CDR2 and CDR3 regions with the amino acid sequences of positions 26-33, positions 51-58 and positions 97-112 of SEQ ID NO: 229, respectively; and light chain CDR1, CDR2 and CDR3 regions having the amino acid sequences of positions 26-31, positions 49-51 and positions 89-96 of SEQ ID NO: 230, respectively;

(cxvi) heavy chain CDR1, CDR2 and CDR3 regions with the amino acid sequences of positions 26-33, positions 51-60 and positions 99-115 of SEQ ID NO: 231, respectively; and light chain CDR1, CDR2 and CDR3 regions having the amino acid sequences of positions 27-33, positions 51-53 and positions 90-99 of SEQ ID NO: 232, respectively;

(cxvii) heavy chain CDR1, CDR2 and CDR3 regions with the amino acid sequences of positions 26-35, positions 53-59 and positions 98-110 of SEQ ID NO: 233, respectively; and light chain CDR1, CDR2 and CDR3 regions having the amino acid sequences of positions 27-33, positions 51-53 and positions 90-98 of SEQ ID NO: 234, respectively;

(cxviii) heavy chain CDR1, CDR2 and CDR3 regions with the amino acid sequences of positions 26-35, positions 53-59 and positions 98-118 of SEQ ID NO: 235, respectively; and light chain CDR1, CDR2 and CDR3 regions having the amino acid sequences of positions 27-32, positions 50-52 and positions 89-97 of SEQ ID NO: 236, respectively;

(cxix) heavy chain CDR1, CDR2 and CDR3 regions with the amino acid sequences of positions 26-33, positions 51-58 and positions 97-112 of SEQ ID NO: 237, respectively; and light chain CDR1, CDR2 and CDR3 regions having the amino acid sequences of positions 26-31, positions 49-51 and positions 88-96 of SEQ ID NO: 238, respectively;

(cxx) heavy chain CDR1, CDR2 and CDR3 regions with the amino acid sequences of positions 26-35, positions 53-59 and positions 98-111 of SEQ ID NO: 239, respectively; and light chain CDR1, CDR2 and CDR3 regions with the amino acid sequences of positions 26-31, positions 49-51 and positions 88-96 of SEQ ID NO: 240, respectively;

(cxxi) heavy chain CDR1, CDR2 and CDR3 regions having the amino acid sequences of positions 26-33, positions 51-57 and positions 96-106 of SEQ ID NO: 241, respectively; and light chain CDR1, CDR2 and CDR3 regions having the amino acid sequences of positions 27-32, positions 50-52 and positions 89-991 of SEQ ID NO: 242, respectively;

(cxxii) heavy chain CDR1, CDR2 and CDR3 regions with the amino acid sequences of positions 26-34, positions 52-58 and positions 97-111 of SEQ ID NO: 243, respectively; and light chain CDR1, CDR2 and CDR3 regions having the amino acid sequences of positions 26-34, positions 52-54 and positions 91-101 of SEQ ID NO: 244, respectively;

(cxxiii) heavy chain CDR1, CDR2 and CDR3 regions with the amino acid sequences of positions 26-35, positions 53-59 and positions 98-113 of SEQ ID NO: 245, respectively; and light chain CDR1, CDR2 and CDR3 regions with the amino acid sequences of positions 27-37, positions 55-57 and positions 94-102 of SEQ ID NO: 246, respectively;

(cxxix) heavy chain CDR1, CDR2 and CDR3 regions with the amino acid sequences of positions 26-35, positions 53-59 and positions 98-113 of SEQ ID NO: 247, respectively; and light chain CDR1, CDR2 and CDR3 regions with the amino acid sequences of positions 27-37, positions 55-57 and positions 94-102 of SEQ ID NO: 248, respectively;

(cxxv) heavy chain CDR1, CDR2 and CDR3 regions with the amino acid sequences of positions 26-33, positions 51-58 and positions 97-112 of SEQ ID NO: 249, respectively; and light chain CDR1, CDR2 and CDR3 regions having the amino acid sequences of positions 27-38, positions 56-58 and positions 95-103 of SEQ ID NO: 250, respectively;

(cxxvi) heavy chain CDR1, CDR2 and CDR3 regions with the amino acid sequences of positions 26-33, positions 51-58 and positions 107-122 of SEQ ID NO: 251, respectively; and light chain CDR1, CDR2 and CDR3 regions having the amino acid sequences of positions 26-33, positions 51-53 and positions 90-99 of SEQ ID NO: 252, respectively;

(cxxvii) heavy chain CDR1, CDR2 and CDR3 regions with the amino acid sequences of positions 26-33, positions 51-58 and positions 97-121 of SEQ ID NO: 253, respectively; and light chain CDR1, CDR2 and CDR3 regions with the amino acid sequences of positions 26-34, positions 52-54 and positions 91-101 of SEQ ID NO: 254, respectively;

(cxxviii) heavy chain CDR1, CDR2 and CDR3 regions with the amino acid sequences of positions 27-33, positions 51-58 and positions 97-118 of SEQ ID NO: 255, respectively; and light chain CDR1, CDR2 and CDR3 regions having the amino acid sequences of positions 26-34, positions 52-54 and positions 91-100 of SEQ ID NO: 256, respectively;

(cxxix) heavy chain CDR1, CDR2 and CDR3 regions with the amino acid sequences of positions 26-32, positions 51-58 and positions 97-118 of SEQ ID NO: 257, respectively; and light chain CDR1, CDR2 and CDR3 regions having the amino acid sequences of positions 26-34, positions 52-54 and positions 91-99 of SEQ ID NO: 258, respectively;

(cxxx) heavy chain CDR1, CDR2 and CDR3 regions with the amino acid sequences of positions 26-33, positions 51-58 and positions 98-108 of SEQ ID NO: 259, respectively; and light chain CDR1, CDR2 and CDR3 regions with the amino acid sequences of positions 27-38, positions 56-58 and positions 95-103 of SEQ ID NO: 260, respectively;

(cxxxi) heavy chain CDR1, CDR2 and CDR3 regions having the amino acid sequences of positions 38-45, positions 63-69 and positions 108-123 of SEQ ID NO: 261, respectively; and light chain CDR1, CDR2 and CDR3 regions having the amino acid sequences of positions 27-32, positions 50-52 and positions 89-99 of SEQ ID NO: 262, respectively;

(cxxxii) heavy chain CDR1, CDR2 and CDR3 regions with the amino acid sequences of positions 26-34, positions 51-58 and positions 97-108 of SEQ ID NO: 263, respectively; and light chain CDR1, CDR2 and CDR3 regions with the amino acid sequences of positions 26-33, positions 51-53 and positions 92-101 of SEQ ID NO: 264, respectively;

(cxxxiii) heavy chain CDR1, CDR2 and CDR3 regions with the amino acid sequences of positions 26-33, positions 51-58 and positions 97-112 of SEQ ID NO: 265, respectively; and light chain CDR1, CDR2 and CDR3 regions with the amino acid sequences of positions 27-37, positions 55-57 and positions 94-101 of SEQ ID NO: 266, respectively;

(cxxxiv) heavy chain CDR1, CDR2 and CDR3 regions with the amino acid sequences of positions 26-34, positions 52-58 and positions 97-113 of SEQ ID NO: 267, respectively; and light chain CDR1, CDR2 and CDR3 regions with the amino acid sequences of positions 26-33, positions 51-53 and positions 90-101 of SEQ ID NO: 268, respectively;

(cxxxv) heavy chain CDR1, CDR2 and CDR3 regions with the amino acid sequences of positions 26-33, positions 51-57 and positions 96-106 of SEQ ID NO: 269, respectively; and light chain CDR1, CDR2 and CDR3 regions having the amino acid sequences of positions 27-32, positions 50-52 and positions 89-99 of SEQ ID NO: 270, respectively;

(cxxxvi) heavy chain CDR1, CDR2 and CDR3 regions with the amino acid sequences of positions 26-33, positions 51-57 and positions 97-108 of SEQ ID NO: 271, respectively; and light chain CDR1, CDR2 and CDR3 regions having the amino acid sequences of positions 27-38, positions 56-58 and positions 95-103 of SEQ ID NO: 272, respectively;

(cxxxvii) heavy chain CDR1, CDR2 and CDR3 regions with the amino acid sequences of positions 26-35, positions 53-59 and positions 98-110 of SEQ ID NO: 273, respectively; and light chain CDR1, CDR2 and CDR3 regions with the amino acid sequences of positions 27-33, positions 51-53 and positions 90-98 of SEQ ID NO: 274, respectively;

(cxxxviii) heavy chain CDR1, CDR2 and CDR3 regions with the amino acid sequences of positions 26-34, positions 52-58 and positions 97-113 of SEQ ID NO: 275, respectively; and light chain CDR1, CDR2 and CDR3 regions with the amino acid sequences of positions 26-33, positions 51-53 and positions 90-101 of SEQ ID NO: 276, respectively;

(cxxxix) heavy chain CDR1, CDR2 and CDR3 regions with the amino acid sequences of positions 26-33, positions 51-57 and positions 96-107 of SEQ ID NO: 277, respectively; and light chain CDR1, CDR2 and CDR3 regions having the amino acid sequences of positions 26-34, positions 52-54 and positions 91-100 of SEQ ID NO: 278, respectively;

(cxl) heavy chain CDR1, CDR2 and CDR3 regions with the amino acid sequences of positions 26-35, positions 54-59 and positions 98-112 of SEQ ID NO: 279, respectively; and light chain CDR1, CDR2 and CDR3 regions with the amino acid sequences of positions 26-31, positions 49-51 and positions 88-96 of SEQ ID NO: 280, respectively;

(cxli) heavy chain CDR1, CDR2 and CDR3 regions with the amino acid sequences of positions 26-33, positions 51-58 and positions 97-112 of SEQ ID NO: 281, respectively; and light chain CDR1, CDR2 and CDR3 regions having the amino acid sequences of positions 27-37, positions 55-57 and positions 94-101 of SEQ ID NO: 282, respectively;

(cxlii) heavy chain CDR1, CDR2 and CDR3 regions with the amino acid sequences of positions 26-33, positions 51-58 and positions 97-120 of SEQ ID NO: 283, respectively; and light chain CDR1, CDR2 and CDR3 regions having the amino acid sequences of positions 26-31, positions 49-51 and positions 88-98 of SEQ ID NO: 284, respectively;

(cxliii) heavy chain CDR1, CDR2 and CDR3 regions with the amino acid sequences of positions 26-33, positions 51-57 and positions 96-106 of SEQ ID NO: 285, respectively; and light chain CDR1, CDR2 and CDR3 regions having the amino acid sequences of positions 26-31, positions 50-52 and positions 89-98 of SEQ ID NO: 286, respectively;

(cxliv) heavy chain CDR1, CDR2 and CDR3 regions with the amino acid sequences of positions 26-35, positions 52-58 and positions 97-111 of SEQ ID NO: 287, respectively; and light chain CDR1, CDR2 and CDR3 regions having the amino acid sequences of positions 26-34, positions 52-54 and positions 95-101 of SEQ ID NO: 288, respectively;

(cxlv) heavy chain CDR1, CDR2 and CDR3 regions with the amino acid sequences of positions 26-33, positions 51-58 and positions 97-116 of SEQ ID NO: 289, respectively; and light chain CDR1, CDR2 and CDR3 regions having the amino acid sequences of positions 28-32, positions 50-52 and positions 89-97 of SEQ ID NO: 290, respectively;

(cxlvi) heavy chain CDR1, CDR2 and CDR3 regions with the amino acid sequences of positions 26-33, positions 51-58 and positions 97-116 of SEQ ID NO: 291, respectively; and light chain CDR1, CDR2 and CDR3 regions having the amino acid sequences of positions 27-32, positions 50-52 and positions 89-96 of SEQ ID NO: 292, respectively;

(cxlvii) heavy chain CDR1, CDR2 and CDR3 regions with the amino acid sequences of positions 26-33, positions 51-58 and positions 97-118 of SEQ ID NO: 293, respectively; and light chain CDR1, CDR2 and CDR3 regions with the amino acid sequences of positions 26-34, positions 52-54 and positions 91-100 of SEQ ID NO: 294, respectively;

(cxlviii) heavy chain CDR1, CDR2 and CDR3 regions with the amino acid sequences of positions 26-33, positions 51-58 and positions 97-112 of SEQ ID NO: 295, respectively; and light chain CDR1, CDR2 and CDR3 regions having the amino acid sequences of positions 27-32, positions 50-52 and positions 89-95 of SEQ ID NO: 296, respectively;

(cxlix) heavy chain CDR1, CDR2 and CDR3 regions with the amino acid sequences of positions 21-28, positions 46-53 and positions 92-106 of SEQ ID NO: 297, respectively; and light chain CDR1, CDR2 and CDR3 regions having the amino acid sequences of positions 17-22, positions 40-42 and positions 79-87 of SEQ ID NO: 298, respectively;

(cl) heavy chain CDR1, CDR2 and CDR3 regions with the amino acid sequences of positions 26-33, positions 51-57 and positions 96-107 of SEQ ID NO: 299, respectively; and light chain CDR1, CDR2 and CDR3 regions with the amino acid sequences of positions 27-37, positions 55-57 and positions 94-101 of SEQ ID NO: 300, respectively;

(cli) heavy chain CDR1, CDR2 and CDR3 regions having the amino acid sequences of positions 26-33, positions 51-57 and positions 96-107 of SEQ ID NO: 301, respectively; and light chain CDR1, CDR2 and CDR3 regions having the amino acid sequences of positions 27-37, positions 55-57 and positions 94-101 of SEQ ID NO: 302, respectively;

(clii) heavy chain CDR1, CDR2 and CDR3 regions with the amino acid sequences of positions 26-33, positions 51-57 and positions 96-113 of SEQ ID NO: 303, respectively; and light chain CDR1, CDR2 and CDR3 regions with the amino acid sequences of positions 27-32, positions 51-54 and positions 90-98 of SEQ ID NO: 304, respectively;

(cliii) heavy chain CDR1, CDR2 and CDR3 regions with the amino acid sequences of positions 26-33, positions 51-57 and positions 96-110 of SEQ ID NO: 305, respectively; and light chain CDR1, CDR2 and CDR3 regions having the amino acid sequences of positions 27-32, positions 50-52 and positions 89-98 of SEQ ID NO: 306, respectively;

(cliv) heavy chain CDR1, CDR2 and CDR3 regions with the amino acid sequences of positions 26-33, positions 51-58 and positions 97-109 of SEQ ID NO: 307, respectively; and light chain CDR1, CDR2 and CDR3 regions having the amino acid sequences of positions 26-33, positions 51-53 and positions 90-99 of SEQ ID NO: 308, respectively;

(clv) heavy chain CDR1, CDR2 and CDR3 regions with the amino acid sequences of positions 26-33, positions 51-58 and positions 97-114 of SEQ ID NO: 309, respectively; and light chain CDR1, CDR2 and CDR3 regions having the amino acid sequences of positions 24-29, positions 48-50 and positions 86-94 of SEQ ID NO: 310, respectively;

(clvi) heavy chain CDR1, CDR2 and CDR3 regions with the amino acid sequences of positions 26-33, positions 51-58 and positions 97-108 of SEQ ID NO: 311, respectively; and light chain CDR1, CDR2 and CDR3 regions having the amino acid sequences of positions 27-38, positions 56-58 and positions 95-103 of SEQ ID NO: 312, respectively;

(clvii) heavy chain CDR1, CDR2 and CDR3 regions with the amino acid sequences of positions 26-33, positions 51-58 and positions 97-115 of SEQ ID NO: 313, respectively; and light chain CDR1, CDR2 and CDR3 regions having the amino acid sequences of positions 27-32, positions 50-52 and positions 89-97 of SEQ ID NO: 314, respectively;

(clviii) heavy chain CDR1, CDR2 and CDR3 regions with the amino acid sequences of positions 26-33, positions 51-58 and positions 97-115 of SEQ ID NO: 315, respectively; and light chain CDR1, CDR2 and CDR3 regions having the amino acid sequences of positions 27-32, positions 50-52 and positions 89-97 of SEQ ID NO: 316, respectively;

(clix) heavy chain CDR1, CDR2 and CDR3 regions with the amino acid sequences of positions 26-33, positions 51-58 and positions 97-113 of SEQ ID NO: 317, respectively; and light chain CDR1, CDR2 and CDR3 regions having the amino acid sequences of positions 26-31, positions 51-53 and positions 90-100 of SEQ ID NO: 318, respectively;

(clx) heavy chain CDR1, CDR2 and CDR3 regions with the amino acid sequences of positions 26-34, positions 53-59 and positions 98-119 of SEQ ID NO: 319, respectively; and light chain CDR1, CDR2 and CDR3 regions with the amino acid sequences of positions 26-31, positions 51-53 and positions 90-101 of SEQ ID NO: 320, respectively;

(clxi) heavy chain CDR1, CDR2 and CDR3 regions having the amino acid sequences of positions 26-33, positions 51-58 and positions 97-110 of SEQ ID NO: 321, respectively; and light chain CDR1, CDR2 and CDR3 regions having the amino acid sequences of positions 27-32, positions 50-52 and positions 89-97 of SEQ ID NO: 322, respectively;

(clxii) heavy chain CDR1, CDR2 and CDR3 regions with the amino acid sequences of positions 26-35, positions 53-59 and positions 98-119 of SEQ ID NO: 323, respectively; and light chain CDR1, CDR2 and CDR3 regions having the amino acid sequences of positions 26-34, positions 52-54 and positions 91-100 of SEQ ID NO: 324, respectively;

(clxiii) heavy chain CDR1, CDR2 and CDR3 regions with the amino acid sequences of positions 26-33, positions 51-57 and positions 96-103 of SEQ ID NO: 325, respectively; and light chain CDR1, CDR2 and CDR3 regions having the amino acid sequences of positions 27-32, positions 50-52 and positions 89-97 of SEQ ID NO: 326, respectively;

(clxiv) heavy chain CDR1, CDR2 and CDR3 regions with the amino acid sequences of positions 26-35, positions 52-59 and positions 98-112 of SEQ ID NO: 327, respectively; and light chain CDR1, CDR2 and CDR3 regions having the amino acid sequences of positions 26-31, positions 49-51 and positions 88-96 of SEQ ID NO: 328, respectively;

(clxv) heavy chain CDR1, CDR2 and CDR3 regions with the amino acid sequences of positions 26-33, positions 51-58 and positions 97-117 of SEQ ID NO: 329, respectively; and light chain CDR1, CDR2 and CDR3 regions having the amino acid sequences of positions 24-29, positions 47-49 and positions 86-94 of SEQ ID NO: 330, respectively;

(clxvi) heavy chain CDR1, CDR2 and CDR3 regions with the amino acid sequences of positions 26-33, positions 51-58 and positions 97-113 of SEQ ID NO: 331, respectively; and light chain CDR1, CDR2 and CDR3 regions having the amino acid sequences of positions 26-33, positions 51-53 and positions 90-100 of SEQ ID NO: 332, respectively;

(clxvii) heavy chain CDR1, CDR2 and CDR3 regions with the amino acid sequences of positions 26-33, positions 51-57 and positions 97-113 of SEQ ID NO: 333, respectively; and light chain CDR1, CDR2 and CDR3 regions having the amino acid sequences of positions 26-33, positions 51-53 and positions 90-100 of SEQ ID NO: 334, respectively;

(clxviii) heavy chain CDR1, CDR2 and CDR3 regions with the amino acid sequences of positions 26-33, positions 51-57 and positions 97-116 of SEQ ID NO: 335, respectively; and light chain CDR1, CDR2 and CDR3 regions having the amino acid sequences of positions 27-31, positions 50-52 and positions 89-98 of SEQ ID NO: 336, respectively;

(clxix) heavy chain CDR1, CDR2 and CDR3 regions with the amino acid sequences of positions 26-35, positions 53-59 and positions 98-121 of SEQ ID NO: 337, respectively; and light chain CDR1, CDR2 and CDR3 regions having the amino acid sequences of positions 27-37, positions 55-57 and positions 94-102 of SEQ ID NO: 338, respectively;

(clxx) heavy chain CDR1, CDR2 and CDR3 regions with the amino acid sequences of positions 26-33, positions 51-60 and positions 99-114 of SEQ ID NO: 339, respectively; and light chain CDR1, CDR2 and CDR3 regions with the amino acid sequences of positions 27-33, positions 51-53 and positions 90-99 of SEQ ID NO: 340, respectively;

(clxxi) heavy chain CDR1, CDR2 and CDR3 regions with the amino acid sequences of positions 26-33, positions 51-58 and positions 97-117 of SEQ ID NO: 341, respectively; and light chain CDR1, CDR2 and CDR3 regions having the amino acid sequences of positions 26-33, positions 51-53 and positions 90-101 of SEQ ID NO: 342, respectively;

(clxxii) heavy chain CDR1, CDR2 and CDR3 regions with the amino acid sequences of positions 27-34, positions 52-58 and positions 98-112 of SEQ ID NO: 343, respectively; and light chain CDR1, CDR2 and CDR3 regions with the amino acid sequences of positions 26-34, positions 52-54 and positions 91-100 of SEQ ID NO: 344, respectively;

(clxxiii) heavy chain CDR1, CDR2 and CDR3 regions with the amino acid sequences of positions 27-35, positions 53-59 and positions 98-118 of SEQ ID NO: 345, respectively; and light chain CDR1, CDR2 and CDR3 regions with the amino acid sequences of positions 26-34, positions 52-54 and positions 91-100 of SEQ ID NO: 346, respectively

(clxxiv) heavy chain CDR1, CDR2 and CDR3 regions with the amino acid sequences of positions 26-35, positions 53-59 and positions 98-122 of SEQ ID NO: 347, respectively; and light chain CDR1, CDR2 and CDR3 regions having the amino acid sequences of positions 27-37, positions 55-57 and positions 94-102 of SEQ ID NO: 348, respectively;

(clxxv) heavy chain CDR1, CDR2 and CDR3 regions with the amino acid sequences of positions 26-33, positions 51-61 and positions 97-106 of SEQ ID NO: 349, respectively; and light chain CDR1, CDR2 and CDR3 regions having the amino acid sequences of positions 26-31, positions 49-51 and positions 88-96 of SEQ ID NO: 350, respectively;

(clxxvi) heavy chain CDR1, CDR2 and CDR3 regions with the amino acid sequences of positions 27-34, positions 52-58 and positions 97-112 of SEQ ID NO: 351, respectively; and light chain CDR1, CDR2 and CDR3 regions having the amino acid sequences of positions 26-34, positions 52-54 and positions 91-101 of SEQ ID NO: 352, respectively;

(clxxvii) heavy chain CDR1, CDR2 and CDR3 regions with the amino acid sequences of positions 26-34, positions 52-59 and positions 97-112 of SEQ ID NO: 353, respectively; and light chain CDR1, CDR2 and CDR3 regions having the amino acid sequences of positions 27-32, positions 50-52 and positions 89-97 of SEQ ID NO: 354, respectively;

(clxxviii) heavy chain CDR1, CDR2 and CDR3 regions with the amino acid sequences of positions 26-33, positions 51-58 and positions 97-117 of SEQ ID NO: 355, respectively; and light chain CDR1, CDR2 and CDR3 regions having the amino acid sequences of positions 27-32, positions 50-52 and positions 89-99 of SEQ ID NO: 356, respectively;

(clxxix) heavy chain CDR1, CDR2 and CDR3 regions with the amino acid sequences of positions 26-32, positions 51-58 and positions 97-110 of SEQ ID NO: 357, respectively; and light chain CDR1, CDR2 and CDR3 regions having the amino acid sequences of positions 26-31, positions 49-51 and positions 88-98 of SEQ ID NO: 358, respectively; or

(clxxx) heavy chain CDR1, CDR2 and CDR3 regions with the amino acid sequences of positions 26-32, positions 51-58 and positions 97-110 of SEQ ID NO: 359, respectively; and light chain CDR1, CDR2 and CDR3 regions with the amino acid sequences of positions 26-31, positions 49-51 and positions 88-98 of SEQ ID NO: 360, respectively.

2. Antibody according to claim 1, wherein the antibody comprises a heavy chain and a light chain selected from (i) - (clxxx):

(i) a heavy chain comprising SEQ ID NO: 1 and a light chain comprising SEQ ID NO: 2;
(ii) a heavy chain comprising SEQ ID NO: 3 and a light chain comprising SEQ ID NO: 4;
(iii) a heavy chain comprising SEQ ID NO: 5 and a light chain comprising SEQ ID NO: 6;
(iv) a heavy chain comprising SEQ ID NO: 7 and a light chain comprising SEQ ID NO: 8;
(v) a heavy chain comprising SEQ ID NO: 9 and a light chain comprising SEQ ID NO: 10;
(vi) a heavy chain comprising SEQ ID NO: 11 and a light chain comprising SEQ ID NO: 12;
(vii) a heavy chain comprising SEQ ID NO: 13 and a light chain comprising SEQ ID NO: 14;
(viii) a heavy chain comprising SEQ ID NO: 15 and a light chain comprising SEQ ID NO: 16;
(ix) a heavy chain comprising SEQ ID NO: 17 and a light chain comprising SEQ ID NO: 18;
(x) a heavy chain comprising SEQ ID NO: 19 and a light chain comprising SEQ ID NO: 20;
(xi) a heavy chain comprising SEQ ID NO: 21 and a light chain comprising SEQ ID NO: 22;
(xii) a heavy chain comprising SEQ ID NO: 23 and a light chain comprising SEQ ID NO: 24;
(xiii) a heavy chain comprising SEQ ID NO: 25 and a light chain comprising SEQ ID NO: 26;
(xiv) a heavy chain comprising SEQ ID NO: 27 and a light chain comprising SEQ ID NO: 28;
(xv) a heavy chain comprising SEQ ID NO: 29 and a light chain comprising SEQ ID NO: 30;
(xvi) a heavy chain comprising SEQ ID NO: 31 and a light chain comprising SEQ ID NO: 32;
(xvii) a heavy chain comprising SEQ ID NO: 33 and a light chain comprising SEQ ID NO: 34;
(xviii) a heavy chain comprising SEQ ID NO: 35 and a light chain comprising SEQ ID NO: 36;
(xxix) a heavy chain comprising SEQ ID NO: 37 and a light chain comprising SEQ ID NO: 38;
(xx) a heavy chain comprising SEQ ID NO: 39 and a light chain comprising SEQ ID NO: 40;
(xxi) a heavy chain comprising SEQ ID NO: 41 and a light chain comprising SEQ ID NO: 42;
(xxii) a heavy chain comprising SEQ ID NO: 43 and a light chain comprising SEQ ID NO: 44;
(xxiii) a heavy chain comprising SEQ ID NO: 45 and a light chain comprising SEQ ID NO: 46;
(xxiv) a heavy chain comprising SEQ ID NO: 47 and a light chain comprising SEQ ID NO: 48;
(xxv) a heavy chain comprising SEQ ID NO: 49 and a light chain comprising SEQ ID NO: 50;
(xxvi) a heavy chain comprising SEQ ID NO: 51 and a light chain comprising SEQ ID NO: 52;
(xxvii) a heavy chain comprising SEQ ID NO: 53 and a light chain comprising SEQ ID NO: 54;
(xxviii) a heavy chain comprising SEQ ID NO: 55 and a light chain comprising SEQ ID NO: 56;
(xxix) a heavy chain comprising SEQ ID NO: 57 and a light chain comprising SEQ ID NO: 58;
(xxx) a heavy chain comprising SEQ ID NO: 59 and a light chain comprising SEQ ID NO: 60;
(xxxi) a heavy chain comprising SEQ ID NO: 61 and a light chain comprising SEQ ID NO: 62;
(xxxii) a heavy chain comprising SEQ ID NO: 63 and a light chain comprising SEQ ID NO: 64;
(xxxiii) a heavy chain comprising SEQ ID NO: 65 and a light chain comprising SEQ ID NO: 66;
(xxxiv) a heavy chain comprising SEQ ID NO: 67 and a light chain comprising SEQ ID NO: 68;
(xxxv) a heavy chain comprising SEQ ID NO: 69 and a light chain comprising SEQ ID NO: 70;
(xxxvi) a heavy chain comprising SEQ ID NO: 71 and a light chain comprising SEQ ID NO: 72;
(xxxvii) a heavy chain comprising SEQ ID NO: 73 and a light chain comprising SEQ ID NO: 74;
(xxxviii) a heavy chain comprising SEQ ID NO: 75 and a light chain comprising SEQ ID NO: 76;
(xxxix) a heavy chain comprising SEQ ID NO: 77 and a light chain comprising SEQ ID NO: 78;
(xl) a heavy chain comprising SEQ ID NO: 79 and a light chain comprising SEQ ID NO: 80;
(xli) a heavy chain comprising SEQ ID NO: 81 and a light chain comprising SEQ ID NO: 82;
(xlii) a heavy chain comprising SEQ ID NO: 83 and a light chain comprising SEQ ID NO: 84;

(xliii) a heavy chain comprising SEQ ID NO: 85 and a light chain comprising SEQ ID NO: 86;

(xliv) a heavy chain comprising SEQ ID NO: 87 and a light chain comprising SEQ ID NO: 88; or

(xlv) a heavy chain comprising SEQ ID NO: 89 and a light chain comprising SEQ ID NO: 90.

(xlvi) a heavy chain comprising SEQ ID NO: 91 and a light chain comprising SEQ ID NO: 92;

(xlvii) a heavy chain comprising SEQ ID NO: 93 and a light chain comprising SEQ ID NO: 94;

(xlviii) a heavy chain comprising SEQ ID NO: 95 and a light chain comprising SEQ ID NO: 96;

(xlix) a heavy chain comprising SEQ ID NO: 97 and a light chain comprising SEQ ID NO: 98;

(l) a heavy chain comprising SEQ ID NO: 99 and a light chain comprising SEQ ID NO: 100;

(li) a heavy chain comprising SEQ ID NO: 101 and a light chain comprising SEQ ID NO: 102;

(lii) a heavy chain comprising SEQ ID NO: 103 and a light chain comprising SEQ ID NO: 104;

(liii) a heavy chain comprising SEQ ID NO: 105 and a light chain comprising SEQ ID NO: 106;

(liv) a heavy chain comprising SEQ ID NO: 107 and a light chain comprising SEQ ID NO: 108;

(lv) a heavy chain comprising SEQ ID NO: 109 and a light chain comprising SEQ ID NO: 110;

(lvi) a heavy chain comprising SEQ ID NO: 111 and a light chain comprising SEQ ID NO: 112;

(lvii) a heavy chain comprising SEQ ID NO: 113 and a light chain comprising SEQ ID NO: 114;

(lviii) a heavy chain comprising SEQ ID NO: 115 and a light chain comprising SEQ ID NO: 116;

(lix) a heavy chain comprising SEQ ID NO: 117 and a light chain comprising SEQ ID NO: 118;

(lx) a heavy chain comprising SEQ ID NO: 119 and a light chain comprising SEQ ID NO: 120;

(lxi) a heavy chain comprising SEQ ID NO: 121 and a light chain comprising SEQ ID NO: 122;

(lxii) a heavy chain comprising SEQ ID NO: 123 and a light chain comprising SEQ ID NO: 124;

(lxiii) a heavy chain comprising SEQ ID NO: 125 and a light chain comprising SEQ ID NO: 126;

(lxiv) a heavy chain comprising SEQ ID NO: 127 and a light chain comprising SEQ ID NO: 128;

(lxv) a heavy chain comprising SEQ ID NO: 129 and a light chain comprising SEQ ID NO: 130;

(lxvi) a heavy chain comprising SEQ ID NO: 131 and a light chain comprising SEQ ID NO: 132;

(lxvii) a heavy chain comprising SEQ ID NO: 133 and a light chain comprising SEQ ID NO: 134;

(lxviii) a heavy chain comprising SEQ ID NO: 135 and a light chain comprising SEQ ID NO: 136;

(lxix) a heavy chain comprising SEQ ID NO: 137 and a light chain comprising SEQ ID NO: 138;

(lxx) a heavy chain comprising SEQ ID NO: 139 and a light chain comprising SEQ ID NO: 140;

(lxxi) a heavy chain comprising SEQ ID NO: 141 and a light chain comprising SEQ ID NO: 142;

(lxxii) a heavy chain comprising SEQ ID NO: 143 and a light chain comprising SEQ ID NO: 144;

(lxxiii) a heavy chain comprising SEQ ID NO: 145 and a light chain comprising SEQ ID NO: 146;

(lxxiv) a heavy chain comprising SEQ ID NO: 147 and a light chain comprising SEQ ID NO: 148;

(lxxv) a heavy chain comprising SEQ ID NO: 149 and a light chain comprising SEQ ID NO: 150;

(lxxvi) a heavy chain comprising SEQ ID NO: 151 and a light chain comprising SEQ ID NO: 152;

(lxxvii) a heavy chain comprising SEQ ID NO: 153 and a light chain comprising SEQ ID NO: 154;

(lxxviii) a heavy chain comprising SEQ ID NO: 155 and a light chain comprising SEQ ID NO: 156;

(lxxix) a heavy chain comprising SEQ ID NO: 157 and a light chain comprising SEQ ID NO: 158;

(lxxx) a heavy chain comprising SEQ ID NO: 159 and a light chain comprising SEQ ID NO: 160;

(lxxxi) a heavy chain comprising SEQ ID NO: 161 and a light chain comprising SEQ ID NO: 162;

(lxxxii) a heavy chain comprising SEQ ID NO: 163 and a light chain comprising SEQ ID NO: 164;

(lxxxiii) a heavy chain comprising SEQ ID NO: 165 and a light chain comprising SEQ ID NO: 166;

(lxxxiv) a heavy chain comprising SEQ ID NO: 167 and a light chain comprising SEQ ID NO: 168;

(lxxxv) a heavy chain comprising SEQ ID NO: 169 and a light chain comprising SEQ ID NO: 170;

(lxxxvi) a heavy chain comprising SEQ ID NO: 171 and a light chain comprising SEQ ID NO: 172;

(lxxxvii) a heavy chain comprising SEQ ID NO: 173 and a light chain comprising SEQ ID NO: 174;

(lxxxviii) a heavy chain comprising SEQ ID NO: 175 and a light chain comprising SEQ ID NO: 176;

(lxxxix) a heavy chain comprising SEQ ID NO: 177 and a light chain comprising SEQ ID NO: 178;

(xc) a heavy chain comprising SEQ ID NO: 179 and a light chain comprising SEQ ID NO: 180;

(xci) a heavy chain comprising SEQ ID NO: 181 and a light chain comprising SEQ ID NO: 182;

(xcii) a heavy chain comprising SEQ ID NO: 183 and a light chain comprising SEQ ID NO: 184;

(xciii) a heavy chain comprising SEQ ID NO: 185 and a light chain comprising SEQ ID NO: 186;

(xciv) a heavy chain comprising SEQ ID NO: 187 and a light chain comprising SEQ ID NO: 188;

(xcv) a heavy chain comprising SEQ ID NO: 189 and a light chain comprising SEQ ID NO: 190;

(xcvi) a heavy chain comprising SEQ ID NO: 191 and a light chain comprising SEQ ID NO: 192;

(xcvii) a heavy chain comprising SEQ ID NO: 193 and a light chain comprising SEQ ID NO: 194;

(xciii) a heavy chain comprising SEQ ID NO: 195 and a light chain comprising SEQ ID NO: 196;

(xcix) a heavy chain comprising SEQ ID NO: 197 and a light chain comprising SEQ ID NO: 198;

(c) a heavy chain comprising SEQ ID NO: 199 and a light chain comprising SEQ ID NO: 200;

(ci) a heavy chain comprising SEQ ID NO: 201 and a light chain comprising SEQ ID NO: 202;

(cii) a heavy chain comprising SEQ ID NO: 203 and a light chain comprising SEQ ID NO: 204;

(ciii) a heavy chain comprising SEQ ID NO: 205 and a light chain comprising SEQ ID NO: 206;

(civ) a heavy chain comprising SEQ ID NO: 207 and a light chain comprising SEQ ID NO: 208;

(cv) a heavy chain comprising SEQ ID NO: 209 and a light chain comprising SEQ ID NO: 210;

(cvi) a heavy chain comprising SEQ ID NO: 211 and a light chain comprising SEQ ID NO: 212;

(cvii) a heavy chain comprising SEQ ID NO: 213 and a light chain comprising SEQ ID NO: 214;

(cxiii) a heavy chain comprising SEQ ID NO: 215 and a light chain comprising SEQ ID NO: 216;

(cix) a heavy chain comprising SEQ ID NO: 217 and a light chain comprising SEQ ID NO: 218;

(cx) a heavy chain comprising SEQ ID NO: 219 and a light chain comprising SEQ ID NO: 220;

(cxi) a heavy chain comprising SEQ ID NO: 221 and a light chain comprising SEQ ID NO: 222;

(cxii) a heavy chain comprising SEQ ID NO: 223 and a light chain comprising SEQ ID NO: 224;

(cxiii) a heavy chain comprising SEQ ID NO: 225 and a light chain comprising SEQ ID NO: 226;

(cxiv) a heavy chain comprising SEQ ID NO: 227 and a light chain comprising SEQ ID NO: 228;

(cxv) a heavy chain comprising SEQ ID NO: 229 and a light chain comprising SEQ ID NO: 230;

(cxvi) a heavy chain comprising SEQ ID NO: 231 and a light chain comprising SEQ ID NO: 232;

(cxvii) a heavy chain comprising SEQ ID NO: 233 and a light chain comprising SEQ ID NO: 234;

(cxiii) a heavy chain comprising SEQ ID NO: 235 and a light chain comprising SEQ ID NO: 236;

(cxix) a heavy chain comprising SEQ ID NO: 237 and a light chain comprising SEQ ID NO: 238;

(cxx) a heavy chain comprising SEQ ID NO: 239 and a light chain comprising SEQ ID NO: 240;

(cxxi) a heavy chain comprising SEQ ID NO: 241 and a light chain comprising SEQ ID NO: 242;

(cxxii) a heavy chain comprising SEQ ID NO: 243 and a light chain comprising SEQ ID NO: 244;

(cxxiii) a heavy chain comprising SEQ ID NO: 245 and a light chain comprising SEQ ID NO: 246;

(cxxiv) a heavy chain comprising SEQ ID NO: 247 and a light chain comprising SEQ ID NO: 248;

(cxxv) a heavy chain comprising SEQ ID NO: 249 and a light chain comprising SEQ ID NO: 250;

(cxxvi) a heavy chain comprising SEQ ID NO: 251 and a light chain comprising SEQ ID NO: 252;

(cxxvii) a heavy chain comprising SEQ ID NO: 253 and a light chain comprising SEQ ID NO: 254;

(cxxviii) a heavy chain comprising SEQ ID NO: 255 and a light chain comprising SEQ ID NO: 256;

(cxxvix) a heavy chain comprising SEQ ID NO: 257 and a light chain comprising SEQ ID NO: 258;

(cxxxx) a heavy chain comprising SEQ ID NO: 259 and a light chain comprising SEQ ID NO: 260;

(cxxxi) a heavy chain comprising SEQ ID NO: 261 and a light chain comprising SEQ ID NO: 262;

(cxxxii) a heavy chain comprising SEQ ID NO: 263 and a light chain comprising SEQ ID NO: 264;

(cxxxiii) a heavy chain comprising SEQ ID NO: 265 and a light chain comprising SEQ ID NO: 266;

(cxxxiv) a heavy chain comprising SEQ ID NO: 267 and a light chain comprising SEQ ID NO: 268;

(cxxxv) a heavy chain comprising SEQ ID NO: 269 and a light chain comprising SEQ ID NO: 270;

(cxxxvi) a heavy chain comprising SEQ ID NO: 271 and a light chain comprising SEQ ID NO: 272;

(cxxxvii) a heavy chain comprising SEQ ID NO: 273 and a light chain comprising SEQ ID NO: 274;

(cxxxviii) a heavy chain comprising SEQ ID NO: 275 and a light chain comprising SEQ ID NO: 276;

(cxxxix) a heavy chain comprising SEQ ID NO: 277 and a light chain comprising SEQ ID NO: 278;

(cxl) a heavy chain comprising SEQ ID NO: 279 and a light chain comprising SEQ ID NO: 280;

(cxli) a heavy chain comprising SEQ ID NO: 281 and a light chain comprising SEQ ID NO: 282;

(cxlii) a heavy chain comprising SEQ ID NO: 283 and a light chain comprising SEQ ID NO: 284;

(cxliii) a heavy chain comprising SEQ ID NO: 285 and a light chain comprising SEQ ID NO: 286;

(cxliv) a heavy chain comprising SEQ ID NO: 287 and a light chain comprising SEQ ID NO: 288;

(cxlv) a heavy chain comprising SEQ ID NO: 289 and a light chain comprising SEQ ID NO: 290;

(cxlvi) a heavy chain comprising SEQ ID NO: 291 and a light chain comprising SEQ ID NO: 292;

(cxlvii) a heavy chain comprising SEQ ID NO: 293 and a light chain comprising SEQ ID NO: 294;

(cxlviii) a heavy chain comprising SEQ ID NO: 295 and a light chain comprising SEQ ID NO: 296;

(cxlvix) a heavy chain comprising SEQ ID NO: 297 and a light chain comprising SEQ ID NO: 298;

(cl) a heavy chain comprising SEQ ID NO: 299 and a light chain comprising SEQ ID NO: 300;

(cli) a heavy chain comprising SEQ ID NO: 301 and a light chain comprising SEQ ID NO: 302;

(clii) a heavy chain comprising SEQ ID NO: 303 and a light chain comprising SEQ ID NO: 304;

(clii) a heavy chain comprising SEQ ID NO: 305 and a light chain comprising SEQ ID NO: 306;

(cliv) a heavy chain comprising SEQ ID NO: 307 and a light chain comprising SEQ ID NO: 308;

(clv) a heavy chain comprising SEQ ID NO: 309 and a light chain comprising SEQ ID NO: 310;

(clvi) a heavy chain comprising SEQ ID NO: 311 and a light chain comprising SEQ ID NO: 312;

(clvii) a heavy chain comprising SEQ ID NO: 313 and a light chain comprising SEQ ID NO: 314;

(clviii) a heavy chain comprising SEQ ID NO: 315 and a light chain comprising SEQ ID NO: 316;

**EP 4 545 559 A2**

(clix) a heavy chain comprising SEQ ID NO: 317 and a light chain comprising SEQ ID NO: 318;
(clx) a heavy chain comprising SEQ ID NO: 319 and a light chain comprising SEQ ID NO: 320;
(clxi) a heavy chain comprising SEQ ID NO: 321 and a light chain comprising SEQ ID NO: 322;
(clxii) a heavy chain comprising SEQ ID NO: 323 and a light chain comprising SEQ ID NO: 324;
(clxiii) a heavy chain comprising SEQ ID NO: 325 and a light chain comprising SEQ ID NO: 326;
(clxiv) a heavy chain comprising SEQ ID NO: 327 and a light chain comprising SEQ ID NO: 328;
(clxv) a heavy chain comprising SEQ ID NO: 329 and a light chain comprising SEQ ID NO: 330;
(clxvi) a heavy chain comprising SEQ ID NO: 331 and a light chain comprising SEQ ID NO: 322;
(clxvii) a heavy chain comprising SEQ ID NO: 333 and a light chain comprising SEQ ID NO: 334;
(clxvii) a heavy chain comprising SEQ ID NO: 335 and a light chain comprising SEQ ID NO: 336;
(clxix) a heavy chain comprising SEQ ID NO: 337 and a light chain comprising SEQ ID NO: 338;
(clxx) a heavy chain comprising SEQ ID NO: 339 and a light chain comprising SEQ ID NO: 340;
(clxxi) a heavy chain comprising SEQ ID NO: 341 and a light chain comprising SEQ ID NO: 342;
(clxxii) a heavy chain comprising SEQ ID NO: 343 and a light chain comprising SEQ ID NO: 344;
(clxxiii) a heavy chain comprising SEQ ID NO: 345 and a light chain comprising SEQ ID NO: 346;
(clxxiv) a heavy chain comprising SEQ ID NO: 347 and a light chain comprising SEQ ID NO: 348;
(clxxv) a heavy chain comprising SEQ ID NO: 349 and a light chain comprising SEQ ID NO: 350;
(clxxvi) a heavy chain comprising SEQ ID NO: 351 and a light chain comprising SEQ ID NO: 352;
(clxxvii) a heavy chain comprising SEQ ID NO: 353 and a light chain comprising SEQ ID NO: 354;
(clxxviii) a heavy chain comprising SEQ ID NO: 355 and a light chain comprising SEQ ID NO: 356;
(clxxix) a heavy chain comprising SEQ ID NO: 357 and a light chain comprising SEQ ID NO: 358; or
(clxxx) a heavy chain comprising SEQ ID NO: 359 and a light chain comprising SEQ ID NO: 360.

3. Pharmaceutical composition comprising the monoclonal antibody as defined by claims 1 and 2 and a pharmaceutically acceptable carrier, excipient or diluent.

4. Use of the monoclonal antibody as defined by items 1 and 2 or of the pharmaceutical composition as defined by claim 3, wherein it is for the preparation of a drug for preventive treatment in the therapy of COVID19.

5. Use, according to claim 4, wherein the treatment of COVID-19 consists of neutralizing the SARS-CoV-2 virus, preventing systemic inflammation, viral inhibition and inhibition of inflammation caused by the viral infection.

6. Use of the monoclonal antibody as defined by claims 1 and 2, wherein it is for the generation of cell lines expressing human monoclonal antibodies, derived from B cells of people infected and/or vaccinated with the SARS-CoV-2 virus.

7. Use of the monoclonal antibody as defined by claims 1 and 2, wherein it is for the production of one or more antibodies that recognize the RBD region of SARS-CoV-2, including variants resulting from virus mutation.

8. Use of the monoclonal antibody as defined by claims 1 and 2, wherein it is for prophylactic use of monoclonal antibodies recognizing the SARS-CoV-2 virus.

9. Use of the monoclonal antibody as defined by claims 1 and 2, wherein it is for use as a diagnosis of such monoclonal antibodies.

10. Kit for detecting COVID-19 in a biological sample comprising a monoclonal antibody or a biologically active fragment thereof, as defined by any one of claims 1 and 2, wherein the monoclonal antibody or fragment thereof is linked to a detectable fraction.

Thus, the embodiments presented in the present invention do not limit the totality of possibilities, and it will be understood that various omissions, substitutions and changes can be made by a person skilled in the art, without departing from the scope of the present invention.

It is expressly provided that all combinations of the elements that perform the same function in substantially the same way to achieve the same results are within the scope of the invention. Substitutions of elements from one described embodiment to another are also fully intended and contemplated.

The person skilled in the art will value the knowledge presented here and will be able to reproduce the invention in the presented embodiments and in other variants, covered in the scope of the claims.

REFERENCES

ALIPRANDINI E, TAKATA DY, LEPIQUE A, KALIL J, BOSCARDIN SB, MORO AM. An oligoclonal combination of human monoclonal antibodies able to neutralize tetanus toxin in vivo. Toxicon X, 2019; 2:100006. doi:10.1016/j.toxcx.2019.100006
GIUDICELLI, V.; BROCHET, X.; LEFRANC, M.-P. IMGT/V-QUEST: IMGT Standardized Analysis of the Immunoglobulin (IG) and T Cell Receptor (TR) Nucleotide Sequences. Cold Spring Harbor Protocols, v. 2011, n. 6, 2011. https://doi.org/10.1101/pdb.prot5633

LOPES DOS SANTOS M, YEDA FP, TSURUTA LR, HORTA BB, PIMENTA AA JR, DEGAKI TL, SOARES IC, TUMA MC, OKAMOTO OK, ALVES VA, OLD LJ, RITTER G, MORO AM. Rebmab200, a humanized monoclonal antibody targeting the sodium phosphate transporter NaPi2b displays strong immune-mediated cytotoxicity against cancer: a novel reagent for targeted antibody therapy of cancer. PLoS One. 2013; 8(7): e70332. doi: 10.1371/journal.pone.0070332. PMID: 23936189

LUCHESE MD, LOPES DOS SANTOS M, GARBUIO A, TARGINO RC, MANSUELI CP, TSURUTA LR, QUINTILIO W, MORO AM. A new CHO (Chinese hamster ovary)-derived cell line expressing anti-TNFα monoclonal antibody with biosimilar potential. Immunol Res. 2018, 66(3):392-405. doi: 10.1007/s12026-018-8997-4

ROBBIANI, D F.; GAEBLER, C; MUECKSCH, F; LORENZI, JC. C.; WANG, Z; CHO, A; AGUDELO, M; BARNES, CO.; GAZUMYAN, A; FINKIN, S. Convergent antibody responses to SARS-CoV-2 in convalescent individuals. Nature, [S.L.], v. 584, 437-442, 2020. http://dx.doi.org/10.1038/s41586-020-2456-9.

TILLER, T. et al. Efficient generation of monoclonal antibodies from single human B cells by single cell RT-PCR and expression vector cloning. Journal of immunological methods, v. 329, n. 1-2, p. 112-124, 2008. https://doi.org/10.1016/j.jim.2007.09.017

WARDEMANN, H; KOFER, J. Expression Cloning of Human B Cell Immunoglobulins. Methods In Molecular Biology, p. 93-111, 2012. Humana Press. http://dx.doi.org/10.1007/978-1-62703-269-8 5.

## Claims

1. Anti-SARS-COV-2 human monoclonal antibody, **characterized in that** the antibody comprises an antibody heavy chain and an antibody light chain, said chains comprising complementarity determining regions (CDRs) selected from (i) - (clxxx):

(i) heavy chain CDR1, CDR2 and CDR3 regions having the amino acid sequences of positions 26-33, positions 51-57 and positions 96-106 of SEQ ID NO: 1, respectively; and light chain CDR1, CDR2 and CDR3 regions with the amino acid sequences of positions 27-32, positions 50-52 and positions 89-97 of SEQ ID NO: 2, respectively;
(ii) heavy chain CDR1, CDR2 and CDR3 regions having the amino acid sequences from positions 26-33, positions 50-57 and positions 96-106 of SEQ ID NO: 3, respectively; and light chain CDR1, CDR2 and CDR3 regions with the amino acid sequences of positions 27-32, positions 50-52 and positions 89-97 of SEQ ID NO: 4, respectively;
(iii) heavy chain CDR1, CDR2 and CDR3 regions having the amino acid sequences from positions 26-33, positions 51-57 and positions 96-107 of SEQ ID NO: 5, respectively; and light chain CDR1, CDR2 and CDR3 regions having the amino acid sequences of positions 26-31, positions 49-51 and positions 88-98 of SEQ ID NO: 6, respectively;
(iv) heavy chain CDR1, CDR2 and CDR3 regions with the amino acid sequences of positions 26-33, positions 51-58 and positions 97-109 of SEQ ID NO: 7, respectively; and light chain CDR1, CDR2 and CDR3 regions having the amino acid sequences of positions 26-31, positions 49-51 and positions 88-97 of SEQ ID NO: 8, respectively;
(v) heavy chain CDR1, CDR2 and CDR3 regions with the amino acid sequences of positions 26-33, positions 51-58 and positions 97-117 of SEQ ID NO: 9, respectively; and light chain CDR1, CDR2 and CDR3 regions having the amino acid sequences of positions 27-32, positions 50-52 and positions 89-97 of SEQ ID NO: 10, respectively;
(vi) heavy chain CDR1, CDR2 and CDR3 regions with the amino acid sequences of positions 26-33, positions 51-58 and positions 97-112 of SEQ ID NO: 11, respectively; and light chain CDR1, CDR2 and CDR3 regions with the amino acid sequences of positions 27-32, positions 50-52 and positions 89-97 of SEQ ID NO: 12, respectively;
(vii) heavy chain CDR1, CDR2 and CDR3 regions with the amino acid sequences of positions 26-33, positions 51-57 and positions 96-107 of SEQ ID NO: 13, respectively; and light chain CDR1, CDR2 and CDR3 regions having the amino acid sequences of positions 26-31, positions 49-51 and positions 88-98 of SEQ ID NO: 14, respectively;
(viii) heavy chain CDR1, CDR2 and CDR3 regions with the amino acid sequences of positions 26-33, positions 51-57 and positions 106-116 of SEQ ID NO: 15, respectively; and light chain CDR1, CDR2 and CDR3 regions having the amino acid sequences of positions 27-33, positions 51-53 and positions 90-98 of SEQ ID NO: 16, respectively;
(ix) heavy chain CDR1, CDR2 and CDR3 regions with the amino acid sequences of positions 26-33, positions 51-58 and positions 97-114 of SEQ ID NO: 17, respectively; and light chain CDR1, CDR2 and CDR3 regions having the amino acid sequences of positions 27-32, positions 50-52 and positions 89-97 of SEQ ID NO: 18, respectively;

(x) heavy chain CDR1, CDR2 and CDR3 regions with the amino acid sequences of positions 26-33, positions 51-58 and positions 97-109 of SEQ ID NO: 19, respectively; and light chain CDR1, CDR2 and CDR3 regions with the amino acid sequences of positions 26-31, positions 49-51 and positions 88-97 of SEQ ID NO: 20, respectively;

(xi) heavy chain CDR1, CDR2 and CDR3 regions with the amino acid sequences of positions 26-33, positions 51-58 and positions 97-112 of SEQ ID NO: 21, respectively; and light chain CDR1, CDR2 and CDR3 regions with the amino acid sequences of positions 27-32, positions 50-52 and positions 89-97 of SEQ ID NO: 22, respectively,

(xii) heavy chain CDR1, CDR2 and CDR3 regions with the amino acid sequences of positions 26-33, positions 51-57 and positions 96-106 of SEQ ID NO: 23, respectively; and light chain CDR1, CDR2 and CDR3 regions with the amino acid sequences of positions 27-32, positions 50-52 and positions 89-98 of SEQ ID NO: 24, respectively,

(xiii) heavy chain CDR1, CDR2 and CDR3 regions with the amino acid sequences of positions 26-33, positions 51-57 and positions 96-106 of SEQ ID NO: 25, respectively; and light chain CDR1, CDR2 and CDR3 regions with the amino acid sequences of positions 27-32, positions 50-52 and positions 89-97 of SEQ ID NO: 26, respectively,

(xiv) heavy chain CDR1, CDR2 and CDR3 regions with the amino acid sequences of positions 26-33, positions 51-58 and positions 97-119 of SEQ ID NO: 27, respectively; and light chain CDR1, CDR2 and CDR3 regions with the amino acid sequences of positions 26-33, positions 51-53 and positions 92-101 of SEQ ID NO: 28, respectively;

(xv) heavy chain CDR1, CDR2 and CDR3 regions with the amino acid sequences of positions 26-33, positions 51-58 and positions 97-116 of SEQ ID NO: 29, respectively; and light chain CDR1, CDR2 and CDR3 regions having the amino acid sequences of positions 27-32, positions 50-52 and positions 89-97 of SEQ ID NO: 30, respectively;

(xvi) heavy chain CDR1, CDR2 and CDR3 regions with the amino acid sequences of positions 26-33, positions 51-58 and positions 97-116 of SEQ ID NO: 31, respectively; and light chain CDR1, CDR2 and CDR3 regions with the amino acid sequences of positions 27-32, positions 50-52 and positions 89-98 of SEQ ID NO: 32, respectively;

(xvii) heavy chain CDR1, CDR2 and CDR3 regions with the amino acid sequences of positions 26-33, positions 51-57 and positions 96-106 of SEQ ID NO: 33, respectively; and light chain CDR1, CDR2 and CDR3 regions having the amino acid sequences of positions 27-32, positions 50-52 and positions 89-98 of SEQ ID NO: 34, respectively;

(xviii) heavy chain CDR1, CDR2 and CDR3 regions with the amino acid sequences of positions 26-35, positions 53-59 and positions 98-109 of SEQ ID NO: 35, respectively; and light chain CDR1, CDR2 and CDR3 regions with the amino acid sequences of positions 25-34, positions 52-54 and positions 91-102 of SEQ ID NO: 36, respectively;

(xix) heavy chain CDR1, CDR2 and CDR3 regions with the amino acid sequences of positions 26-37, positions 51-57 and positions 96-107 of SEQ ID NO: 37, respectively; and light chain CDR1, CDR2 and CDR3 regions having the amino acid sequences of positions 27-32, positions 50-52 and positions 89-97 of SEQ ID NO: 38, respectively;

(xx) heavy chain CDR1, CDR2 and CDR3 regions with the amino acid sequences of positions 26-33, positions 51-57 and positions 96-107 of SEQ ID NO: 39, respectively; and light chain CDR1, CDR2 and CDR3 regions with the amino acid sequences of positions 27-33, positions 51-53 and positions 90-98 of SEQ ID NO: 40, respectively;

(xxi) heavy chain CDR1, CDR2 and CDR3 regions having the amino acid sequences of positions 26-33, positions 51-58 and positions 97-112 of SEQ ID NO: 41, respectively; and light chain CDR1, CDR2 and CDR3 regions having the amino acid sequences of positions 27-37, positions 55-57 and positions 94-101 of SEQ ID NO: 42, respectively;

(xxii) heavy chain CDR1, CDR2 and CDR3 regions with the amino acid sequences of positions 26-33, positions 51-58 and positions 97-115 of SEQ ID NO: 43, respectively; and light chain CDR1, CDR2 and CDR3 regions having the amino acid sequences of positions 27-32, positions 50-52 and positions 89-96 of SEQ ID NO: 44, respectively;

(xxiii) heavy chain CDR1, CDR2 and CDR3 regions with the amino acid sequences of positions 26-33, positions 51-58 and positions 97-116 of SEQ ID NO: 45, respectively; and light chain CDR1, CDR2 and CDR3 regions having the amino acid sequences of positions 27-32, positions 50-52 and positions 89-98 of SEQ ID NO: 46, respectively;

(xxiv) heavy chain CDR1, CDR2 and CDR3 regions with the amino acid sequences of positions 26-33, positions 51-58 and positions 97-121 of SEQ ID NO: 47, respectively; and light chain CDR1, CDR2 and CDR3 regions having the amino acid sequences of positions 27-32, positions 50-52 and positions 89-97 of SEQ ID NO: 48, respectively;

(xxv) heavy chain CDR1, CDR2 and CDR3 regions with the amino acid sequences of positions 26-33, positions 51-57 and positions 96-106 of SEQ ID NO: 49, respectively; and light chain CDR1, CDR2 and CDR3 regions having the amino acid sequences of positions 27-33, positions 51-53 and positions 90-97 of SEQ ID NO: 50, respectively;

(xxvi) heavy chain CDR1, CDR2 and CDR3 regions with the amino acid sequences of positions 26-33, positions 51-58 and positions 97-109 of SEQ ID NO: 51, respectively; and light chain CDR1, CDR2 and CDR3 regions having the amino acid sequences of positions 27-32, positions 50-52 and positions 89-98 of SEQ ID NO: 52, respectively;

(xxvii) heavy chain CDR1, CDR2 and CDR3 regions with the amino acid sequences of positions 26-33, positions 51-58 and positions 97-112 of SEQ ID NO: 53, respectively; and light chain CDR1, CDR2 and CDR3 regions having the amino acid sequences of positions 27-38, positions 56-58 and positions 95-103 of SEQ ID NO: 54, respectively;

(xxviii) heavy chain CDR1, CDR2 and CDR3 regions with the amino acid sequences of positions 26-33, positions 51-58 and positions 97-108 of SEQ ID NO: 55, respectively; and light chain CDR1, CDR2 and CDR3 regions with the amino acid sequences of positions 27-32, positions 50-52 and positions 89-98 of SEQ ID NO: 56, respectively

(xxix) heavy chain CDR1, CDR2 and CDR3 regions with the amino acid sequences of positions 26-33, positions 51-58 and positions 97-115 of SEQ ID NO: 57, respectively; and light chain CDR1, CDR2 and CDR3 regions having the amino acid sequences of positions 27-38, positions 56-58 and positions 95-104 of SEQ ID NO: 58, respectively;

(xxx) heavy chain CDR1, CDR2 and CDR3 regions with the amino acid sequences of positions 26-33, positions 51-58 and positions 97-110 of SEQ ID NO: 59, respectively; and light chain CDR1, CDR2 and CDR3 regions with the amino acid sequences of positions 27-31, positions 50-52 and positions 89-97 of SEQ ID NO: 60, respectively;

(xxxi) heavy chain CDR1, CDR2 and CDR3 regions with the amino acid sequences of positions 26-33, positions 51-58 and positions 97-112 of SEQ ID NO: 61, respectively; and light chain CDR1, CDR2 and CDR3 regions with the amino acid sequences of positions 27-37, positions 55-57 and positions 94-101 of SEQ ID NO: 62, respectively;

(xxxii) heavy chain CDR1, CDR2 and CDR3 regions with the amino acid sequences of positions 26-33, positions 51-58 and positions 97-106 of SEQ ID NO: 63, respectively; and light chain CDR1, CDR2 and CDR3 regions with the amino acid sequences of positions 27-32, positions 50-52 and positions 89-98 of SEQ ID NO: 64, respectively;

(xxxiii) heavy chain CDR1, CDR2 and CDR3 regions with the amino acid sequences of positions 26-35, positions 53-59 and positions 98-112 of SEQ ID NO: 65, respectively; and light chain CDR1, CDR2 and CDR3 regions having the amino acid sequences of positions 26-31, positions 49-51 and positions 88-96 of SEQ ID NO: 66, respectively;

(xxxiv) heavy chain CDR1, CDR2 and CDR3 regions with the amino acid sequences of positions 26-33, positions 51-60 and positions 99-114 of SEQ ID NO: 67, respectively; and light chain CDR1, CDR2 and CDR3 regions with the amino acid sequences of positions 27-33, positions 51-53 and positions 90-98 of SEQ ID NO: 68, respectively;

(xxxv) heavy chain CDR1, CDR2 and CDR3 regions with the amino acid sequences of positions 26-33, positions 51-58 and positions 97-113 of SEQ ID NO: 69, respectively; and light chain CDR1, CDR2 and CDR3 regions having the amino acid sequences of positions 27-32, positions 50-52 and positions 89-96 of SEQ ID NO: 70, respectively;

(xxxvi) heavy chain CDR1, CDR2 and CDR3 regions with the amino acid sequences of positions 26-33, positions 51-58 and positions 97-115 of SEQ ID NO: 71, respectively; and light chain CDR1, CDR2 and CDR3 regions having the amino acid sequences of positions 27-32, positions 50-52 and positions 89-98 of SEQ ID NO: 72, respectively;

(xxxvii) heavy chain CDR1, CDR2 and CDR3 regions with the amino acid sequences of positions 26-33, positions 51-58 and positions 97-114 of SEQ ID NO: 73, respectively; and light chain CDR1, CDR2 and CDR3 regions having the amino acid sequences of positions 26-31, positions 49-51 and positions 88-96 of SEQ ID NO: 74, respectively;

(xxxviii) heavy chain CDR1, CDR2 and CDR3 regions with the amino acid sequences of positions 26-33, positions 51-58 and positions 97-120 of SEQ ID NO: 75, respectively; and light chain CDR1, CDR2 and CDR3 regions having the amino acid sequences of positions 27-32, positions 50-52 and positions 89-98 of SEQ ID NO: 76, respectively;

(xxxxi) heavy chain CDR1, CDR2 and CDR3 regions with the amino acid sequences of positions 26-33, positions 51-58 and positions 97-111 of SEQ ID NO: 77, respectively; and light chain CDR1, CDR2 and CDR3 regions having the amino acid sequences of positions 27-32, positions 50-52 and positions 89-97 of SEQ ID NO: 78, respectively;

(xl) heavy chain CDR1, CDR2 and CDR3 regions with the amino acid sequences of positions 26-33, positions 51-58 and positions 97-121 of SEQ ID NO: 79, respectively; and light chain CDR1, CDR2 and CDR3 regions with the amino acid sequences of positions 26-31, positions 49-51 and positions 88-96 of SEQ ID NO: 80, respectively;

(xli) heavy chain CDR1, CDR2 and CDR3 regions having the amino acid sequences of positions 26-33, positions 51-58 and positions 97-110 of SEQ ID NO: 81, respectively; and light chain CDR1, CDR2 and CDR3 regions having the amino acid sequences of positions 27-32, positions 50-52 and positions 89-98 of SEQ ID NO: 82, respectively;

(xlii) heavy chain CDR1, CDR2 and CDR3 regions with the amino acid sequences of positions 26-33, positions 51-58 and positions 97-114 of SEQ ID NO: 83, respectively; and light chain CDR1, CDR2 and CDR3 regions having the amino acid sequences of positions 27-37, positions 55-57 and positions 94-102 of SEQ ID NO: 84, respectively;

(xliii) heavy chain CDR1, CDR2 and CDR3 regions with the amino acid sequences of positions 27-33, positions 51-57 and positions 96-106 of SEQ ID NO: 85, respectively; and light chain CDR1, CDR2 and CDR3 regions having the amino acid sequences of positions 27-32, positions 50-52 and positions 89-96 of SEQ ID NO: 86, respectively;

(xliv) heavy chain CDR1, CDR2 and CDR3 regions with the amino acid sequences of positions 29-36, positions 54-60 and positions 99-116 of SEQ ID NO: 87, respectively; and light chain CDR1, CDR2 and CDR3 regions having the amino acid sequences of positions 27-33, positions 51-53 and positions 91-98 of SEQ ID NO: 88, respectively;

(xlv) heavy chain CDR1, CDR2 and CDR3 regions with the amino acid sequences of positions 26-33, positions 51-58 and positions 97-118 of SEQ ID NO: 89, respectively; and light chain CDR1, CDR2 and CDR3 regions having the amino acid sequences of positions 31-39, positions 57-59 and positions 99-108 of SEQ ID NO: 90, respectively; or

(xlvi) heavy chain CDR1, CDR2 and CDR3 regions with the amino acid sequences of positions 26-33, positions 51-57 and positions 96-115 of SEQ ID NO: 91, respectively; and light chain CDR1, CDR2 and CDR3 regions with the amino acid sequences of positions 27-32, positions 50-52 and positions 89-98 of SEQ ID NO: 92, respectively.

(xlvii) heavy chain CDR1, CDR2 and CDR3 regions with the amino acid sequences of positions 26-33, positions 51-58 and positions 97-107 of SEQ ID NO: 93, respectively; and light chain CDR1, CDR2 and CDR3 regions having the amino acid sequences of positions 27-32, positions 51-53 and positions 90-99 of SEQ ID NO: 94, respectively;

(xlxi) heavy chain CDR1, CDR2 and CDR3 regions with the amino acid sequences of positions 26-33, positions 51-57 and positions 96-106 of SEQ ID NO: 95, respectively; and light chain CDR1, CDR2 and CDR3 regions having the amino acid sequences of positions 27-32, positions 50-52 and positions 89-99 of SEQ ID NO: 96, respectively;

(l) heavy chain CDR1, CDR2 and CDR3 regions with the amino acid sequences of positions 26-33, positions 51-57 and positions 96-105 of SEQ ID NO: 97, respectively; and light chain CDR1, CDR2 and CDR3 regions with the amino acid sequences of positions 26-31, positions 49-51 and positions 88-96 of SEQ ID NO: 98, respectively;

(li) heavy chain CDR1, CDR2 and CDR3 regions having the amino acid sequences of positions 26-33, positions 51-57 and positions 96-106 of SEQ ID NO: 99, respectively; and light chain CDR1, CDR2 and CDR3 regions having the amino acid sequences of positions 27-32, positions 50-52 and positions 89-96 of SEQ ID NO: 100, respectively;

(lii) heavy chain CDR1, CDR2 and CDR3 regions with the amino acid sequences of positions 26-35, positions 53-59 and positions 98-109 of SEQ ID NO: 101, respectively; and light chain CDR1, CDR2 and CDR3 regions having the amino acid sequences of positions 26-34, positions 52-524 and positions 91-102 of SEQ ID NO: 102, respectively;

(liii) heavy chain CDR1, CDR2 and CDR3 regions with the amino acid sequences of positions 26-33, positions 51-58 and positions 97-115 of SEQ ID NO: 103, respectively; and light chain CDR1, CDR2 and CDR3 regions having the amino acid sequences of positions 26-31, positions 49-51 and positions 88-96 of SEQ ID NO: 104, respectively;

(liii) heavy chain CDR1, CDR2 and CDR3 regions with the amino acid sequences of positions 26-33, positions 51-58 and positions 97-117 of SEQ ID NO: 105, respectively; and light chain CDR1, CDR2 and CDR3 regions having the amino acid sequences of positions 26-31, positions 49-51 and positions 88-95 of SEQ ID NO: 106, respectively;

(liv) heavy chain CDR1, CDR2 and CDR3 regions with the amino acid sequences of positions 26-33, positions 51-58 and positions 97-112 of SEQ ID NO: 107, respectively; and light chain CDR1, CDR2 and CDR3 regions having the amino acid sequences of positions 27-37, positions 55-57 and positions 89-101 of SEQ ID NO: 108, respectively;

(lv) heavy chain CDR1, CDR2 and CDR3 regions with the amino acid sequences of positions 26-33, positions 51-58 and positions 97-110 of SEQ ID NO: 107, respectively; and light chain CDR1, CDR2 and CDR3 regions having the amino acid sequences of positions 26-31, positions 49-51 and positions 88-98 of SEQ ID NO: 110, respectively;

(lvi) heavy chain CDR1, CDR2 and CDR3 regions with the amino acid sequences of positions 26-35, positions 53-59 and positions 98-115 of SEQ ID NO: 109, respectively; and light chain CDR1, CDR2 and CDR3 regions with the amino acid sequences of positions 26-34, positions 52-54 and positions 91-100 of SEQ ID NO: 112,

respectively;

(Ivii) heavy chain CDR1, CDR2 and CDR3 regions with the amino acid sequences of positions 26-33, positions 51-57 and positions 96-106 of SEQ ID NO: 113, respectively; and light chain CDR1, CDR2 and CDR3 regions with the amino acid sequences of positions 27-32, positions 50-52 and positions 89-98 of SEQ ID NO: 114, respectively;

(Iviii) heavy chain CDR1, CDR2 and CDR3 regions with the amino acid sequences of positions 26-33, positions 51-57 and positions 96-106 of SEQ ID NO: 115, respectively; and light chain CDR1, CDR2 and CDR3 regions having the amino acid sequences of positions 27-32, positions 50-52 and positions 89-96 of SEQ ID NO: 116, respectively;

(Ivix) heavy chain CDR1, CDR2 and CDR3 regions with the amino acid sequences of positions 26-33, positions 51-58 and positions 97-113 of SEQ ID NO: 117, respectively; and light chain CDR1, CDR2 and CDR3 regions having the amino acid sequences of positions 27-32, positions 50-52 and positions 89-93 of SEQ ID NO: 118, respectively;

(Ix) heavy chain CDR1, CDR2 and CDR3 regions with the amino acid sequences of positions 26-33, positions 51-58 and positions 97-110 of SEQ ID NO: 119, respectively; and light chain CDR1, CDR2 and CDR3 regions with the amino acid sequences of positions 27-32, positions 50-52 and positions 89-97 of SEQ ID NO: 120, respectively;

(Ixi) heavy chain CDR1, CDR2 and CDR3 regions having the amino acid sequences of positions 26-35, positions 53-59 and positions 98-113 of SEQ ID NO: 121, respectively; and light chain CDR1, CDR2 and CDR3 regions having the amino acid sequences of positions 26-34, positions 52-54 and positions 91-102 of SEQ ID NO: 122, respectively;

(Ixii) heavy chain CDR1, CDR2 and CDR3 regions with the amino acid sequences of positions 26-33, positions 51-57 and positions 96-106 of SEQ ID NO: 123, respectively; and light chain CDR1, CDR2 and CDR3 regions with the amino acid sequences of positions 27-32, positions 50-52 and positions 89-97 of SEQ ID NO: 124, respectively;

(Ixiii) heavy chain CDR1, CDR2 and CDR3 regions with the amino acid sequences of positions 26-33, positions 51-57 and positions 96-106 of SEQ ID NO: 125, respectively; and light chain CDR1, CDR2 and CDR3 regions with the amino acid sequences of positions 27-32, positions 50-52 and positions 89-97 of SEQ ID NO: 126, respectively;

(Ixiv) heavy chain CDR1, CDR2 and CDR3 regions with the amino acid sequences of positions 26-33, positions 51-58 and positions 97-114 of SEQ ID NO: 127, respectively; and light chain CDR1, CDR2 and CDR3 regions having the amino acid sequences of positions 27-32, positions 50-52 and positions 89-97 of SEQ ID NO: 128, respectively;

(Ixv) heavy chain CDR1, CDR2 and CDR3 regions with the amino acid sequences of positions 26-33, positions 51-58 and positions 97-110 of SEQ ID NO: 129, respectively; and light chain CDR1, CDR2 and CDR3 regions having the amino acid sequences of positions 26-31, positions 49-51 and positions 88-98 of SEQ ID NO: 130, respectively;

(Ixvi) heavy chain CDR1, CDR2 and CDR3 regions with the amino acid sequences of positions 26-33, positions 51-67 and positions 106-125 of SEQ ID NO: 131, respectively; and light chain CDR1, CDR2 and CDR3 regions having the amino acid sequences of positions 27-32, positions 50-52 and positions 89-97 of SEQ ID NO: 132, respectively;

(Ixvii) heavy chain CDR1, CDR2 and CDR3 regions with the amino acid sequences of positions 26-33, positions 51-58 and positions 97-110 of SEQ ID NO: 133, respectively; and light chain CDR1, CDR2 and CDR3 regions having the amino acid sequences of positions 26-31, positions 49-51 and positions 88-97 of SEQ ID NO: 134, respectively;

(Ixviii) heavy chain CDR1, CDR2 and CDR3 regions with the amino acid sequences of positions 26-33, positions 51-58 and positions 97-109 of SEQ ID NO: 135, respectively; and light chain CDR1, CDR2 and CDR3 regions having the amino acid sequences of positions 27-32, positions 50-52 and positions 89-98 of SEQ ID NO: 136, respectively;

(Ixix) heavy chain CDR1, CDR2 and CDR3 regions with the amino acid sequences of positions 26-33, positions 51-58 and positions 97-108 of SEQ ID NO: 137, respectively; and light chain CDR1, CDR2 and CDR3 regions having the amino acid sequences of positions 26-33, positions 51-53 and positions 92-101 of SEQ ID NO: 138, respectively;

(Ixx) heavy chain CDR1, CDR2 and CDR3 regions with the amino acid sequences of positions 26-33, positions 51-58 and positions 97-112 of SEQ ID NO: 139, respectively; and light chain CDR1, CDR2 and CDR3 regions with the amino acid sequences of positions 27-38, positions 56-58 and positions 95-103 of SEQ ID NO: 140, respectively;

(Ixxi) heavy chain CDR1, CDR2 and CDR3 regions with the amino acid sequences of positions 26-33, positions 51-58 and positions 97-115 of SEQ ID NO: 141, respectively; and light chain CDR1, CDR2 and CDR3 regions with the amino acid sequences of positions 27-32, positions 50-52 and positions 89-97 of SEQ ID NO: 142, respectively;

(lxxii) heavy chain CDR1, CDR2 and CDR3 regions with the amino acid sequences of positions 26-33, positions 51-58 and positions 98-112 of SEQ ID NO: 143, respectively; and light chain CDR1, CDR2 and CDR3 regions with the amino acid sequences of positions 27-37, positions 55-57 and positions 94-101 of SEQ ID NO: 144, respectively;

(lxxiii) heavy chain CDR1, CDR2 and CDR3 regions with the amino acid sequences of positions 26-33, positions 51-57 and positions 99-106 of SEQ ID NO: 145, respectively; and light chain CDR1, CDR2 and CDR3 regions with the amino acid sequences of positions 27-32, positions 50-52 and positions 99-108 of SEQ ID NO: 146, respectively;

(lxxiv) heavy chain CDR1, CDR2 and CDR3 regions with the amino acid sequences of positions 26-35, positions 53-59 and positions 98-114 of SEQ ID NO: 147, respectively; and light chain CDR1, CDR2 and CDR3 regions having the amino acid sequences of positions 27-34, positions 52-54 and positions 91-100 of SEQ ID NO: 148, respectively;

(lxxv) heavy chain CDR1, CDR2 and CDR3 regions with the amino acid sequences of positions 26-35, positions 53-59 and positions 98-112 of SEQ ID NO: 149, respectively; and light chain CDR1, CDR2 and CDR3 regions having the amino acid sequences of positions 26-34, positions 52-54 and positions 91-102 of SEQ ID NO: 150, respectively;

(lxxvi) heavy chain CDR1, CDR2 and CDR3 regions with the amino acid sequences of positions 26-35, positions 53-59 and positions 98-110 of SEQ ID NO: 151, respectively; and light chain CDR1, CDR2 and CDR3 regions having the amino acid sequences of positions 27-32, positions 50-52 and positions 89-98 of SEQ ID NO: 152, respectively;

(lxxvii) heavy chain CDR1, CDR2 and CDR3 regions with the amino acid sequences of positions 26-33, positions 52-57 and positions 96-106 of SEQ ID NO: 153, respectively; and light chain CDR1, CDR2 and CDR3 regions having the amino acid sequences of positions 27-32, positions 50-52 and positions 89-97 of SEQ ID NO: 154, respectively;

(lxxviii) heavy chain CDR1, CDR2 and CDR3 regions with the amino acid sequences of positions 26-33, positions 51-58 and positions 97-112 of SEQ ID NO: 155, respectively; and light chain CDR1, CDR2 and CDR3 regions with the amino acid sequences of positions 27-37, positions 55-57 and positions 94-101 of SEQ ID NO: 156, respectively;

(lxxix) heavy chain CDR1, CDR2 and CDR3 regions with the amino acid sequences of positions 26-33, positions 51-58 and positions 97-121 of SEQ ID NO: 157, respectively; and light chain CDR1, CDR2 and CDR3 regions having the amino acid sequences of positions 27-32, positions 50-52 and positions 89-97 of SEQ ID NO: 158, respectively;

(lxxx) heavy chain CDR1, CDR2 and CDR3 regions with the amino acid sequences of positions 26-33, positions 51-57 and positions 96-106 of SEQ ID NO: 159, respectively; and light chain CDR1, CDR2 and CDR3 regions with the amino acid sequences of positions 27-32, positions 50-52 and positions 89-97 of SEQ ID NO: 160, respectively;

(lxxxi) heavy chain CDR1, CDR2 and CDR3 regions having the amino acid sequences of positions 26-33, positions 51-59 and positions 97-109 of SEQ ID NO: 161, respectively; and light chain CDR1, CDR2 and CDR3 regions with the amino acid sequences of positions 27-32, positions 50-52 and positions 89-98 of SEQ ID NO: 162, respectively;

(lxxxii) heavy chain CDR1, CDR2 and CDR3 regions with the amino acid sequences of positions 26-33, positions 51-58 and positions 97-112 of SEQ ID NO: 163, respectively; and light chain CDR1, CDR2 and CDR3 regions with the amino acid sequences of positions 27-33, positions 51-53 and positions 90-98 of SEQ ID NO: 164, respectively;

(lxxxiii) heavy chain CDR1, CDR2 and CDR3 regions with the amino acid sequences of positions 26-33, positions 51-57 and positions 96-109 of SEQ ID NO: 165, respectively; and light chain CDR1, CDR2 and CDR3 regions having the amino acid sequences of positions 26-31, positions 49-51 and positions 88-98 of SEQ ID NO: 166, respectively;

(lxxxiv) heavy chain CDR1, CDR2 and CDR3 regions with the amino acid sequences of positions 26-33, positions 51-58 and positions 97-112 of SEQ ID NO: 167, respectively; and light chain CDR1, CDR2 and CDR3 regions with the amino acid sequences of positions 27-32, positions 55-57 and positions 94-101 of SEQ ID NO: 168, respectively;

(lxxxv) heavy chain CDR1, CDR2 and CDR3 regions with the amino acid sequences of positions 26-33, positions 51-58 and positions 97-115 of SEQ ID NO: 169, respectively; and light chain CDR1, CDR2 and CDR3 regions having the amino acid sequences of positions 27-32, positions 50-52 and positions 89-96 of SEQ ID NO: 170, respectively;

(lxxxvi) heavy chain CDR1, CDR2 and CDR3 regions with the amino acid sequences of positions 26-33, positions 51-57 and positions 97-113 of SEQ ID NO: 171, respectively; and light chain CDR1, CDR2 and CDR3 regions with the amino acid sequences of positions 27-32, positions 50-52 and positions 89-93 of SEQ ID NO: 172, respectively;

(lxxxvii) heavy chain CDR1, CDR2 and CDR3 regions with the amino acid sequences of positions 26-33, positions 51-57 and positions 96-107 of SEQ ID NO: 173, respectively; and light chain CDR1, CDR2 and CDR3 regions with the amino acid sequences of positions 27-33, positions 51-53 and positions 90-98 of SEQ ID NO: 174, respectively;

(lxxxviii) heavy chain CDR1, CDR2 and CDR3 regions with the amino acid sequences of positions 26-33, positions 51-58 and positions 97-115 of SEQ ID NO: 175, respectively; and light chain CDR1, CDR2 and CDR3 regions having the amino acid sequences of positions 27-32, positions 50-52 and positions 89-96 of SEQ ID NO: 176, respectively;

(lxxxix) heavy chain CDR1, CDR2 and CDR3 regions with the amino acid sequences of positions 26-33, positions 51-58 and positions 97-108 of SEQ ID NO: 177, respectively; and light chain CDR1, CDR2 and CDR3 regions having the amino acid sequences of positions 27-38, positions 56-58 and positions 95-102 of SEQ ID NO: 178, respectively;

(xc) heavy chain CDR1, CDR2 and CDR3 regions with the amino acid sequences from positions 26-34, positions 52-58 and positions 97-113 of SEQ ID NO: 179, respectively; and light chain CDR1, CDR2 and CDR3 regions with the amino acid sequences of positions 26-33, positions 51-53 and positions 90-101 of SEQ ID NO: 180, respectively;

(xci) heavy chain CDR1, CDR2 and CDR3 regions having the amino acid sequences of positions 26-33, positions 51-58 and positions 97-111 of SEQ ID NO: 181, respectively; and light chain CDR1, CDR2 and CDR3 regions having the amino acid sequences of positions 27-32, positions 50-52 and positions 89-97 of SEQ ID NO: 182, respectively;

(xcii) heavy chain CDR1, CDR2 and CDR3 regions with the amino acid sequences of positions 26-33, positions 51-58 and positions 97-112 of SEQ ID NO: 183, respectively; and light chain CDR1, CDR2 and CDR3 regions with the amino acid sequences of positions 27-32, positions 50-52 and positions 89-99 of SEQ ID NO: 184, respectively;

(xciii) heavy chain CDR1, CDR2 and CDR3 regions with the amino acid sequences of positions 26-33, positions 51-57 and positions 96-117 of SEQ ID NO: 185, respectively; and light chain CDR1, CDR2 and CDR3 regions with the amino acid sequences of positions 27-32, positions 50-52 and positions 89-97 of SEQ ID NO: 186, respectively;

(xciv) heavy chain CDR1, CDR2 and CDR3 regions with the amino acid sequences from positions 26-35, positions 53-59 and positions 98-112 of SEQ ID NO: 187, respectively; and light chain CDR1, CDR2 and CDR3 regions with the amino acid sequences of positions 27-32, positions 50-52 and positions 89-97 of SEQ ID NO: 188, respectively;

(xcv) heavy chain CDR1, CDR2 and CDR3 regions with the amino acid sequences of positions 26-33, positions 51-57 and positions 98-119 of SEQ ID NO: 189, respectively; and light chain CDR1, CDR2 and CDR3 regions having the amino acid sequences of positions 26-34, positions 52-54 and positions 91-100 of SEQ ID NO: 190, respectively;

(xcvi) heavy chain CDR1, CDR2 and CDR3 regions with the amino acid sequences of positions 26-33, positions 51-58 and positions 97-107 of SEQ ID NO: 191, respectively; and light chain CDR1, CDR2 and CDR3 regions with the amino acid sequences of positions 26-33, positions 51-53 and positions 90-100 of SEQ ID NO: 192, respectively;

(xcvii) heavy chain CDR1, CDR2 and CDR3 regions with the amino acid sequences of positions 26-33, positions 51-58 and positions 97-112 of SEQ ID NO: 193, respectively; and light chain CDR1, CDR2 and CDR3 regions with the amino acid sequences of positions 26-36, positions 54-56 and positions 93-100 of SEQ ID NO: 194, respectively;

(xcviii) heavy chain CDR1, CDR2 and CDR3 regions with the amino acid sequences of positions 26-33, positions 51-58 and positions 97-113 of SEQ ID NO: 195, respectively; and light chain CDR1, CDR2 and CDR3 regions with the amino acid sequences of positions 26-34, positions 52-54 and positions 91-102 of SEQ ID NO: 196, respectively;

(xcix) heavy chain CDR1, CDR2 and CDR3 regions with the amino acid sequences of positions 26-33, positions 51-58 and positions 97-112 of SEQ ID NO: 197, respectively; and light chain CDR1, CDR2 and CDR3 regions with the amino acid sequences of positions 27-37, positions 55-57 and positions 94-101 of SEQ ID NO: 198, respectively;

(c) heavy chain CDR1, CDR2 and CDR3 regions with the amino acid sequences of positions 26-33, positions 51-57 and positions 96-106 of SEQ ID NO: 199, respectively; and light chain CDR1, CDR2 and CDR3 regions with the amino acid sequences of positions 27-32, positions 50-52 and positions 89-98 of SEQ ID NO: 200, respectively;

(ci) heavy chain CDR1, CDR2 and CDR3 regions with the amino acid sequences of positions 26-33, positions 51-58 and positions 97-113 of SEQ ID NO: 201, respectively; and light chain CDR1, CDR2 and CDR3 regions having the amino acid sequences of positions 26-33, positions 52-54 and positions 91-102 of SEQ ID NO: 202, respectively;

(cii) heavy chain CDR1, CDR2 and CDR3 regions with the amino acid sequences of positions 26-33, positions 51-57 and positions 96-109 of SEQ ID NO: 203, respectively; and light chain CDR1, CDR2 and CDR3 regions having the amino acid sequences of positions 26-31, positions 49-51 and positions 88-98 of SEQ ID NO: 204, respectively;

(ciii) heavy chain CDR1, CDR2 and CDR3 regions with the amino acid sequences of positions 26-34, positions 52-58 and positions 97-111 of SEQ ID NO: 205, respectively; and light chain CDR1, CDR2 and CDR3 regions having the amino acid sequences of positions 26-34, positions 52-54 and positions 91-101 of SEQ ID NO: 206, respectively;

(civ) heavy chain CDR1, CDR2 and CDR3 regions with the amino acid sequences of positions 26-33, positions 51-58 and positions 97-109 of SEQ ID NO: 207, respectively; and light chain CDR1, CDR2 and CDR3 regions having the amino acid sequences of positions 26-31, positions 49-51 and positions 88-99 of SEQ ID NO: 208, respectively;

(cv) heavy chain CDR1, CDR2 and CDR3 regions with the amino acid sequences of positions 26-33, positions 51-58 and positions 97-113 of SEQ ID NO: 209, respectively; and light chain CDR1, CDR2 and CDR3 regions having the amino acid sequences of positions 26-32, positions 52-54 and positions 91-102 of SEQ ID NO: 210, respectively;

(cvi) heavy chain CDR1, CDR2 and CDR3 regions with the amino acid sequences of positions 26-33, positions 51-58 and positions 97-109 of SEQ ID NO: 211, respectively; and light chain CDR1, CDR2 and CDR3 regions having the amino acid sequences of positions 27-32, positions 50-52 and positions 91-112 of SEQ ID NO: 212, respectively;

(cvii) heavy chain CDR1, CDR2 and CDR3 regions with the amino acid sequences of positions 26-33, positions 51-57 and positions 96-106 of SEQ ID NO: 213, respectively; and light chain CDR1, CDR2 and CDR3 regions with the amino acid sequences of positions 27-32, positions 50-52 and positions 89-98 of SEQ ID NO: 214, respectively;

(cviii) heavy chain CDR1, CDR2 and CDR3 regions with the amino acid sequences of positions 26-33, positions 51-58 and positions 97-117 of SEQ ID NO: 215, respectively; and light chain CDR1, CDR2 and CDR3 regions with the amino acid sequences of positions 27-32, positions 50-52 and positions 89-97 of SEQ ID NO: 216, respectively;

(cix) heavy chain CDR1, CDR2 and CDR3 regions with the amino acid sequences of positions 26-33, positions 51-58 and positions 97-115 of SEQ ID NO: 217, respectively; and light chain CDR1, CDR2 and CDR3 regions with the amino acid sequences of positions 27-36, positions 55-57 and positions 94-102 of SEQ ID NO: 218, respectively;

(cx) heavy chain CDR1, CDR2 and CDR3 regions with the amino acid sequences of positions 26-33, positions 51-58 and positions 97-106 of SEQ ID NO: 219, respectively; and light chain CDR1, CDR2 and CDR3 regions with the amino acid sequences of positions 27-38, positions 56-58 and positions 95-104 of SEQ ID NO: 220, respectively;

(cxi) heavy chain CDR1, CDR2 and CDR3 regions having the amino acid sequences of positions 26-33, positions 51-58 and positions 97-119 of SEQ ID NO: 221, respectively; and light chain CDR1, CDR2 and CDR3 regions having the amino acid sequences of positions 25-33, positions 51-53 and positions 90-100 of SEQ ID NO: 222, respectively;

(cxii) heavy chain CDR1, CDR2 and CDR3 regions with the amino acid sequences of positions 26-33, positions 51-58 and positions 97-111 of SEQ ID NO: 223, respectively; and light chain CDR1, CDR2 and CDR3 regions with the amino acid sequences of positions 27-32, positions 50-52 and positions 89-97 of SEQ ID NO: 224, respectively;

(cxiii) heavy chain CDR1, CDR2 and CDR3 regions with the amino acid sequences of positions 26-33, positions 51-60 and positions 99-115 of SEQ ID NO: 225, respectively; and light chain CDR1, CDR2 and CDR3 regions with the amino acid sequences of positions 27-33, positions 51-53 and positions 90-99 of SEQ ID NO: 226, respectively;

(cxiv) heavy chain CDR1, CDR2 and CDR3 regions with the amino acid sequences of positions 26-33, positions 51-60 and positions 99-115 of SEQ ID NO: 227, respectively; and light chain CDR1, CDR2 and CDR3 regions with the amino acid sequences of positions 27-33, positions 51-53 and positions 90-99 of SEQ ID NO: 228, respectively;

(cxv) heavy chain CDR1, CDR2 and CDR3 regions with the amino acid sequences of positions 26-33, positions 51-58 and positions 97-112 of SEQ ID NO: 229, respectively; and light chain CDR1, CDR2 and CDR3 regions having the amino acid sequences of positions 26-31, positions 49-51 and positions 89-96 of SEQ ID NO: 230, respectively;

(cxvi) heavy chain CDR1, CDR2 and CDR3 regions with the amino acid sequences of positions 26-33, positions 51-60 and positions 99-115 of SEQ ID NO: 231, respectively; and light chain CDR1, CDR2 and CDR3 regions having the amino acid sequences of positions 27-33, positions 51-53 and positions 90-99 of SEQ ID NO: 232, respectively;

(cxvii) heavy chain CDR1, CDR2 and CDR3 regions with the amino acid sequences of positions 26-35, positions 53-59 and positions 98-110 of SEQ ID NO: 233, respectively; and light chain CDR1, CDR2 and CDR3 regions having the amino acid sequences of positions 27-33, positions 51-53 and positions 90-98 of SEQ ID NO: 234, respectively;

(cxviii) heavy chain CDR1, CDR2 and CDR3 regions with the amino acid sequences of positions 26-35, positions 53-59 and positions 98-118 of SEQ ID NO: 235, respectively; and light chain CDR1, CDR2 and CDR3 regions having the amino acid sequences of positions 27-32, positions 50-52 and positions 89-97 of SEQ ID NO: 236, respectively;

(cxix) heavy chain CDR1, CDR2 and CDR3 regions with the amino acid sequences of positions 26-33, positions 51-58 and positions 97-112 of SEQ ID NO: 237, respectively; and light chain CDR1, CDR2 and CDR3 regions having the amino acid sequences of positions 26-31, positions 49-51 and positions 88-96 of SEQ ID NO: 238, respectively;

(cxx) heavy chain CDR1, CDR2 and CDR3 regions with the amino acid sequences of positions 26-35, positions 53-59 and positions 98-111 of SEQ ID NO: 239, respectively; and light chain CDR1, CDR2 and CDR3 regions with the amino acid sequences of positions 26-31, positions 49-51 and positions 88-96 of SEQ ID NO: 240, respectively;

(cxxi) heavy chain CDR1, CDR2 and CDR3 regions having the amino acid sequences of positions 26-33, positions 51-57 and positions 96-106 of SEQ ID NO: 241, respectively; and light chain CDR1, CDR2 and CDR3 regions having the amino acid sequences of positions 27-32, positions 50-52 and positions 89-991 of SEQ ID NO: 242, respectively;

(cxxii) heavy chain CDR1, CDR2 and CDR3 regions with the amino acid sequences of positions 26-34, positions 52-58 and positions 97-111 of SEQ ID NO: 243, respectively; and light chain CDR1, CDR2 and CDR3 regions having the amino acid sequences of positions 26-34, positions 52-54 and positions 91-101 of SEQ ID NO: 244, respectively;

(cxxiii) heavy chain CDR1, CDR2 and CDR3 regions with the amino acid sequences of positions 26-35, positions 53-59 and positions 98-113 of SEQ ID NO: 245, respectively; and light chain CDR1, CDR2 and CDR3 regions with the amino acid sequences of positions 27-37, positions 55-57 and positions 94-102 of SEQ ID NO: 246, respectively;

(cxxix) heavy chain CDR1, CDR2 and CDR3 regions with the amino acid sequences of positions 26-35, positions 53-59 and positions 98-113 of SEQ ID NO: 247, respectively; and light chain CDR1, CDR2 and CDR3 regions with the amino acid sequences of positions 27-37, positions 55-57 and positions 94-102 of SEQ ID NO: 248, respectively;

(cxxv) heavy chain CDR1, CDR2 and CDR3 regions with the amino acid sequences of positions 26-33, positions 51-58 and positions 97-112 of SEQ ID NO: 249, respectively; and light chain CDR1, CDR2 and CDR3 regions having the amino acid sequences of positions 27-38, positions 56-58 and positions 95-103 of SEQ ID NO: 250, respectively;

(cxxvi) heavy chain CDR1, CDR2 and CDR3 regions with the amino acid sequences of positions 26-33, positions 51-58 and positions 107-122 of SEQ ID NO: 251, respectively; and light chain CDR1, CDR2 and CDR3 regions having the amino acid sequences of positions 26-33, positions 51-53 and positions 90-99 of SEQ ID NO: 252, respectively;

(cxxvii) heavy chain CDR1, CDR2 and CDR3 regions with the amino acid sequences of positions 26-33, positions 51-58 and positions 97-121 of SEQ ID NO: 253, respectively; and light chain CDR1, CDR2 and CDR3 regions with the amino acid sequences of positions 26-34, positions 52-54 and positions 91-101 of SEQ ID NO: 254, respectively;

(cxxviii) heavy chain CDR1, CDR2 and CDR3 regions with the amino acid sequences of positions 27-33, positions 51-58 and positions 97-118 of SEQ ID NO: 255, respectively; and light chain CDR1, CDR2 and CDR3 regions having the amino acid sequences of positions 26-34, positions 52-54 and positions 91-100 of SEQ ID NO: 256, respectively;

(cxxvix) heavy chain CDR1, CDR2 and CDR3 regions with the amino acid sequences of positions 26-32, positions 51-58 and positions 97-118 of SEQ ID NO: 257, respectively; and light chain CDR1, CDR2 and CDR3 regions having the amino acid sequences of positions 26-34, positions 52-54 and positions 91-99 of SEQ ID NO: 258, respectively;

(cxxx) heavy chain CDR1, CDR2 and CDR3 regions with the amino acid sequences of positions 26-33, positions 51-58 and positions 98-108 of SEQ ID NO: 259, respectively; and light chain CDR1, CDR2 and CDR3 regions with the amino acid sequences of positions 27-38, positions 56-58 and positions 95-103 of SEQ ID NO: 260, respectively;

(cxxxi) heavy chain CDR1, CDR2 and CDR3 regions having the amino acid sequences of positions 38-45, positions 63-69 and positions 108-123 of SEQ ID NO: 261, respectively; and light chain CDR1, CDR2 and CDR3 regions having the amino acid sequences of positions 27-32, positions 50-52 and positions 89-99 of SEQ ID NO: 262, respectively;

(cxxxii) heavy chain CDR1, CDR2 and CDR3 regions with the amino acid sequences of positions 26-34, positions 51-58 and positions 97-108 of SEQ ID NO: 263, respectively; and light chain CDR1, CDR2 and CDR3 regions with the amino acid sequences of positions 26-33, positions 51-53 and positions 92-101 of SEQ ID NO: 264, respectively;

(cxxxiii) heavy chain CDR1, CDR2 and CDR3 regions with the amino acid sequences of positions 26-33, positions 51-58 and positions 97-112 of SEQ ID NO: 265, respectively; and light chain CDR1, CDR2 and CDR3 regions with the amino acid sequences of positions 27-37, positions 55-57 and positions 94-101 of SEQ ID NO: 266, respectively;

(cxxxiv) heavy chain CDR1, CDR2 and CDR3 regions with the amino acid sequences of positions 26-34, positions 52-58 and positions 97-113 of SEQ ID NO: 267, respectively; and light chain CDR1, CDR2 and CDR3 regions with the amino acid sequences of positions 26-33, positions 51-53 and positions 90-101 of SEQ ID NO: 268, respectively;

(cxxxv) heavy chain CDR1, CDR2 and CDR3 regions with the amino acid sequences of positions 26-33, positions 51-57 and positions 96-106 of SEQ ID NO: 269, respectively; and light chain CDR1, CDR2 and CDR3 regions having the amino acid sequences of positions 27-32, positions 50-52 and positions 89-99 of SEQ ID NO: 270, respectively;

(cxxxvi) heavy chain CDR1, CDR2 and CDR3 regions with the amino acid sequences of positions 26-33, positions 51-57 and positions 97-108 of SEQ ID NO: 271, respectively; and light chain CDR1, CDR2 and CDR3 regions having the amino acid sequences of positions 27-38, positions 56-58 and positions 95-103 of SEQ ID NO: 272, respectively;

(cxxxvii) heavy chain CDR1, CDR2 and CDR3 regions with the amino acid sequences of positions 26-35, positions 53-59 and positions 98-110 of SEQ ID NO: 273, respectively; and light chain CDR1, CDR2 and CDR3 regions with the amino acid sequences of positions 27-33, positions 51-53 and positions 90-98 of SEQ ID NO: 274, respectively;

(cxxxviii) heavy chain CDR1, CDR2 and CDR3 regions with the amino acid sequences of positions 26-34, positions 52-58 and positions 97-113 of SEQ ID NO: 275, respectively; and light chain CDR1, CDR2 and CDR3 regions with the amino acid sequences of positions 26-33, positions 51-53 and positions 90-101 of SEQ ID NO: 276, respectively;

(cxxxix) heavy chain CDR1, CDR2 and CDR3 regions with the amino acid sequences of positions 26-33, positions 51-57 and positions 96-107 of SEQ ID NO: 277, respectively; and light chain CDR1, CDR2 and CDR3 regions having the amino acid sequences of positions 26-34, positions 52-54 and positions 91-100 of SEQ ID NO: 278, respectively;

(cxl) heavy chain CDR1, CDR2 and CDR3 regions with the amino acid sequences of positions 26-35, positions 54-59 and positions 98-112 of SEQ ID NO: 279, respectively; and light chain CDR1, CDR2 and CDR3 regions with the amino acid sequences of positions 26-31, positions 49-51 and positions 88-96 of SEQ ID NO: 280, respectively;

(cxli) heavy chain CDR1, CDR2 and CDR3 regions with the amino acid sequences of positions 26-33, positions 51-58 and positions 97-112 of SEQ ID NO: 281, respectively; and light chain CDR1, CDR2 and CDR3 regions having the amino acid sequences of positions 27-37, positions 55-57 and positions 94-101 of SEQ ID NO: 282, respectively;

(cxlii) heavy chain CDR1, CDR2 and CDR3 regions with the amino acid sequences of positions 26-33, positions 51-58 and positions 97-120 of SEQ ID NO: 283, respectively; and light chain CDR1, CDR2 and CDR3 regions having the amino acid sequences of positions 26-31, positions 49-51 and positions 88-98 of SEQ ID NO: 284, respectively;

(cxliii) heavy chain CDR1, CDR2 and CDR3 regions with the amino acid sequences of positions 26-33, positions 51-57 and positions 96-106 of SEQ ID NO: 285, respectively; and light chain CDR1, CDR2 and CDR3 regions having the amino acid sequences of positions 26-31, positions 50-52 and positions 89-98 of SEQ ID NO: 286, respectively;

(cxliv) heavy chain CDR1, CDR2 and CDR3 regions with the amino acid sequences of positions 26-35, positions 52-58 and positions 97-111 of SEQ ID NO: 287, respectively; and light chain CDR1, CDR2 and CDR3 regions having the amino acid sequences of positions 26-34, positions 52-54 and positions 95-101 of SEQ ID NO: 288, respectively;

(cxlv) heavy chain CDR1, CDR2 and CDR3 regions with the amino acid sequences of positions 26-33, positions 51-58 and positions 97-116 of SEQ ID NO: 289, respectively; and light chain CDR1, CDR2 and CDR3 regions having the amino acid sequences of positions 28-32, positions 50-52 and positions 89-97 of SEQ ID NO: 290, respectively;

(cxlvi) heavy chain CDR1, CDR2 and CDR3 regions with the amino acid sequences of positions 26-33, positions 51-58 and positions 97-116 of SEQ ID NO: 291, respectively; and light chain CDR1, CDR2 and CDR3 regions having the amino acid sequences of positions 27-32, positions 50-52 and positions 89-96 of SEQ ID NO: 292, respectively;

(cxlvii) heavy chain CDR1, CDR2 and CDR3 regions with the amino acid sequences of positions 26-33, positions 51-58 and positions 97-118 of SEQ ID NO: 293, respectively; and light chain CDR1, CDR2 and CDR3 regions with the amino acid sequences of positions 26-34, positions 52-54 and positions 91-100 of SEQ ID NO: 294, respectively;

(cxlviii) heavy chain CDR1, CDR2 and CDR3 regions with the amino acid sequences of positions 26-33, positions 51-58 and positions 97-112 of SEQ ID NO: 295, respectively; and light chain CDR1, CDR2 and CDR3 regions having the amino acid sequences of positions 27-32, positions 50-52 and positions 89-95 of SEQ ID NO: 296, respectively;

(cxlix) heavy chain CDR1, CDR2 and CDR3 regions with the amino acid sequences of positions 21-28, positions 46-53 and positions 92-106 of SEQ ID NO: 297, respectively; and light chain CDR1, CDR2 and CDR3 regions having the amino acid sequences of positions 17-22, positions 40-42 and positions 79-87 of SEQ ID NO: 298, respectively;

(cl) heavy chain CDR1, CDR2 and CDR3 regions with the amino acid sequences of positions 26-33, positions 51-57 and positions 96-107 of SEQ ID NO: 299, respectively; and light chain CDR1, CDR2 and CDR3 regions with the amino acid sequences of positions 27-37, positions 55-57 and positions 94-101 of SEQ ID NO: 300, respectively;

(cli) heavy chain CDR1, CDR2 and CDR3 regions having the amino acid sequences of positions 26-33, positions 51-57 and positions 96-107 of SEQ ID NO: 301, respectively; and light chain CDR1, CDR2 and CDR3 regions having the amino acid sequences of positions 27-37, positions 55-57 and positions 94-101 of SEQ ID NO: 302, respectively;

(clii) heavy chain CDR1, CDR2 and CDR3 regions with the amino acid sequences of positions 26-33, positions 51-57 and positions 96-113 of SEQ ID NO: 303, respectively; and light chain CDR1, CDR2 and CDR3 regions with the amino acid sequences of positions 27-32, positions 51-54 and positions 90-98 of SEQ ID NO: 304, respectively;

(cliii) heavy chain CDR1, CDR2 and CDR3 regions with the amino acid sequences of positions 26-33, positions 51-57 and positions 96-110 of SEQ ID NO: 305, respectively; and light chain CDR1, CDR2 and CDR3 regions having the amino acid sequences of positions 27-32, positions 50-52 and positions 89-98 of SEQ ID NO: 306, respectively;

(cliv) heavy chain CDR1, CDR2 and CDR3 regions with the amino acid sequences of positions 26-33, positions 51-58 and positions 97-109 of SEQ ID NO: 307, respectively; and light chain CDR1, CDR2 and CDR3 regions having the amino acid sequences of positions 26-33, positions 51-53 and positions 90-99 of SEQ ID NO: 308, respectively;

(clv) heavy chain CDR1, CDR2 and CDR3 regions with the amino acid sequences of positions 26-33, positions 51-58 and positions 97-114 of SEQ ID NO: 309, respectively; and light chain CDR1, CDR2 and CDR3 regions having the amino acid sequences of positions 24-29, positions 48-50 and positions 86-94 of SEQ ID NO: 310, respectively;

(clvi) heavy chain CDR1, CDR2 and CDR3 regions with the amino acid sequences of positions 26-33, positions 51-58 and positions 97-108 of SEQ ID NO: 311, respectively; and light chain CDR1, CDR2 and CDR3 regions having the amino acid sequences of positions 27-38, positions 56-58 and positions 95-103 of SEQ ID NO: 312, respectively;

(clvii) heavy chain CDR1, CDR2 and CDR3 regions with the amino acid sequences of positions 26-33, positions 51-58 and positions 97-115 of SEQ ID NO: 313, respectively; and light chain CDR1, CDR2 and CDR3 regions having the amino acid sequences of positions 27-32, positions 50-52 and positions 89-97 of SEQ ID NO: 314, respectively;

(clviii) heavy chain CDR1, CDR2 and CDR3 regions with the amino acid sequences of positions 26-33, positions 51-58 and positions 97-115 of SEQ ID NO: 315, respectively; and light chain CDR1, CDR2 and CDR3 regions having the amino acid sequences of positions 27-32, positions 50-52 and positions 89-97 of SEQ ID NO: 316, respectively;

(clix) heavy chain CDR1, CDR2 and CDR3 regions with the amino acid sequences of positions 26-33, positions 51-58 and positions 97-113 of SEQ ID NO: 317, respectively; and light chain CDR1, CDR2 and CDR3 regions having the amino acid sequences of positions 26-31, positions 51-53 and positions 90-100 of SEQ ID NO: 318, respectively;

(clx) heavy chain CDR1, CDR2 and CDR3 regions with the amino acid sequences of positions 26-34, positions 53-59 and positions 98-119 of SEQ ID NO: 319, respectively; and light chain CDR1, CDR2 and CDR3 regions with the amino acid sequences of positions 26-31, positions 51-53 and positions 90-101 of SEQ ID NO: 320, respectively;

(clxi) heavy chain CDR1, CDR2 and CDR3 regions having the amino acid sequences of positions 26-33, positions 51-58 and positions 97-110 of SEQ ID NO: 321, respectively; and light chain CDR1, CDR2 and CDR3 regions having the amino acid sequences of positions 27-32, positions 50-52 and positions 89-97 of SEQ ID NO: 322, respectively;

(clxii) heavy chain CDR1, CDR2 and CDR3 regions with the amino acid sequences of positions 26-35, positions 53-59 and positions 98-119 of SEQ ID NO: 323, respectively; and light chain CDR1, CDR2 and CDR3 regions having the amino acid sequences of positions 26-34, positions 52-54 and positions 91-100 of SEQ ID NO: 324, respectively;

(clxiii) heavy chain CDR1, CDR2 and CDR3 regions with the amino acid sequences of positions 26-33, positions 51-57 and positions 96-103 of SEQ ID NO: 325, respectively; and light chain CDR1, CDR2 and CDR3 regions having the amino acid sequences of positions 27-32, positions 50-52 and positions 89-97 of SEQ ID NO: 326, respectively;

(clxiv) heavy chain CDR1, CDR2 and CDR3 regions with the amino acid sequences of positions 26-35, positions 52-59 and positions 98-112 of SEQ ID NO: 327, respectively; and light chain CDR1, CDR2 and CDR3 regions having the amino acid sequences of positions 26-31, positions 49-51 and positions 88-96 of SEQ ID NO: 328, respectively;

(clxv) heavy chain CDR1, CDR2 and CDR3 regions with the amino acid sequences of positions 26-33, positions 51-58 and positions 97-117 of SEQ ID NO: 329, respectively; and light chain CDR1, CDR2 and CDR3 regions having the amino acid sequences of positions 24-29, positions 47-49 and positions 86-94 of SEQ ID NO: 330, respectively;

(clxvi) heavy chain CDR1, CDR2 and CDR3 regions with the amino acid sequences of positions 26-33, positions 51-58 and positions 97-113 of SEQ ID NO: 331, respectively; and light chain CDR1, CDR2 and CDR3 regions having the amino acid sequences of positions 26-33, positions 51-53 and positions 90-100 of SEQ ID NO: 332, respectively;

(clxvii) heavy chain CDR1, CDR2 and CDR3 regions with the amino acid sequences of positions 26-33, positions 51-57 and positions 97-113 of SEQ ID NO: 333, respectively; and light chain CDR1, CDR2 and CDR3 regions having the amino acid sequences of positions 26-33, positions 51-53 and positions 90-100 of SEQ ID NO: 334, respectively;

(clxviii) heavy chain CDR1, CDR2 and CDR3 regions with the amino acid sequences of positions 26-33, positions 51-57 and positions 97-116 of SEQ ID NO: 335, respectively; and light chain CDR1, CDR2 and CDR3 regions having the amino acid sequences of positions 27-31, positions 50-52 and positions 89-98 of SEQ ID NO: 336, respectively;

(clxix) heavy chain CDR1, CDR2 and CDR3 regions with the amino acid sequences of positions 26-35, positions 53-59 and positions 98-121 of SEQ ID NO: 337, respectively; and light chain CDR1, CDR2 and CDR3 regions having the amino acid sequences of positions 27-37, positions 55-57 and positions 94-102 of SEQ ID NO: 338, respectively;

(clxx) heavy chain CDR1, CDR2 and CDR3 regions with the amino acid sequences of positions 26-33, positions 51-60 and positions 99-114 of SEQ ID NO: 339, respectively; and light chain CDR1, CDR2 and CDR3 regions with the amino acid sequences of positions 27-33, positions 51-53 and positions 90-99 of SEQ ID NO: 340, respectively;

(clxxi) heavy chain CDR1, CDR2 and CDR3 regions with the amino acid sequences of positions 26-33, positions 51-58 and positions 97-117 of SEQ ID NO: 341, respectively; and light chain CDR1, CDR2 and CDR3 regions having the amino acid sequences of positions 26-33, positions 51-53 and positions 90-101 of SEQ ID NO: 342, respectively;

(clxxii) heavy chain CDR1, CDR2 and CDR3 regions with the amino acid sequences of positions 27-34, positions 52-58 and positions 98-112 of SEQ ID NO: 343, respectively; and light chain CDR1, CDR2 and CDR3 regions with the amino acid sequences of positions 26-34, positions 52-54 and positions 91-100 of SEQ ID NO: 344, respectively;

(clxxiii) heavy chain CDR1, CDR2 and CDR3 regions with the amino acid sequences of positions 27-35, positions 53-59 and positions 98-118 of SEQ ID NO: 345, respectively; and light chain CDR1, CDR2 and CDR3 regions with the amino acid sequences of positions 26-34, positions 52-54 and positions 91-100 of SEQ ID NO: 346, respectively

(clxxiv) heavy chain CDR1, CDR2 and CDR3 regions with the amino acid sequences of positions 26-35, positions 53-59 and positions 98-122 of SEQ ID NO: 347, respectively; and light chain CDR1, CDR2 and CDR3 regions having the amino acid sequences of positions 27-37, positions 55-57 and positions 94-102 of SEQ ID NO: 348, respectively;

(clxxv) heavy chain CDR1, CDR2 and CDR3 regions with the amino acid sequences of positions 26-33, positions 51-61 and positions 97-106 of SEQ ID NO: 349, respectively; and light chain CDR1, CDR2 and CDR3 regions having the amino acid sequences of positions 26-31, positions 49-51 and positions 88-96 of SEQ ID NO: 350, respectively;

(clxxvi) heavy chain CDR1, CDR2 and CDR3 regions with the amino acid sequences of positions 27-34, positions 52-58 and positions 97-112 of SEQ ID NO: 351, respectively; and light chain CDR1, CDR2 and CDR3 regions having the amino acid sequences of positions 26-34, positions 52-54 and positions 91-101 of SEQ ID NO: 352, respectively;

(clxxvii) heavy chain CDR1, CDR2 and CDR3 regions with the amino acid sequences of positions 26-34, positions 52-59 and positions 97-112 of SEQ ID NO: 353, respectively; and light chain CDR1, CDR2 and CDR3 regions having the amino acid sequences of positions 27-32, positions 50-52 and positions 89-97 of SEQ ID NO: 354, respectively;

(clxxviii) heavy chain CDR1, CDR2 and CDR3 regions with the amino acid sequences of positions 26-33, positions 51-58 and positions 97-117 of SEQ ID NO: 355, respectively; and light chain CDR1, CDR2 and CDR3 regions having the amino acid sequences of positions 27-32, positions 50-52 and positions 89-99 of SEQ ID NO: 356, respectively;

(clxxix) heavy chain CDR1, CDR2 and CDR3 regions with the amino acid sequences of positions 26-32, positions 51-58 and positions 97-110 of SEQ ID NO: 357, respectively; and light chain CDR1, CDR2 and CDR3 regions having the amino acid sequences of positions 26-31, positions 49-51 and positions 88-98 of SEQ ID NO: 358, respectively; or

(clxxx) heavy chain CDR1, CDR2 and CDR3 regions with the amino acid sequences of positions 26-32, positions 51-58 and positions 97-110 of SEQ ID NO: 359, respectively; and light chain CDR1, CDR2 and CDR3 regions with the amino acid sequences of positions 26-31, positions 49-51 and positions 88-98 of SEQ ID NO: 360, respectively.

2. Antibody according to claim 1, **characterized in that** the antibody comprises a heavy chain and a light chain selected from (i) - (clxxx):

(i) a heavy chain comprising SEQ ID NO: 1 and a light chain comprising SEQ ID NO: 2;
(ii) a heavy chain comprising SEQ ID NO: 3 and a light chain comprising SEQ ID NO: 4;
(iii) a heavy chain comprising SEQ ID NO: 5 and a light chain comprising SEQ ID NO: 6;
(iv) a heavy chain comprising SEQ ID NO: 7 and a light chain comprising SEQ ID NO: 8;
(v) a heavy chain comprising SEQ ID NO: 9 and a light chain comprising SEQ ID NO: 10;
(vi) a heavy chain comprising SEQ ID NO: 11 and a light chain comprising SEQ ID NO: 12;
(vii) a heavy chain comprising SEQ ID NO: 13 and a light chain comprising SEQ ID NO: 14;
(viii) a heavy chain comprising SEQ ID NO: 15 and a light chain comprising SEQ ID NO: 16;
(ix) a heavy chain comprising SEQ ID NO: 17 and a light chain comprising SEQ ID NO: 18;
(x) a heavy chain comprising SEQ ID NO: 19 and a light chain comprising SEQ ID NO: 20;
(xi) a heavy chain comprising SEQ ID NO: 21 and a light chain comprising SEQ ID NO: 22;
(xii) a heavy chain comprising SEQ ID NO: 23 and a light chain comprising SEQ ID NO: 24;
(xiii) a heavy chain comprising SEQ ID NO: 25 and a light chain comprising SEQ ID NO: 26;
(xiv) a heavy chain comprising SEQ ID NO: 27 and a light chain comprising SEQ ID NO: 28;
(xv) a heavy chain comprising SEQ ID NO: 29 and a light chain comprising SEQ ID NO: 30;
(xvi) a heavy chain comprising SEQ ID NO: 31 and a light chain comprising SEQ ID NO: 32;
(xvii) a heavy chain comprising SEQ ID NO: 33 and a light chain comprising SEQ ID NO: 34;
(xviii) a heavy chain comprising SEQ ID NO: 35 and a light chain comprising SEQ ID NO: 36;
(xxix) a heavy chain comprising SEQ ID NO: 37 and a light chain comprising SEQ ID NO: 38;
(xx) a heavy chain comprising SEQ ID NO: 39 and a light chain comprising SEQ ID NO: 40;
(xxi) a heavy chain comprising SEQ ID NO: 41 and a light chain comprising SEQ ID NO: 42;

(xxii) a heavy chain comprising SEQ ID NO: 43 and a light chain comprising SEQ ID NO: 44;
(xxiii) a heavy chain comprising SEQ ID NO: 45 and a light chain comprising SEQ ID NO: 46;
(xxiv) a heavy chain comprising SEQ ID NO: 47 and a light chain comprising SEQ ID NO: 48;
(xxv) a heavy chain comprising SEQ ID NO: 49 and a light chain comprising SEQ ID NO: 50;
(xxvi) a heavy chain comprising SEQ ID NO: 51 and a light chain comprising SEQ ID NO: 52;
(xxvii) a heavy chain comprising SEQ ID NO: 53 and a light chain comprising SEQ ID NO: 54;
(xxviii) a heavy chain comprising SEQ ID NO: 55 and a light chain comprising SEQ ID NO: 56;
(xxix) a heavy chain comprising SEQ ID NO: 57 and a light chain comprising SEQ ID NO: 58;
(xxx) a heavy chain comprising SEQ ID NO: 59 and a light chain comprising SEQ ID NO: 60;
(xxxi) a heavy chain comprising SEQ ID NO: 61 and a light chain comprising SEQ ID NO: 62;
(xxxii) a heavy chain comprising SEQ ID NO: 63 and a light chain comprising SEQ ID NO: 64;
(xxxiii) a heavy chain comprising SEQ ID NO: 65 and a light chain comprising SEQ ID NO: 66;
(xxxiv) a heavy chain comprising SEQ ID NO: 67 and a light chain comprising SEQ ID NO: 68;
(xxxv) a heavy chain comprising SEQ ID NO: 69 and a light chain comprising SEQ ID NO: 70;
(xxxvi) a heavy chain comprising SEQ ID NO: 71 and a light chain comprising SEQ ID NO: 72;
(xxxvii) a heavy chain comprising SEQ ID NO: 73 and a light chain comprising SEQ ID NO: 74;
(xxxviii) a heavy chain comprising SEQ ID NO: 75 and a light chain comprising SEQ ID NO: 76;
(xxxix) a heavy chain comprising SEQ ID NO: 77 and a light chain comprising SEQ ID NO: 78;
(xl) a heavy chain comprising SEQ ID NO: 79 and a light chain comprising SEQ ID NO: 80;
(xli) a heavy chain comprising SEQ ID NO: 81 and a light chain comprising SEQ ID NO: 82;
(xlii) a heavy chain comprising SEQ ID NO: 83 and a light chain comprising SEQ ID NO: 84;
(xliii) a heavy chain comprising SEQ ID NO: 85 and a light chain comprising SEQ ID NO: 86;
(xliv) a heavy chain comprising SEQ ID NO: 87 and a light chain comprising SEQ ID NO: 88; or
(xlv) a heavy chain comprising SEQ ID NO: 89 and a light chain comprising SEQ ID NO: 90.
(xlvi) a heavy chain comprising SEQ ID NO: 91 and a light chain comprising SEQ ID NO: 92;
(xlvii) a heavy chain comprising SEQ ID NO: 93 and a light chain comprising SEQ ID NO: 94;
(xlviii) a heavy chain comprising SEQ ID NO: 95 and a light chain comprising SEQ ID NO: 96;
(xlix) a heavy chain comprising SEQ ID NO: 97 and a light chain comprising SEQ ID NO: 98;
(l) a heavy chain comprising SEQ ID NO: 99 and a light chain comprising SEQ ID NO: 100;
(li) a heavy chain comprising SEQ ID NO: 101 and a light chain comprising SEQ ID NO: 102;
(lii) a heavy chain comprising SEQ ID NO: 103 and a light chain comprising SEQ ID NO: 104;
(liii) a heavy chain comprising SEQ ID NO: 105 and a light chain comprising SEQ ID NO: 106;
(liv) a heavy chain comprising SEQ ID NO: 107 and a light chain comprising SEQ ID NO: 108;
(lv) a heavy chain comprising SEQ ID NO: 109 and a light chain comprising SEQ ID NO: 110;
(lvi) a heavy chain comprising SEQ ID NO: 111 and a light chain comprising SEQ ID NO: 112;
(lvii) a heavy chain comprising SEQ ID NO: 113 and a light chain comprising SEQ ID NO: 114;
(lviii) a heavy chain comprising SEQ ID NO: 115 and a light chain comprising SEQ ID NO: 116;
(lix) a heavy chain comprising SEQ ID NO: 117 and a light chain comprising SEQ ID NO: 118;
(lx) a heavy chain comprising SEQ ID NO: 119 and a light chain comprising SEQ ID NO: 120;
(lxi) a heavy chain comprising SEQ ID NO: 121 and a light chain comprising SEQ ID NO: 122;
(lxii) a heavy chain comprising SEQ ID NO: 123 and a light chain comprising SEQ ID NO: 124;
(lxiii) a heavy chain comprising SEQ ID NO: 125 and a light chain comprising SEQ ID NO: 126;
(lxiv) a heavy chain comprising SEQ ID NO: 127 and a light chain comprising SEQ ID NO: 128;
(lxv) a heavy chain comprising SEQ ID NO: 129 and a light chain comprising SEQ ID NO: 130;
(lxvi) a heavy chain comprising SEQ ID NO: 131 and a light chain comprising SEQ ID NO: 132;
(lxvii) a heavy chain comprising SEQ ID NO: 133 and a light chain comprising SEQ ID NO: 134;
(lxviii) a heavy chain comprising SEQ ID NO: 135 and a light chain comprising SEQ ID NO: 136;
(lxix) a heavy chain comprising SEQ ID NO: 137 and a light chain comprising SEQ ID NO: 138;
(lxx) a heavy chain comprising SEQ ID NO: 139 and a light chain comprising SEQ ID NO: 140;
(lxxi) a heavy chain comprising SEQ ID NO: 141 and a light chain comprising SEQ ID NO: 142;
(lxxii) a heavy chain comprising SEQ ID NO: 143 and a light chain comprising SEQ ID NO: 144;
(lxxiii) a heavy chain comprising SEQ ID NO: 145 and a light chain comprising SEQ ID NO: 146;
(lxxiv) a heavy chain comprising SEQ ID NO: 147 and a light chain comprising SEQ ID NO: 148;
(lxxv) a heavy chain comprising SEQ ID NO: 149 and a light chain comprising SEQ ID NO: 150;
(lxxvi) a heavy chain comprising SEQ ID NO: 151 and a light chain comprising SEQ ID NO: 152;
(lxxvii) a heavy chain comprising SEQ ID NO: 153 and a light chain comprising SEQ ID NO: 154;
(lxxviii) a heavy chain comprising SEQ ID NO: 155 and a light chain comprising SEQ ID NO: 156;
(lxxix) a heavy chain comprising SEQ ID NO: 157 and a light chain comprising SEQ ID NO: 158;

(lxxx) a heavy chain comprising SEQ ID NO: 159 and a light chain comprising SEQ ID NO: 160;

(lxxxi) a heavy chain comprising SEQ ID NO: 161 and a light chain comprising SEQ ID NO: 162;

(lxxxii) a heavy chain comprising SEQ ID NO: 163 and a light chain comprising SEQ ID NO: 164;

(lxxxiii) a heavy chain comprising SEQ ID NO: 165 and a light chain comprising SEQ ID NO: 166;

(lxxxiv) a heavy chain comprising SEQ ID NO: 167 and a light chain comprising SEQ ID NO: 168;

(lxxxv) a heavy chain comprising SEQ ID NO: 169 and a light chain comprising SEQ ID NO: 170;

(lxxxvi) a heavy chain comprising SEQ ID NO: 171 and a light chain comprising SEQ ID NO: 172;

(lxxxvii) a heavy chain comprising SEQ ID NO: 173 and a light chain comprising SEQ ID NO: 174;

(lxxxviii) a heavy chain comprising SEQ ID NO: 175 and a light chain comprising SEQ ID NO: 176;

(lxxxix) a heavy chain comprising SEQ ID NO: 177 and a light chain comprising SEQ ID NO: 178;

(xc) a heavy chain comprising SEQ ID NO: 179 and a light chain comprising SEQ ID NO: 180;

(xci) a heavy chain comprising SEQ ID NO: 181 and a light chain comprising SEQ ID NO: 182;

(xcii) a heavy chain comprising SEQ ID NO: 183 and a light chain comprising SEQ ID NO: 184;

(xciii) a heavy chain comprising SEQ ID NO: 185 and a light chain comprising SEQ ID NO: 186;

(xciv) a heavy chain comprising SEQ ID NO: 187 and a light chain comprising SEQ ID NO: 188;

(xcv) a heavy chain comprising SEQ ID NO: 189 and a light chain comprising SEQ ID NO: 190;

(xcvi) a heavy chain comprising SEQ ID NO: 191 and a light chain comprising SEQ ID NO: 192;

(xcvii) a heavy chain comprising SEQ ID NO: 193 and a light chain comprising SEQ ID NO: 194;

(xciii) a heavy chain comprising SEQ ID NO: 195 and a light chain comprising SEQ ID NO: 196;

(xcix) a heavy chain comprising SEQ ID NO: 197 and a light chain comprising SEQ ID NO: 198;

(c) a heavy chain comprising SEQ ID NO: 199 and a light chain comprising SEQ ID NO: 200;

(ci) a heavy chain comprising SEQ ID NO: 201 and a light chain comprising SEQ ID NO: 202;

(cii) a heavy chain comprising SEQ ID NO: 203 and a light chain comprising SEQ ID NO: 204;

(ciii) a heavy chain comprising SEQ ID NO: 205 and a light chain comprising SEQ ID NO: 206;

(civ) a heavy chain comprising SEQ ID NO: 207 and a light chain comprising SEQ ID NO: 208;

(cv) a heavy chain comprising SEQ ID NO: 209 and a light chain comprising SEQ ID NO: 210;

(cvi) a heavy chain comprising SEQ ID NO: 211 and a light chain comprising SEQ ID NO: 212;

(cvii) a heavy chain comprising SEQ ID NO: 213 and a light chain comprising SEQ ID NO: 214;

(cxiii) a heavy chain comprising SEQ ID NO: 215 and a light chain comprising SEQ ID NO: 216;

(cix) a heavy chain comprising SEQ ID NO: 217 and a light chain comprising SEQ ID NO: 218;

(cx) a heavy chain comprising SEQ ID NO: 219 and a light chain comprising SEQ ID NO: 220;

(cxi) a heavy chain comprising SEQ ID NO: 221 and a light chain comprising SEQ ID NO: 222;

(cxii) a heavy chain comprising SEQ ID NO: 223 and a light chain comprising SEQ ID NO: 224;

(cxiii) a heavy chain comprising SEQ ID NO: 225 and a light chain comprising SEQ ID NO: 226;

(cxiv) a heavy chain comprising SEQ ID NO: 227 and a light chain comprising SEQ ID NO: 228;

(cxv) a heavy chain comprising SEQ ID NO: 229 and a light chain comprising SEQ ID NO: 230;

(cxvi) a heavy chain comprising SEQ ID NO: 231 and a light chain comprising SEQ ID NO: 232;

(cxvii) a heavy chain comprising SEQ ID NO: 233 and a light chain comprising SEQ ID NO: 234;

(cxiii) a heavy chain comprising SEQ ID NO: 235 and a light chain comprising SEQ ID NO: 236;

(cxix) a heavy chain comprising SEQ ID NO: 237 and a light chain comprising SEQ ID NO: 238;

(cxx) a heavy chain comprising SEQ ID NO: 239 and a light chain comprising SEQ ID NO: 240;

(cxxi) a heavy chain comprising SEQ ID NO: 241 and a light chain comprising SEQ ID NO: 242;

(cxxii) a heavy chain comprising SEQ ID NO: 243 and a light chain comprising SEQ ID NO: 244;

(cxxiii) a heavy chain comprising SEQ ID NO: 245 and a light chain comprising SEQ ID NO: 246;

(cxxiv) a heavy chain comprising SEQ ID NO: 247 and a light chain comprising SEQ ID NO: 248;

(cxxv) a heavy chain comprising SEQ ID NO: 249 and a light chain comprising SEQ ID NO: 250;

(cxxvi) a heavy chain comprising SEQ ID NO: 251 and a light chain comprising SEQ ID NO: 252;

(cxxvii) a heavy chain comprising SEQ ID NO: 253 and a light chain comprising SEQ ID NO: 254;

(cxxviii) a heavy chain comprising SEQ ID NO: 255 and a light chain comprising SEQ ID NO: 256;

(cxxvix) a heavy chain comprising SEQ ID NO: 257 and a light chain comprising SEQ ID NO: 258;

(cxxxx) a heavy chain comprising SEQ ID NO: 259 and a light chain comprising SEQ ID NO: 260;

(cxxxi) a heavy chain comprising SEQ ID NO: 261 and a light chain comprising SEQ ID NO: 262;

(cxxxii) a heavy chain comprising SEQ ID NO: 263 and a light chain comprising SEQ ID NO: 264;

(cxxxiii) a heavy chain comprising SEQ ID NO: 265 and a light chain comprising SEQ ID NO: 266;

(cxxxiv) a heavy chain comprising SEQ ID NO: 267 and a light chain comprising SEQ ID NO: 268;

(cxxxv) a heavy chain comprising SEQ ID NO: 269 and a light chain comprising SEQ ID NO: 270;

(cxxxvi) a heavy chain comprising SEQ ID NO: 271 and a light chain comprising SEQ ID NO: 272;

(cxxxvii) a heavy chain comprising SEQ ID NO: 273 and a light chain comprising SEQ ID NO: 274;

(cxxxviii) a heavy chain comprising SEQ ID NO: 275 and a light chain comprising SEQ ID NO: 276;
(cxxxix) a heavy chain comprising SEQ ID NO: 277 and a light chain comprising SEQ ID NO: 278;
(cxl) a heavy chain comprising SEQ ID NO: 279 and a light chain comprising SEQ ID NO: 280;
(cxli) a heavy chain comprising SEQ ID NO: 281 and a light chain comprising SEQ ID NO: 282;
(cxlii) a heavy chain comprising SEQ ID NO: 283 and a light chain comprising SEQ ID NO: 284;
(cxliii) a heavy chain comprising SEQ ID NO: 285 and a light chain comprising SEQ ID NO: 286;
(cxliv) a heavy chain comprising SEQ ID NO: 287 and a light chain comprising SEQ ID NO: 288;
(cxlv) a heavy chain comprising SEQ ID NO: 289 and a light chain comprising SEQ ID NO: 290;
(cxlvi) a heavy chain comprising SEQ ID NO: 291 and a light chain comprising SEQ ID NO: 292;
(cxlvii) a heavy chain comprising SEQ ID NO: 293 and a light chain comprising SEQ ID NO: 294;
(cxlviii) a heavy chain comprising SEQ ID NO: 295 and a light chain comprising SEQ ID NO: 296;
(cxlvix) a heavy chain comprising SEQ ID NO: 297 and a light chain comprising SEQ ID NO: 298;
(cl) a heavy chain comprising SEQ ID NO: 299 and a light chain comprising SEQ ID NO: 300;
(cli) a heavy chain comprising SEQ ID NO: 301 and a light chain comprising SEQ ID NO: 302;
(clii) a heavy chain comprising SEQ ID NO: 303 and a light chain comprising SEQ ID NO: 304;
(clii) a heavy chain comprising SEQ ID NO: 305 and a light chain comprising SEQ ID NO: 306;
(cliv) a heavy chain comprising SEQ ID NO: 307 and a light chain comprising SEQ ID NO: 308;
(clv) a heavy chain comprising SEQ ID NO: 309 and a light chain comprising SEQ ID NO: 310;
(clvi) a heavy chain comprising SEQ ID NO: 311 and a light chain comprising SEQ ID NO: 312;
(clvii) a heavy chain comprising SEQ ID NO: 313 and a light chain comprising SEQ ID NO: 314;
(clviii) a heavy chain comprising SEQ ID NO: 315 and a light chain comprising SEQ ID NO: 316;
(clix) a heavy chain comprising SEQ ID NO: 317 and a light chain comprising SEQ ID NO: 318;
(clx) a heavy chain comprising SEQ ID NO: 319 and a light chain comprising SEQ ID NO: 320;
(clxi) a heavy chain comprising SEQ ID NO: 321 and a light chain comprising SEQ ID NO: 322;
(clxii) a heavy chain comprising SEQ ID NO: 323 and a light chain comprising SEQ ID NO: 324;
(clxiii) a heavy chain comprising SEQ ID NO: 325 and a light chain comprising SEQ ID NO: 326;
(clxiv) a heavy chain comprising SEQ ID NO: 327 and a light chain comprising SEQ ID NO: 328;
(clxv) a heavy chain comprising SEQ ID NO: 329 and a light chain comprising SEQ ID NO: 330;
(clxvi) a heavy chain comprising SEQ ID NO: 331 and a light chain comprising SEQ ID NO: 322;
(clxvii) a heavy chain comprising SEQ ID NO: 333 and a light chain comprising SEQ ID NO: 334;
(clxvii) a heavy chain comprising SEQ ID NO: 335 and a light chain comprising SEQ ID NO: 336;
(clxix) a heavy chain comprising SEQ ID NO: 337 and a light chain comprising SEQ ID NO: 338;
(clxx) a heavy chain comprising SEQ ID NO: 339 and a light chain comprising SEQ ID NO: 340;
(clxxi) a heavy chain comprising SEQ ID NO: 341 and a light chain comprising SEQ ID NO: 342;
(clxxii) a heavy chain comprising SEQ ID NO: 343 and a light chain comprising SEQ ID NO: 344;
(clxxiii) a heavy chain comprising SEQ ID NO: 345 and a light chain comprising SEQ ID NO: 346;
(clxxiv) a heavy chain comprising SEQ ID NO: 347 and a light chain comprising SEQ ID NO: 348;
(clxxv) a heavy chain comprising SEQ ID NO: 349 and a light chain comprising SEQ ID NO: 350;
(clxxvi) a heavy chain comprising SEQ ID NO: 351 and a light chain comprising SEQ ID NO: 352;
(clxxvii) a heavy chain comprising SEQ ID NO: 353 and a light chain comprising SEQ ID NO: 354;
(clxxviii) a heavy chain comprising SEQ ID NO: 355 and a light chain comprising SEQ ID NO: 356;
(clxxix) a heavy chain comprising SEQ ID NO: 357 and a light chain comprising SEQ ID NO: 358; or
(clxxx) a heavy chain comprising SEQ ID NO: 359 and a light chain comprising SEQ ID NO: 360.

3. Pharmaceutical composition **characterized in that** it comprises the monoclonal antibody as defined by any one of claims 1 and 2 and a pharmaceutically acceptable carrier, excipient or diluent.

4. Use of the monoclonal antibody as defined by items 1 and 2 or of the pharmaceutical composition as defined by claim 3, **characterized in that** it is for the preparation of a drug for preventive treatment in the therapy of COVID19.

5. Use, according to claim 4, **characterized by** the fact that the treatment of COVID-19 consists of neutralizing the SARS-CoV-2 virus, preventing systemic inflammation, viral inhibition and inhibition of inflammation caused by the viral infection.

6. Use of the monoclonal antibody as defined by any one of claims 1 and 2, **characterized in that** it is for the generation of cell lines expressing human monoclonal antibodies, derived from B cells of people infected and/or vaccinated with the SARS-CoV-2 virus.

7. Use of the monoclonal antibody as defined by any one of claims 1 and 2, **characterized in that** it is for the production of one or more antibodies that recognize the RBD region of SARS-CoV-2, including variants resulting from virus mutation.

8. Use of the monoclonal antibody as defined by any one of claims 1 and 2, **characterized in that** it is for prophylactic use of monoclonal antibodies recognizing the SARS-CoV-2 virus.

9. Use of the monoclonal antibody as defined by any one of claims 1 and 2, **characterized in that** it is for use as a diagnosis of such monoclonal antibodies.

10. Kit for detecting COVID-19 in a biological sample comprising a monoclonal antibody or a biologically active fragment thereof, as defined by any one of claims 1 and 2, **characterized in that** the monoclonal antibody or fragment thereof is linked to a detectable fraction.

RT-PCR

Reverse transcription

HC -Igγ

LC - Igκ

LC - Igλ

B-actin

Figure 1

## Daily weight variation of hamsters

Figure 2

Figure 3

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2021218947 A
- WO 2021228135 A
- WO 2021207152 A
- WO 2022046888 A
- WO 2021242815 A
- WO 2021195326 A
- WO 2021244852 A
- WO 2021226560 A
- CN 113402602
- CN 112159469
- WO 2021226405 A
- WO 2021183947 A

### Non-patent literature cited in the description

- **ALIPRANDINI E** ; **TAKATA DY** ; **LEPIQUE A** ; **KALIL J** ; **BOSCARDIN SB** ; **MORO AM**. An oligoclonal combination of human monoclonal antibodies able to neutralize tetanus toxin in vivo. *Toxicon X*, 2019, vol. 2, 100006
- **GIUDICELLI, V.** ; **BROCHET, X.** ; **LEFRANC, M.-P.** IMGT/V-QUEST: IMGT Standardized Analysis of the Immunoglobulin (IG) and T Cell Receptor (TR) Nucleotide Sequences. Cold Spring Harbor Protocols, 2011, vol. 2011
- **LOPES DOS SANTOS M** ; **YEDA FP** ; **TSURUTA LR** ; **HORTA BB** ; **PIMENTA AA JR** ; **DEGAKI TL** ; **SOARES IC** ; **TUMA MC** ; **OKAMOTO OK** ; **ALVES VA**. Rebmab200, a humanized monoclonal antibody targeting the sodium phosphate transporter NaPi2b displays strong immune-mediated cytotoxicity against cancer: a novel reagent for targeted antibody therapy of cancer. *PLoS One.*, 2013, vol. 8 (7), e70332
- **LUCHESE MD** ; **LOPES DOS SANTOS M** ; **GARBUIO A** ; **TARGINO RC** ; **MANSUELI CP** ; **TSURUTA LR** ; **QUINTILIO W** ; **MORO AM**. A new CHO (Chinese hamster ovary)-derived cell line expressing anti-TNFα monoclonal antibody with biosimilar potential. *Immunol Res.*, 2018, vol. 66 (3), 392-405
- **ROBBIANI, D F.** ; **GAEBLER, C** ; **MUECKSCH, F** ; **LORENZI, JC. C.** ; **WANG, Z** ; **CHO, A** ; **AGUDELO, M** ; **BARNES, CO.** ; **GAZUMYAN, A** ; **FINKIN, S**. Convergent antibody responses to SARS-CoV-2 in convalescent individuals. *Nature*, 2020, vol. 584, 437-442, http://dx.doi.org/10.1038/s41586-020-2456-9.
- **TILLER, T. et al.** Efficient generation of monoclonal antibodies from single human B cells by single cell RT-PCR and expression vector cloning. *Journal of immunological methods*, 2008, vol. 329 (1-2), 112-124, https://doi.org/10.1016/j.jim.2007.09.017
- Expression Cloning of Human B Cell Immunoglobulins. **WARDEMANN, H** ; **KOFER, J**. Methods In Molecular Biology. Humana Press, 2012, 93-111